# EUROPEAN PATENT SPECIFICATION

(11) **EP 2 096 169 B1**
(45) Date of publication and mention of the grant of the patent: **18.11.2020**
(21) Application number: 08832782.0
(22) Date of filing: 31.10.2008
(51) Int. Cl.: C12N 5/10

(54) **NUCLEAR REPROGRAMMING METHOD**
NUKLEAR-NEUPROGRAMMIERUNGSVERFAHREN
PROCEDE DE RE-PROGRAMMATION NUCLEAIRE

(30) Priority: 31.10.2007 US 1108; 09.11.2007 US 996289 P; 13.06.2008 US 213035
(43) Date of publication of application: 02.09.2009
(73) Proprietor: Kyoto University, Kyoto-shi Kyoto 606-8501 (JP)
(72) Inventor: YAMANAKA, Shinya, Kyoto-shi Kyoto 606-8507 (JP); TAKAHASHI, Kazutoshi, Kyoto-shi Kyoto 606-8507 (JP); NAKAGAWA, Masato, Kyoto-shi Kyoto 606-8507 (JP)
(74) Representative: Hoffmann Eitle
(86) International application number: PCT/JP2008/070365
(87) International publication number: WO 2009/057831

(56) References cited:
- EP-A1- 1 970 446
- WO-A1-2007/069666
- WO-A2-2008/118820
- CN-A- 101 250 502
- BLELLOCH R ET AL: "Generation of induced pluripotent stem cells in the absence of drug selection", CELL STEM CELL, vol. 1, no. 3, September 2007 (2007-09), pages 245-247, XP009147426, ISSN: 1934-5909, DOI: 10.1016/J.STEM.2007.08.008 [retrieved on 2007-09-06]
- YANG J ET AL: "Genome-Wide Analysis Reveals Sall4 to Be a Major Regulator of Pluripotency in Murine-Embryonic Stem Cells", PROCEEDINGS OF THE NATIONAL ACADEMY OF SCIENCES, vol. 105, no. 50, 16 December 2008 (2008-12-16), pages 19762-19761, XP009174987, ISSN: 0027-8424
- TSUBOOKA N ET AL: "Roles of Sall4 in the generation of pluripotent stem cells from blastocysts and fibroblasts", GENES TO CELLS, vol. 14, no. 6, 19 May 2009 (2009-05-19), pages 683-694, XP55201963, ISSN: 1356-9597, DOI: 10.1111/j.1365-2443.2009.01301.x
- TAKAHASHI K ET AL.: 'Induction of pluripotent stem cells from mouse embryonic and adult fibroblast cultures by defined factors.' CELL vol. 126, no. 4, 2006, pages 663 - 676, XP003013968
- TAKAHASHI K ET AL.: 'Induction of pluripotent stem cells from adult human fibroblasts by defined factors.' CELL vol. 131, no. 5, 30 November 2007, pages 861 - 872, XP002530547
- YU J. ET AL.: 'Induced pluripotent stem cell lines derived from human somatic cells.' SCIENCE vol. 318, no. 5858, 21 December 2007, pages 1917 - 1920, XP009105055
- NAKAGAWA M. ET AL.: 'Generation of induced pluripotent stem cells without Myc from mouse and human fibroblasts.' NAT BIOTECHNOL. vol. 26, no. 1, January 2008, pages 101 - 106, XP009098334
- WU Q ET AL.: 'Sall4 interacts with Nanog and co-occupies Nanog genomic sites in embryonic stem cells.' J BIOL CHEM. vol. 281, no. 34, 2006, pages 24090 - 24094, XP008109817

### SEQUENCE LISTING

<110> Kyoto University
<120> Nuclear reprogramming method
<130> A81342M
<150> 61/001, 108
   <151> 2007-10-31
<150> 60/996,289
   <151> 2007-11-9
<150> 12/213,035
   <151> 2008-06-13
<160> 184
<170> PatentIn version 3.4
<210> 1
   <211> 24
   <212> DNA
   <213> Artificial sequence
<220>
   <223> Primer for hOct3/4-S1165
<400> 1
   gacaggggga ggggaggagc tagg 24
<210> 2
   <211> 26
   <212> DNA
   <213> Artificial sequence
<220>
   <223> Primer for hOct3/4-AS1283
<400> 2
   cttccctcca accagttgcc ccaaac 26
<210> 3
   <211> 26
   <212> DNA
   <213> Artificial sequence
<220>
   <223> Primer for hSox2-S1430
<400> 3
   gggaaatggg aggggtgcaa aagagg 26
<210> 4
   <211> 26
   <212> DNA
   <213> Artificial sequence
<220>
   <223> Primer for hSox2-AS1555
<400> 4
   ttgcgtgagt gtggatggga ttggtg 26
<210> 5
   <211> 25
   <212> DNA
   <213> Artificial sequence
<220>
   <223> Primer for ECAT4(Nanog)-macaca-968S
<400> 5
   cagccccgat tcttccacca gtccc 25
<210> 6
   <211> 25
   <212> DNA
   <213> Artificial sequence
<220>
   <223> Primer for ECAT4(Nanog)-macaca-1334AS
<400> 6
   cggaagattc ccagtcgggt tcacc 25
<210> 7
   <211> 24
   <212> DNA
   <213> Artificial sequence
<220>
   <223> Primer for hREXI-RT-U
<400> 7
   cagatcctaa acagctcgca gaat 24
<210> 8
   <211> 23
   <212> DNA
   <213> Artificial sequence
<220>
   <223> Primer for hREXI-RT-L
<400> 8
   gcgtacgcaa attaaagtcc aga 23
<210> 9
   <211> 23
   <212> DNA
   <213> Artificial sequence
<220>
   <223> Primer for hFGF4-RT-U
<400> 9
   ctacaacgcc tacgagtcct aca 23
<210> 10
   <211> 24
   <212> DNA
   <213> Artificial sequence
<220>
   <223> Primer for hFGF4-RT-L
<400> 10
   gttgcaccag aaaagtcaga gttg 24
<210> 11
   <211> 24
   <212> DNA
   <213> Artificial sequence
<220>
   <223> Primer for hGDF3-S243
<400> 11
   cttatgctac gtaaaggagc tggg 24
<210> 12
   <211> 24
   <212> DNA
   <213> Artificial sequence
<220>
   <223> Primer for hGDF3-AS850
<400> 12
   gtgccaaccc aggtcccgga agtt 24
<210> 13
   <211> 24
   <212> DNA
   <213> Artificial sequence
<220>
   <223> Primer for hECATI5-1-S532
<400> 13
   ggagccgcct gccctggaaa attc 24
<210> 14
   <211> 24
   <212> DNA
   <213> Artificial sequence
<220>
   <223> Primer for hECAT15-1-AS916
<400> 14
   tttttcctga tattctattc ccat 24
<210> 15
   <211> 24
   <212> DNA
   <213> Artificial sequence
<220>
   <223> Primer for hECAT15-2-S85
<400> 15
   ccgtccccgc aatctccttc catc 24
<210> 16
   <211> 24
   <212> DNA
   <213> Artificial sequence
<220>
   <223> Primer for hECAT15-2-AS667
<400> 16
   atgatgccaa catggctccc ggtg 24
<210> 17
<211> 24
<212> DNA
<213> Artificial sequence
<220>
<223> Primer for hpH34-S40
<400> 17
   atatcccgcc gtgggtgaaa gttc 24
<210> 18
<211> 24
<212> DNA
<213> Artificial sequence
<220>
<223> Primer for hpH34-AS259
<400> 18
   actcagccat ggactggagc atcc 24
<210> 19
<211> 26
<212> DNA
<213> Artificial sequence
<220>
<223> Primer for hTERT-S3234
<400> 19
   cctgctcaag ctgactcgac accgtg 26
<210> 20
<211> 25
<212> DNA
<213> Artificial sequence
<220>
<223> Primer for hTERT-AS3713
<400> 20
   ggaaaagctg gccctggggt ggagc 25
<210> 21
   <211> 20
   <212> DNA
   <213> Artificial sequence
<220>
   <223> Primer for G3PDH-F
<400> 21
   accacagtcc atgccatcac 20
<210> 22
   <211> 20
   <212> DNA
   <213> Artificial sequence
<220>
   <223> Primer for G3PDH-R
<400> 22
   tccaccaccc tgttgctgta 20
<210> 23
   <211> 24
   <212> DNA
   <213> Artificial sequence
<220>
   <223> Primer for hOct3/4-S1165
<400> 23
   gacaggggga ggggaggagc tagg 24
<210> 24
   <211> 26
   <212> DNA
   <213> Artificial sequence
<220>
   <223> Primer for hOct3/4-AS1283
<400> 24
   cttccctcca accagttgcc ccaaac 26
<210> 25
   <211> 26
   <212> DNA
   <213> Artificial sequence
<220>
   <223> Primer for hSox2-S1430
<400> 25
   gggaaatggg aggggtgcaa aagagg 26
<210> 26
   <211> 26
   <212> DNA
   <213> Artificial sequence
<220>
   <223> Primer for hSox2-AS1555
<400> 26
   ttgcgtgagt gtggatggga ttggtg 26
<210> 27
   <211> 25
   <212> DNA
   <213> Artificial sequence
<220>
   <223> Primer for ECAT4(Nanog)-macaca-968S
<400> 27
   cagccccgat tcttccacca gtccc 25
<210> 28
   <211> 25
   <212> DNA
   <213> Artificial sequence
<220>
   <223> Primer for ECAT4(Nanog)-macaca-1334AS
<400> 28
   cggaagattc ccagtcgggt tcacc 25
<210> 29
   <211> 24
   <212> DNA
   <213> Artificial sequence
<220>
   <223> Primer for hREXI-RT-U
<400> 29
   cagatcctaa acagctcgca gaat 24
<210> 30
   <211> 23
   <212> DNA
   <213> Artificial sequence
<220>
   <223> Primer for hREXI-RT-L
<400> 30
   gcgtacgcaa attaaagtcc aga 23
<210> 31
   <211> 23
   <212> DNA
   <213> Artificial sequence
<220>
   <223> Primer for hFGF4-RT-U
<400> 31
   ctacaacgcc tacgagtcct aca 23
<210> 32
   <211> 24
   <212> DNA
   <213> Artificial sequence
<220>
   <223> Primer for hFGF4-RT-L
<400> 32
   gttgcaccag aaaagtcaga gttg 24
<210> 33
   <211> 24
   <212> DNA
   <213> Artificial sequence
<220>
   <223> Primer for hGDF3-S243
<400> 33
   cttatgctac gtaaaggagc tggg 24
<210> 34
   <211> 24
   <212> DNA
   <213> Artificial sequence
<220>
   <223> Primer for hGDF3-AS850
<400> 34
   gtgccaaccc aggtcccgga agtt 24
<210> 35
   <211> 24
   <212> DNA
   <213> Artificial sequence
<220>
   <223> Primer for hECAT15-1-S532
<400> 35
   ggagccgcct gccctggaaa attc 24
<210> 36
   <211> 24
   <212> DNA
   <213> Artificial sequence
<220>
   <223> Primer for hECAT15-1-AS916
<400> 36
   tttttcctga tattctattc ccat 24
<210> 37
   <211> 24
   <212> DNA
   <213> Artificial sequence
<220>
   <223> Primer for hECAT15-2-S85
<400> 37
   ccgtccccgc aatctccttc catc 24
<210> 38
   <211> 24
   <212> DNA
   <213> Artificial sequence
<220>
   <223> Primer for hECAT15-2-AS667
<400> 38
   atgatgccaa catggctccc ggtg 24
<210> 39
   <211> 24
   <212> DNA
   <213> Artificial sequence
<220>
   <223> Primer for hpH34-S40
<400> 39
   atatcccgcc gtgggtgaaa gttc 24
<210> 40
   <211> 24
   <212> DNA
   <213> Artificial sequence
<220>
   <223> Primer for hpH34-AS259
<400> 40
   actcagccat ggactggagc atcc 24
<210> 41
   <211> 26
   <212> DNA
   <213> Artificial sequence
<220>
   <223> Primer for hTERT-S3234
<400> 41
   cctgctcaag ctgactcgac accgtg 26
<210> 42
   <211> 25
   <212> DNA
   <213> Artificial sequence
<220>
   <223> Primer for hTERT-AS3713
<400> 42
   ggaaaagctg gccctggggt ggagc 25
<210> 43
   <211> 20
   <212> DNA
   <213> Artificial sequence
<220>
   <223> Primer for G3PDH-F
<400> 43
   accacagtcc atgccatcac 20
<210> 44
   <211> 20
   <212> DNA
   <213> Artificial sequence
<220>
   <223> Primer for G3PDH-R
<400> 44
   tccaccaccc tgttgctgta 20
<210> 45
   <211> 23
   <212> DNA
   <213> Artificial sequence
<220>
   <223> Primer for hOCT3/4-S944
<400> 45
   ccccagggcc ccattttggt acc 23
<210> 46
   <211> 26
   <212> DNA
   <213> Artificial sequence
<220>
   <223> Primer for hSOX2-S691
<400> 46
   ggcacccctg gcatggctct tggctc 26
<210> 47
   <211> 25
   <212> DNA
   <213> Artificial sequence
<220>
   <223> Primer for hKLF4-S1128
<400> 47
   acgatcgtgg ccccggaaaa ggacc 25
<210> 48
   <211> 27
   <212> DNA
   <213> Artificial sequence
<220>
   <223> Primer for hMYC-S1011
<400> 48
   caacaaccga aaatgcacca gccccag 27
<210> 49
   <211> 24
   <212> DNA
   <213> Artificial sequence
<220>
   <223> Primer for pMXs-AS3200
<400> 49
   ttatcgtcga ccactgtgct gctg 24
<210> 50
   <211> 24
   <212> DNA
   <213> Artificial sequence
<220>
   <223> Primer for pMXs-L3205
<400> 50
   ccctttttct ggagactaaa taaa 24
<210> 51
   <211> 24
   <212> DNA
   <213> Artificial sequence
<220>
   <223> Primer for hOCT3/4-S1165
<400> 51
   gacaggggga ggggaggagc tagg 24
<210> 52
   <211> 26
   <212> DNA
   <213> Artificial sequence
<220>
   <223> Primer for hOCT3/4-AS1283
<400> 52
   cttccctcca accagttgcc ccaaac 26
<210> 53
   <211> 26
   <212> DNA
   <213> Artificial sequence
<220>
   <223> Primer for hSOX2-S1430
<400> 53
   gggaaatggg aggggtgcaa aagagg 26
<210> 54
   <211> 26
   <212> DNA
   <213> Artificial sequence
<220>
   <223> Primer for hSOX2-AS1555
<400> 54
   ttgcgtgagt gtggatggga ttggtg 26
<210> 55
   <211> 25
   <212> DNA
   <213> Artificial sequence
<220>
   <223> Primer for ECAT4-macaca-968S
<400> 55
   cagccccgat tcttccacca gtccc 25
<210> 56
   <211> 25
   <212> DNA
   <213> Artificial sequence
<220>
   <223> Primer for ECAT4-macaca-1334AS
<400> 56
   cggaagattc ccagtcgggt tcacc 25
<210> 57
   <211> 24
   <212> DNA
   <213> Artificial sequence
<220>
   <223> Primer for hGDF3-S243
<400> 57
   cttatgctac gtaaaggagc tggg 24
<210> 58
   <211> 24
   <212> DNA
   <213> Artificial sequence
<220>
   <223> Primer for hGDF3-AS850
<400> 58
   gtgccaaccc aggtcccgga agtt 24
<210> 59
   <211> 24
   <212> DNA
   <213> Artificial sequence
<220>
   <223> Primer for hREXI-RT-U
<400> 59
   cagatcctaa acagctcgca gaat 24
<210> 60
   <211> 23
   <212> DNA
   <213> Artificial sequence
<220>
   <223> Primer for hREXI-RT-L
<400> 60
   gcgtacgcaa attaaagtcc aga 23
<210> 61
   <211> 23
   <212> DNA
   <213> Artificial sequence
<220>
   <223> Primer for hFGF4-RT-U
<400> 61
   ctacaacgcc tacgagtcct aca 23
<210> 62
   <211> 24
   <212> DNA
   <213> Artificial sequence
<220>
   <223> Primer for hFGF4-RT-L
<400> 62
   gttgcaccag aaaagtcaga gttg 24
<210> 63
   <211> 24
   <212> DNA
   <213> Artificial sequence
<220>
   <223> Primer for hpH34-S40
<400> 63
   atatcccgcc gtgggtgaaa gttc 24
<210> 64
   <211> 24
   <212> DNA
   <213> Artificial sequence
<220>
   <223> Primer for hpH34-AS259
<400> 64
   actcagccat ggactggagc atcc 24
<210> 65
   <211> 24
   <212> DNA
   <213> Artificial sequence
<220>
   <223> Primer for hECAT15-1-S532
<400> 65
   ggagccgcct gccctggaaa attc 24
<210> 66
   <211> 24
   <212> DNA
   <213> Artificial sequence
<220>
   <223> Primer for hECAT15-1-AS916.
<400> 66
   tttttcctga tattctattc ccat 24
<210> 67
   <211> 24
   <212> DNA
   <213> Artificial sequence
<220>
   <223> Primer for hECAT15-2-S85
<400> 67
   ccgtccccgc aatctccttc catc 24
<210> 68
   <211> 24
   <212> DNA
   <213> Artificial sequence
<220>
   <223> Primer for hECAT15-2-AS667
<400> 68
   atgatgccaa catggctccc ggtg 24
<210> 69
   <211> 26
   <212> DNA
   <213> Artificial sequence
<220>
   <223> Primer for hTERT-S3234
<400> 69
   cctgctcaag ctgactcgac accgtg 26
<210> 70
   <211> 25
   <212> DNA
   <213> Artificial sequence
<220>
   <223> Primer for hTERT-AS3713
<400> 70
   ggaaaagctg gccctggggt ggagc 25
<210> 71
   <211> 28
   <212> DNA
   <213> Artificial sequence
<220>
   <223> Primer for hKLF4-AS1826
<400> 71
   tgattgtagt gctttctggc tgggctcc 28
<210> 71
   <211> 26
   <212> DNA
   <213> Artificial sequence
<220>
   <223> Primer for hMYC-S253
<400> 72
   gcgtcctggg aagggagatc cggagc 26
<210> 73
   <211> 26
   <212> DNA
   <213> Artificial sequence
<220>
   <223> Primer for hMYC-AS555
<400> 73
   ttgaggggca tcgtcgcggg aggctg 26
<210> 74
   <211> 24
   <212> DNA
   <213> Artificial sequence
<220>
   <223> Primer for hMSX1-S665
<400> 74
   cgagaggacc ccgtggatgc agag 24
<210> 75
   <211> 24
   <212> DNA
   <213> Artificial sequence
<220>
   <223> Primer for hMSX1-AS938
<400> 75
   ggcggccatc ttcagcttct ccag 24
<210> 76
   <211> 26
   <212> DNA
   <213> Artificial sequence
<220>
   <223> Primer for hBRACHYURY-S1292
<400> 76
   gccctctccc tcccctccac gcacag 26
<210> 77
   <211> 26
   <212> DNA
   <213> Artificial sequence
<220>
   <223> Primer for hBRACHYURY-AS1540
<400> 77
   cggcgccgtt gctcacagac cacagg 26
<210> 78
   <211> 26
   <212> DNA
   <213> Artificial sequence
<220>
   <223> Primer for hGFAP-S1040
<400> 78
   ggcccgccac ttgcaggagt accagg 26
<210> 79
   <211> 26
   <212> DNA
   <213> Artificial sequence
<220>
   <223> Primer for hGFAP-AS1342
<400> 79
   cttctgctcg ggcccctcat gagacg 26
<210> 80
   <211> 28
   <212> DNA
   <213> Artificial sequence
<220>
   <223> Primer for hPAX6-S1206
<400> 80
   acccattatc cagatgtgtt tgcccgag 28
<210> 81
   <211> 26
   <212> DNA
   <213> Artificial sequence
<220>
   <223> Primer for hPAX6-AS1497
<400> 81
   atggtgaagc tgggcatagg cggcag 26
<210> 82
   <211> 26
   <212> DNA
   <213> Artificial sequence
<220>
   <223> Primer for hF0XA2-S208
<400> 82
   tgggagcggt gaagatggaa gggcac 26
<210> 83
   <211> 26
   <212> DNA
   <213> Artificial sequence
<220>
   <223> Primer for hF0XA2-AS398
<400> 83
   tcatgccagc gcccacgtac gacgac 26
<210> 84
   <211> 26
   <212> DNA
   <213> Artificial sequence
<220>
   <223> Primer for hS0X17-S423
<400> 84
   cgctttcatg gtgtgggcta aggacg 26
<210> 85
   <211> 26
   <212> DNA
   <213> Artificial sequence
<220>
   <223> Primer for hSOX17-AS583
<400> 85
   tagttggggt ggtcctgcat gtgctg 26
<210> 86
   <211> 26
   <212> DNA
   <213> Artificial sequence
<220>
   <223> Primer for hAADC-S1378
<400> 86
   cgccaggatc cccgctttga aatctg 26
<210> 87
   <211> 26
   <212> DNA
   <213> Artificial sequence
<220>
   <223> Primer for hAADC-AS1594
<400> 87
   tcggccgcca gctctttgat gtgttc 26
<210> 88
   <211> 24
   <212> DNA
   <213> Artificial sequence
<220>
   <223> Primer for hChAT-S1360
<400> 88
   ggaggcgtgg agctcagcga cacc 24
<210> 89
   <211> 24
   <212> DNA
   <213> Artificial sequence
<220>
   <223> Primer for hChAT-AS1592
<400> 89
   cggggagctc gctgacggag tctg 24
<210> 90
   <211> 30
   <212> DNA
   <213> Artificial sequence
<220>
   <223> Primer for hMAP2-S5401
<400> 90
   caggtggcgg acgtgtgaaa attgagagtg 30
<210> 91
   <211> 26
   <212> DNA
   <213> Artificial sequence
<220>
   <223> Primer for hMAP2-AS5587
<400> 91
   cacgctggat ctgcctgggg actgtg 26
<210> 92
   <211> 26
   <212> DNA
   <213> Artificial sequence
<220>
   <223> Primer for hDAT-S 1935
<400> 92
   acagagggga ggtgcgccag ttcacg 26
<210> 93
   <211> 26
   <212> DNA
   <213> Artificial sequence
<220>
   <223> Primer for hDAT-AS2207
<400> 93
   acggggtgga cctcgctgca cagatc 26
<210> 94
   <211> 26
   <212> DNA
   <213> Artificial sequence
<220>
   <223> Primer for hLMX1B-S770
<400> 94
   ggcaccagca gcagcaggag cagcag 26
<210> 95
   <211> 26
   <212> DNA
   <213> Artificial sequence
<220>
   <223> Primer for hLMXIB-AS1020
<400> 95
   ccacgtctga ggagccgagg aagcag 26
<210> 96
   <211> 26
   <212> DNA
   <213> Artificial sequence
<220>
   <223> Primer for hMYL2A-S258
<400> 96
   gggccccatc aacttcaccg tcttcc 26
<210> 97
   <211> 26
   <212> DNA
   <213> Artificial sequence
<220>
   <223> Primer for hMYL2A-AS468
<400> 97
   tgtagtcgat gttccccgcc aggtcc 26
<210> 98
   <211> 26
   <212> DNA
   <213> Artificial sequence
<220>
   <223> Primer for hTnTc-S524
<400> 98
   atgagcggga gaaggagcgg cagaac 26
<210> 99
   <211> 26
   <212> DNA
   <213> Artificial sequence
<220>
   <223> Primer for hTnTc-AS730
<400> 99
   tcaatggcca gcaccttcct cctctc 26
<210> 100
   <211> 28
   <212> DNA
   <213> Artificial sequence
<220>
   <223> Primer for hMEF2C-S1407
<400> 100
   tttaacaccg ccagcgctct tcaccttg 28
<210> 101
   <211> 28
   <212> DNA
   <213> Artificial sequence
<220>
   <223> Primer for hMEF2C-AS1618
<400> 101
   tcgtggcgcg tgtgttgtgg gtatctcg 28
<210> 102
   <211> 25
   <212> DNA
   <213> Artificial sequence
<220>
   <223> Primer for hMYHCB-S5582
<400> 102
   ctggaggccg agcagaagcg caacg 25
<210> 103
   <211> 25
   <212> DNA
   <213> Artificial sequence
<220>
   <223> Primer for hMYHCB-AS5815
<400> 103
   gtccgcccgc tcctctgcct catcc 25
<210> 104
   <211> 20
   <212> DNA
   <213> Artificial sequence
<220>
   <223> Primer for dT20
<400> 104
   tttttttttt tttttttttt 20
<210> 105
   <211> 27
   <212> DNA
   <213> Artificial sequence
<220>
   <223> Primer for hMYC-S857
<400> 105
   gccacagcaa acctcctcac agcccac 27
<210> 106
   <211> 28
   <212> DNA
   <213> Artificial sequence
<220>
   <223> Primer for hMYC-AS1246
<400> 106
   ctcgtcgttt ccgcaacaag tcctcttc 28
<210> 107
   <211> 26
   <212> DNA
   <213> Artificial sequence
<220>
   <223> Primer for h0CT3/4-S
<400> 107
   caccatggcg ggacacctgg cttcag 26
<210> 108
   <211> 26
   <212> DNA
   <213> Artificial sequence
<220>
   <223> Primer for h0CT3/4-AS
<400> 108
   acctcagttt gaatgcatgg gagagc 26
<210> 109
   <211> 31
   <212> DNA
   <213> Artificial sequence
<220>
   <223> Primer for hS0X2-S
<400> 109
   caccatgtac aacatgatgg agacggagct g 31
<210> 110
   <211> 26
   <212> DNA
   <213> Artificial sequence
<220>
   <223> Primer for hS0X2-AS
<400> 110
   tcacatgtgt gagaggggca gtgtgc 26
<210> 111
   <211> 30
   <212> DNA
   <213> Artificial sequence
<220>
   <223> Primer for hKLF4-S
<400> 111
   caccatggct gtcagtgacg cgctgctccc 30
<210> 112
   <211> 30
   <212> DNA
   <213> Artificial sequence
<220>
   <223> Primer for hKLF4-AS
<400> 112
   ttaaaaatgt ctcttcatgt gtaaggcgag 30
<210> 113
   <211> 30
   <212> DNA
   <213> Artificial sequence
<220>
   <223> Primer for hMYC-S
<400> 113
   caccatgccc ctcaacgtta gcttcaccaa 30
<210> 114
   <211> 30
   <212> DNA
   <213> Artificial sequence
<220>
   <223> Primer for hMYC-AS
<400> 114
   tcacgcacaa gagttccgta gctgttcaag 30
<210> 115
   <211> 27
   <212> DNA
   <213> Artificial sequence
<220>
   <223> Primer for Slc7al-S
<400> 115
   caccatgggc tgcaaaaacc tgctcgg 27
<210> 116
   <211> 27
   <212> DNA
   <213> Artificial sequence
<220>
   <223> Primer for Slc7al-AS
<400> 116
   tcatttgcac tggtccaagt tgctgtc 27
<210> 117
   <211> 33
   <212> DNA
   <213> Artificial sequence
<220>
   <223> Primer for hREX1-pro5K-S
<400> 117
   attgtcgacg gggatttggc agggtcacag gac 33
<210> 118
   <211> 32
   <212> DNA
   <213> Artificial sequence
<220>
   <223> Primer for hREXx1-pro5K-AS
<400> 118
   cccagatctc caatgccacc tcctcccaaa cg 32
<210> 119
   <211> 32
   <212> DNA
   <213> Artificial sequence
<220>
   <223> Primer for h0CT3/4-pro5K-S
<400> 119
   cactcgaggt ggaggagctg agggcactgt gg 32
<210> 120
   <211> 32
   <212> DNA
   <213> Artificial sequence
<220>
   <223> Primer for h0CT3/4-pro5K-AS
<400> 120
   cacagatctg aaatgagggc ttgcgaaggg ac 32
<210> 121
   <211> 30
   <212> DNA
   <213> Artificial sequence
<220>
   <223> Primer for mehREXl-F1-S
<400> 121
   ggtttaaaag ggtaaatgtg attatattta 30
<210> 122
   <211> 22
   <212> DNA
   <213> Artificial sequence
<220>
   <223> Primer for mehREXl-F1-AS
<400> 122
   caaactacaa ccacccatca ac 22
<210> 123
   <211> 30
   <212> DNA
   <213> Artificial sequence
<220>
   <223> Primer for meh0CT3/4 F2-S
<400> 123
   gaggttggag tagaaggatt gttttggttt 30
<210> 124
   <211> 30
   <212> DNA
   <213> Artificial sequence
<220>
   <223> Primer for meh0CT3/4 F2-AS
<400> 124
   cccccctaac ccatcacctc caccacctaa 30
<210> 125
   <211> 25
   <212> DNA
   <213> Artificial sequence
<220>
   <223> Primer for mehNAN0G-FI-S
<400> 125
   tggttaggtt ggttttaaat ttttg 25
<210> 126
   <211> 26
   <212> DNA
   <213> Artificial sequence
<220>
   <223> Primer for mehNAN0G-FI-AS
<400> 126
   aacccaccct tataaattct caatta 26
<210> 127
   <211> 33
   <212> DNA
   <213> Artificial sequence
<220>
   <223> Primer for Sox1
<400> 127
   caccatgtac agcatgatga tggagaccga cct 33
<210> 128
   <211> 26
   <212> DNA
   <213> Artificial sequence
<220>
   <223> Primer for Sox1
<400> 128
   ctagatatgc gtcaggggca ccgtgc 26
<210> 129
   <211> 34
   <212> DNA
   <213> Artificial sequence
<220>
   <223> Primer for Sox3
<400> 129
   caccatgtac agcctgctgg agactgaact caag 34
<210> 130
   <211> 30
   <212> DNA
   <213> Artificial sequence
<220>
   <223> Primer for Sox3
<400> 130
   tcagatgtgg gtcagcggca ccgttccatt 30
<210> 131
   <211> 27
   <212> DNA
   <213> Artificial sequence
<220>
   <223> Primer for Sox7
<400> 131
   cacctcggcc atggcctcgc tgctggg 27
<210> 132
   <211> 27
   <212> DNA
   <213> Artificial sequence
<220>
   <223> Primer for Sox7
<400> 132
   ctccattcct ccagctctat gacacac 27
<210> 133
   <211> 28
   <212> DNA
   <213> Artificial sequence
<220>
   <223> Primer for Sox15
<400> 133
   caccatggcg ctgaccagct cctcacaa 28
<210> 134
   <211> 24
   <212> DNA
   <213> Artificial sequence
<220>
   <223> Primer for Sox15
<400> 134
   ttaaaggtgg gttactggca tggg 24
<210> 135
   <211> 26
   <212> DNA
   <213> Artificial sequence
<220>
   <223> Primer for Sox17
<400> 135
   caccagagcc atgagcagcc cggatg 26
<210> 136
   <211> 26
   <212> DNA
   <213> Artificial sequence
<220>
   <223> Primer for Sox17
<400> 136
   cgtcaaatgt cggggtagtt gcaata 26
<210> 137
   <211> 29
   <212> DNA
   <213> Artificial sequence
<220>
   <223> Primer for Sox18
<400> 137
   caccatgcag agatcgccgc ccggctacg 29
<210> 138
   <211> 30
   <212> DNA
   <213> Artificial sequence
<220>
   <223> Primer for Sox18
<400> 138
   ctagcctgag atgcaagcac tgtaatagac 30
<210> 139
   <211> 28
   <212> DNA
   <213> Artificial sequence
<220>
   <223> Primer for Oct1
<400> 139
   caccatgaat aatccatcag aaaccaat 28
<210> 140
   <211> 24
   <212> DNA
   <213> Artificial sequence
<220>
   <223> Primer for Oct1
<400> 140
   gctctgcact cagctcactg tgcc 24
<210> 141
   <211> 28
   <212> DNA
   <213> Artificial sequence
<220>
   <223> Primer for Oct6
<400> 141
   caccatggcc accaccgcgc agtatctg 28
<210> 142
   <211> 24
   <212> DNA
   <213> Artificial sequence
<220>
   <223> Primer for Oct6
<400> 142
   ggaacccagt ccgcagggtc actg 24
<210> 143
   <211> 29
   <212> DNA
   <213> Artificial sequence
<220>
   <223> Primer for Klf1
<400> 143
   caccatgagg cagaagagag agaggaggc 29
<210> 144
   <211> 30
   <212> DNA
   <213> Artificial sequence
<220>
   <223> Primer for Klf1
<400> 144
   tcagaggtga cgcttcatgt gcagagctaa 30
<210> 145
   <211> 30
   <212> DNA
   <213> Artificial sequence
<220>
   <223> Primer for Klf2
<400> 145
   caccatggcg ctcagcgagc ctatcttgcc 30
<210> 146
   <211> 30
   <212> DNA
   <213> Artificial sequence
<220>
   <223> Primer for Klf2
<400> 146
   ctacatatgt cgcttcatgt gcaaggccag 30
<210> 147
   <211> 28
   <212> DNA
   <213> Artificial sequence
<220>
   <223> Primer for Klf5
<400> 147
   caccatgccc acgcgggtgc tgaccatg 28
<210> 148
   <211> 24
   <212> DNA
   <213> Artificial sequence
<220>
   <223> Primer for Klf5
<400> 148
   tcgctcagtt ctggtggcgc ttca 24
<210> 149
   <211> 34
   <212> DNA
   <213> Artificial sequence
<220>
   <223> Primer for L-MycWT
<400> 149
   caccatggac ttcgactcgt atcagcacta tttc 34
<210> 150
   <211> 30
   <212> DNA
   <213> Artificial sequence
<220>
   <223> Primer for L-MycWT
<400> 150
   ttagtagcca ctgaggtacg cgattctctt 30
<210> 151
   <211> 30
   <212> DNA
   <213> Artificial sequence
<220>
   <223> Primer for N-MycWT
<400> 151
   caccatgccc agctgcaccg cgtccaccat 30
<210> 152
   <211> 26
   <212> DNA
   <213> Artificial sequence
<220>
   <223> Primer for N-MycWT
<400> 152
   ttagcaagtc cgagcgtgtt cgatct 26
<210> 153
   <211> 1320
   <212> DNA
   <213> Mouse
<220>
   <221> CDS
   <222> (1)..(1320)
   <223>
<400> 153
   atg ccc ctc aac gtg aac ttc acc aac agg aac tat gac ctc gac tac      48
   Met Pro Leu Asn Val Asn Phe Thr Asn Arg Asn Tyr Asp Leu Asp Tyr
   1               5                   10                  15
   gac tcc gta cag ccc tat ttc atc tgc gac gag gaa gag aat ttc tat      96
   Asp Ser Val Gln Pro Tyr Phe Ile Cys Asp Glu Glu Glu Asn Phe Tyr
               20                  25                  30
   cac cag caa cag cag agc gag ctg cag ccg ccc gcg ccc agt gag gat      144
   His Gln Gln Gln Gln Ser Glu Leu Gln Pro Pro Ala Pro Ser Glu Asp
           35                  40                  45
   atc tgg aag aaa ttc gag ctg ctt ccc acc ccg ccc ctg tcc ccg agc      192
   Ile Trp Lys Lys Phe Glu Leu Leu Pro Thr Pro Pro Leu Ser Pro Ser
       50                  55                  60
   cgc cgc tcc ggg ctc tgc tct cca tcc tat gtt gcg gtc gct acg tcc      240
   Arg Arg Ser Gly Leu Cys Ser Pro Ser Tyr Val Ala Val Ala Thr Ser
   65                  70                  75                  80
   ttc tcc cca agg gaa gac gat gac ggc ggc ggt ggc aac ttc tcc acc      288
   Phe Ser Pro Arg Glu Asp Asp Asp Gly Gly Gly Gly Asn Phe Ser Thr
                   85                  90                  95
   gcc gat cag ctg gag atg atg acc gag tta ctt gga gga gac atg gtg      336
   Ala Asp Gln Leu Glu Met Met Thr Glu Leu Leu Gly Gly Asp Met Val
               100                 105                 110
   aac cag agc ttc atc tgc gat cct gac gac gag acc ttc atc aag aac      384
   Asn Gln Ser Phe Ile Cys Asp Pro Asp Asp Glu Thr Phe Ile Lys Asn
           115                 120                 125
   atc atc atc cag gac tgt atg tgg agc ggt ttc tca gcc gct gcc aag      432
   Ile Ile Ile Gln Asp Cys Met Trp Ser Gly Phe Ser Ala Ala Ala Lys
       130                 135                 140
   ctg gtc tcg gag aag ctg gcc tcc tac cag gct gcg cgc aaa gac agc      480
   Leu Val Ser Glu Lys Leu Ala Ser Tyr Gln Ala Ala Arg Lys Asp Ser
   145                 150                 155                 160
   acc agc ctg agc ccc gcc cgc ggg cac agc gtc tgc tcc acc tcc agc      528
   Thr Ser Leu Ser Pro Ala Arg Gly His Ser Val Cys Ser Thr Ser Ser
                   165                 170                 175
   ctg tac ctg cag gac ctc acc gcc gcc gcg tcc gag tgc att gac ccc      576
   Leu Tyr Leu Gln Asp Leu Thr Ala Ala Ala Ser Glu Cys Ile Asp Pro
               180                 185                 190
   tca gtg gtc ttt ccc tac ccg ctc aac gac agc agc tcg ccc aaa tcc      624
   Ser Val Val Phe Pro Tyr Pro Leu Asn Asp Ser Ser Ser Pro Lys Ser
           195                 200                 205
   tgt acc tcg tcc gat tcc acg gcc ttc tct cct tcc tcg gac tcg ctg      672
   Cys Thr Ser Ser Asp Ser Thr Ala Phe Ser Pro Ser Ser Asp Ser Leu
       210                 215                 220
   ctg tcc tcc gag tcc tcc cca cgg gcc agc cct gag ccc cta gtg ctg      720
   Leu Ser Ser Glu Ser Ser Pro Arg Ala Ser Pro Glu Pro Leu Val Leu
   225                 230                 235                 240
   cat gag gag aca ccg ccc acc acc agc agc gac tct gaa gaa gag caa      768
   His Glu Glu Thr Pro Pro Thr Thr Ser Ser Asp Ser Glu Glu Glu Gln
                   245                 250                 255
   gaa gat gag gaa gaa att gat gtg gtg tct gtg gag aag agg caa acc      816
   Glu Asp Glu Glu Glu Ile Asp Val Val Ser Val Glu Lys Arg Gln Thr
               260                 265                 270
   cct gcc aag agg tcg gag tcg ggc tca tct cca tcc cga ggc cac agc 864
   Pro Ala Lys Arg Ser Glu Ser Gly Ser Ser Pro Ser Arg Gly His Ser
   275 280 285
   aaa cct ccg cac agc cca ctg gtc ctc aag agg tgc cac gtc tcc act 912
   Lys Pro Pro His Ser Pro Leu Val Leu Lys Arg Cys His Val Ser Thr
   290 295 300
   cac cag cac aac tac gcc gca ccc ccc tcc aca agg aag gac tat cca 960
   His Gln His Asn Tyr Ala Ala Pro Pro Ser Thr Arg Lys Asp Tyr Pro
   305 310 315 320
   gct gcc aag agg gcc aag ttg gac agt ggc agg gtc ctg aag cag atc 1008
   Ala Ala Lys Arg Ala Lys Leu Asp Ser Gly Arg Val Leu Lys Gln Ile
   325 330 335
   agc aac aac cgc aag tgc tcc agc ccc agg tcc tca gac acg gag gaa 1056
   Ser Asn Asn Arg Lys Cys Ser Ser Pro Arg Ser Ser Asp Thr Glu Glu
   340 345 350
   aac gac aag agg cgg aca cac aac gtc ttg gaa cgt cag agg agg aac 1104
   Asn Asp Lys Arg Arg Thr His Asn Val Leu Glu Arg Gln Arg Arg Asn
   355 360 365
   gag ctg aag cgc agc ttt ttt gcc ctg cgt gac cag atc cct gaa ttg 1152
   Glu Leu Lys Arg Ser Phe Phe Ala Leu Arg Asp Gln Ile Pro Glu Leu
   370 375 380
   gaa aac aac gaa aag gcc ccc aag gta gtg atc ctc aaa aaa gcc acc 1200
   Glu Asn Asn Glu Lys Ala Pro Lys Val Val Ile Leu Lys Lys Ala Thr
   385 390 395 400
   gcc tac atc ctg tcc att caa gca gac gag cac aag ctc acc tct gaa 1248
   Ala Tyr Ile Leu Ser Ile Gln Ala Asp Glu His Lys Leu Thr Ser Glu
   405 410 415
   aag gac tta ttg agg aaa cga cga gaa cag ttg aaa cac aaa ctc gaa 1296
   Lys Asp Leu Leu Arg Lys Arg Arg Glu Gln Leu Lys His Lys Leu Glu
   420 425 430
   cag ctt cga aac tct ggt gca taa 1320
   Gln Leu Arg Asn Ser Gly Ala
   435
<210> 154
   <211> 439
   <212> PRT
   <213> Mouse
<400> 154
   Met Pro Leu Asn Val Asn Phe Thr Asn Arg Asn Tyr Asp Leu Asp Tyr
   1               5                   10                  15
   Asp Ser Val Gln Pro Tyr Phe Ile Cys Asp Glu Glu Glu Asn Phe Tyr
               20                  25                  30
   His Gln Gln Gln Gln Ser Glu Leu Gln Pro Pro Ala Pro Ser Glu Asp
           35                  40                  45
   Ile Trp Lys Lys Phe Glu Leu Leu Pro Thr Pro Pro Leu Ser Pro Ser
       50                  55                  60
   Arg Arg Ser Gly Leu Cys Ser Pro Ser Tyr Val Ala Val Ala Thr Ser
   65                  70                  75                  80
   Phe Ser Pro Arg Glu Asp Asp Asp Gly Gly Gly Gly Asn Phe Ser Thr
                   85                  90                  95
   Ala Asp Gln Leu Glu Met Met Thr Glu Leu Leu Gly Gly Asp Met Val
               100                 105                 110
   Asn Gln Ser Phe Ile Cys Asp Pro Asp Asp Glu Thr Phe Ile Lys Asn
           115                 120                 125
   Ile Ile Ile Gln Asp Cys Met Trp Ser Gly Phe Ser Ala Ala Ala Lys
       130                 135                 140
   Leu Val Ser Glu Lys Leu Ala Ser Tyr Gln Ala Ala Arg Lys Asp Ser
   145                 150                 155                 160
   Thr Ser Leu Ser Pro Ala Arg Gly His Ser Val Cys Ser Thr Ser Ser
                   165                 170                 175
   Leu Tyr Leu Gln Asp Leu Thr Ala Ala Ala Ser Glu Cys Ile Asp Pro
               180                 185                 190
   Ser Val Val Phe Pro Tyr Pro Leu Asn Asp Ser Ser Ser Pro Lys Ser
           195                 200                 205
   Cys Thr Ser Ser Asp Ser Thr Ala Phe Ser Pro Ser Ser Asp Ser Leu
       210                 215                 220
   Leu Ser Ser Glu Ser Ser Pro Arg Ala Ser Pro Glu Pro Leu Val Leu
   225                 230                 235                 240
   His Glu Glu Thr Pro Pro Thr Thr Ser Ser Asp Ser Glu Glu Glu Gln
                   245                 250                 255
   Glu Asp Glu Glu Glu Ile Asp Val Val Ser Val Glu Lys Arg Gln Thr
               260                 265                 270
   Pro Ala Lys Arg Ser Glu Ser Gly Ser Ser Pro Ser Arg Gly His Ser
           275                 280                 285
   Lys Pro Pro His Ser Pro Leu Val Leu Lys Arg Cys His Val Ser Thr
       290                 295                 300
   His Gln His Asn Tyr Ala Ala Pro Pro Ser Thr Arg Lys Asp Tyr Pro
   305 310 315 320
   Ala Ala Lys Arg Ala Lys Leu Asp Ser Gly Arg Val Leu Lys Gln Ile
   325 330 335
   Ser Asn Asn Arg Lys Cys Ser Ser Pro Arg Ser Ser Asp Thr Glu Glu
   340 345 350
   Asn Asp Lys Arg Arg Thr His Asn Val Leu Glu Arg Gln Arg Arg Asn
   355 360 365
   Glu Leu Lys Arg Ser Phe Phe Ala Leu Arg Asp Gln Ile Pro Glu Leu
   370 375 380
   Glu Asn Asn Glu Lys Ala Pro Lys Val Val Ile Leu Lys Lys Ala Thr
   385 390 395 400
   Ala Tyr Ile Leu Ser Ile Gln Ala Asp Glu His Lys Leu Thr Ser Glu
   405 410 415
   Lys Asp Leu Leu Arg Lys Arg Arg Glu Gln Leu Lys His Lys Leu Glu
   420 425 430
   Gln Leu Arg Asn Ser Gly Ala
   435
<210> 155
   <211> 1107
   <212> DNA
   <213> Mouse
<220>
   <221> CDS
   <222> (1).. (1107)
   <223>
<400> 155
   atg gac ttc gac tcg tat cag cac tat ttc tac gac tat gac tgc gga 48
   Met Asp Phe Asp Ser Tyr Gln His Tyr Phe Tyr Asp Tyr Asp Cys Gly
   1 5 10 15
   gag gat ttc tac cgc tcc acg gcg ccc agc gag gac atc tgg aag aaa 96
   Glu Asp Phe Tyr Arg Ser Thr Ala Pro Ser Glu Asp Ile Trp Lys Lys
   20 25 30
   ttc gag ctg gtg ccg tcg ccc ccc acg tcg ccg ccc tgg ggc tcc ggt 144
   Phe Glu Leu Val Pro Ser Pro Pro Thr Ser Pro Pro Trp Gly Ser Gly
   35 40 45
   ccc ggc gcc gtg gac cca gcc tct ggg att aat ccc ggg gag ccg tgg 192
   Pro Gly Ala Val Asp Pro Ala Ser Gly Ile Asn Pro Gly Glu Pro Trp
   50 55 60
   cct gga ggg ggt gcc ggg gac gag gcg gaa tct cgg ggc cat tcg aaa      240
   Pro Gly Gly Gly Ala Gly Asp Glu Ala Glu Ser Arg Gly His Ser Lys
   65                  70                  75                  80
   gcc tgg ggc agg aat tat gct tcc atc att cgc cgt gac tgc atg tgg      288
   Ala Trp Gly Arg Asn Tyr Ala Ser Ile Ile Arg Arg Asp Cys Met Trp
                   85                  90                  95
   agc ggc ttc tcc gcc cga gaa cgg ctg gag aga gtg gtg agc gac agg      336
   Ser Gly Phe Ser Ala Arg Glu Arg Leu Glu Arg Val Val Ser Asp Arg
               100                 105                 110
   ctg gcc cca ggc gcg ccc cgg ggg aac ccg ccc aaa gcg ccc gct acc      384
   Leu Ala Pro Gly Ala Pro Arg Gly Asn Pro Pro Lys Ala Pro Ala Thr
           115                 120                 125
   ccg gac ggc act cct agt ctg gaa gcc agt aac ccg gcg ccc gcc acc      432
   Pro Asp Gly Thr Pro Ser Leu Glu Ala Ser Asn Pro Ala Pro Ala Thr
       130                 135                 140
   caa tgt cag ctg ggc gag ccc aag act cag gcc tgc tcc ggg tcc gag      480
   Gln Cys Gln Leu Gly Glu Pro Lys Thr Gln Ala Cys Ser Gly Ser Glu
   145                 150                 155                 160
   agc ccc agc gat tct gaa ggt gaa gag att gac gtg gtg acc gtg gag      528
   Ser Pro Ser Asp Ser Glu Gly Glu Glu Ile Asp Val Val Thr Val Glu
                   165                 170                 175
   aag agg cga tct ctg gac atc cga aag cca gtc acc atc acg gtg cga      576
   Lys Arg Arg Ser Leu Asp Ile Arg Lys Pro Val Thr Ile Thr Val Arg
               180                 185                 190
   gca gac ccc ctg gac ccc tgc atg aag cac ttc cat atc tct atc cac      624
   Ala Asp Pro Leu Asp Pro Cys Met Lys His Phe His Ile Ser Ile His
           195                 200                 205
   caa cag cag cat aac tat gct gcc cgt ttt cct cca gaa agt tgc tct      672
   Gln Gln Gln His Asn Tyr Ala Ala Arg Phe Pro Pro Glu Ser Cys Ser
       210                 215                 220
   caa gag ggg gat cct gag cca ggt ccc cag gaa gag gct ccg gag ata      720
   Gln Glu Gly Asp Pro Glu Pro Gly Pro Gln Glu Glu Ala Pro Glu Ile
   225                 230                 235                 240
   gaa gct ccc aag gag aaa gag gag gag gaa gag gaa gag gag gaa gaa      768
   Glu Ala Pro Lys Glu Lys Glu Glu Glu Glu Glu Glu Glu Glu Glu Glu
                   245                 250                 255
   gag att gtg agc ccc cca cct gtc gga agt gag gct ccc cag tcc tgc      816
   Glu Ile Val Ser Pro Pro Pro Val Gly Ser Glu Ala Pro Gln Ser Cys
               260                 265                 270
   cac ccc aaa cct gtc agt tct gac act gag gac gtg acc aag agg aag 864
   His Pro Lys Pro Val Ser Ser Asp Thr Glu Asp Val Thr Lys Arg Lys
   275 280 285
   aac cat aac ttc ttg gaa cga aaa agg agg aat gac ctc cgc tcc cgg 912
   Asn His Asn Phe Leu Glu Arg Lys Arg Arg Asn Asp Leu Arg Ser Arg
   290 295 300
   ttc cta gcc ctg cgg gac cag gtt ccc acc ctg gcc agc tgc tct aag 960
   Phe Leu Ala Leu Arg Asp Gln Val Pro Thr Leu Ala Ser Cys Ser Lys
   305 310 315 320
   gcc ccc aaa gtc gtg atc ctc agc aag gcg tta gaa tac ttg cag gct 1008
   Ala Pro Lys Val Val Ile Leu Ser Lys Ala Leu Glu Tyr Leu Gln Ala
   325 330 335
   ttg gtg ggg gct gaa aag aaa atg gct aca gag aaa agg cag ctc cgg 1056
   Leu Val Gly Ala Glu Lys Lys Met Ala Thr Glu Lys Arg Gln Leu Arg
   340 345 350
   tgt cgg caa cag caa ctg caa aag aga atc gcg tac ctc agt ggc tac 1104
   Cys Arg Gln Gln Gln Leu Gln Lys Arg Ile Ala Tyr Leu Ser Gly Tyr
   355 360 365
   taa 1107
<210> 156
   <211> 368
   <212> PRT
   <213> Mouse
<400> 156
   Met Asp Phe Asp Ser Tyr Gln His Tyr Phe Tyr Asp Tyr Asp Cys Gly
   1 5 10 15
   Glu Asp Phe Tyr Arg Ser Thr Ala Pro Ser Glu Asp Ile Trp Lys Lys
   20 25 30
   Phe Glu Leu Val Pro Ser Pro Pro Thr Ser Pro Pro Trp Gly Ser Gly
   35 40 45
   Pro Gly Ala Val Asp Pro Ala Ser Gly Ile Asn Pro Gly Glu Pro Trp
   50 55 60
   Pro Gly Gly Gly Ala Gly Asp Glu Ala Glu Ser Arg Gly His Ser Lys
   65 70 75 80
   Ala Trp Gly Arg Asn Tyr Ala Ser Ile Ile Arg Arg Asp Cys Met Trp
   85 90 95
   Ser Gly Phe Ser Ala Arg Glu Arg Leu Glu Arg Val Val Ser Asp Arg
   100 105 110
   Leu Ala Pro Gly Ala Pro Arg Gly Asn Pro Pro Lys Ala Pro Ala Thr
   115 120 125
   Pro Asp Gly Thr Pro Ser Leu Glu Ala Ser Asn Pro Ala Pro Ala Thr
   130 135 140
   Gln Cys Gln Leu Gly Glu Pro Lys Thr Gln Ala Cys Ser Gly Ser Glu
   145 150 155 160
   Ser Pro Ser Asp Ser Glu Gly Glu Glu Ile Asp Val Val Thr Val Glu
   165 170 175
   Lys Arg Arg Ser Leu Asp Ile Arg Lys Pro Val Thr Ile Thr Val Arg
   180 185 190
   Ala Asp Pro Leu Asp Pro Cys Met Lys His Phe His Ile Ser Ile His
   195 200 205
   Gln Gln Gln His Asn Tyr Ala Ala Arg Phe Pro Pro Glu Ser Cys Ser
   210 215 220
   Gln Glu Gly Asp Pro Glu Pro Gly Pro Gln Glu Glu Ala Pro Glu Ile
   225 230 235 240
   Glu Ala Pro Lys Glu Lys Glu Glu Glu Glu Glu Glu Glu Glu Glu Glu
   245 250 255
   Glu Ile Val Ser Pro Pro Pro Val Gly Ser Glu Ala Pro Gln Ser Cys
   260 265 270
   His Pro Lys Pro Val Ser Ser Asp Thr Glu Asp Val Thr Lys Arg Lys
   275 280 285
   Asn His Asn Phe Leu Glu Arg Lys Arg Arg Asn Asp Leu Arg Ser Arg
   290 295 300
   Phe Leu Ala Leu Arg Asp Gln Val Pro Thr Leu Ala Ser Cys Ser Lys
   305 310 315 320
   Ala Pro Lys Val Val Ile Leu Ser Lys Ala Leu Glu Tyr Leu Gln Ala
   325 330 335
   Leu Val Gly Ala Glu Lys Lys Met Ala Thr Glu Lys Arg Gln Leu Arg
   340 345 350
   Cys Arg Gln Gln Gln Leu Gln Lys Arg Ile Ala Tyr Leu Ser Gly Tyr
   355 360 365
<210> 157
   <211> 1275
   <212> DNA
   <213> Mouse
<220>
   <221> CDS
   <222> (1).. (1275)
   <223>
<400> 157
   atg ccc ctc aac gtg aac ttc acc aac agg aac tat gac ctc gac tac        48
   Met Pro Leu Asn Val Asn Phe Thr Asn Arg Asn Tyr Asp Leu Asp Tyr
   1               5                   10                  15
   gac tcc gta cag ccc tat ttc atc tgc gac gag gaa gag aat ttc tat        96
   Asp Ser Val Gln Pro Tyr Phe Ile Cys Asp Glu Glu Glu Asn Phe Tyr
               20                  25                  30
   cac cag caa cag cag agc gag ctg cag ccg ccc gcg ccc agt gag gat        144
   His Gln Gln Gln Gln Ser Glu Leu Gln Pro Pro Ala Pro Ser Glu Asp
           35                  40                  45
   atc tgg aag aaa ttc gag ctg ctt ccc acc ccg ccc ctg tcc ccg agc        192
   Ile Trp Lys Lys Phe Glu Leu Leu Pro Thr Pro Pro Leu Ser Pro Ser
       50                  55                  60
   cgc cgc tcc ggg ctc tgc tct cca tcc tat gtt gcg gtc gct acg tcc        240
   Arg Arg Ser Gly Leu Cys Ser Pro Ser Tyr Val Ala Val Ala Thr Ser
   65                  70                  75                  80
   ttc tcc cca agg gaa gac gat gac ggc ggc ggt ggc aac ttc tcc acc        288
   Phe Ser Pro Arg Glu Asp Asp Asp Gly Gly Gly Gly Asn Phe Ser Thr
                   85                  90                  95
   gcc gat cag ctg gag atg atg acc gag tta ctt gga gga gac atg gtg        336
   Ala Asp Gln Leu Glu Met Met Thr Glu Leu Leu Gly Gly Asp Met Val
               100                 105                 110
   aac cag agc ttc atc tgc gat cct gac gac gag acc ttc atc aag aac        384
   Asn Gln Ser Phe Ile Cys Asp Pro Asp Asp Glu Thr Phe Ile Lys Asn
           115                 120                 125
   atc atc atc cag gac tgt atg tgg agc ggt ttc tca gcc gct gcc aag        432
   Ile Ile Ile Gln Asp Cys Met Trp Ser Gly Phe Ser Ala Ala Ala Lys
       130                 135                 140
   ctg gtc tcg gag aag ctg gcc tcc tac cag gct gcg cgc aaa gac agc        480
   Leu Val Ser Glu Lys Leu Ala Ser Tyr Gln Ala Ala Arg Lys Asp Ser
   145                 150                 155                 160
   acc agc ctg agc ccc gcc cgc ggg cac agc gtc tgc tcc acc tcc agc        528
   Thr Ser Leu Ser Pro Ala Arg Gly His Ser Val Cys Ser Thr Ser Ser
                   165                 170                 175
   ctg tac ctg cag gac ctc acc gcc gcc gcg tcc gag tgc att gac ccc        576
   Leu Tyr Leu Gln Asp Leu Thr Ala Ala Ala Ser Glu Cys Ile Asp Pro
               180                 185                 190
   tca gtg gtc ttt ccc tac ccg ctc aac gac agc agc tcg ccc aaa tcc 624
   Ser Val Val Phe Pro Tyr Pro Leu Asn Asp Ser Ser Ser Pro Lys Ser
   195 200 205
   tgt acc tcg tcc gat tcc acg gcc ttc tct agc gat tct gaa ggt gaa 672
   Cys Thr Ser Ser Asp Ser Thr Ala Phe Ser Ser Asp Ser Glu Gly Glu
   210 215 220
   gag att gac gtg gtg acc gtg gag aag agg cga tct ctg gac atc cga 720
   Glu Ile Asp Val Val Thr Val Glu Lys Arg Arg Ser Leu Asp Ile Arg
   225 230 235 240
   aag cca gtc acc atc acg gtg cga gca gac ccc ctg gac ccc tgc atg 768
   Lys Pro Val Thr Ile Thr Val Arg Ala Asp Pro Leu Asp Pro Cys Met
   245 250 255
   aag cac ttc cat atc tct atc cac caa cag cag cat aac tat gct gcc 816
   Lys His Phe His Ile Ser Ile His Gln Gln Gln His Asn Tyr Ala Ala
   260 265 270
   cgt ttt cct cca gaa agt tgc tct caa gag ggg gat cct gag cca ggt 864
   Arg Phe Pro Pro Glu Ser Cys Ser Gln Glu Gly Asp Pro Glu Pro Gly
   275 280 285
   ccc cag gaa gag gct ccg gag ata gaa gct ccc aag gag aaa gag gag 912
   Pro Gln Glu Glu Ala Pro Glu Ile Glu Ala Pro Lys Glu Lys Glu Glu
   290 295 300
   gag gaa gag gaa gag gag gaa gaa gag att gtg agc ccc cca cct gtc 960
   Glu Glu Glu Glu Glu Glu Glu Glu Glu Ile Val Ser Pro Pro Pro Val
   305 310 315 320
   gga agt gag gct ccc cag tcc tgc cac ccc aaa cct gtc agt tct gac 1008
   Gly Ser Glu Ala Pro Gln Ser Cys His Pro Lys Pro Val Ser Ser Asp
   325 330 335
   act gag gac gtg acc aag agg aag aac cat aac ttc ttg gaa cga aaa 1056
   Thr Glu Asp Val Thr Lys Arg Lys Asn His Asn Phe Leu Glu Arg Lys
   340 345 350
   agg agg aat gac ctc cgc tcc cgg ttc cta gcc ctg cgg gac cag gtt 1104
   Arg Arg Asn Asp Leu Arg Ser Arg Phe Leu Ala Leu Arg Asp Gln Val
   355 360 365
   ccc acc ctg gcc agc tgc tct aag gcc ccc aaa gtc gtg atc ctc agc 1152
   Pro Thr Leu Ala Ser Cys Ser Lys Ala Pro Lys Val Val Ile Leu Ser
   370 375 380
   aag gcg tta gaa tac ttg cag gct ttg gtg ggg gct gaa aag aaa atg 1200
   Lys Ala Leu Glu Tyr Leu Gln Ala Leu Val Gly Ala Glu Lys Lys Met
   385 390 395 400
   gct aca gag aaa agg cag ctc cgg tgt cgg caa cag caa ctg caa aag 1248
   Ala Thr Glu Lys Arg Gln Leu Arg Cys Arg Gln Gln Gln Leu Gln Lys
   405 410 415
   aga atc gcg tac ctc agt ggc tac taa 1275
   Arg Ile Ala Tyr Leu Ser Gly Tyr
   420
<210> 158
   <211> 424
   <212> PRT
   <213> Mouse
<400> 158
   Met Pro Leu Asn Val Asn Phe Thr Asn Arg Asn Tyr Asp Leu Asp Tyr
   1 5 10 15
   Asp Ser Val Gln Pro Tyr Phe Ile Cys Asp Glu Glu Glu Asn Phe Tyr
   20 25 30
   His Gln Gln Gln Gln Ser Glu Leu Gln Pro Pro Ala Pro Ser Glu Asp
   35 40 45
   Ile Trp Lys Lys Phe Glu Leu Leu Pro Thr Pro Pro Leu Ser Pro Ser
   50 55 60
   Arg Arg Ser Gly Leu Cys Ser Pro Ser Tyr Val Ala Val Ala Thr Ser
   65 70 75 80
   Phe Ser Pro Arg Glu Asp Asp Asp Gly Gly Gly Gly Asn Phe Ser Thr
   85 90 95
   Ala Asp Gln Leu Glu Met Met Thr Glu Leu Leu Gly Gly Asp Met Val
   100 105 110
   Asn Gln Ser Phe Ile Cys Asp Pro Asp Asp Glu Thr Phe Ile Lys Asn
   115 120 125
   Ile Ile Ile Gln Asp Cys Met Trp Ser Gly Phe Ser Ala Ala Ala Lys
   130 135 140
   Leu Val Ser Glu Lys Leu Ala Ser Tyr Gln Ala Ala Arg Lys Asp Ser
   145 150 155 160
   Thr Ser Leu Ser Pro Ala Arg Gly His Ser Val Cys Ser Thr Ser Ser
   165 170 175
   Leu Tyr Leu Gln Asp Leu Thr Ala Ala Ala Ser Glu Cys Ile Asp Pro
   180 185 190
   Ser Val Val Phe Pro Tyr Pro Leu Asn Asp Ser Ser Ser Pro Lys Ser
   195 200 205
   Cys Thr Ser Ser Asp Ser Thr Ala Phe Ser Ser Asp Ser Glu Gly Glu
   210 215 220
   Glu Ile Asp Val Val Thr Val Glu Lys Arg Arg Ser Leu Asp Ile Arg
   225 230 235 240
   Lys Pro Val Thr Ile Thr Val Arg Ala Asp Pro Leu Asp Pro Cys Met
   245 250 255
   Lys His Phe His Ile Ser Ile His Gln Gln Gln His Asn Tyr Ala Ala
   260 265 270
   Arg Phe Pro Pro Glu Ser Cys Ser Gln Glu Gly Asp Pro Glu Pro Gly
   275 280 285
   Pro Gln Glu Glu Ala Pro Glu Ile Glu Ala Pro Lys Glu Lys Glu Glu
   290 295 300
   Glu Glu Glu Glu Glu Glu Glu Glu Glu Ile Val Ser Pro Pro Pro Val
   305 310 315 320
   Gly Ser Glu Ala Pro Gln Ser Cys His Pro Lys Pro Val Ser Ser Asp
   325 330 335
   Thr Glu Asp Val Thr Lys Arg Lys Asn His Asn Phe Leu Glu Arg Lys
   340 345 350
   Arg Arg Asn Asp Leu Arg Ser Arg Phe Leu Ala Leu Arg Asp Gln Val
   355 360 365
   Pro Thr Leu Ala Ser Cys Ser Lys Ala Pro Lys Val Val Ile Leu Ser
   370 375 380
   Lys Ala Leu Glu Tyr Leu Gln Ala Leu Val Gly Ala Glu Lys Lys Met
   385 390 395 400
   Ala Thr Glu Lys Arg Gln Leu Arg Cys Arg Gln Gln Gln Leu Gln Lys
   405 410 415
   Arg Ile Ala Tyr Leu Ser Gly Tyr
   420
<210> 159
   <211> 1146
   <212> DNA
   <213> Mouse
<220>
   <221> CDS
   <222> (1).. (1146)
   <223>
<400> 159
   atg gac ttc gac tcg tat cag cac tat ttc tac gac tat gac tgc gga 48
   Met Asp Phe Asp Ser Tyr Gln His Tyr Phe Tyr Asp Tyr Asp Cys Gly
   1               5                   10                  15
   gag gat ttc tac cgc tcc acg gcg ccc agc gag gac atc tgg aag aaa        96
   Glu Asp Phe Tyr Arg Ser Thr Ala Pro Ser Glu Asp Ile Trp Lys Lys
               20                  25                  30
   ttc gag ctg gtg ccg tcg ccc ccc acg tcg ccg ccc tgg ggc tcc ggt       144
   Phe Glu Leu Val Pro Ser Pro Pro Thr Ser Pro Pro Trp Gly Ser Gly
           35                  40                  45
   ccc ggc gcc gtg gac cca gcc tct ggg att aat ccc ggg gag ccg tgg       192
   Pro Gly Ala Val Asp Pro Ala Ser Gly Ile Asn Pro Gly Glu Pro Trp
       50                  55                  60
   cct gga ggg ggt gcc ggg gac gag gcg gaa tct cgg ggc cat tcg aaa       240
   Pro Gly Gly Gly Ala Gly Asp Glu Ala Glu Ser Arg Gly His Ser Lys
   65                  70                  75                  80
   gcc tgg ggc agg aat tat gct tcc atc att cgc cgt gac tgc atg tgg       288
   Ala Trp Gly Arg Asn Tyr Ala Ser Ile Ile Arg Arg Asp Cys Met Trp
                   85                  90                  95
   agc ggc ttc tcc gcc cga gaa cgg ctg gag aga gtg gtg agc gac agg       336
   Ser Gly Phe Ser Ala Arg Glu Arg Leu Glu Arg Val Val Ser Asp Arg
               100                 105                 110
   ctg gcc cca ggc gcg ccc cgg ggg aac ccg ccc aaa gcg ccc gct acc       384
   Leu Ala Pro Gly Ala Pro Arg Gly Asn Pro Pro Lys Ala Pro Ala Thr
           115                 120                 125
   ccg gac ggc act cct agt ctg gaa gcc agt aac ccg gcg ccc gcc acc       432
   Pro Asp Gly Thr Pro Ser Leu Glu Ala Ser Asn Pro Ala Pro Ala Thr
       130                 135                 140
   caa tgt cag ctg ggc gag ccc aag act cag gcc tgc tcc ggg tcc gag       480
   Gln Cys Gln Leu Gly Glu Pro Lys Thr Gln Ala Cys Ser Gly Ser Glu
   145                 150                 155                 160
   agc ccc tcg gac tcg ctg ctg tcc tcc gag tcc tcc cca cgg gcc agc       528
   Ser Pro Ser Asp Ser Leu Leu Ser Ser Glu Ser Ser Pro Arg Ala Ser
                   165                 170                 175
   cct gag ccc cta gtg ctg cat gag gag aca ccg ccc acc acc agc agc       576
   Pro Glu Pro Leu Val Leu His Glu Glu Thr Pro Pro Thr Thr Ser Ser
               180                 185                 190
   gac tct gaa gaa gag caa gaa gat gag gaa gaa att gat gtg gtg tct       624
   Asp Ser Glu Glu Glu Gln Glu Asp Glu Glu Glu Ile Asp Val Val Ser
           195                 200                 205
   gtg gag aag agg caa acc cct gcc aag agg tcg gag tcg ggc tca tct       672
   Val Glu Lys Arg Gln Thr Pro Ala Lys Arg Ser Glu Ser Gly Ser Ser
       210                 215                 220
   cca tcc cga ggc cac agc aaa cct ccg cac agc cca ctg gtc ctc aag      720
   Pro Ser Arg Gly His Ser Lys Pro Pro His Ser Pro Leu Val Leu Lys
   225                 230                 235                 240
   agg tgc cac gtc tcc act cac cag cac aac tac gcc gca ccc ccc tcc      768
   Arg Cys His Val Ser Thr His Gln His Asn Tyr Ala Ala Pro Pro Ser
                   245                 250                 255
   aca agg aag gac tat cca gct gcc aag agg gcc aag ttg gac agt ggc      816
   Thr Arg Lys Asp Tyr Pro Ala Ala Lys Arg Ala Lys Leu Asp Ser Gly
               260                 265                 270
   agg gtc ctg aag cag atc agc aac aac cgc aag tgc tcc agc ccc agg      864
   Arg Val Leu Lys Gln Ile Ser Asn Asn Arg Lys Cys Ser Ser Pro Arg
           275                 280                 285
   tcc tca gac acg gag gaa aac gac aag agg cgg aca cac aac gtc ttg      912
   Ser Ser Asp Thr Glu Glu Asn Asp Lys Arg Arg Thr His Asn Val Leu
       290                 295                 300
   gaa cgt cag agg agg aac gag ctg aag cgc agc ttt ttt gcc ctg cgt      960
   Glu Arg Gln Arg Arg Asn Glu Leu Lys Arg Ser Phe Phe Ala Leu Arg
   305                 310                 315                 320
   gac cag atc cct gaa ttg gaa aac aac gaa aag gcc ccc aag gta gtg     1008
   Asp Gln Ile Pro Glu Leu Glu Asn Asn Glu Lys Ala Pro Lys Val Val
                   325                 330                 335
   atc ctc aaa aaa gcc acc gcc tac atc ctg tcc att caa gca gac gag     1056
   Ile Leu Lys Lys Ala Thr Ala Tyr Ile Leu Ser Ile Gln Ala Asp Glu
               340                 345                 350
   cac aag ctc acc tct gaa aag gac tta ttg agg aaa cga cga gaa cag     1104
   His Lys Leu Thr Ser Glu Lys Asp Leu Leu Arg Lys Arg Arg Glu Gln
           355                 360                 365
   ttg aaa cac aaa ctc gaa cag ctt cga aac tct ggt gca taa             1146
   Leu Lys His Lys Leu Glu Gln Leu Arg Asn Ser Gly Ala
       370                 375                 380
<210> 160
   <211> 381
   <212> PRT
   <213> Mouse
<400> 160
   Met Asp Phe Asp Ser Tyr Gln His Tyr Phe Tyr Asp Tyr Asp Cys Gly
   1               5                   10                  15
   Glu Asp Phe Tyr Arg Ser Thr Ala Pro Ser Glu Asp Ile Trp Lys Lys
   20 25 30
   Phe Glu Leu Val Pro Ser Pro Pro Thr Ser Pro Pro Trp Gly Ser Gly
   35 40 45
   Pro Gly Ala Val Asp Pro Ala Ser Gly Ile Asn Pro Gly Glu Pro Trp
   50 55 60
   Pro Gly Gly Gly Ala Gly Asp Glu Ala Glu Ser Arg Gly His Ser Lys
   65 70 75 80
   Ala Trp Gly Arg Asn Tyr Ala Ser Ile Ile Arg Arg Asp Cys Met Trp
   85 90 95
   Ser Gly Phe Ser Ala Arg Glu Arg Leu Glu Arg Val Val Ser Asp Arg
   100 105 110
   Leu Ala Pro Gly Ala Pro Arg Gly Asn Pro Pro Lys Ala Pro Ala Thr
   115 120 125
   Pro Asp Gly Thr Pro Ser Leu Glu Ala Ser Asn Pro Ala Pro Ala Thr
   130 135 140
   Gln Cys Gln Leu Gly Glu Pro Lys Thr Gln Ala Cys Ser Gly Ser Glu
   145 150 155 160
   Ser Pro Ser Asp Ser Leu Leu Ser Ser Glu Ser Ser Pro Arg Ala Ser
   165 170 175
   Pro Glu Pro Leu Val Leu His Glu Glu Thr Pro Pro Thr Thr Ser Ser
   180 185 190
   Asp Ser Glu Glu Glu Gln Glu Asp Glu Glu Glu Ile Asp Val Val Ser
   195 200 205
   Val Glu Lys Arg Gln Thr Pro Ala Lys Arg Ser Glu Ser Gly Ser Ser
   210 215 220
   Pro Ser Arg Gly His Ser Lys Pro Pro His Ser Pro Leu Val Leu Lys
   225 230 235 240
   Arg Cys His Val Ser Thr His Gln His Asn Tyr Ala Ala Pro Pro Ser
   245 250 255
   Thr Arg Lys Asp Tyr Pro Ala Ala Lys Arg Ala Lys Leu Asp Ser Gly
   260 265 270
   Arg Val Leu Lys Gln Ile Ser Asn Asn Arg Lys Cys Ser Ser Pro Arg
   275 280 285
   Ser Ser Asp Thr Glu Glu Asn Asp Lys Arg Arg Thr His Asn Val Leu
   290 295 300
   Glu Arg Gln Arg Arg Asn Glu Leu Lys Arg Ser Phe Phe Ala Leu Arg
   305 310 315 320
   Asp Gln Ile Pro Glu Leu Glu Asn Asn Glu Lys Ala Pro Lys Val Val
   325 330 335
   Ile Leu Lys Lys Ala Thr Ala Tyr Ile Leu Ser Ile Gln Ala Asp Glu
   340 345 350
   His Lys Leu Thr Ser Glu Lys Asp Leu Leu Arg Lys Arg Arg Glu Gln
   355 360 365
   Leu Lys His Lys Leu Glu Gln Leu Arg Asn Ser Gly Ala
   370 375 380
<210> 161
   <211> 1233
   <212> DNA
   <213> Mouse
<220>
   <221> CDS
   <222> (1).. (1233)
   <223>
<400> 161
   atg ccc ctc aac gtg aac ttc acc aac agg aac tat gac ctc gac tac 48
   Met Pro Leu Asn Val Asn Phe Thr Asn Arg Asn Tyr Asp Leu Asp Tyr
   1 5 10 15
   gac tcc gta cag ccc tat ttc atc tgc gac gag gaa gag aat ttc tat 96
   Asp Ser Val Gln Pro Tyr Phe Ile Cys Asp Glu Glu Glu Asn Phe Tyr
   20 25 30
   cac cag caa cag cag agc gag ctg cag ccg ccc gcg ccc agt gag gat 144
   His Gln Gln Gln Gln Ser Glu Leu Gln Pro Pro Ala Pro Ser Glu Asp
   35 40 45
   atc tgg aag aaa ttc gag ctg ctt ccc acc ccg ccc ctg tcc ccg agc 192
   Ile Trp Lys Lys Phe Glu Leu Leu Pro Thr Pro Pro Leu Ser Pro Ser
   50 55 60
   cgc cgc tcc ggg ctc tgc tct cca tcc tat gtt gcg gtc gct acg tcc 240
   Arg Arg Ser Gly Leu Cys Ser Pro Ser Tyr Val Ala Val Ala Thr Ser
   65 70 75 80
   ttc tcc cca agg gaa gac gat gac ggc ggc ggt ggc aac ttc tcc acc 288
   Phe Ser Pro Arg Glu Asp Asp Asp Gly Gly Gly Gly Asn Phe Ser Thr
   85 90 95
   gcc gat cag ctg gag atg atg acc gag tta ctt gga gga gac atg gtg 336
   Ala Asp Gln Leu Glu Met Met Thr Glu Leu Leu Gly Gly Asp Met Val
   100 105 110
   aac cag agc ttc atc tgc gat cct gac gac gag acc ttc atc aag aac 384
   Asn Gln Ser Phe Ile Cys Asp Pro Asp Asp Glu Thr Phe Ile Lys Asn
           115                 120                 125
   atc atc atc cag gac tgt atg tgg agc ggt ttc tca gcc gct gcc aag      432
   Ile Ile Ile Gln Asp Cys Met Trp Ser Gly Phe Ser Ala Ala Ala Lys
       130                 135                 140
   ctg gag aga gtg gtg agc gac agg ctg gcc cca ggc gcg ccc cgg ggg      480
   Leu Glu Arg Val Val Ser Asp Arg Leu Ala Pro Gly Ala Pro Arg Gly
   145                 150                 155                 160
   aac ccg ccc aaa gcg ccc gct acc ccg gac ggc act cct agt ctg gaa      528
   Asn Pro Pro Lys Ala Pro Ala Thr Pro Asp Gly Thr Pro Ser Leu Glu
                   165                 170                 175
   gcc agt aac ccg gcg ccc gcc acc caa tgt cag ctg ggc gag ccc aag      576
   Ala Ser Asn Pro Ala Pro Ala Thr Gln Cys Gln Leu Gly Glu Pro Lys
               180                 185                 190
   act cag gcc tgc tcc ggg tcc gag agc ccc agc gat tct gaa ggt gaa      624
   Thr Gln Ala Cys Ser Gly Ser Glu Ser Pro Ser Asp Ser Glu Gly Glu
           195                 200                 205
   gag att gac gtg gtg acc gtg gag aag agg cga tct ctg gac atc cga      672
   Glu Ile Asp Val Val Thr Val Glu Lys Arg Arg Ser Leu Asp Ile Arg
       210                 215                 220
   aag cca gtc acc atc acg gtg cga gca gac ccc ctg gac ccc tgc atg      720
   Lys Pro Val Thr Ile Thr Val Arg Ala Asp Pro Leu Asp Pro Cys Met
   225                 230                 235                 240
   aag cac ttc cat atc tct atc cac caa cag cag cat aac tat gct gcc      768
   Lys His Phe His Ile Ser Ile His Gln Gln Gln His Asn Tyr Ala Ala
                   245                 250                 255
   cgt ttt cct cca gaa agt tgc tct caa gag ggg gat cct gag cca ggt      816
   Arg Phe Pro Pro Glu Ser Cys Ser Gln Glu Gly Asp Pro Glu Pro Gly
               260                 265                 270
   ccc cag gaa gag gct ccg gag ata gaa gct ccc aag gag aaa gag gag      864
   Pro Gln Glu Glu Ala Pro Glu Ile Glu Ala Pro Lys Glu Lys Glu Glu
           275                 280                 285
   gag gaa gag gaa gag gag gaa gaa gag att gtg agc ccc cca cct gtc      912
   Glu Glu Glu Glu Glu Glu Glu Glu Glu Ile Val Ser Pro Pro Pro Val
       290                 295                 300
   gga agt gag gct ccc cag tcc tgc cac ccc aaa cct gtc tcc tca gac      960
   Gly Ser Glu Ala Pro Gln Ser Cys His Pro Lys Pro Val Ser Ser Asp
   305                 310                 315                 320
   acg gag gaa aac gac aag agg cgg aca cac aac gtc ttg gaa cgt cag     1008
   Thr Glu Glu Asn Asp Lys Arg Arg Thr His Asn Val Leu Glu Arg Gln
   325 330 335
   agg agg aac gag ctg aag cgc agc ttt ttt gcc ctg cgt gac cag atc 1056
   Arg Arg Asn Glu Leu Lys Arg Ser Phe Phe Ala Leu Arg Asp Gln Ile
   340 345 350
   cct gaa ttg gaa aac aac gaa aag gcc ccc aag gta gtg atc ctc aaa 1104
   Pro Glu Leu Glu Asn Asn Glu Lys Ala Pro Lys Val Val Ile Leu Lys
   355 360 365
   aaa gcc acc gcc tac atc ctg tcc att caa gca gac gag cac aag ctc 1152
   Lys Ala Thr Ala Tyr Ile Leu Ser Ile Gln Ala Asp Glu His Lys Leu
   370 375 380
   acc tct gaa aag gac tta ttg agg aaa cga cga gaa cag ttg aaa cac 1200
   Thr Ser Glu Lys Asp Leu Leu Arg Lys Arg Arg Glu Gln Leu Lys His
   385 390 395 400
   aaa ctc gaa cag ctt cga aac tct ggt gca taa 1233
   Lys Leu Glu Gln Leu Arg Asn Ser Gly Ala
   405 410
<210> 162
   <211> 410
   <212> PRT
   <213> Mouse
<400> 162
   Met Pro Leu Asn Val Asn Phe Thr Asn Arg Asn Tyr Asp Leu Asp Tyr
   1 5 10 15
   Asp Ser Val Gln Pro Tyr Phe Ile Cys Asp Glu Glu Glu Asn Phe Tyr
   20 25 30
   His Gln Gln Gln Gln Ser Glu Leu Gln Pro Pro Ala Pro Ser Glu Asp
   35 40 45
   Ile Trp Lys Lys Phe Glu Leu Leu Pro Thr Pro Pro Leu Ser Pro Ser
   50 55 60
   Arg Arg Ser Gly Leu Cys Ser Pro Ser Tyr Val Ala Val Ala Thr Ser
   65 70 75 80
   Phe Ser Pro Arg Glu Asp Asp Asp Gly Gly Gly Gly Asn Phe Ser Thr
   85 90 95
   Ala Asp Gln Leu Glu Met Met Thr Glu Leu Leu Gly Gly Asp Met Val
   100 105 110
   Asn Gln Ser Phe Ile Cys Asp Pro Asp Asp Glu Thr Phe Ile Lys Asn
   115 120 125
   Ile Ile Ile Gln Asp Cys Met Trp Ser Gly Phe Ser Ala Ala Ala Lys
   130 135 140
   Leu Glu Arg Val Val Ser Asp Arg Leu Ala Pro Gly Ala Pro Arg Gly
   145 150 155 160
   Asn Pro Pro Lys Ala Pro Ala Thr Pro Asp Gly Thr Pro Ser Leu Glu
   165 170 175
   Ala Ser Asn Pro Ala Pro Ala Thr Gln Cys Gln Leu Gly Glu Pro Lys
   180 185 190
   Thr Gln Ala Cys Ser Gly Ser Glu Ser Pro Ser Asp Ser Glu Gly Glu
   195 200 205
   Glu Ile Asp Val Val Thr Val Glu Lys Arg Arg Ser Leu Asp Ile Arg
   210 215 220
   Lys Pro Val Thr Ile Thr Val Arg Ala Asp Pro Leu Asp Pro Cys Met
   225 230 235 240
   Lys His Phe His Ile Ser Ile His Gln Gln Gln His Asn Tyr Ala Ala
   245 250 255
   Arg Phe Pro Pro Glu Ser Cys Ser Gln Glu Gly Asp Pro Glu Pro Gly
   260 265 270
   Pro Gln Glu Glu Ala Pro Glu Ile Glu Ala Pro Lys Glu Lys Glu Glu
   275 280 285
   Glu Glu Glu Glu Glu Glu Glu Glu Glu Ile Val Ser Pro Pro Pro Val
   290 295 300
   Gly Ser Glu Ala Pro Gln Ser Cys His Pro Lys Pro Val Ser Ser Asp
   305 310 315 320
   Thr Glu Glu Asn Asp Lys Arg Arg Thr His Asn Val Leu Glu Arg Gln
   325 330 335
   Arg Arg Asn Glu Leu Lys Arg Ser Phe Phe Ala Leu Arg Asp Gln Ile
   340 345 350
   Pro Glu Leu Glu Asn Asn Glu Lys Ala Pro Lys Val Val Ile Leu Lys
   355 360 365
   Lys Ala Thr Ala Tyr Ile Leu Ser Ile Gln Ala Asp Glu His Lys Leu
   370 375 380
   Thr Ser Glu Lys Asp Leu Leu Arg Lys Arg Arg Glu Gln Leu Lys His
   385 390 395 400
   Lys Leu Glu Gln Leu Arg Asn Ser Gly Ala
   405 410
<210> 163
   <211> 1194
   <212> DNA
   <213> Mouse
<220>
   <221> CDS
   <222> (1).. (1194)
   <223>
<400> 163
   atg gac ttc gac tcg tat cag cac tat ttc tac gac tat gac tgc gga      48
   Met Asp Phe Asp Ser Tyr Gln His Tyr Phe Tyr Asp Tyr Asp Cys Gly
   1               5                   10                  15
   gag gat ttc tac cgc tcc acg gcg ccc agc gag gac atc tgg aag aaa      96
   Glu Asp Phe Tyr Arg Ser Thr Ala Pro Ser Glu Asp Ile Trp Lys Lys
               20                  25                  30
   ttc gag ctg gtg ccg tcg ccc ccc acg tcg ccg ccc tgg ggc tcc ggt      144
   Phe Glu Leu Val Pro Ser Pro Pro Thr Ser Pro Pro Trp Gly Ser Gly
           35                  40                  45
   ccc ggc gcc gtg gac cca gcc tct ggg att aat ccc ggg gag ccg tgg      192
   Pro Gly Ala Val Asp Pro Ala Ser Gly Ile Asn Pro Gly Glu Pro Trp
       50                  55                  60
   cct gga ggg ggt gcc ggg gac gag gcg gaa tct cgg ggc cat tcg aaa      240
   Pro Gly Gly Gly Ala Gly Asp Glu Ala Glu Ser Arg Gly His Ser Lys
   65                  70                  75                  80
   gcc tgg ggc agg aat tat gct tcc atc att cgc cgt gac tgc atg tgg      288
   Ala Trp Gly Arg Asn Tyr Ala Ser Ile Ile Arg Arg Asp Cys Met Trp
                   85                  90                  95
   agc ggc ttc tcc gcc cga gaa cgg ctg gtc tcg gag aag ctg gcc tcc      336
   Ser Gly Phe Ser Ala Arg Glu Arg Leu Val Ser Glu Lys Leu Ala Ser
               100                 105                 110
   tac cag gct gcg cgc aaa gac agc acc agc ctg agc ccc gcc cgc ggg      384
   Tyr Gln Ala Ala Arg Lys Asp Ser Thr Ser Leu Ser Pro Ala Arg Gly
           115                 120                 125
   cac agc gtc tgc tcc acc tcc agc ctg tac ctg cag gac ctc acc gcc      432
   His Ser Val Cys Ser Thr Ser Ser Leu Tyr Leu Gln Asp Leu Thr Ala
       130                 135                 140
   gcc gcg tcc gag tgc att gac ccc tca gtg gtc ttt ccc tac ccg ctc      480
   Ala Ala Ser Glu Cys Ile Asp Pro Ser Val Val Phe Pro Tyr Pro Leu
   145                 150                 155                 160
   aac gac agc agc tcg ccc aaa tcc tgt acc tcg tcc gat tcc acg gcc      528
   Asn Asp Ser Ser Ser Pro Lys Ser Cys Thr Ser Ser Asp Ser Thr Ala
   165 170 175
   ttc tct cct tcc tcg gac tcg ctg ctg tcc tcc gag tcc tcc cca cgg 576
   Phe Ser Pro Ser Ser Asp Ser Leu Leu Ser Ser Glu Ser Ser Pro Arg
   180 185 190
   gcc agc cct gag ccc cta gtg ctg cat gag gag aca ccg ccc acc acc 624
   Ala Ser Pro Glu Pro Leu Val Leu His Glu Glu Thr Pro Pro Thr Thr
   195 200 205
   agc agc gac tct gaa gaa gag caa gaa gat gag gaa gaa att gat gtg 672
   Ser Ser Asp Ser Glu Glu Glu Gln Glu Asp Glu Glu Glu Ile Asp Val
   210 215 220
   gtg tct gtg gag aag agg caa acc cct gcc aag agg tcg gag tcg ggc 720
   Val Ser Val Glu Lys Arg Gln Thr Pro Ala Lys Arg Ser Glu Ser Gly
   225 230 235 240
   tca tct cca tcc cga ggc cac agc aaa cct ccg cac agc cca ctg gtc 768
   Ser Ser Pro Ser Arg Gly His Ser Lys Pro Pro His Ser Pro Leu Val
   245 250 255
   ctc aag agg tgc cac gtc tcc act cac cag cac aac tac gcc gca ccc 816
   Leu Lys Arg Cys His Val Ser Thr His Gln His Asn Tyr Ala Ala Pro
   260 265 270
   ccc tcc aca agg aag gac tat cca gct gcc aag agg gcc aag ttg gac 864
   Pro Ser Thr Arg Lys Asp Tyr Pro Ala Ala Lys Arg Ala Lys Leu Asp
   275 280 285
   agt ggc agg gtc ctg aag cag atc agc aac aac cgc aag tgc tcc agc 912
   Ser Gly Arg Val Leu Lys Gln Ile Ser Asn Asn Arg Lys Cys Ser Ser
   290 295 300
   ccc agg agt tct gac act gag gac gtg acc aag agg aag aac cat aac 960
   Pro Arg Ser Ser Asp Thr Glu Asp Val Thr Lys Arg Lys Asn His Asn
   305 310 315 320
   ttc ttg gaa cga aaa agg agg aat gac ctc cgc tcc cgg ttc cta gcc 1008
   Phe Leu Glu Arg Lys Arg Arg Asn Asp Leu Arg Ser Arg Phe Leu Ala
   325 330 335
   ctg cgg gac cag gtt ccc acc ctg gcc agc tgc tct aag gcc ccc aaa 1056
   Leu Arg Asp Gln Val Pro Thr Leu Ala Ser Cys Ser Lys Ala Pro Lys
   340 345 350
   gtc gtg atc ctc agc aag gcg tta gaa tac ttg cag gct ttg gtg ggg 1104
   Val Val Ile Leu Ser Lys Ala Leu Glu Tyr Leu Gln Ala Leu Val Gly
   355 360 365
   gct gaa aag aaa atg gct aca gag aaa agg cag ctc cgg tgt cgg caa 1152
   Ala Glu Lys Lys Met Ala Thr Glu Lys Arg Gln Leu Arg Cys Arg Gln
   370 375 380
   cag caa ctg caa aag aga atc gcg tac ctc agt ggc tac taa 1194
   Gln Gln Leu Gln Lys Arg Ile Ala Tyr Leu Ser Gly Tyr
   385 390 395
<210> 164
   <211> 397
   <212> PRT
   <213> Mouse
<400> 164
   Met Asp Phe Asp Ser Tyr Gln His Tyr Phe Tyr Asp Tyr Asp Cys Gly
   1 5 10 15
   Glu Asp Phe Tyr Arg Ser Thr Ala Pro Ser Glu Asp Ile Trp Lys Lys
   20 25 30
   Phe Glu Leu Val Pro Ser Pro Pro Thr Ser Pro Pro Trp Gly Ser Gly
   35 40 45
   Pro Gly Ala Val Asp Pro Ala Ser Gly Ile Asn Pro Gly Glu Pro Trp
   50 55 60
   Pro Gly Gly Gly Ala Gly Asp Glu Ala Glu Ser Arg Gly His Ser Lys
   65 70 75 80
   Ala Trp Gly Arg Asn Tyr Ala Ser Ile Ile Arg Arg Asp Cys Met Trp
   85 90 95
   Ser Gly Phe Ser Ala Arg Glu Arg Leu Val Ser Glu Lys Leu Ala Ser
   100 105 110
   Tyr Gln Ala Ala Arg Lys Asp Ser Thr Ser Leu Ser Pro Ala Arg Gly
   115 120 125
   His Ser Val Cys Ser Thr Ser Ser Leu Tyr Leu Gln Asp Leu Thr Ala
   130 135 140
   Ala Ala Ser Glu Cys Ile Asp Pro Ser Val Val Phe Pro Tyr Pro Leu
   145 150 155 160
   Asn Asp Ser Ser Ser Pro Lys Ser Cys Thr Ser Ser Asp Ser Thr Ala
   165 170 175
   Phe Ser Pro Ser Ser Asp Ser Leu Leu Ser Ser Glu Ser Ser Pro Arg
   180 185 190
   Ala Ser Pro Glu Pro Leu Val Leu His Glu Glu Thr Pro Pro Thr Thr
   195 200 205
   Ser Ser Asp Ser Glu Glu Glu Gln Glu Asp Glu Glu Glu Ile Asp Val
   210 215 220
   Val Ser Val Glu Lys Arg Gln Thr Pro Ala Lys Arg Ser Glu Ser Gly
   225 230 235 240
   Ser Ser Pro Ser Arg Gly His Ser Lys Pro Pro His Ser Pro Leu Val
   245 250 255
   Leu Lys Arg Cys His Val Ser Thr His Gln His Asn Tyr Ala Ala Pro
   260 265 270
   Pro Ser Thr Arg Lys Asp Tyr Pro Ala Ala Lys Arg Ala Lys Leu Asp
   275 280 285
   Ser Gly Arg Val Leu Lys Gln Ile Ser Asn Asn Arg Lys Cys Ser Ser
   290 295 300
   Pro Arg Ser Ser Asp Thr Glu Asp Val Thr Lys Arg Lys Asn His Asn
   305 310 315 320
   Phe Leu Glu Arg Lys Arg Arg Asn Asp Leu Arg Ser Arg Phe Leu Ala
   325 330 335
   Leu Arg Asp Gln Val Pro Thr Leu Ala Ser Cys Ser Lys Ala Pro Lys
   340 345 350
   Val Val Ile Leu Ser Lys Ala Leu Glu Tyr Leu Gln Ala Leu Val Gly
   355 360 365
   Ala Glu Lys Lys Met Ala Thr Glu Lys Arg Gln Leu Arg Cys Arg Gln
   370 375 380
   Gln Gln Leu Gln Lys Arg Ile Ala Tyr Leu Ser Gly Tyr
   385 390 395
<210> 165
   <211> 1314
   <212> DNA
   <213> Mouse
<220>
   <221> CDS
   <222> (1).. (1314)
   <223>
<400> 165
   atg ccc ctc aac gtg aac ttc acc aac agg aac tat gac ctc gac tac 48
   Met Pro Leu Asn Val Asn Phe Thr Asn Arg Asn Tyr Asp Leu Asp Tyr
   1 5 10 15
   gac tcc gta cag ccc tat ttc atc tgc gac gag gaa gag aat ttc tat 96
   Asp Ser Val Gln Pro Tyr Phe Ile Cys Asp Glu Glu Glu Asn Phe Tyr
   20 25 30
   cac cag caa cag cag agc gag ctg cag ccg ccc gcg ccc agt gag gat 144
   His Gln Gln Gln Gln Ser Glu Leu Gln Pro Pro Ala Pro Ser Glu Asp
               35                  40                  45
   atc tgg aag aaa ttc gag ctg ctt ccc acc ccg ccc ctg tcc ccg agc      192
   Ile Trp Lys Lys Phe Glu Leu Leu Pro Thr Pro Pro Leu Ser Pro Ser
           50                  55                  60
   cgc cgc tcc ggg ctc tgc tct cca tcc tat gtt gcg gtc gct acg tcc      240
   Arg Arg Ser Gly Leu Cys Ser Pro Ser Tyr Val Ala Val Ala Thr Ser
   65                  70                  75                  80
   ttc tcc cca agg gaa gac gat gac ggc ggc ggt ggc aac ttc tcc acc      288
   Phe Ser Pro Arg Glu Asp Asp Asp Gly Gly Gly Gly Asn Phe Ser Thr
                   85                  90                  95
   gcc gat cag ctg gag atg atg acc gag tta ctt gga gga gac atg gtg      336
   Ala Asp Gln Leu Glu Met Met Thr Glu Leu Leu Gly Gly Asp Met Val
               100                 105                 110
   aac cag agc ttc atc tgc gat cct gac gac gag acc ttc atc aag aac      384
   Asn Gln Ser Phe Ile Cys Asp Pro Asp Asp Glu Thr Phe Ile Lys Asn
           115                 120                 125
   atc atc atc cag gac tgt atg tgg agc ggt ttc tca gcc gct gcc aag      432
   Ile Ile Ile Gln Asp Cys Met Trp Ser Gly Phe Ser Ala Ala Ala Lys
       130                 135                 140
   ctg gtc tcg gag aag ctg gcc tcc tac cag gct gcg cgc aaa gac agc      480
   Leu Val Ser Glu Lys Leu Ala Ser Tyr Gln Ala Ala Arg Lys Asp Ser
   145                 150                 155                 160
   acc agc ctg agc ccc gcc cgc ggg cac agc gtc tgc tcc acc tcc agc      528
   Thr Ser Leu Ser Pro Ala Arg Gly His Ser Val Cys Ser Thr Ser Ser
                   165                 170                 175
   ctg tac ctg cag gac ctc acc gcc gcc gcg tcc gag tgc att gac ccc      576
   Leu Tyr Leu Gln Asp Leu Thr Ala Ala Ala Ser Glu Cys Ile Asp Pro
               180                 185                 190
   tca gtg gtc ttt ccc tac ccg ctc aac gac agc agc tcg ccc aaa tcc      624
   Ser Val Val Phe Pro Tyr Pro Leu Asn Asp Ser Ser Ser Pro Lys Ser
           195                 200                 205
   tgt acc tcg tcc gat tcc acg gcc ttc tct cct tcc tcg gac tcg ctg      672
   Cys Thr Ser Ser Asp Ser Thr Ala Phe Ser Pro Ser Ser Asp Ser Leu
       210                 215                 220
   ctg tcc tcc gag tcc tcc cca cgg gcc agc cct gag ccc cta gtg ctg      720
   Leu Ser Ser Glu Ser Ser Pro Arg Ala Ser Pro Glu Pro Leu Val Leu
   225                 230                 235                 240
   cat gag gag aca ccg ccc acc acc agc agc gac tct gaa gaa gag caa      768
   His Glu Glu Thr Pro Pro Thr Thr Ser Ser Asp Ser Glu Glu Glu Gln
   245 250 255
   gaa gat gag gaa gaa att gat gtg gtg tct gtg gag aag agg caa acc 816
   Glu Asp Glu Glu Glu Ile Asp Val Val Ser Val Glu Lys Arg Gln Thr
   260 265 270
   cct gcc aag agg tcg gag tcg ggc tca tct cca tcc cga ggc cac agc 864
   Pro Ala Lys Arg Ser Glu Ser Gly Ser Ser Pro Ser Arg Gly His Ser
   275 280 285
   aaa cct ccg cac agc cca ctg gtc ctc aag agg tgc cac gtc tcc act 912
   Lys Pro Pro His Ser Pro Leu Val Leu Lys Arg Cys His Val Ser Thr
   290 295 300
   cac cag cac aac tac gcc gca ccc ccc tcc aca agg aag gac tat cca 960
   His Gln His Asn Tyr Ala Ala Pro Pro Ser Thr Arg Lys Asp Tyr Pro
   305 310 315 320
   gct gcc aag agg gcc aag ttg gac agt ggc agg gtc ctg aag cag atc 1008
   Ala Ala Lys Arg Ala Lys Leu Asp Ser Gly Arg Val Leu Lys Gln Ile
   325 330 335
   agc aac aac cgc aag tgc tcc agc ccc agg agt tct gac act gag gac 1056
   Ser Asn Asn Arg Lys Cys Ser Ser Pro Arg Ser Ser Asp Thr Glu Asp
   340 345 350
   gtg acc aag agg aag aac cat aac ttc ttg gaa cga aaa agg agg aat 1104
   Val Thr Lys Arg Lys Asn His Asn Phe Leu Glu Arg Lys Arg Arg Asn
   355 360 365
   gac ctc cgc tcc cgg ttc cta gcc ctg cgg gac cag gtt ccc acc ctg 1152
   Asp Leu Arg Ser Arg Phe Leu Ala Leu Arg Asp Gln Val Pro Thr Leu
   370 375 380
   gcc agc tgc tct aag gcc ccc aaa gtc gtg atc ctc agc aag gcg tta 1200
   Ala Ser Cys Ser Lys Ala Pro Lys Val Val Ile Leu Ser Lys Ala Leu
   385 390 395 400
   gaa tac ttg cag gct ttg gtg ggg gct gaa aag aaa atg gct aca gag 1248
   Glu Tyr Leu Gln Ala Leu Val Gly Ala Glu Lys Lys Met Ala Thr Glu
   405 410 415
   aaa agg cag ctc cgg tgt cgg caa cag caa ctg caa aag aga atc gcg 1296
   Lys Arg Gln Leu Arg Cys Arg Gln Gln Gln Leu Gln Lys Arg Ile Ala
   420 425 430
   tac ctc agt ggc tac taa 1314
   Tyr Leu Ser Gly Tyr
   435
<210> 166
   <211> 437
   <212> PRT
   <213> Mouse
<400> 166
   Met Pro Leu Asn Val Asn Phe Thr Asn Arg Asn Tyr Asp Leu Asp Tyr
   1               5                   10                  15
   Asp Ser Val Gln Pro Tyr Phe Ile Cys Asp Glu Glu Glu Asn Phe Tyr
               20                  25                  30
   His Gln Gln Gln Gln Ser Glu Leu Gln Pro Pro Ala Pro Ser Glu Asp
           35                  40                  45
   Ile Trp Lys Lys Phe Glu Leu Leu Pro Thr Pro Pro Leu Ser Pro Ser
       50                  55                  60
   Arg Arg Ser Gly Leu Cys Ser Pro Ser Tyr Val Ala Val Ala Thr Ser
   65                  70                  75                  80
   Phe Ser Pro Arg Glu Asp Asp Asp Gly Gly Gly Gly Asn Phe Ser Thr
                   85                  90                  95
   Ala Asp Gln Leu Glu Met Met Thr Glu Leu Leu Gly Gly Asp Met Val
               100                 105                 110
   Asn Gln Ser Phe Ile Cys Asp Pro Asp Asp Glu Thr Phe Ile Lys Asn
           115                 120                 125
   Ile Ile Ile Gln Asp Cys Met Trp Ser Gly Phe Ser Ala Ala Ala Lys
       130                 135                 140
   Leu Val Ser Glu Lys Leu Ala Ser Tyr Gln Ala Ala Arg Lys Asp Ser
   145                 150                 155                 160
   Thr Ser Leu Ser Pro Ala Arg Gly His Ser Val Cys Ser Thr Ser Ser
                   165                 170                 175
   Leu Tyr Leu Gln Asp Leu Thr Ala Ala Ala Ser Glu Cys Ile Asp Pro
               180                 185                 190
   Ser Val Val Phe Pro Tyr Pro Leu Asn Asp Ser Ser Ser Pro Lys Ser
           195                 200                 205
   Cys Thr Ser Ser Asp Ser Thr Ala Phe Ser Pro Ser Ser Asp Ser Leu
       210                 215                 220
   Leu Ser Ser Glu Ser Ser Pro Arg Ala Ser Pro Glu Pro Leu Val Leu
   225                 230                 235                 240
   His Glu Glu Thr Pro Pro Thr Thr Ser Ser Asp Ser Glu Glu Glu Gln
                   245                 250                 255
   Glu Asp Glu Glu Glu Ile Asp Val Val Ser Val Glu Lys Arg Gln Thr
               260                 265                 270
   Pro Ala Lys Arg Ser Glu Ser Gly Ser Ser Pro Ser Arg Gly His Ser
   275 280 285
   Lys Pro Pro His Ser Pro Leu Val Leu Lys Arg Cys His Val Ser Thr
   290 295 300
   His Gln His Asn Tyr Ala Ala Pro Pro Ser Thr Arg Lys Asp Tyr Pro
   305 310 315 320
   Ala Ala Lys Arg Ala Lys Leu Asp Ser Gly Arg Val Leu Lys Gln Ile
   325 330 335
   Ser Asn Asn Arg Lys Cys Ser Ser Pro Arg Ser Ser Asp Thr Glu Asp
   340 345 350
   Val Thr Lys Arg Lys Asn His Asn Phe Leu Glu Arg Lys Arg Arg Asn
   355 360 365
   Asp Leu Arg Ser Arg Phe Leu Ala Leu Arg Asp Gln Val Pro Thr Leu
   370 375 380
   Ala Ser Cys Ser Lys Ala Pro Lys Val Val Ile Leu Ser Lys Ala Leu
   385 390 395 400
   Glu Tyr Leu Gln Ala Leu Val Gly Ala Glu Lys Lys Met Ala Thr Glu
   405 410 415
   Lys Arg Gln Leu Arg Cys Arg Gln Gln Gln Leu Gln Lys Arg Ile Ala
   420 425 430
   Tyr Leu Ser Gly Tyr
   435
<210> 167
   <211> 1227
   <212> DNA
   <213> Mouse
<220>
   <221> CDS
   <222> (1).. (1227)
   <223>
<400> 167
   atg ccc ctc aac gtg aac ttc acc aac agg aac tat gac ctc gac tac 48
   Met Pro Leu Asn Val Asn Phe Thr Asn Arg Asn Tyr Asp Leu Asp Tyr
   1 5 10 15
   gac tcc gta cag ccc tat ttc atc tgc gac gag gaa gag aat ttc tat 96
   Asp Ser Val Gln Pro Tyr Phe Ile Cys Asp Glu Glu Glu Asn Phe Tyr
   20 25 30
   cac cag caa cag cag agc gag ctg cag ccg ccc gcg ccc agt gag gat 144
   His Gln Gln Gln Gln Ser Glu Leu Gln Pro Pro Ala Pro Ser Glu Asp
           35                  40                  45
   atc tgg aag aaa ttc gag ctg ctt ccc acc ccg ccc ctg tcc ccg agc      192
   Ile Trp Lys Lys Phe Glu Leu Leu Pro Thr Pro Pro Leu Ser Pro Ser
       50                  55                  60
   cgc cgc tcc ggg ctc tgc tct cca tcc tat gtt gcg gtc gct acg tcc      240
   Arg Arg Ser Gly Leu Cys Ser Pro Ser Tyr Val Ala Val Ala Thr Ser
   65                  70                  75                  80
   ttc tcc cca agg gaa gac gat gac ggc ggc ggt ggc aac ttc tcc acc      288
   Phe Ser Pro Arg Glu Asp Asp Asp Gly Gly Gly Gly Asn Phe Ser Thr
                   85                  90                  95
   gcc gat cag ctg gag atg atg acc gag tta ctt gga gga gac atg gtg      336
   Ala Asp Gln Leu Glu Met Met Thr Glu Leu Leu Gly Gly Asp Met Val
               100                 105                 110
   aac cag agc ttc atc tgc gat cct gac gac gag acc ttc atc aag aac      384
   Asn Gln Ser Phe Ile Cys Asp Pro Asp Asp Glu Thr Phe Ile Lys Asn
           115                 120                 125
   atc atc atc cag gac tgt atg tgg agc ggt ttc tca gcc gct gcc aag      432
   Ile Ile Ile Gln Asp Cys Met Trp Ser Gly Phe Ser Ala Ala Ala Lys
       130                 135                 140
   ctg gag aga gtg gtg agc gac agg ctg gcc cca ggc gcg ccc cgg ggg      480
   Leu Glu Arg Val Val Ser Asp Arg Leu Ala Pro Gly Ala Pro Arg Gly
   145                 150                 155                 160
   aac ccg ccc aaa gcg ccc gct acc ccg gac ggc act cct agt ctg gaa      528
   Asn Pro Pro Lys Ala Pro Ala Thr Pro Asp Gly Thr Pro Ser Leu Glu
                   165                 170                 175
   gcc agt aac ccg gcg ccc gcc acc caa tgt cag ctg ggc gag ccc aag      576
   Ala Ser Asn Pro Ala Pro Ala Thr Gln Cys Gln Leu Gly Glu Pro Lys
               180                 185                 190
   act cag gcc tgc tcc ggg tcc gag agc ccc agc gat tct gaa ggt gaa      624
   Thr Gln Ala Cys Ser Gly Ser Glu Ser Pro Ser Asp Ser Glu Gly Glu
           195                 200                 205
   gag att gac gtg gtg acc gtg gag aag agg cga tct ctg gac atc cga      672
   Glu Ile Asp Val Val Thr Val Glu Lys Arg Arg Ser Leu Asp Ile Arg
       210                 215                 220
   aag cca gtc acc atc acg gtg cga gca gac ccc ctg gac ccc tgc atg      720
   Lys Pro Val Thr Ile Thr Val Arg Ala Asp Pro Leu Asp Pro Cys Met
   225                 230                 235                 240
   aag cac ttc cat atc tct atc cac caa cag cag cat aac tat gct gcc      768
   Lys His Phe His Ile Ser Ile His Gln Gln Gln His Asn Tyr Ala Ala
   245 250 255
   cgt ttt cct cca gaa agt tgc tct caa gag ggg gat cct gag cca ggt 816
   Arg Phe Pro Pro Glu Ser Cys Ser Gln Glu Gly Asp Pro Glu Pro Gly
   260 265 270
   ccc cag gaa gag gct ccg gag ata gaa gct ccc aag gag aaa gag gag 864
   Pro Gln Glu Glu Ala Pro Glu Ile Glu Ala Pro Lys Glu Lys Glu Glu
   275 280 285
   gag gaa gag gaa gag gag gaa gaa gag att gtg agc ccc cca cct gtc 912
   Glu Glu Glu Glu Glu Glu Glu Glu Glu Ile Val Ser Pro Pro Pro Val
   290 295 300
   gga agt gag gct ccc cag tcc tgc cac ccc aaa cct gtc agt tct gac 960
   Gly Ser Glu Ala Pro Gln Ser Cys His Pro Lys Pro Val Ser Ser Asp
   305 310 315 320
   act gag gac gtg acc aag agg aag aac cat aac ttc ttg gaa cga aaa 1008
   Thr Glu Asp Val Thr Lys Arg Lys Asn His Asn Phe Leu Glu Arg Lys
   325 330 335
   agg agg aat gac ctc cgc tcc cgg ttc cta gcc ctg cgg gac cag gtt 1056
   Arg Arg Asn Asp Leu Arg Ser Arg Phe Leu Ala Leu Arg Asp Gln Val
   340 345 350
   ccc acc ctg gcc agc tgc tct aag gcc ccc aaa gtc gtg atc ctc agc 1104
   Pro Thr Leu Ala Ser Cys Ser Lys Ala Pro Lys Val Val Ile Leu Ser
   355 360 365
   aag gcg tta gaa tac ttg cag gct ttg gtg ggg gct gaa aag aaa atg 1152
   Lys Ala Leu Glu Tyr Leu Gln Ala Leu Val Gly Ala Glu Lys Lys Met
   370 375 380
   gct aca gag aaa agg cag ctc cgg tgt cgg caa cag caa ctg caa aag 1200
   Ala Thr Glu Lys Arg Gln Leu Arg Cys Arg Gln Gln Gln Leu Gln Lys
   385 390 395 400
   aga atc gcg tac ctc agt ggc tac taa 1227
   Arg Ile Ala Tyr Leu Ser Gly Tyr
   405
<210> 168
   <211> 408
   <212> PRT
   <213> Mouse
<400> 168
   Met Pro Leu Asn Val Asn Phe Thr Asn Arg Asn Tyr Asp Leu Asp Tyr
   1 5 10 15
   Asp Ser Val Gln Pro Tyr Phe Ile Cys Asp Glu Glu Glu Asn Phe Tyr
   20 25 30
   His Gln Gln Gln Gln Ser Glu Leu Gln Pro Pro Ala Pro Ser Glu Asp
   35 40 45
   Ile Trp Lys Lys Phe Glu Leu Leu Pro Thr Pro Pro Leu Ser Pro Ser
   50 55 60
   Arg Arg Ser Gly Leu Cys Ser Pro Ser Tyr Val Ala Val Ala Thr Ser
   65 70 75 80
   Phe Ser Pro Arg Glu Asp Asp Asp Gly Gly Gly Gly Asn Phe Ser Thr
   85 90 95
   Ala Asp Gln Leu Glu Met Met Thr Glu Leu Leu Gly Gly Asp Met Val
   100 105 110
   Asn Gln Ser Phe Ile Cys Asp Pro Asp Asp Glu Thr Phe Ile Lys Asn
   115 120 125
   Ile Ile Ile Gln Asp Cys Met Trp Ser Gly Phe Ser Ala Ala Ala Lys
   130 135 140
   Leu Glu Arg Val Val Ser Asp Arg Leu Ala Pro Gly Ala Pro Arg Gly
   145 150 155 160
   Asn Pro Pro Lys Ala Pro Ala Thr Pro Asp Gly Thr Pro Ser Leu Glu
   165 170 175
   Ala Ser Asn Pro Ala Pro Ala Thr Gln Cys Gln Leu Gly Glu Pro Lys
   180 185 190
   Thr Gln Ala Cys Ser Gly Ser Glu Ser Pro Ser Asp Ser Glu Gly Glu
   195 200 205
   Glu Ile Asp Val Val Thr Val Glu Lys Arg Arg Ser Leu Asp Ile Arg
   210 215 220
   Lys Pro Val Thr Ile Thr Val Arg Ala Asp Pro Leu Asp Pro Cys Met
   225 230 235 240
   Lys His Phe His Ile Ser Ile His Gln Gln Gln His Asn Tyr Ala Ala
   245 250 255
   Arg Phe Pro Pro Glu Ser Cys Ser Gln Glu Gly Asp Pro Glu Pro Gly
   260 265 270
   Pro Gln Glu Glu Ala Pro Glu Ile Glu Ala Pro Lys Glu Lys Glu Glu
   275 280 285
   Glu Glu Glu Glu Glu Glu Glu Glu Glu Ile Val Ser Pro Pro Pro Val
   290 295 300
   Gly Ser Glu Ala Pro Gln Ser Cys His Pro Lys Pro Val Ser Ser Asp
   305 310 315 320
   Thr Glu Asp Val Thr Lys Arg Lys Asn His Asn Phe Leu Glu Arg Lys
   325 330 335
   Arg Arg Asn Asp Leu Arg Ser Arg Phe Leu Ala Leu Arg Asp Gln Val
   340 345 350
   Pro Thr Leu Ala Ser Cys Ser Lys Ala Pro Lys Val Val Ile Leu Ser
   355 360 365
   Lys Ala Leu Glu Tyr Leu Gln Ala Leu Val Gly Ala Glu Lys Lys Met
   370 375 380
   Ala Thr Glu Lys Arg Gln Leu Arg Cys Arg Gln Gln Gln Leu Gln Lys
   385 390 395 400
   Arg Ile Ala Tyr Leu Ser Gly Tyr
   405
<210> 169
   <211> 1113
   <212> DNA
   <213> Mouse
<220>
   <221> CDS
   <222> (1).. (1113)
   <223>
<400> 169
   atg gac ttc gac tcg tat cag cac tat ttc tac gac tat gac tgc gga 48
   Met Asp Phe Asp Ser Tyr Gln His Tyr Phe Tyr Asp Tyr Asp Cys Gly
   1 5 10 15
   gag gat ttc tac cgc tcc acg gcg ccc agc gag gac atc tgg aag aaa 96
   Glu Asp Phe Tyr Arg Ser Thr Ala Pro Ser Glu Asp Ile Trp Lys Lys
   20 25 30
   ttc gag ctg gtg ccg tcg ccc ccc acg tcg ccg ccc tgg ggc tcc ggt 144
   Phe Glu Leu Val Pro Ser Pro Pro Thr Ser Pro Pro Trp Gly Ser Gly
   35 40 45
   ccc ggc gcc gtg gac cca gcc tct ggg att aat ccc ggg gag ccg tgg 192
   Pro Gly Ala Val Asp Pro Ala Ser Gly Ile Asn Pro Gly Glu Pro Trp
   50 55 60
   cct gga ggg ggt gcc ggg gac gag gcg gaa tct cgg ggc cat tcg aaa 240
   Pro Gly Gly Gly Ala Gly Asp Glu Ala Glu Ser Arg Gly His Ser Lys
   65 70 75 80
   gcc tgg ggc agg aat tat gct tcc atc att cgc cgt gac tgc atg tgg 288
   Ala Trp Gly Arg Asn Tyr Ala Ser Ile Ile Arg Arg Asp Cys Met Trp
   85 90 95
   agc ggc ttc tcc gcc cga gaa cgg ctg gag aga gtg gtg agc gac agg      336
   Ser Gly Phe Ser Ala Arg Glu Arg Leu Glu Arg Val Val Ser Asp Arg
                   100                 105                 110
   ctg gcc cca ggc gcg ccc cgg ggg aac ccg ccc aaa gcg ccc gct acc      384
   Leu Ala Pro Gly Ala Pro Arg Gly Asn Pro Pro Lys Ala Pro Ala Thr
               115                 120                 125
   ccg gac ggc act cct agt ctg gaa gcc agt aac ccg gcg ccc gcc acc      432
   Pro Asp Gly Thr Pro Ser Leu Glu Ala Ser Asn Pro Ala Pro Ala Thr
           130                 135                 140
   caa tgt cag ctg ggc gag ccc aag act cag gcc tgc tcc ggg tcc gag      480
   Gln Cys Gln Leu Gly Glu Pro Lys Thr Gln Ala Cys Ser Gly Ser Glu
   145                 150                 155                 160
   agc ccc agc gat tct gaa ggt gaa gag att gac gtg gtg acc gtg gag      528
   Ser Pro Ser Asp Ser Glu Gly Glu Glu Ile Asp Val Val Thr Val Glu
                       165                 170                 175
   aag agg cga tct ctg gac atc cga aag cca gtc acc atc acg gtg cga      576
   Lys Arg Arg Ser Leu Asp Ile Arg Lys Pro Val Thr Ile Thr Val Arg
                   180                 185                 190
   gca gac ccc ctg gac ccc tgc atg aag cac ttc cat atc tct atc cac      624
   Ala Asp Pro Leu Asp Pro Cys Met Lys His Phe His Ile Ser Ile His
               195                 200                 205
   caa cag cag cat aac tat gct gcc cgt ttt cct cca gaa agt tgc tct      672
   Gln Gln Gln His Asn Tyr Ala Ala Arg Phe Pro Pro Glu Ser Cys Ser
           210                 215                 220
   caa gag ggg gat cct gag cca ggt ccc cag gaa gag gct ccg gag ata      720
   Gln Glu Gly Asp Pro Glu Pro Gly Pro Gln Glu Glu Ala Pro Glu Ile
   225                 230                 235                 240
   gaa gct ccc aag gag aaa gag gag gag gaa gag gaa gag gag gaa gaa      768
   Glu Ala Pro Lys Glu Lys Glu Glu Glu Glu Glu Glu Glu Glu Glu Glu
                       245                 250                 255
   gag att gtg agc ccc cca cct gtc gga agt gag gct ccc cag tcc tgc      816
   Glu Ile Val Ser Pro Pro Pro Val Gly Ser Glu Ala Pro Gln Ser Cys
                   260                 265                 270
   cac ccc aaa cct gtc tcc tca gac acg gag gaa aac gac aag agg cgg      864
   His Pro Lys Pro Val Ser Ser Asp Thr Glu Glu Asn Asp Lys Arg Arg
               275                 280                 285
   aca cac aac gtc ttg gaa cgt cag agg agg aac gag ctg aag cgc agc      912
   Thr His Asn Val Leu Glu Arg Gln Arg Arg Asn Glu Leu Lys Arg Ser
           290                 295                 300
   ttt ttt gcc ctg cgt gac cag atc cct gaa ttg gaa aac aac gaa aag 960
   Phe Phe Ala Leu Arg Asp Gln Ile Pro Glu Leu Glu Asn Asn Glu Lys
   305 310 315 320
   gcc ccc aag gta gtg atc ctc aaa aaa gcc acc gcc tac atc ctg tcc 1008
   Ala Pro Lys Val Val Ile Leu Lys Lys Ala Thr Ala Tyr Ile Leu Ser
   325 330 335
   att caa gca gac gag cac aag ctc acc tct gaa aag gac tta ttg agg 1056
   Ile Gln Ala Asp Glu His Lys Leu Thr Ser Glu Lys Asp Leu Leu Arg
   340 345 350
   aaa cga cga gaa cag ttg aaa cac aaa ctc gaa cag ctt cga aac tct 1104
   Lys Arg Arg Glu Gln Leu Lys His Lys Leu Glu Gln Leu Arg Asn Ser
   355 360 365
   ggt gca taa 1113
   Gly Ala
   370
<210> 170
   <211> 370
   <212> PRT
   <213> Mouse
<400> 170
   Met Asp Phe Asp Ser Tyr Gln His Tyr Phe Tyr Asp Tyr Asp Cys Gly
   1 5 10 15
   Glu Asp Phe Tyr Arg Ser Thr Ala Pro Ser Glu Asp Ile Trp Lys Lys
   20 25 30
   Phe Glu Leu Val Pro Ser Pro Pro Thr Ser Pro Pro Trp Gly Ser Gly
   35 40 45
   Pro Gly Ala Val Asp Pro Ala Ser Gly Ile Asn Pro Gly Glu Pro Trp
   50 55 60
   Pro Gly Gly Gly Ala Gly Asp Glu Ala Glu Ser Arg Gly His Ser Lys
   65 70 75 80
   Ala Trp Gly Arg Asn Tyr Ala Ser Ile Ile Arg Arg Asp Cys Met Trp
   85 90 95
   Ser Gly Phe Ser Ala Arg Glu Arg Leu Glu Arg Val Val Ser Asp Arg
   100 105 110
   Leu Ala Pro Gly Ala Pro Arg Gly Asn Pro Pro Lys Ala Pro Ala Thr
   115 120 125
   Pro Asp Gly Thr Pro Ser Leu Glu Ala Ser Asn Pro Ala Pro Ala Thr
   130 135 140
   Gln Cys Gln Leu Gly Glu Pro Lys Thr Gln Ala Cys Ser Gly Ser Glu
   145 150 155 160
   Ser Pro Ser Asp Ser Glu Gly Glu Glu Ile Asp Val Val Thr Val Glu
   165 170 175
   Lys Arg Arg Ser Leu Asp Ile Arg Lys Pro Val Thr Ile Thr Val Arg
   180 185 190
   Ala Asp Pro Leu Asp Pro Cys Met Lys His Phe His Ile Ser Ile His
   195 200 205
   Gln Gln Gln His Asn Tyr Ala Ala Arg Phe Pro Pro Glu Ser Cys Ser
   210 215 220
   Gln Glu Gly Asp Pro Glu Pro Gly Pro Gln Glu Glu Ala Pro Glu Ile
   225 230 235 240
   Glu Ala Pro Lys Glu Lys Glu Glu Glu Glu Glu Glu Glu Glu Glu Glu
   245 250 255
   Glu Ile Val Ser Pro Pro Pro Val Gly Ser Glu Ala Pro Gln Ser Cys
   260 265 270
   His Pro Lys Pro Val Ser Ser Asp Thr Glu Glu Asn Asp Lys Arg Arg
   275 280 285
   Thr His Asn Val Leu Glu Arg Gln Arg Arg Asn Glu Leu Lys Arg Ser
   290 295 300
   Phe Phe Ala Leu Arg Asp Gln Ile Pro Glu Leu Glu Asn Asn Glu Lys
   305 310 315 320
   Ala Pro Lys Val Val Ile Leu Lys Lys Ala Thr Ala Tyr Ile Leu Ser
   325 330 335
   Ile Gln Ala Asp Glu His Lys Leu Thr Ser Glu Lys Asp Leu Leu Arg
   340 345 350
   Lys Arg Arg Glu Gln Leu Lys His Lys Leu Glu Gln Leu Arg Asn Ser
   355 360 365
   Gly Ala
   370
<210> 171
   <211> 1200
   <212> DNA
   <213> Mouse
<220>
   <221> CDS
   <222> (1).. (1200)
   <223>
<400> 171
   atg gac ttc gac tcg tat cag cac tat ttc tac gac tat gac tgc gga 48
   Met Asp Phe Asp Ser Tyr Gln His Tyr Phe Tyr Asp Tyr Asp Cys Gly
   1 5 10 15
   gag gat ttc tac cgc tcc acg gcg ccc agc gag gac atc tgg aag aaa 96
   Glu Asp Phe Tyr Arg Ser Thr Ala Pro Ser Glu Asp Ile Trp Lys Lys
   20 25 30
   ttc gag ctg gtg ccg tcg ccc ccc acg tcg ccg ccc tgg ggc tcc ggt 144
   Phe Glu Leu Val Pro Ser Pro Pro Thr Ser Pro Pro Trp Gly Ser Gly
   35 40 45
   ccc ggc gcc gtg gac cca gcc tct ggg att aat ccc ggg gag ccg tgg 192
   Pro Gly Ala Val Asp Pro Ala Ser Gly Ile Asn Pro Gly Glu Pro Trp
   50 55 60
   cct gga ggg ggt gcc ggg gac gag gcg gaa tct cgg ggc cat tcg aaa 240
   Pro Gly Gly Gly Ala Gly Asp Glu Ala Glu Ser Arg Gly His Ser Lys
   65 70 75 80
   gcc tgg ggc agg aat tat gct tcc atc att cgc cgt gac tgc atg tgg 288
   Ala Trp Gly Arg Asn Tyr Ala Ser Ile Ile Arg Arg Asp Cys Met Trp
   85 90 95
   agc ggc ttc tcc gcc cga gaa cgg ctg gtc tcg gag aag ctg gcc tcc 336
   Ser Gly Phe Ser Ala Arg Glu Arg Leu Val Ser Glu Lys Leu Ala Ser
   100 105 110
   tac cag gct gcg cgc aaa gac agc acc agc ctg agc ccc gcc cgc ggg 384
   Tyr Gln Ala Ala Arg Lys Asp Ser Thr Ser Leu Ser Pro Ala Arg Gly
   115 120 125
   cac agc gtc tgc tcc acc tcc agc ctg tac ctg cag gac ctc acc gcc 432
   His Ser Val Cys Ser Thr Ser Ser Leu Tyr Leu Gln Asp Leu Thr Ala
   130 135 140
   gcc gcg tcc gag tgc att gac ccc tca gtg gtc ttt ccc tac ccg ctc 480
   Ala Ala Ser Glu Cys Ile Asp Pro Ser Val Val Phe Pro Tyr Pro Leu
   145 150 155 160
   aac gac agc agc tcg ccc aaa tcc tgt acc tcg tcc gat tcc acg gcc 528
   Asn Asp Ser Ser Ser Pro Lys Ser Cys Thr Ser Ser Asp Ser Thr Ala
   165 170 175
   ttc tct cct tcc tcg gac tcg ctg ctg tcc tcc gag tcc tcc cca cgg 576
   Phe Ser Pro Ser Ser Asp Ser Leu Leu Ser Ser Glu Ser Ser Pro Arg
   180 185 190
   gcc agc cct gag ccc cta gtg ctg cat gag gag aca ccg ccc acc acc 624
   Ala Ser Pro Glu Pro Leu Val Leu His Glu Glu Thr Pro Pro Thr Thr
               195                 200                 205
   agc agc gac tct gaa gaa gag caa gaa gat gag gaa gaa att gat gtg      672
   Ser Ser Asp Ser Glu Glu Glu Gln Glu Asp Glu Glu Glu Ile Asp Val
       210                 215                 220
   gtg tct gtg gag aag agg caa acc cct gcc aag agg tcg gag tcg ggc      720
   Val Ser Val Glu Lys Arg Gln Thr Pro Ala Lys Arg Ser Glu Ser Gly
   225                 230                 235                 240
   tca tct cca tcc cga ggc cac agc aaa cct ccg cac agc cca ctg gtc      768
   Ser Ser Pro Ser Arg Gly His Ser Lys Pro Pro His Ser Pro Leu Val
                   245                 250                 255
   ctc aag agg tgc cac gtc tcc act cac cag cac aac tac gcc gca ccc      816
   Leu Lys Arg Cys His Val Ser Thr His Gln His Asn Tyr Ala Ala Pro
               260                 265                 270
   ccc tcc aca agg aag gac tat cca gct gcc aag agg gcc aag ttg gac      864
   Pro Ser Thr Arg Lys Asp Tyr Pro Ala Ala Lys Arg Ala Lys Leu Asp
           275                 280                 285
   agt ggc agg gtc ctg aag cag atc agc aac aac cgc aag tgc tcc agc      912
   Ser Gly Arg Val Leu Lys Gln Ile Ser Asn Asn Arg Lys Cys Ser Ser
       290                 295                 300
   ccc agg tcc tca gac acg gag gaa aac gac aag agg cgg aca cac aac      960
   Pro Arg Ser Ser Asp Thr Glu Glu Asn Asp Lys Arg Arg Thr His Asn
   305                 310                 315                 320
   gtc ttg gaa cgt cag agg agg aac gag ctg aag cgc agc ttt ttt gcc     1008
   Val Leu Glu Arg Gln Arg Arg Asn Glu Leu Lys Arg Ser Phe Phe Ala
                   325                 330                 335
   ctg cgt gac cag atc cct gaa ttg gaa aac aac gaa aag gcc ccc aag     1056
   Leu Arg Asp Gln Ile Pro Glu Leu Glu Asn Asn Glu Lys Ala Pro Lys
               340                 345                 350
   gta gtg atc ctc aaa aaa gcc acc gcc tac atc ctg tcc att caa gca     1104
   Val Val Ile Leu Lys Lys Ala Thr Ala Tyr Ile Leu Ser Ile Gln Ala
           355                 360                 365
   gac gag cac aag ctc acc tct gaa aag gac tta ttg agg aaa cga cga     1152
   Asp Glu His Lys Leu Thr Ser Glu Lys Asp Leu Leu Arg Lys Arg Arg
       370                 375                 380
   gaa cag ttg aaa cac aaa ctc gaa cag ctt cga aac tct ggt gca taa     1200
   Glu Gln Leu Lys His Lys Leu Glu Gln Leu Arg Asn Ser Gly Ala
   385                 390                 395
<210> 172
   <211> 399
   <212> PRT
   <213> Mouse
<400> 172
   Met Asp Phe Asp Ser Tyr Gln His Tyr Phe Tyr Asp Tyr Asp Cys Gly
   1 5 10 15
   Glu Asp Phe Tyr Arg Ser Thr Ala Pro Ser Glu Asp Ile Trp Lys Lys
   20 25 30
   Phe Glu Leu Val Pro Ser Pro Pro Thr Ser Pro Pro Trp Gly Ser Gly
   35 40 45
   Pro Gly Ala Val Asp Pro Ala Ser Gly Ile Asn Pro Gly Glu Pro Trp
   50 55 60
   Pro Gly Gly Gly Ala Gly Asp Glu Ala Glu Ser Arg Gly His Ser Lys
   65 70 75 80
   Ala Trp Gly Arg Asn Tyr Ala Ser Ile Ile Arg Arg Asp Cys Met Trp
   85 90 95
   Ser Gly Phe Ser Ala Arg Glu Arg Leu Val Ser Glu Lys Leu Ala Ser
   100 105 110
   Tyr Gln Ala Ala Arg Lys Asp Ser Thr Ser Leu Ser Pro Ala Arg Gly
   115 120 125
   His Ser Val Cys Ser Thr Ser Ser Leu Tyr Leu Gln Asp Leu Thr Ala
   130 135 140
   Ala Ala Ser Glu Cys Ile Asp Pro Ser Val Val Phe Pro Tyr Pro Leu
   145 150 155 160
   Asn Asp Ser Ser Ser Pro Lys Ser Cys Thr Ser Ser Asp Ser Thr Ala
   165 170 175
   Phe Ser Pro Ser Ser Asp Ser Leu Leu Ser Ser Glu Ser Ser Pro Arg
   180 185 190
   Ala Ser Pro Glu Pro Leu Val Leu His Glu Glu Thr Pro Pro Thr Thr
   195 200 205
   Ser Ser Asp Ser Glu Glu Glu Gln Glu Asp Glu Glu Glu Ile Asp Val
   210 215 220
   Val Ser Val Glu Lys Arg Gln Thr Pro Ala Lys Arg Ser Glu Ser Gly
   225 230 235 240
   Ser Ser Pro Ser Arg Gly His Ser Lys Pro Pro His Ser Pro Leu Val
   245 250 255
   Leu Lys Arg Cys His Val Ser Thr His Gln His Asn Tyr Ala Ala Pro
   260 265 270
   Pro Ser Thr Arg Lys Asp Tyr Pro Ala Ala Lys Arg Ala Lys Leu Asp
   275 280 285
   Ser Gly Arg Val Leu Lys Gln Ile Ser Asn Asn Arg Lys Cys Ser Ser
   290 295 300
   Pro Arg Ser Ser Asp Thr Glu Glu Asn Asp Lys Arg Arg Thr His Asn
   305 310 315 320
   Val Leu Glu Arg Gln Arg Arg Asn Glu Leu Lys Arg Ser Phe Phe Ala
   325 330 335
   Leu Arg Asp Gln Ile Pro Glu Leu Glu Asn Asn Glu Lys Ala Pro Lys
   340 345 350
   Val Val Ile Leu Lys Lys Ala Thr Ala Tyr Ile Leu Ser Ile Gln Ala
   355 360 365
   Asp Glu His Lys Leu Thr Ser Glu Lys Asp Leu Leu Arg Lys Arg Arg
   370 375 380
   Glu Gln Leu Lys His Lys Leu Glu Gln Leu Arg Asn Ser Gly Ala
   385 390 395
<210> 173
   <211> 1320
   <212> DNA
   <213> Mouse
<220>
   <221> CDS
   <222> (1)..(1320)
   <223>
<400> 173
   atg ccc ctc aac gtg aac ttc acc aac agg aac tat gac ctc gac tac 48
   Met Pro Leu Asn Val Asn Phe Thr Asn Arg Asn Tyr Asp Leu Asp Tyr
   1 5 10 15
   gac tcc gta cag ccc tat ttc atc tgc gac gag gaa gag aat ttc tat 96
   Asp Ser Val Gln Pro Tyr Phe Ile Cys Asp Glu Glu Glu Asn Phe Tyr
   20 25 30
   cac cag caa cag cag agc gag ctg cag ccg ccc gcg ccc agt gag gat 144
   His Gln Gln Gln Gln Ser Glu Leu Gln Pro Pro Ala Pro Ser Glu Asp
   35 40 45
   atc tgg aag aaa ttc gag ctg ctt ccc acc ccg ccc ctg tcc ccg agc 192
   Ile Trp Lys Lys Phe Glu Leu Leu Pro Thr Pro Pro Leu Ser Pro Ser
   50 55 60
   cgc cgc tcc ggg ctc tgc tct cca tcc tat gtt gcg gtc gct acg tcc 240
   Arg Arg Ser Gly Leu Cys Ser Pro Ser Tyr Val Ala Val Ala Thr Ser
   65                  70                  75                  80
   ttc tcc cca agg gaa gac gat gac ggc ggc ggt ggc aac ttc tcc acc      288
   Phe Ser Pro Arg Glu Asp Asp Asp Gly Gly Gly Gly Asn Phe Ser Thr
                   85                  90                  95
   gcc gat cag ctg gag atg atg acc gag tta ctt gga gga gac atg gtg      336
   Ala Asp Gln Leu Glu Met Met Thr Glu Leu Leu Gly Gly Asp Met Val
               100                 105                 110
   aac cag agc ttc atc tgc gat cct gac gac gag acc ttc atc aag aac      384
   Asn Gln Ser Phe Ile Cys Asp Pro Asp Asp Glu Thr Phe Ile Lys Asn
           115                 120                 125
   atc atc atc cag gac tgt atg gag agc ggt ttc tca gcc gct gcc aag      432
   Ile Ile Ile Gln Asp Cys Met Glu Ser Gly Phe Ser Ala Ala Ala Lys
       130                 135                 140
   ctg gtc tcg gag aag ctg gcc tcc tac cag gct gcg cgc aaa gac agc      480
   Leu Val Ser Glu Lys Leu Ala Ser Tyr Gln Ala Ala Arg Lys Asp Ser
   145                 150                 155                 160
   acc agc ctg agc ccc gcc cgc ggg cac agc gtc tgc tcc acc tcc agc      528
   Thr Ser Leu Ser Pro Ala Arg Gly His Ser Val Cys Ser Thr Ser Ser
                   165                 170                 175
   ctg tac ctg cag gac ctc acc gcc gcc gcg tcc gag tgc att gac ccc      576
   Leu Tyr Leu Gln Asp Leu Thr Ala Ala Ala Ser Glu Cys Ile Asp Pro
               180                 185                 190
   tca gtg gtc ttt ccc tac ccg ctc aac gac agc agc tcg ccc aaa tcc      624
   Ser Val Val Phe Pro Tyr Pro Leu Asn Asp Ser Ser Ser Pro Lys Ser
           195                 200                 205
   tgt acc tcg tcc gat tcc acg gcc ttc tct cct tcc tcg gac tcg ctg      672
   Cys Thr Ser Ser Asp Ser Thr Ala Phe Ser Pro Ser Ser Asp Ser Leu
       210                 215                 220
   ctg tcc tcc gag tcc tcc cca cgg gcc agc cct gag ccc cta gtg ctg      720
   Leu Ser Ser Glu Ser Ser Pro Arg Ala Ser Pro Glu Pro Leu Val Leu
   225                 230                 235                 240
   cat gag gag aca ccg ccc acc acc agc agc gac tct gaa gaa gag caa      768
   His Glu Glu Thr Pro Pro Thr Thr Ser Ser Asp Ser Glu Glu Glu Gln
                   245                 250                 255
   gaa gat gag gaa gaa att gat gtg gtg tct gtg gag aag agg caa acc      816
   Glu Asp Glu Glu Glu Ile Asp Val Val Ser Val Glu Lys Arg Gln Thr
               260                 265                 270
   cct gcc aag agg tcg gag tcg ggc tca tct cca tcc cga ggc cac agc      864
   Pro Ala Lys Arg Ser Glu Ser Gly Ser Ser Pro Ser Arg Gly His Ser
   275 280 285
   aaa cct ccg cac agc cca ctg gtc ctc aag agg tgc cac gtc tcc act 912
   Lys Pro Pro His Ser Pro Leu Val Leu Lys Arg Cys His Val Ser Thr
   290 295 300
   cac cag cac aac tac gcc gca ccc ccc tcc aca agg aag gac tat cca 960
   His Gln His Asn Tyr Ala Ala Pro Pro Ser Thr Arg Lys Asp Tyr Pro
   305 310 315 320
   gct gcc aag agg gcc aag ttg gac agt ggc agg gtc ctg aag cag atc 1008
   Ala Ala Lys Arg Ala Lys Leu Asp Ser Gly Arg Val Leu Lys Gln Ile
   325 330 335
   agc aac aac cgc aag tgc tcc agc ccc agg tcc tca gac acg gag gaa 1056
   Ser Asn Asn Arg Lys Cys Ser Ser Pro Arg Ser Ser Asp Thr Glu Glu
   340 345 350
   aac gac aag agg cgg aca cac aac gtc ttg gaa cgt cag agg agg aac 1104
   Asn Asp Lys Arg Arg Thr His Asn Val Leu Glu Arg Gln Arg Arg Asn
   355 360 365
   gag ctg aag cgc agc ttt ttt gcc ctg cgt gac cag atc cct gaa ttg 1152
   Glu Leu Lys Arg Ser Phe Phe Ala Leu Arg Asp Gln Ile Pro Glu Leu
   370 375 380
   gaa aac aac gaa aag gcc ccc aag gta gtg atc ctc aaa aaa gcc acc 1200
   Glu Asn Asn Glu Lys Ala Pro Lys Val Val Ile Leu Lys Lys Ala Thr
   385 390 395 400
   gcc tac atc ctg tcc att caa gca gac gag cac aag ctc acc tct gaa 1248
   Ala Tyr Ile Leu Ser Ile Gln Ala Asp Glu His Lys Leu Thr Ser Glu
   405 410 415
   aag gac tta ttg agg aaa cga cga gaa cag ttg aaa cac aaa ctc gaa 1296
   Lys Asp Leu Leu Arg Lys Arg Arg Glu Gln Leu Lys His Lys Leu Glu
   420 425 430
   cag ctt cga aac tct ggt gca taa 1320
   Gln Leu Arg Asn Ser Gly Ala
   435
<210> 174
   <211> 439
   <212> PRT
   <213> Mouse
<400> 174
   Met Pro Leu Asn Val Asn Phe Thr Asn Arg Asn Tyr Asp Leu Asp Tyr
   1 5 10 15
   Asp Ser Val Gln Pro Tyr Phe Ile Cys Asp Glu Glu Glu Asn Phe Tyr
   20 25 30
   His Gln Gln Gln Gln Ser Glu Leu Gln Pro Pro Ala Pro Ser Glu Asp
   35 40 45
   Ile Trp Lys Lys Phe Glu Leu Leu Pro Thr Pro Pro Leu Ser Pro Ser
   50 55 60
   Arg Arg Ser Gly Leu Cys Ser Pro Ser Tyr Val Ala Val Ala Thr Ser
   65 70 75 80
   Phe Ser Pro Arg Glu Asp Asp Asp Gly Gly Gly Gly Asn Phe Ser Thr
   85 90 95
   Ala Asp Gln Leu Glu Met Met Thr Glu Leu Leu Gly Gly Asp Met Val
   100 105 110
   Asn Gln Ser Phe Ile Cys Asp Pro Asp Asp Glu Thr Phe Ile Lys Asn
   115 120 125
   Ile Ile Ile Gln Asp Cys Met Glu Ser Gly Phe Ser Ala Ala Ala Lys
   130 135 140
   Leu Val Ser Glu Lys Leu Ala Ser Tyr Gln Ala Ala Arg Lys Asp Ser
   145 150 155 160
   Thr Ser Leu Ser Pro Ala Arg Gly His Ser Val Cys Ser Thr Ser Ser
   165 170 175
   Leu Tyr Leu Gln Asp Leu Thr Ala Ala Ala Ser Glu Cys Ile Asp Pro
   180 185 190
   Ser Val Val Phe Pro Tyr Pro Leu Asn Asp Ser Ser Ser Pro Lys Ser
   195 200 205
   Cys Thr Ser Ser Asp Ser Thr Ala Phe Ser Pro Ser Ser Asp Ser Leu
   210 215 220
   Leu Ser Ser Glu Ser Ser Pro Arg Ala Ser Pro Glu Pro Leu Val Leu
   225 230 235 240
   His Glu Glu Thr Pro Pro Thr Thr Ser Ser Asp Ser Glu Glu Glu Gln
   245 250 255
   Glu Asp Glu Glu Glu Ile Asp Val Val Ser Val Glu Lys Arg Gln Thr
   260 265 270
   Pro Ala Lys Arg Ser Glu Ser Gly Ser Ser Pro Ser Arg Gly His Ser
   275 280 285
   Lys Pro Pro His Ser Pro Leu Val Leu Lys Arg Cys His Val Ser Thr
   290 295 300
   His Gln His Asn Tyr Ala Ala Pro Pro Ser Thr Arg Lys Asp Tyr Pro
   305 310 315 320
   Ala Ala Lys Arg Ala Lys Leu Asp Ser Gly Arg Val Leu Lys Gln Ile
   325 330 335
   Ser Asn Asn Arg Lys Cys Ser Ser Pro Arg Ser Ser Asp Thr Glu Glu
   340 345 350
   Asn Asp Lys Arg Arg Thr His Asn Val Leu Glu Arg Gln Arg Arg Asn
   355 360 365
   Glu Leu Lys Arg Ser Phe Phe Ala Leu Arg Asp Gln Ile Pro Glu Leu
   370 375 380
   Glu Asn Asn Glu Lys Ala Pro Lys Val Val Ile Leu Lys Lys Ala Thr
   385 390 395 400
   Ala Tyr Ile Leu Ser Ile Gln Ala Asp Glu His Lys Leu Thr Ser Glu
   405 410 415
   Lys Asp Leu Leu Arg Lys Arg Arg Glu Gln Leu Lys His Lys Leu Glu
   420 425 430
   Gln Leu Arg Asn Ser Gly Ala
   435
<210> 175
   <211> 1320
   <212> DNA
   <213> Mouse
<220>
   <221> CDS
   <222> (1).. (1320)
   <223>
<400> 175
   atg ccc ctc aac gtg aac ttc acc aac agg aac tat gac ctc gac tac 48
   Met Pro Leu Asn Val Asn Phe Thr Asn Arg Asn Tyr Asp Leu Asp Tyr
   1 5 10 15
   gac tcc gta cag ccc tat ttc atc tgc gac gag gaa gag aat ttc tat 96
   Asp Ser Val Gln Pro Tyr Phe Ile Cys Asp Glu Glu Glu Asn Phe Tyr
   20 25 30
   cac cag caa cag cag agc gag ctg cag ccg ccc gcg ccc agt gag gat 144
   His Gln Gln Gln Gln Ser Glu Leu Gln Pro Pro Ala Pro Ser Glu Asp
   35 40 45
   atc tgg aag aaa ttc gag ctg ctt ccc acc ccg ccc ctg tcc ccg agc 192
   Ile Trp Lys Lys Phe Glu Leu Leu Pro Thr Pro Pro Leu Ser Pro Ser
   50 55 60
   cgc cgc tcc ggg ctc tgc tct cca tcc tat gtt gcg gtc gct acg tcc 240
   Arg Arg Ser Gly Leu Cys Ser Pro Ser Tyr Val Ala Val Ala Thr Ser
   65 70 75 80
   ttc tcc cca agg gaa gac gat gac ggc ggc ggt ggc aac ttc tcc acc 288
   Phe Ser Pro Arg Glu Asp Asp Asp Gly Gly Gly Gly Asn Phe Ser Thr
   85 90 95
   gcc gat cag ctg gag atg atg acc gag tta ctt gga gga gac atg gtg 336
   Ala Asp Gln Leu Glu Met Met Thr Glu Leu Leu Gly Gly Asp Met Val
   100 105 110
   aac cag agc ttc atc tgc gat cct gac gac gag acc ttc atc aag aac 384
   Asn Gln Ser Phe Ile Cys Asp Pro Asp Asp Glu Thr Phe Ile Lys Asn
   115 120 125
   atc atc atc cag gac tgt atg tgg agc ggt ttc tca gcc gct gcc aag 432
   Ile Ile Ile Gln Asp Cys Met Trp Ser Gly Phe Ser Ala Ala Ala Lys
   130 135 140
   ctg gtc tcg gag aag ctg gcc tcc tac cag gct gcg cgc aaa gac agc 480
   Leu Val Ser Glu Lys Leu Ala Ser Tyr Gln Ala Ala Arg Lys Asp Ser
   145 150 155 160
   acc agc ctg agc ccc gcc cgc ggg cac agc gtc tgc tcc acc tcc agc 528
   Thr Ser Leu Ser Pro Ala Arg Gly His Ser Val Cys Ser Thr Ser Ser
   165 170 175
   ctg tac ctg cag gac ctc acc gcc gcc gcg tcc gag tgc att gac ccc 576
   Leu Tyr Leu Gln Asp Leu Thr Ala Ala Ala Ser Glu Cys Ile Asp Pro
   180 185 190
   tca gtg gtc ttt ccc tac ccg ctc aac gac agc agc tcg ccc aaa tcc 624
   Ser Val Val Phe Pro Tyr Pro Leu Asn Asp Ser Ser Ser Pro Lys Ser
   195 200 205
   tgt acc tcg tcc gat tcc acg gcc ttc tct cct tcc tcg gac tcg ctg 672
   Cys Thr Ser Ser Asp Ser Thr Ala Phe Ser Pro Ser Ser Asp Ser Leu
   210 215 220
   ctg tcc tcc gag tcc tcc cca cgg gcc agc cct gag ccc cta gtg ctg 720
   Leu Ser Ser Glu Ser Ser Pro Arg Ala Ser Pro Glu Pro Leu Val Leu
   225 230 235 240
   cat gag gag aca ccg ccc acc acc agc agc gac tct gaa gaa gag caa 768
   His Glu Glu Thr Pro Pro Thr Thr Ser Ser Asp Ser Glu Glu Glu Gln
   245 250 255
   gaa gat gag gaa gaa att gat gtg gtg tct gtg gag aag agg caa acc 816
   Glu Asp Glu Glu Glu Ile Asp Val Val Ser Val Glu Lys Arg Gln Thr
   260 265 270
   cct gcc aag agg tcg gag tcg ggc tca tct cca tcc cga ggc cac agc 864
   Pro Ala Lys Arg Ser Glu Ser Gly Ser Ser Pro Ser Arg Gly His Ser
           275                 280                 285
   aaa cct ccg cac agc cca ctg gtc ctc aag agg tgc cac gtc tcc act      912
   Lys Pro Pro His Ser Pro Leu Val Leu Lys Arg Cys His Val Ser Thr
       290                 295                 300
   cac cag cac aac tac gcc gca ccc ccc tcc aca agg aag gac tat cca      960
   His Gln His Asn Tyr Ala Ala Pro Pro Ser Thr Arg Lys Asp Tyr Pro
   305                 310                 315                 320
   gct gcc aag agg gcc aag ttg gac agt ggc agg gtc ctg aag cag atc     1008
   Ala Ala Lys Arg Ala Lys Leu Asp Ser Gly Arg Val Leu Lys Gln Ile
                   325                 330                 335
   agc aac aac cgc aag tgc tcc agc ccc agg tcc tca gac acg gag gaa     1056
   Ser Asn Asn Arg Lys Cys Ser Ser Pro Arg Ser Ser Asp Thr Glu Glu
               340                 345                 350
   aac gac aag agg cgg aca cac aac gtc ttg gaa cgt cag agg agg aac     1104
   Asn Asp Lys Arg Arg Thr His Asn Val Leu Glu Arg Gln Arg Arg Asn
           355                 360                 365
   gag ctg aag cgc agc ttt ttt gcc ctg cgt gac cag atc cct gaa ttg     1152
   Glu Leu Lys Arg Ser Phe Phe Ala Leu Arg Asp Gln Ile Pro Glu Leu
       370                 375                 380
   gaa aac aac gaa aag gcc ccc aag gta gat atc ctc aaa aaa gcc acc     1200
   Glu Asn Asn Glu Lys Ala Pro Lys Val Asp Ile Leu Lys Lys Ala Thr
   385                 390                 395                 400
   gcc tac atc ctg tcc att caa gca gac gag cac aag ctc acc tct gaa     1248
   Ala Tyr Ile Leu Ser Ile Gln Ala Asp Glu His Lys Leu Thr Ser Glu
                   405                 410                 415
   aag gac tta ttg agg aaa cga cga gaa cag ttg aaa cac aaa ctc gaa     1296
   Lys Asp Leu Leu Arg Lys Arg Arg Glu Gln Leu Lys His Lys Leu Glu
               420                 425                 430
   cag ctt cga aac tct ggt gca taa                                     1320
   Gln Leu Arg Asn Ser Gly Ala
           435
<210> 176
   <211> 439
   <212> PRT
   <213> Mouse
<400> 176
   Met Pro Leu Asn Val Asn Phe Thr Asn Arg Asn Tyr Asp Leu Asp Tyr
   1               5                   10                  15
   Asp Ser Val Gln Pro Tyr Phe Ile Cys Asp Glu Glu Glu Asn Phe Tyr
   20 25 30
   His Gln Gln Gln Gln Ser Glu Leu Gln Pro Pro Ala Pro Ser Glu Asp
   35 40 45
   Ile Trp Lys Lys Phe Glu Leu Leu Pro Thr Pro Pro Leu Ser Pro Ser
   50 55 60
   Arg Arg Ser Gly Leu Cys Ser Pro Ser Tyr Val Ala Val Ala Thr Ser
   65 70 75 80
   Phe Ser Pro Arg Glu Asp Asp Asp Gly Gly Gly Gly Asn Phe Ser Thr
   85 90 95
   Ala Asp Gln Leu Glu Met Met Thr Glu Leu Leu Gly Gly Asp Met Val
   100 105 110
   Asn Gln Ser Phe Ile Cys Asp Pro Asp Asp Glu Thr Phe Ile Lys Asn
   115 120 125
   Ile Ile Ile Gln Asp Cys Met Trp Ser Gly Phe Ser Ala Ala Ala Lys
   130 135 140
   Leu Val Ser Glu Lys Leu Ala Ser Tyr Gln Ala Ala Arg Lys Asp Ser
   145 150 155 160
   Thr Ser Leu Ser Pro Ala Arg Gly His Ser Val Cys Ser Thr Ser Ser
   165 170 175
   Leu Tyr Leu Gln Asp Leu Thr Ala Ala Ala Ser Glu Cys Ile Asp Pro
   180 185 190
   Ser Val Val Phe Pro Tyr Pro Leu Asn Asp Ser Ser Ser Pro Lys Ser
   195 200 205
   Cys Thr Ser Ser Asp Ser Thr Ala Phe Ser Pro Ser Ser Asp Ser Leu
   210 215 220
   Leu Ser Ser Glu Ser Ser Pro Arg Ala Ser Pro Glu Pro Leu Val Leu
   225 230 235 240
   His Glu Glu Thr Pro Pro Thr Thr Ser Ser Asp Ser Glu Glu Glu Gln
   245 250 255
   Glu Asp Glu Glu Glu Ile Asp Val Val Ser Val Glu Lys Arg Gln Thr
   260 265 270
   Pro Ala Lys Arg Ser Glu Ser Gly Ser Ser Pro Ser Arg Gly His Ser
   275 280 285
   Lys Pro Pro His Ser Pro Leu Val Leu Lys Arg Cys His Val Ser Thr
   290 295 300
   His Gln His Asn Tyr Ala Ala Pro Pro Ser Thr Arg Lys Asp Tyr Pro
   305 310 315 320
   Ala Ala Lys Arg Ala Lys Leu Asp Ser Gly Arg Val Leu Lys Gln Ile
   325 330 335
   Ser Asn Asn Arg Lys Cys Ser Ser Pro Arg Ser Ser Asp Thr Glu Glu
   340 345 350
   Asn Asp Lys Arg Arg Thr His Asn Val Leu Glu Arg Gln Arg Arg Asn
   355 360 365
   Glu Leu Lys Arg Ser Phe Phe Ala Leu Arg Asp Gln Ile Pro Glu Leu
   370 375 380
   Glu Asn Asn Glu Lys Ala Pro Lys Val Asp Ile Leu Lys Lys Ala Thr
   385 390 395 400
   Ala Tyr Ile Leu Ser Ile Gln Ala Asp Glu His Lys Leu Thr Ser Glu
   405 410 415
   Lys Asp Leu Leu Arg Lys Arg Arg Glu Gln Leu Lys His Lys Leu Glu
   420 425 430
   Gln Leu Arg Asn Ser Gly Ala
   435
<210> 177
   <211> 1320
   <212> DNA
   <213> Mouse
<220>
   <221> CDS
   <222> (1).. (1320)
   <223>
<400> 177
   atg ccc ctc aac gtg aac ttc acc aac agg aac tat gac ctc gac tac 48
   Met Pro Leu Asn Val Asn Phe Thr Asn Arg Asn Tyr Asp Leu Asp Tyr
   1 5 10 15
   gac tcc gta cag ccc tat ttc atc tgc gac gag gaa gag aat ttc tat 96
   Asp Ser Val Gln Pro Tyr Phe Ile Cys Asp Glu Glu Glu Asn Phe Tyr
   20 25 30
   cac cag caa cag cag agc gag ctg cag ccg ccc gcg ccc agt gag gat 144
   His Gln Gln Gln Gln Ser Glu Leu Gln Pro Pro Ala Pro Ser Glu Asp
   35 40 45
   atc tgg aag aaa ttc gag ctg ctt ccc acc ccg ccc ctg tcc ccg agc 192
   Ile Trp Lys Lys Phe Glu Leu Leu Pro Thr Pro Pro Leu Ser Pro Ser
   50 55 60
   cgc cgc tcc ggg ctc tgc tct cca tcc tat gtt gcg gtc gct acg tcc 240
   Arg Arg Ser Gly Leu Cys Ser Pro Ser Tyr Val Ala Val Ala Thr Ser
   65 70 75 80
   ttc tcc cca agg gaa gac gat gac ggc ggc ggt ggc aac ttc tcc acc 288
   Phe Ser Pro Arg Glu Asp Asp Asp Gly Gly Gly Gly Asn Phe Ser Thr
   85 90 95
   gcc gat cag ctg gag atg atg acc gag tta ctt gga gga gac atg gtg 336
   Ala Asp Gln Leu Glu Met Met Thr Glu Leu Leu Gly Gly Asp Met Val
   100 105 110
   aac cag agc ttc atc tgc gat cct gac gac gag acc ttc atc aag aac 384
   Asn Gln Ser Phe Ile Cys Asp Pro Asp Asp Glu Thr Phe Ile Lys Asn
   115 120 125
   atc atc atc cag gac tgt atg tgg agc ggt ttc tca gcc gct gcc aag 432
   Ile Ile Ile Gln Asp Cys Met Trp Ser Gly Phe Ser Ala Ala Ala Lys
   130 135 140
   ctg gtc tcg gag aag ctg gcc tcc tac cag gct gcg cgc aaa gac agc 480
   Leu Val Ser Glu Lys Leu Ala Ser Tyr Gln Ala Ala Arg Lys Asp Ser
   145 150 155 160
   acc agc ctg agc ccc gcc cgc ggg cac agc gtc tgc tcc acc tcc agc 528
   Thr Ser Leu Ser Pro Ala Arg Gly His Ser Val Cys Ser Thr Ser Ser
   165 170 175
   ctg tac ctg cag gac ctc acc gcc gcc gcg tcc gag tgc att gac ccc 576
   Leu Tyr Leu Gln Asp Leu Thr Ala Ala Ala Ser Glu Cys Ile Asp Pro
   180 185 190
   tca gtg gtc ttt ccc tac ccg ctc aac gac agc agc tcg ccc aaa tcc 624
   Ser Val Val Phe Pro Tyr Pro Leu Asn Asp Ser Ser Ser Pro Lys Ser
   195 200 205
   tgt acc tcg tcc gat tcc acg gcc ttc tct cct tcc tcg gac tcg ctg 672
   Cys Thr Ser Ser Asp Ser Thr Ala Phe Ser Pro Ser Ser Asp Ser Leu
   210 215 220
   ctg tcc tcc gag tcc tcc cca cgg gcc agc cct gag ccc cta gtg ctg 720
   Leu Ser Ser Glu Ser Ser Pro Arg Ala Ser Pro Glu Pro Leu Val Leu
   225 230 235 240
   cat gag gag aca ccg ccc acc acc agc agc gac tct gaa gaa gag caa 768
   His Glu Glu Thr Pro Pro Thr Thr Ser Ser Asp Ser Glu Glu Glu Gln
   245 250 255
   gaa gat gag gaa gaa att gat gtg gtg tct gtg gag aag agg caa acc 816
   Glu Asp Glu Glu Glu Ile Asp Val Val Ser Val Glu Lys Arg Gln Thr
   260 265 270
   cct gcc aag agg tcg gag tcg ggc tca tct cca tcc cga ggc cac agc 864
   Pro Ala Lys Arg Ser Glu Ser Gly Ser Ser Pro Ser Arg Gly His Ser
   275 280 285
   aaa cct ccg cac agc cca ctg gtc ctc aag agg tgc cac gtc tcc act 912
   Lys Pro Pro His Ser Pro Leu Val Leu Lys Arg Cys His Val Ser Thr
   290 295 300
   cac cag cac aac tac gcc gca ccc ccc tcc aca agg aag gac tat cca 960
   His Gln His Asn Tyr Ala Ala Pro Pro Ser Thr Arg Lys Asp Tyr Pro
   305 310 315 320
   gct gcc aag agg gcc aag ttg gac agt ggc agg gtc ctg aag cag atc 1008
   Ala Ala Lys Arg Ala Lys Leu Asp Ser Gly Arg Val Leu Lys Gln Ile
   325 330 335
   agc aac aac cgc aag tgc tcc agc ccc agg tcc tca gac acg gag gaa 1056
   Ser Asn Asn Arg Lys Cys Ser Ser Pro Arg Ser Ser Asp Thr Glu Glu
   340 345 350
   aac gac aag agg cgg aca cac aac gtc ttg gaa cgt cag agg agg aac 1104
   Asn Asp Lys Arg Arg Thr His Asn Val Leu Glu Arg Gln Arg Arg Asn
   355 360 365
   gag ctg aag cgc agc ttt ttt gcc ctg cgt gac cag atc cct gaa ttg 1152
   Glu Leu Lys Arg Ser Phe Phe Ala Leu Arg Asp Gln Ile Pro Glu Leu
   370 375 380
   gaa aac aac gaa aag gcc ccc aag gta gtg atc ctc aaa aaa gcc acc 1200
   Glu Asn Asn Glu Lys Ala Pro Lys Val Val Ile Leu Lys Lys Ala Thr
   385 390 395 400
   gcc tac atc ctg tcc att caa gca gac gag cac aag ctc acc tct gaa 1248
   Ala Tyr Ile Leu Ser Ile Gln Ala Asp Glu His Lys Leu Thr Ser Glu
   405 410 415
   aag gac tta ccg agg aaa cga cga gaa cag ttg aaa cac aaa ctc gaa 1296
   Lys Asp Leu Pro Arg Lys Arg Arg Glu Gln Leu Lys His Lys Leu Glu
   420 425 430
   cag ctt cga aac tct ggt gca taa 1320
   Gln Leu Arg Asn Ser Gly Ala
   435
<210> 178
   <211> 439
   <212> PRT
   <213> Mouse
<400> 178
   Met Pro Leu Asn Val Asn Phe Thr Asn Arg Asn Tyr Asp Leu Asp Tyr
   1 5 10 15
   Asp Ser Val Gln Pro Tyr Phe Ile Cys Asp Glu Glu Glu Asn Phe Tyr
   20 25 30
   His Gln Gln Gln Gln Ser Glu Leu Gln Pro Pro Ala Pro Ser Glu Asp
   35 40 45
   Ile Trp Lys Lys Phe Glu Leu Leu Pro Thr Pro Pro Leu Ser Pro Ser
   50 55 60
   Arg Arg Ser Gly Leu Cys Ser Pro Ser Tyr Val Ala Val Ala Thr Ser
   65 70 75 80
   Phe Ser Pro Arg Glu Asp Asp Asp Gly Gly Gly Gly Asn Phe Ser Thr
   85 90 95
   Ala Asp Gln Leu Glu Met Met Thr Glu Leu Leu Gly Gly Asp Met Val
   100 105 110
   Asn Gln Ser Phe Ile Cys Asp Pro Asp Asp Glu Thr Phe Ile Lys Asn
   115 120 125
   Ile Ile Ile Gln Asp Cys Met Trp Ser Gly Phe Ser Ala Ala Ala Lys
   130 135 140
   Leu Val Ser Glu Lys Leu Ala Ser Tyr Gln Ala Ala Arg Lys Asp Ser
   145 150 155 160
   Thr Ser Leu Ser Pro Ala Arg Gly His Ser Val Cys Ser Thr Ser Ser
   165 170 175
   Leu Tyr Leu Gln Asp Leu Thr Ala Ala Ala Ser Glu Cys Ile Asp Pro
   180 185 190
   Ser Val Val Phe Pro Tyr Pro Leu Asn Asp Ser Ser Ser Pro Lys Ser
   195 200 205
   Cys Thr Ser Ser Asp Ser Thr Ala Phe Ser Pro Ser Ser Asp Ser Leu
   210 215 220
   Leu Ser Ser Glu Ser Ser Pro Arg Ala Ser Pro Glu Pro Leu Val Leu
   225 230 235 240
   His Glu Glu Thr Pro Pro Thr Thr Ser Ser Asp Ser Glu Glu Glu Gln
   245 250 255
   Glu Asp Glu Glu Glu Ile Asp Val Val Ser Val Glu Lys Arg Gln Thr
   260 265 270
   Pro Ala Lys Arg Ser Glu Ser Gly Ser Ser Pro Ser Arg Gly His Ser
   275 280 285
   Lys Pro Pro His Ser Pro Leu Val Leu Lys Arg Cys His Val Ser Thr
   290 295 300
   His Gln His Asn Tyr Ala Ala Pro Pro Ser Thr Arg Lys Asp Tyr Pro
   305 310 315 320
   Ala Ala Lys Arg Ala Lys Leu Asp Ser Gly Arg Val Leu Lys Gln Ile
   325 330 335
   Ser Asn Asn Arg Lys Cys Ser Ser Pro Arg Ser Ser Asp Thr Glu Glu
   340 345 350
   Asn Asp Lys Arg Arg Thr His Asn Val Leu Glu Arg Gln Arg Arg Asn
   355 360 365
   Glu Leu Lys Arg Ser Phe Phe Ala Leu Arg Asp Gln Ile Pro Glu Leu
   370 375 380
   Glu Asn Asn Glu Lys Ala Pro Lys Val Val Ile Leu Lys Lys Ala Thr
   385 390 395 400
   Ala Tyr Ile Leu Ser Ile Gln Ala Asp Glu His Lys Leu Thr Ser Glu
   405 410 415
   Lys Asp Leu Pro Arg Lys Arg Arg Glu Gln Leu Lys His Lys Leu Glu
   420 425 430
   Gln Leu Arg Asn Ser Gly Ala
   435
<210> 179
   <211> 1107
   <212> DNA
   <213> Mouse
<220>
   <221> CDS
   <222> (1).. (1107)
   <223>
<400> 179
   atg gac ttc gac tcg tat cag cac tat ttc tac gac tat gac tgc gga 48
   Met Asp Phe Asp Ser Tyr Gln His Tyr Phe Tyr Asp Tyr Asp Cys Gly
   1 5 10 15
   gag gat ttc tac cgc tcc acg gcg ccc agc gag gac atc tgg aag aaa 96
   Glu Asp Phe Tyr Arg Ser Thr Ala Pro Ser Glu Asp Ile Trp Lys Lys
   20 25 30
   ttc gag ctg gtg ccg tcg ccc ccc acg tcg ccg ccc tgg ggc tcc ggt 144
   Phe Glu Leu Val Pro Ser Pro Pro Thr Ser Pro Pro Trp Gly Ser Gly
   35 40 45
   ccc ggc gcc gtg gac cca gcc tct ggg att aat ccc ggg gag ccg tgg 192
   Pro Gly Ala Val Asp Pro Ala Ser Gly Ile Asn Pro Gly Glu Pro Trp
   50 55 60
   cct gga ggg ggt gcc ggg gac gag gcg gaa tct cgg ggc cat tcg aaa 240
   Pro Gly Gly Gly Ala Gly Asp Glu Ala Glu Ser Arg Gly His Ser Lys
   65                  70                  75                  80
   gcc tgg ggc agg aat tat gct tcc atc att cgc cgt gac tgc atg gag        288
   Ala Trp Gly Arg Asn Tyr Ala Ser Ile Ile Arg Arg Asp Cys Met Glu
                   85                  90                  95
   agc ggc ttc tcc gcc cga gaa cgg ctg gag aga gtg gtg agc gac agg        336
   Ser Gly Phe Ser Ala Arg Glu Arg Leu Glu Arg Val Val Ser Asp Arg
               100                 105                 110
   ctg gcc cca ggc gcg ccc cgg ggg aac ccg ccc aaa gcg ccc gct acc        384
   Leu Ala Pro Gly Ala Pro Arg Gly Asn Pro Pro Lys Ala Pro Ala Thr
           115                 120                 125
   ccg gac ggc act cct agt ctg gaa gcc agt aac ccg gcg ccc gcc acc        432
   Pro Asp Gly Thr Pro Ser Leu Glu Ala Ser Asn Pro Ala Pro Ala Thr
       130                 135                 140
   caa tgt cag ctg ggc gag ccc aag act cag gcc tgc tcc ggg tcc gag        480
   Gln Cys Gln Leu Gly Glu Pro Lys Thr Gln Ala Cys Ser Gly Ser Glu
   145                 150                 155                 160
   agc ccc agc gat tct gaa ggt gaa gag att gac gtg gtg acc gtg gag        528
   Ser Pro Ser Asp Ser Glu Gly Glu Glu Ile Asp Val Val Thr Val Glu
                   165                 170                 175
   aag agg cga tct ctg gac atc cga aag cca gtc acc atc acg gtg cga        576
   Lys Arg Arg Ser Leu Asp Ile Arg Lys Pro Val Thr Ile Thr Val Arg
               180                 185                 190
   gca gac ccc ctg gac ccc tgc atg aag cac ttc cat atc tct atc cac        624
   Ala Asp Pro Leu Asp Pro Cys Met Lys His Phe His Ile Ser Ile His
           195                 200                 205
   caa cag cag cat aac tat gct gcc cgt ttt cct cca gaa agt tgc tct        672
   Gln Gln Gln His Asn Tyr Ala Ala Arg Phe Pro Pro Glu Ser Cys Ser
       210                 215                 220
   caa gag ggg gat cct gag cca ggt ccc cag gaa gag gct ccg gag ata        720
   Gln Glu Gly Asp Pro Glu Pro Gly Pro Gln Glu Glu Ala Pro Glu Ile
   225                 230                 235                 240
   gaa gct ccc aag gag aaa gag gag gag gaa gag gaa gag gag gaa gaa        768
   Glu Ala Pro Lys Glu Lys Glu Glu Glu Glu Glu Glu Glu Glu Glu Glu
                   245                 250                 255
   gag att gtg agc ccc cca cct gtc gga agt gag gct ccc cag tcc tgc        816
   Glu Ile Val Ser Pro Pro Pro Val Gly Ser Glu Ala Pro Gln Ser Cys
               260                 265                 270
   cac ccc aaa cct gtc agt tct gac act gag gac gtg acc aag agg aag        864
   His Pro Lys Pro Val Ser Ser Asp Thr Glu Asp Val Thr Lys Arg Lys
   275 280 285
   aac cat aac ttc ttg gaa cga aaa agg agg aat gac ctc cgc tcc cgg 912
   Asn His Asn Phe Leu Glu Arg Lys Arg Arg Asn Asp Leu Arg Ser Arg
   290 295 300
   ttc cta gcc ctg cgg gac cag gtt ccc acc ctg gcc agc tgc tct aag 960
   Phe Leu Ala Leu Arg Asp Gln Val Pro Thr Leu Ala Ser Cys Ser Lys
   305 310 315 320
   gcc ccc aaa gtc gtg atc ctc agc aag gcg tta gaa tac ttg cag gct 1008
   Ala Pro Lys Val Val Ile Leu Ser Lys Ala Leu Glu Tyr Leu Gln Ala
   325 330 335
   ttg gtg ggg gct gaa aag aaa atg gct aca gag aaa agg cag ctc cgg 1056
   Leu Val Gly Ala Glu Lys Lys Met Ala Thr Glu Lys Arg Gln Leu Arg
   340 345 350
   tgt cgg caa cag caa ctg caa aag aga atc gcg tac ctc agt ggc tac 1104
   Cys Arg Gln Gln Gln Leu Gln Lys Arg Ile Ala Tyr Leu Ser Gly Tyr
   355 360 365
   taa 1107
<210> 180
   <211> 368
   <212> PRT
   <213> Mouse
<400> 180
   Met Asp Phe Asp Ser Tyr Gln His Tyr Phe Tyr Asp Tyr Asp Cys Gly
   1 5 10 15
   Glu Asp Phe Tyr Arg Ser Thr Ala Pro Ser Glu Asp Ile Trp Lys Lys
   20 25 30
   Phe Glu Leu Val Pro Ser Pro Pro Thr Ser Pro Pro Trp Gly Ser Gly
   35 40 45
   Pro Gly Ala Val Asp Pro Ala Ser Gly Ile Asn Pro Gly Glu Pro Trp
   50 55 60
   Pro Gly Gly Gly Ala Gly Asp Glu Ala Glu Ser Arg Gly His Ser Lys
   65 70 75 80
   Ala Trp Gly Arg Asn Tyr Ala Ser Ile Ile Arg Arg Asp Cys Met Glu
   85 90 95
   Ser Gly Phe Ser Ala Arg Glu Arg Leu Glu Arg Val Val Ser Asp Arg
   100 105 110
   Leu Ala Pro Gly Ala Pro Arg Gly Asn Pro Pro Lys Ala Pro Ala Thr
   115 120 125
   Pro Asp Gly Thr Pro Ser Leu Glu Ala Ser Asn Pro Ala Pro Ala Thr
   130 135 140
   Gln Cys Gln Leu Gly Glu Pro Lys Thr Gln Ala Cys Ser Gly Ser Glu
   145 150 155 160
   Ser Pro Ser Asp Ser Glu Gly Glu Glu Ile Asp Val Val Thr Val Glu
   165 170 175
   Lys Arg Arg Ser Leu Asp Ile Arg Lys Pro Val Thr Ile Thr Val Arg
   180 185 190
   Ala Asp Pro Leu Asp Pro Cys Met Lys His Phe His Ile Ser Ile His
   195 200 205
   Gln Gln Gln His Asn Tyr Ala Ala Arg Phe Pro Pro Glu Ser Cys Ser
   210 215 220
   Gln Glu Gly Asp Pro Glu Pro Gly Pro Gln Glu Glu Ala Pro Glu Ile
   225 230 235 240
   Glu Ala Pro Lys Glu Lys Glu Glu Glu Glu Glu Glu Glu Glu Glu Glu
   245 250 255
   Glu Ile Val Ser Pro Pro Pro Val Gly Ser Glu Ala Pro Gln Ser Cys
   260 265 270
   His Pro Lys Pro Val Ser Ser Asp Thr Glu Asp Val Thr Lys Arg Lys
   275 280 285
   Asn His Asn Phe Leu Glu Arg Lys Arg Arg Asn Asp Leu Arg Ser Arg
   290 295 300
   Phe Leu Ala Leu Arg Asp Gln Val Pro Thr Leu Ala Ser Cys Ser Lys
   305 310 315 320
   Ala Pro Lys Val Val Ile Leu Ser Lys Ala Leu Glu Tyr Leu Gln Ala
   325 330 335
   Leu Val Gly Ala Glu Lys Lys Met Ala Thr Glu Lys Arg Gln Leu Arg
   340 345 350
   Cys Arg Gln Gln Gln Leu Gln Lys Arg Ile Ala Tyr Leu Ser Gly Tyr
   355 360 365
<210> 181
   <211> 1107
   <212> DNA
   <213> Mouse
<220>
   <221> CDS
   <222> (1).. (1107)
   <223>
<400> 181
   atg gac ttc gac tcg tat cag cac tat ttc tac gac tat gac tgc gga       48
   Met Asp Phe Asp Ser Tyr Gln His Tyr Phe Tyr Asp Tyr Asp Cys Gly
   1               5                   10                  15
   gag gat ttc tac cgc tcc acg gcg ccc agc gag gac atc tgg aag aaa       96
   Glu Asp Phe Tyr Arg Ser Thr Ala Pro Ser Glu Asp Ile Trp Lys Lys
               20                  25                  30
   ttc gag ctg gtg ccg tcg ccc ccc acg tcg ccg ccc tgg ggc tcc ggt      144
   Phe Glu Leu Val Pro Ser Pro Pro Thr Ser Pro Pro Trp Gly Ser Gly
           35                  40                  45
   ccc ggc gcc gtg gac cca gcc tct ggg att aat ccc ggg gag ccg tgg      192
   Pro Gly Ala Val Asp Pro Ala Ser Gly Ile Asn Pro Gly Glu Pro Trp
       50                  55                  60
   cct gga ggg ggt gcc ggg gac gag gcg gaa tct cgg ggc cat tcg aaa      240
   Pro Gly Gly Gly Ala Gly Asp Glu Ala Glu Ser Arg Gly His Ser Lys
   65                  70                  75                  80
   gcc tgg ggc agg aat tat gct tcc atc att cgc cgt gac tgc atg tgg      288
   Ala Trp Gly Arg Asn Tyr Ala Ser Ile Ile Arg Arg Asp Cys Met Trp
                   85                  90                  95
   agc ggc ttc tcc gcc cga gaa cgg ctg gag aga gtg gtg agc gac agg      336
   Ser Gly Phe Ser Ala Arg Glu Arg Leu Glu Arg Val Val Ser Asp Arg
               100                 105                 110
   ctg gcc cca ggc gcg ccc cgg ggg aac ccg ccc aaa gcg ccc gct acc      384
   Leu Ala Pro Gly Ala Pro Arg Gly Asn Pro Pro Lys Ala Pro Ala Thr
           115                 120                 125
   ccg gac ggc act cct agt ctg gaa gcc agt aac ccg gcg ccc gcc acc      432
   Pro Asp Gly Thr Pro Ser Leu Glu Ala Ser Asn Pro Ala Pro Ala Thr
       130                 135                 140
   caa tgt cag ctg ggc gag ccc aag act cag gcc tgc tcc ggg tcc gag      480
   Gln Cys Gln Leu Gly Glu Pro Lys Thr Gln Ala Cys Ser Gly Ser Glu
   145                 150                 155                 160
   agc ccc agc gat tct gaa ggt gaa gag att gac gtg gtg acc gtg gag      528
   Ser Pro Ser Asp Ser Glu Gly Glu Glu Ile Asp Val Val Thr Val Glu
                   165                 170                 175
   aag agg cga tct ctg gac atc cga aag cca gtc acc atc acg gtg cga      576
   Lys Arg Arg Ser Leu Asp Ile Arg Lys Pro Val Thr Ile Thr Val Arg
               180                 185                 190
   gca gac ccc ctg gac ccc tgc atg aag cac ttc cat atc tct atc cac      624
   Ala Asp Pro Leu Asp Pro Cys Met Lys His Phe His Ile Ser Ile His
   195 200 205
   caa cag cag cat aac tat gct gcc cgt ttt cct cca gaa agt tgc tct 672
   Gln Gln Gln His Asn Tyr Ala Ala Arg Phe Pro Pro Glu Ser Cys Ser
   210 215 220
   caa gag ggg gat cct gag cca ggt ccc cag gaa gag gct ccg gag ata 720
   Gln Glu Gly Asp Pro Glu Pro Gly Pro Gln Glu Glu Ala Pro Glu Ile
   225 230 235 240
   gaa gct ccc aag gag aaa gag gag gag gaa gag gaa gag gag gaa gaa 768
   Glu Ala Pro Lys Glu Lys Glu Glu Glu Glu Glu Glu Glu Glu Glu Glu
   245 250 255
   gag att gtg agc ccc cca cct gtc gga agt gag gct ccc cag tcc tgc 816
   Glu Ile Val Ser Pro Pro Pro Val Gly Ser Glu Ala Pro Gln Ser Cys
   260 265 270
   cac ccc aaa cct gtc agt tct gac act gag gac gtg acc aag agg aag 864
   His Pro Lys Pro Val Ser Ser Asp Thr Glu Asp Val Thr Lys Arg Lys
   275 280 285
   aac cat aac ttc ttg gaa cga aaa agg agg aat gac ctc cgc tcc cgg 912
   Asn His Asn Phe Leu Glu Arg Lys Arg Arg Asn Asp Leu Arg Ser Arg
   290 295 300
   ttc cta gcc ctg cgg gac cag gtt ccc acc ctg gcc agc tgc tct aag 960
   Phe Leu Ala Leu Arg Asp Gln Val Pro Thr Leu Ala Ser Cys Ser Lys
   305 310 315 320
   gcc ccc aaa gtc gac atc ctc agc aag gcg tta gaa tac ttg cag gct 1008
   Ala Pro Lys Val Asp Ile Leu Ser Lys Ala Leu Glu Tyr Leu Gln Ala
   325 330 335
   ttg gtg ggg gct gaa aag aaa atg gct aca gag aaa agg cag ctc cgg 1056
   Leu Val Gly Ala Glu Lys Lys Met Ala Thr Glu Lys Arg Gln Leu Arg
   340 345 350
   tgt cgg caa cag caa ctg caa aag aga atc gcg tac ctc agt ggc tac 1104
   Cys Arg Gln Gln Gln Leu Gln Lys Arg Ile Ala Tyr Leu Ser Gly Tyr
   355 360 365
   taa 1107
<210> 182
   <211> 368
   <212> PRT
   <213> Mouse
<400> 182
   Met Asp Phe Asp Ser Tyr Gln His Tyr Phe Tyr Asp Tyr Asp Cys Gly
   1               5                   10                  15
   Glu Asp Phe Tyr Arg Ser Thr Ala Pro Ser Glu Asp Ile Trp Lys Lys
               20                  25                  30
   Phe Glu Leu Val Pro Ser Pro Pro Thr Ser Pro Pro Trp Gly Ser Gly
           35                  40                  45
   Pro Gly Ala Val Asp Pro Ala Ser Gly Ile Asn Pro Gly Glu Pro Trp
       50                  55                  60
   Pro Gly Gly Gly Ala Gly Asp Glu Ala Glu Ser Arg Gly His Ser Lys
   65                  70                  75                  80
   Ala Trp Gly Arg Asn Tyr Ala Ser Ile Ile Arg Arg Asp Cys Met Trp
                   85                  90                  95
   Ser Gly Phe Ser Ala Arg Glu Arg Leu Glu Arg Val Val Ser Asp Arg
               100                 105                 110
   Leu Ala Pro Gly Ala Pro Arg Gly Asn Pro Pro Lys Ala Pro Ala Thr
           115                 120                 125
   Pro Asp Gly Thr Pro Ser Leu Glu Ala Ser Asn Pro Ala Pro Ala Thr
       130                 135                 140
   Gln Cys Gln Leu Gly Glu Pro Lys Thr Gln Ala Cys Ser Gly Ser Glu
   145                 150                 155                 160
   Ser Pro Ser Asp Ser Glu Gly Glu Glu Ile Asp Val Val Thr Val Glu
                   165                 170                 175
   Lys Arg Arg Ser Leu Asp Ile Arg Lys Pro Val Thr Ile Thr Val Arg
               180                 185                 190
   Ala Asp Pro Leu Asp Pro Cys Met Lys His Phe His Ile Ser Ile His
           195                 200                 205
   Gln Gln Gln His Asn Tyr Ala Ala Arg Phe Pro Pro Glu Ser Cys Ser
       210                 215                 220
   Gln Glu Gly Asp Pro Glu Pro Gly Pro Gln Glu Glu Ala Pro Glu Ile
   225                 230                 235                 240
   Glu Ala Pro Lys Glu Lys Glu Glu Glu Glu Glu Glu Glu Glu Glu Glu
                   245                 250                 255
   Glu Ile Val Ser Pro Pro Pro Val Gly Ser Glu Ala Pro Gln Ser Cys
               260                 265                 270
   His Pro Lys Pro Val Ser Ser Asp Thr Glu Asp Val Thr Lys Arg Lys
           275                 280                 285
   Asn His Asn Phe Leu Glu Arg Lys Arg Arg Asn Asp Leu Arg Ser Arg
       290                 295                 300
   Phe Leu Ala Leu Arg Asp Gln Val Pro Thr Leu Ala Ser Cys Ser Lys
   305                 310                 315                 320
   Ala Pro Lys Val Asp Ile Leu Ser Lys Ala Leu Glu Tyr Leu Gln Ala
   325 330 335
   Leu Val Gly Ala Glu Lys Lys Met Ala Thr Glu Lys Arg Gln Leu Arg
   340 345 350
   Cys Arg Gln Gln Gln Leu Gln Lys Arg Ile Ala Tyr Leu Ser Gly Tyr
   355 360 365
<210> 183
   <211> 1107
   <212> DNA
   <213> Mouse
<220>
   <221> CDS
   <222> (1).. (1107)
   <223>
<400> 183
   atg gac ttc gac tcg tat cag cac tat ttc tac gac tat gac tgc gga 48
   Met Asp Phe Asp Ser Tyr Gln His Tyr Phe Tyr Asp Tyr Asp Cys Gly
   1 5 10 15
   gag gat ttc tac cgc tcc acg gcg ccc agc gag gac atc tgg aag aaa 96
   Glu Asp Phe Tyr Arg Ser Thr Ala Pro Ser Glu Asp Ile Trp Lys Lys
   20 25 30
   ttc gag ctg gtg ccg tcg ccc ccc acg tcg ccg ccc tgg ggc tcc ggt 144
   Phe Glu Leu Val Pro Ser Pro Pro Thr Ser Pro Pro Trp Gly Ser Gly
   35 40 45
   ccc ggc gcc gtg gac cca gcc tct ggg att aat ccc ggg gag ccg tgg 192
   Pro Gly Ala Val Asp Pro Ala Ser Gly Ile Asn Pro Gly Glu Pro Trp
   50 55 60
   cct gga ggg ggt gcc ggg gac gag gcg gaa tct cgg ggc cat tcg aaa 240
   Pro Gly Gly Gly Ala Gly Asp Glu Ala Glu Ser Arg Gly His Ser Lys
   65 70 75 80
   gcc tgg ggc agg aat tat gct tcc atc att cgc cgt gac tgc atg tgg 288
   Ala Trp Gly Arg Asn Tyr Ala Ser Ile Ile Arg Arg Asp Cys Met Trp
   85 90 95
   agc ggc ttc tcc gcc cga gaa cgg ctg gag aga gtg gtg agc gac agg 336
   Ser Gly Phe Ser Ala Arg Glu Arg Leu Glu Arg Val Val Ser Asp Arg
   100 105 110
   ctg gcc cca ggc gcg ccc cgg ggg aac ccg ccc aaa gcg ccc gct acc 384
   Leu Ala Pro Gly Ala Pro Arg Gly Asn Pro Pro Lys Ala Pro Ala Thr
   115 120 125
   ccg gac ggc act cct agt ctg gaa gcc agt aac ccg gcg ccc gcc acc 432
   Pro Asp Gly Thr Pro Ser Leu Glu Ala Ser Asn Pro Ala Pro Ala Thr
   130 135 140
   caa tgt cag ctg ggc gag ccc aag act cag gcc tgc tcc ggg tcc gag 480
   Gln Cys Gln Leu Gly Glu Pro Lys Thr Gln Ala Cys Ser Gly Ser Glu
   145 150 155 160
   agc ccc agc gat tct gaa ggt gaa gag att gac gtg gtg acc gtg gag 528
   Ser Pro Ser Asp Ser Glu Gly Glu Glu Ile Asp Val Val Thr Val Glu
   165 170 175
   aag agg cga tct ctg gac atc cga aag cca gtc acc atc acg gtg cga 576
   Lys Arg Arg Ser Leu Asp Ile Arg Lys Pro Val Thr Ile Thr Val Arg
   180 185 190
   gca gac ccc ctg gac ccc tgc atg aag cac ttc cat atc tct atc cac 624
   Ala Asp Pro Leu Asp Pro Cys Met Lys His Phe His Ile Ser Ile His
   195 200 205
   caa cag cag cat aac tat gct gcc cgt ttt cct cca gaa agt tgc tct 672
   Gln Gln Gln His Asn Tyr Ala Ala Arg Phe Pro Pro Glu Ser Cys Ser
   210 215 220
   caa gag ggg gat cct gag cca ggt ccc cag gaa gag gct ccg gag ata 720
   Gln Glu Gly Asp Pro Glu Pro Gly Pro Gln Glu Glu Ala Pro Glu Ile
   225 230 235 240
   gaa gct ccc aag gag aaa gag gag gag gaa gag gaa gag gag gaa gaa 768
   Glu Ala Pro Lys Glu Lys Glu Glu Glu Glu Glu Glu Glu Glu Glu Glu
   245 250 255
   gag att gtg agc ccc cca cct gtc gga agt gag gct ccc cag tcc tgc 816
   Glu Ile Val Ser Pro Pro Pro Val Gly Ser Glu Ala Pro Gln Ser Cys
   260 265 270
   cac ccc aaa cct gtc agt tct gac act gag gac gtg acc aag agg aag 864
   His Pro Lys Pro Val Ser Ser Asp Thr Glu Asp Val Thr Lys Arg Lys
   275 280 285
   aac cat aac ttc ttg gaa cga aaa agg agg aat gac ctc cgc tcc cgg 912
   Asn His Asn Phe Leu Glu Arg Lys Arg Arg Asn Asp Leu Arg Ser Arg
   290 295 300
   ttc cta gcc ctg cgg gac cag gtt ccc acc ctg gcc agc tgc tct aag 960
   Phe Leu Ala Leu Arg Asp Gln Val Pro Thr Leu Ala Ser Cys Ser Lys
   305 310 315 320
   gcc ccc aaa gtc gtg atc ctc agc aag gcg tta gaa tac ttg cag gct 1008
   Ala Pro Lys Val Val Ile Leu Ser Lys Ala Leu Glu Tyr Leu Gln Ala
   325 330 335
   ttg gtg ggg gct gaa aag aaa atg gct aca gag aaa agg cag ccc cgg 1056
   Leu Val Gly Ala Glu Lys Lys Met Ala Thr Glu Lys Arg Gln Pro Arg
   340 345 350
   tgt cgg caa cag caa ctg caa aag aga atc gcg tac ctc agt ggc tac 1104
   Cys Arg Gln Gln Gln Leu Gln Lys Arg Ile Ala Tyr Leu Ser Gly Tyr
   355 360 365
   taa 1107
<210> 184
   <211> 368
   <212> PRT
   <213> Mouse
<400> 184
   Met Asp Phe Asp Ser Tyr Gln His Tyr Phe Tyr Asp Tyr Asp Cys Gly
   1 5 10 15
   Glu Asp Phe Tyr Arg Ser Thr Ala Pro Ser Glu Asp Ile Trp Lys Lys
   20 25 30
   Phe Glu Leu Val Pro Ser Pro Pro Thr Ser Pro Pro Trp Gly Ser Gly
   35 40 45
   Pro Gly Ala Val Asp Pro Ala Ser Gly Ile Asn Pro Gly Glu Pro Trp
   50 55 60
   Pro Gly Gly Gly Ala Gly Asp Glu Ala Glu Ser Arg Gly His Ser Lys
   65 70 75 80
   Ala Trp Gly Arg Asn Tyr Ala Ser Ile Ile Arg Arg Asp Cys Met Trp
   85 90 95
   Ser Gly Phe Ser Ala Arg Glu Arg Leu Glu Arg Val Val Ser Asp Arg
   100 105 110
   Leu Ala Pro Gly Ala Pro Arg Gly Asn Pro Pro Lys Ala Pro Ala Thr
   115 120 125
   Pro Asp Gly Thr Pro Ser Leu Glu Ala Ser Asn Pro Ala Pro Ala Thr
   130 135 140
   Gln Cys Gln Leu Gly Glu Pro Lys Thr Gln Ala Cys Ser Gly Ser Glu
   145 150 155 160
   Ser Pro Ser Asp Ser Glu Gly Glu Glu Ile Asp Val Val Thr Val Glu
   165 170 175
   Lys Arg Arg Ser Leu Asp Ile Arg Lys Pro Val Thr Ile Thr Val Arg
   180 185 190
   Ala Asp Pro Leu Asp Pro Cys Met Lys His Phe His Ile Ser Ile His
           195                 200                 205
   Gln Gln Gln His Asn Tyr Ala Ala Arg Phe Pro Pro Glu Ser Cys Ser
       210                 215                 220
   Gln Glu Gly Asp Pro Glu Pro Gly Pro Gln Glu Glu Ala Pro Glu Ile
   225                 230                 235                 240
   Glu Ala Pro Lys Glu Lys Glu Glu Glu Glu Glu Glu Glu Glu Glu Glu
                   245                 250                 255
   Glu Ile Val Ser Pro Pro Pro Val Gly Ser Glu Ala Pro Gln Ser Cys
               260                 265                 270
   His Pro Lys Pro Val Ser Ser Asp Thr Glu Asp Val Thr Lys Arg Lys
           275                 280                 285
   Asn His Asn Phe Leu Glu Arg Lys Arg Arg Asn Asp Leu Arg Ser Arg
       290                 295                 300
   Phe Leu Ala Leu Arg Asp Gln Val Pro Thr Leu Ala Ser Cys Ser Lys
   305                 310                 315                 320
   Ala Pro Lys Val Val Ile Leu Ser Lys Ala Leu Glu Tyr Leu Gln Ala
                   325                 330                 335
   Leu Val Gly Ala Glu Lys Lys Met Ala Thr Glu Lys Arg Gln Pro Arg
               340                 345                 350
   Cys Arg Gln Gln Gln Leu Gln Lys Arg Ile Ala Tyr Leu Ser Gly Tyr
           355                 360                 365

## Description

### Technical Field

The present invention relates to a process for generating induced pluripotent stem cells by reprogramming differentiated somatic cells.

### Background Art

Embryonic stem cell (ES cell), which are derived from human or mouse early embryos, have the ability to proliferate indefinitely while maintaining pluripotency and the ability to differentiate into all cells existsing in a live body. By using these properties, human ES cells are expected as sources of cell transplantation therapies for many diseases such as Parkinson's disease, juvenile diabetes, and leukemia. However, there is a problem that transplantation of ES cells lead to the tissue rejection after transplantation in patients. In addition, there are many counter opinions regarding the use of human ES cells established by destructing human embryos from an ethical point of view.

If patients' own differentiated somatic cells can be used to induce dedifferentiation and establish cells having the pluripotency and ability to proliferate like the ES cells (in the present specification, this cell is referred to as "artificial pluripotent stem cell" or "iPS cell", and is also referred to as "induced pluripotent stem cell", "embryonic stem cell-like cell", or "ES-like cell"), they will be expected to allow using as an ideal pluripotent cell having neither rejection reaction nor ethical problem. Recently, it has been reported that this iPS cell can be generated from mouse and human somatic cell, and this has evoked an extremely massive response (International Publication WO2007/69666; Cell, 126, pp.663-676, 2006; Cell, 131, pp.861-872, 2007).

The above method comprises the step of reprogramming by introducing a plurality of specified factors (four factors of Oct3/4, Sox2, Klf4, and c-Myc are used as described in Cell, 126, pp.663-676, 2006) into somatic cells, and retrovirus vectors are used for introducing the factors. However, as c-Myc is a cancer-associated transcription factor, when the four factors comprising c-Myc are introduced to somatic cells for reprogramming, a possibility that the cells or tissues differentiation-induced from the obtained induced pluripotent stem cells have tumorigenicity can not be denied. It has been known that genes introduced into ES cells by using retrovirus vectors have silencing function, and it has been confirmed that iPS cells generated by using retrovirus vectors also have silencing function (see Example 7 of International Publication WO2007/69666). However, there is no assurance that the introduced exogenous c-Myc is not expressed in their offspring. A method for nuclear reprogramming with the four factors of Oct3/4, Sox2, Lin28, and Nanog without using c-Myc has been proposed by Thomson et al. (Science, 318, pp.1917-1920, 2007; International Publication WO2008/118820). However, research and development for iPS cells are currently progressing by still using the four factors of Oct3/4, Sox2, Klf4, and c-Myc worldwide.

In addition, the International Publication WO2007/69666 teaches that Myc family gene can be replaced with cytokines, and Examples 5 of the above specification shows that the nuclear reprogramming can be achieved by using basic Fibroblast Growth Factor (bFGF) instead of c-Myc. In addition, the above International Publication shows that 24 genes selected as a candidate of the reprogramming factor include sall4 (Table 4 No.13). However, it does not indicate that Sall4 has the nuclear programming activity. Moreover, it shows that iPS cells can be efficiently established by combining L-Myc or N-Myc, which is one of Myc family gene, with Oct3/4, Sox2, and Klf4 compared to those generated by using c-Myc (Example 6).
Patent Publication 1: International Publication WO2007/69666
Patent Publication 2: International Publication WO2008/118820
Non-Patent Publication 1: Cell, 126, pp.663-676, 2006
Non-Patent Publication 2: Cell, 131, pp.861-872, 2007
Non-Patent Publication 3: Science, 318, pp.1917-1920, 2007

### Disclosure of Invention

### Problems to be Solved by the Invention

A problem of the present invention is to provide a process for generating safe induced pluripotent stem cells. Concretely described, a problem of the present invention is to provide safe induced pluripotent stem cells having no fear of tumorigenesis in the cells or tissues obtained by differentiation-inducing the induced pluripotent stem cells.

In addition, another object of the present invention is to provide a process for effectively generating induced pluripotent stem cells.

A particularly preferably object of the present invention is to provide a process for effectively generating the safe induced pluripotent stem cells.

### Means to Solve the Problems

The present inventors intensively studied in order to solve the aforementioned problems and, as a result, found out that a safe induced pluripotent stem cell can be generated without using c-Myc.

In addition, the present inventors found out that safe induced pluripotent stem cells having no fear of tumorigenesis in the cells or tissues obtained by the induction of differentiation can be provided by performing nuclear reprogramming using L-Myc, Salll, or Sall4 without using c-Myc, and a safe induced pluripotent cells can be extremely effectively generated by performing this process.

The present invention was completed based on the above findings.

That is, according to the present invention, a process for generating induced pluripotent stem cells from somatic cells, comprising the step of introducing the following genes: Oct family gene, Klf family gene, Sox family gene and L-Myc into somatic cells, further comprising the step of introducing Lin28 into somatic cells, is provided.

According to a preferable aspect of this process, the process comprising the step of, after introduction of the following genes: Oct family gene, Klf family gene, Sox family gene, L-Myc and Lin28 into somatic cells, culturing the cells over sufficient time for the cells to acquire pluripotency and proliferate is provided.

According to another preferable aspect of this process, the process comprising the step of introducing the following genes: Oct3/4, Klf4, Sox2 and L-Myc into somatic cells, further comprising the step of introducing Lin28 into somatic cells; and the process comprising the step of, after introduction of the following genes: Oct3/4, Klf4, Sox2, L-Myc and Lin28 into somatic cells, culturing the cells over sufficient time for the cells to acquire pluripotency and proliferate is provided.

In addition, according to further another preferable aspect of this process, the process comprising the step of transfecting virus vectors containing Oct family gene, virus vectors containing Klf family gene, and virus vectors containing Sox family gene into separate packaging cells, respectively, and culturing the cells to obtain three culture supernatants, and the step of preparing a mixture of three culture supernatants obtained by the above step, and reprogramming somatic cells using the mixture is provided.

Further, according to another preferable aspect of this process, the process comprising the step of reprogramming the somatic cells without using drug selection is provided.

In the above disclosed, the gene introduction can be performed optionally in the absence or the presence of cytokines, preferably in the absence of cytokines. In addition, the culturing can be performed optionally in the absence or the presence of cytokines, preferably in the absence of cytokines.

In induced pluripotent stem cells provided by the present disclosure development of tumor is substantially reduced or eliminated in cells or tissues obtained after the induction of differentiation. Therefore, according to the present disclosure, a process for generating induced pluripotent stem cells in which development of tumor is substantially reduced or eliminated in the cells or tissues obtained after the induction of differentiation, comprising the step of introducing the following genes: Oct family gene, Klf family gene, and Sox family gene, preferably Oct3/4 family gene, Klf4 gene and Sox2 gene and L-Myc into somatic cells, further comprising the step of introducing Lin28 into somatic cells, is further provided.

From another point of view, according to the present invention, a process for generating induced pluripotent stem cells from somatic cells, comprising
I)
   a) the step of introducing Lin28 into somatic cells; and
   b) the step of introducing a combination of the following two genes: Oct family gene and Sox family gene, and an L-Myc gene, into somatic cells, or a combination of the following two genes: Oct family gene and Klf family gene, and an L-Myc gene into somatic cells; or
II)
   a) the step of introducing Lin28 into somatic cells; and
   b) the step of introducing a combination of the following three genes: Oct family gene, Sox family gene and Klf gene, and at least one of genes selected from the following two genes:
      Sall1, and into somatic cells is provided.

According to a preferable aspect of the invention, the process comprising the step of introducing a combination of the following two genes: Oct3/4 and Sox2 or a combination of the following two genes: Oct3/4 and Klf4, and at least one kind of genes selected from the following three genes: L-Myc, Salll, and Sall4 into somatic cells, further comprising the step of introducing Lin28 into somatic cells, is provided. A process comprising the step of introducing the following gene: Nanog in addition to the above genes into somatic cells is also a preferable aspect. In the above disclosed process, it is also possible to use c-Myc optionally by appropriate combination.

According to a further preferable aspect of the disclosure, the process comprising the step of introducing a combination of the following three genes: Oct family gene, Sox family gene, and Klf gene, preferably a combination of Oct3/4, Sox2, and Klf4, and at least one kind of genes selected from the following three genes: L-Myc, Salll, and Sall4 into somatic cells is provided.

More concretely, the process comprising the step of introducing the following four genes: Oct3/4, Sox2, Klf4, and Salll into somatic cells; the process comprising the step of introducing the following four genes: Oct3/4, Sox2, Klf4, and Sall4 into somatic cells, the process comprising the step of introducing the following four genes: Oct3/4, Sox2, Klf4, and L-Myc into somatic cells; the process comprising the step of introducing the following five genes: Oct3/4, Sox2, Klf4, Salll, and L-Myc into somatic cells; the process comprising the step of introducing the following five genes: Oct3/4, Sox2, Klf4, Sall4, and L-Myc into somatic cells; the process comprising the step of introducing the following five genes: Oct3/4, Sox2, Klf4, Salll and Sall4; and the process comprising the step of introducing the following six genes: Oct3/4, Sox2, Klf4, Salll, Sall4, and L-Myc into somatic cells are provided.

If L-Myc is used, preferably the somatic cell is a human somatic cell.

In any of the above processes, a process comprising the step of further introducing Lin28 into somatic cells is also provided according to the present invention and, as a preferable aspect, the process comprising the step of introducing Oct3/4, Sox2, Klf4, L-Myc, and Lin28 into somatic cells is provided.

In an induced pluripotent stem cell provided according to the present disclosure development of tumor is substantially reduced or eliminated in the cells or tissues obtained after the induction of differentiation. Therefore, further, according to the present disclosure a process for generating induced pluripotent stem cells in which development of tumor is substantially reduced or eliminated in cells or tissues obtained after the induction of differentiation, comprising the step of introducing the following two genes: Oct family gene and Sox family gene, preferably Oct3/4 family gene and Sox2 gene, and at least one kind of genes selected from the following three genes: L-Myc, Salll and into somatic cells, further comprising the step of introducing Lin28 into somatic cells, is provided. A process comprising the step of further introducing Klf family gene, for example, Klf4 into somatic cells in this process is also a preferable aspect, and a process comprising the step of introducing Lin28 together with Klf family gene or instead of Klf family gene into somatic cells is also a preferable aspect.

Further, from another point of view, according to the present disclosure, a process for generating induced pluripotent stem cells from somatic cells, comprising the step of introducing the following six genes: Oct family gene, Klf family gene, Sox family gene, Myc family gene, Lin28 and Nanog, preferably the following six genes: Oct3/4, Klf4, Sox2, c-Myc, Lin28 and Nanog into somatic cells is provided. In this process, at least one kind of genes selected from the following three genes: L-Myc, Salll and can be used instead of c-Myc, and this process is also a preferable aspect.

In each of the above processes, introduction of the genes into somatic cells can be performed with a recombinant vector, and preferably with a recombinant vector including at least one kind selected from a combination of genes as defined in each of the processes, which the genes are introduced into somatic cells. As the vector, any of a virus vector and a non-virus vector may be used.

Further, the nuclear programming factors comprising a combination of genes as defined in each of the above inventions are provided according to the present disclosure. It is also preferable to further combine TERT gene and/or SV40 Large T antigen gene with the aforementioned combination.

In addition, the nuclear reprogramming factors comprising a combination of gene products of genes as defined in each of the above inventions are also provided according to the present disclosure, and a process comprising the step of contacting the nuclear reprogramming factors comprising a combination of gene products of genes as defined in each of the above inventions with somatic cells is also provided according to the present disclosure. As a preferable aspect, the process comprising the step of adding the nuclear reprogramming factor to a culture of somatic cells is also provided.

In each of the above inventions, the process in which the somatic cell is somatic cell of the mammal comprising human is provided. The process disclosed herein, in which the somatic cell is preferably primate somatic cell, more preferably human somatic cell; the process in which the somatic cell is human fetal cell or somatic cells derived from adult human; the process in which the somatic cell is somatic cells collected from a patient are provided.

From another point of view, an induced pluripotent stem cell obtainable by the aforementioned processes is provided according to the present invention. In addition, somatic cell which is differentiation-induced from an induced pluripotent stem cell obtainable by the above processes is also provided according to the present invention. A tissue, an organ, a body fluid, and an individual which is a population of a differentiation-induced somatic cell are also provided according to the present invention.

Further, a stem cell therapy comprising the step of differentiation-inducing an induced pluripotent stem cell obtained according to the above process by using somatic cells recovered and collected from a patient, and transplanting the resulting somatic cell or a tissue, an organ, or a body liquid which is a population of the cell, into the patient is provided according to the present disclosure.

Further, a method for assaying the physiological activity or toxicity of a compound, a drug, or a toxin by using various cells obtained from differentiation-inducing induced pluripotent stem cell obtained according to the above processes is provided according to the present disclosure.

### Brief Description of Drawings

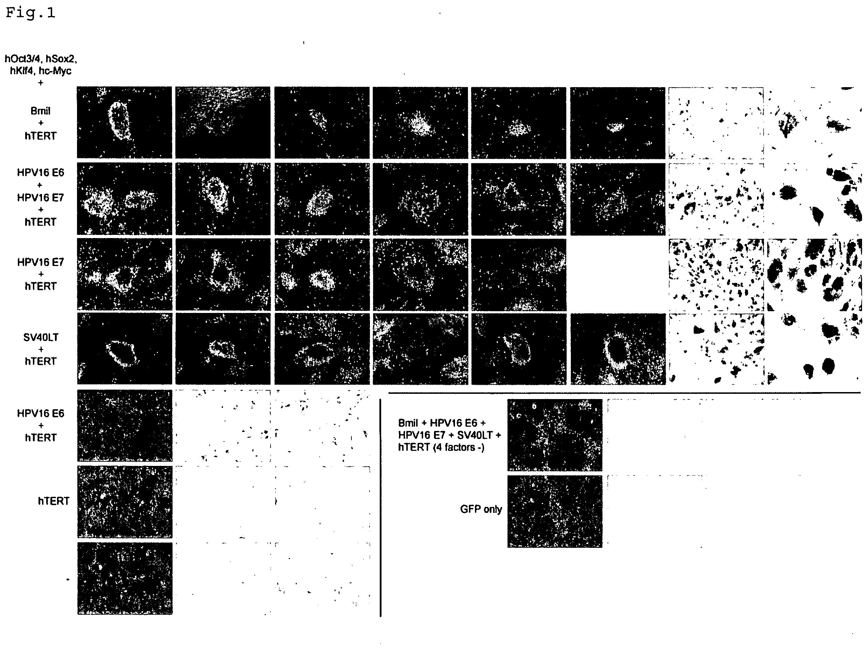

[Fig.1] Fig.1 is a view showing results of alkaline phosphatase staining of iPS cells induced from human adult skin fibroblast (adult HDF). To adult HDF (HDFa-Slc7a1), which was obtained by expressing mouse retrovirus receptor (Slc7a1) with lentivirus infection, the genes shown in a left column were introduced by using retrovirus vectors. "-" shows results of staining at HDFa-Slc7al cells into which the four genes of "Oct3/4, Sox2, Klf4, and c-Myc" were introduced. A rightmost column and a second column from the right of a photograph show results of alkaline phosphatase staining observed with phase-contrast in 10 days after gene introduction.

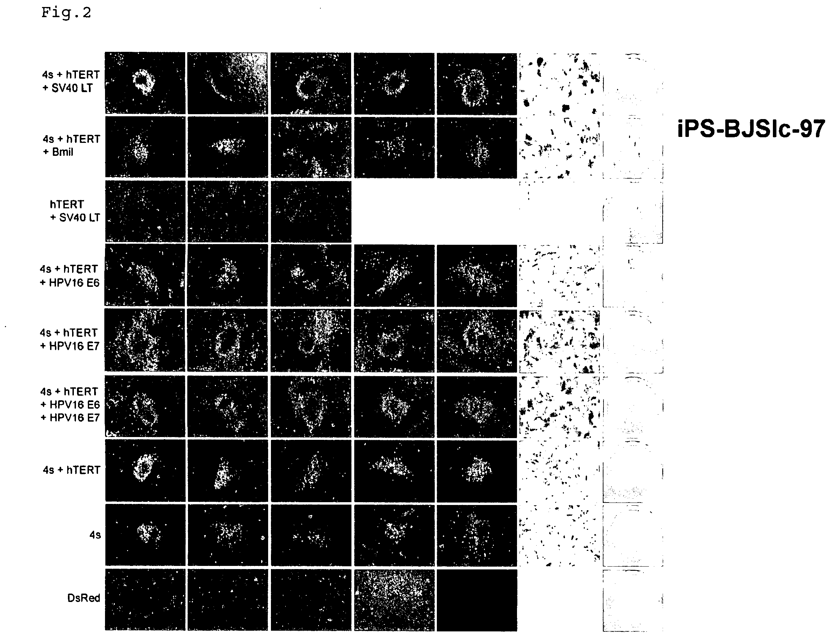

[Fig.2] Fig.2 is a view showing results of alkaline phosphatase staining of iPS cells induced from neonate preputium-derived fibroblasts (BJ). To BJ (BJ-Slc7a1) obtained by expressing mouse retrovirus receptors (Slc7al) with lentivirus infection, the genes shown in a left column was introduced by using retrovirus vectors. A second column from the right of the photograph shows results of alkaline phosphatase staining observed with a phase-contrast in 10 days after gene introduction.

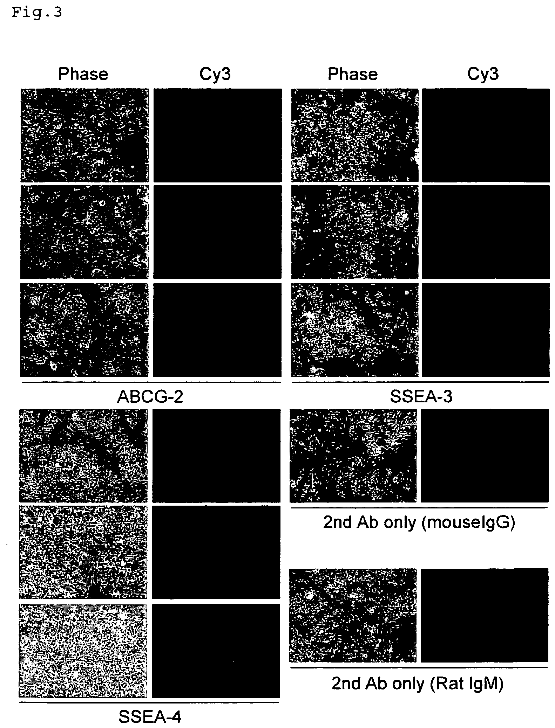

[Fig.3] Fig.3 is a view showing results of immunostaining analysis for expression of ABCG-2, SSEA-3, and SSEA-4 in iPS cells derived from adult HDF (clone iPS-HDFaSlc-87E6), and results of staining of only a secondary antibody (mouse IgG or rat IgM) as a negative control. A photograph indicates phase-contrast images, and a phase-contrast images via a rhodamine filter (objective X 10, respectively).

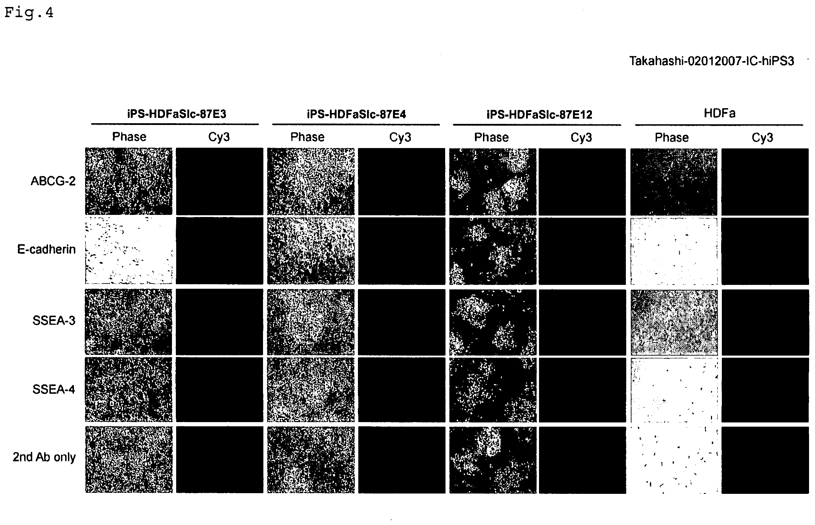

[Fig.4] Fig.4 is a view showing results of immunostaining for expression of ABCG-2, E-cadherin, SSEA-3, and SSEA-4 in iPS cells derived from adult HDF (clone iPS-HDFaSlc-87E3, 4 and 12) and adult HDF expressing mouse Slc7a1 gene (HDFa). A photograph shows phase-contrast images and phase-contrast images via a rhodamine filter (objective X 10, respectively).

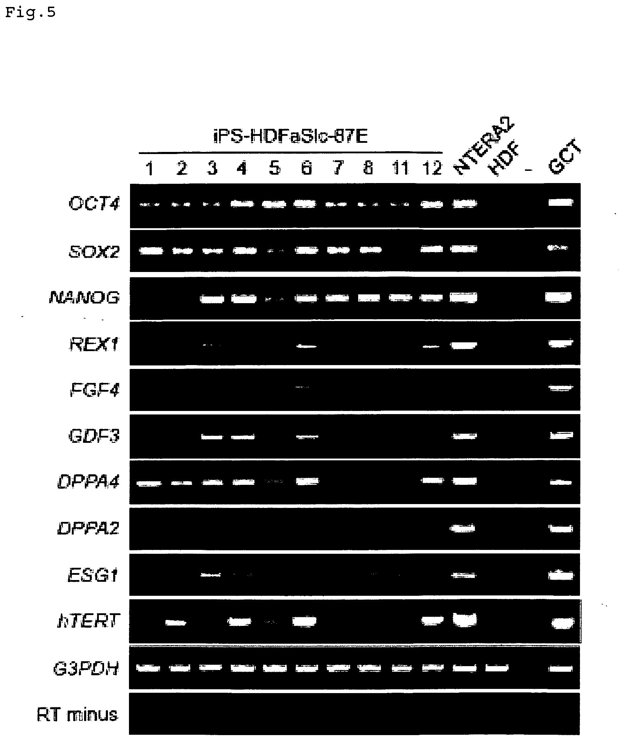

[Fig.5] Fig.5 is a view showing results of PCR amplification of each region for OCT4, Sox2, Nanog, REX1, FGF4, GDF3, DPPA4, DPPA2, ESG1, hTERT, and G3PDH, in cDNA obtained based on RNA isolated from iPS cells derived from adult HDF (clone iPS-HDFaSlc-87E1 to 8, 11 and 12), NTERA2 clone D1 human embryonic cancer cell (NTERA2), and adult HDF expressing mouse Slc7a gene (HDF).

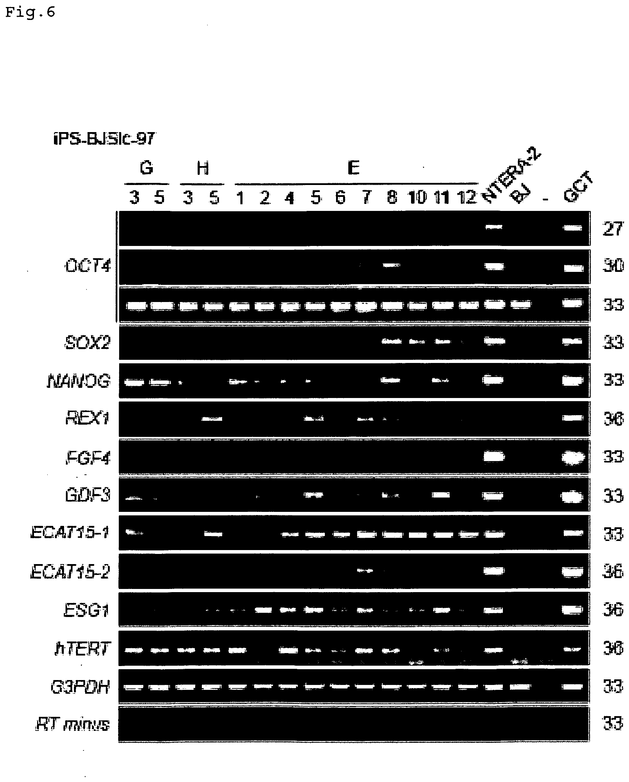

[Fig.6] Fig.6 is a view showing results of PCR amplification of each region for OCT4, Sox2, Nanog, REX1, FGF4, GDF3, ECAT15-1, ECAT15-2, ESG1, hTERT, and G3 in cDNA obtained based on RNA isolated from iPS cells derived from BJ (clone iPS-BJSlc-97G3, G5, H3, H5, E1-10, E11, and E12), a NTERA2 clone D1 human embryonic cancer cell (NTERA2), and BJ expressing mouse Slc7a1 gene(BJ-Slc7a1).

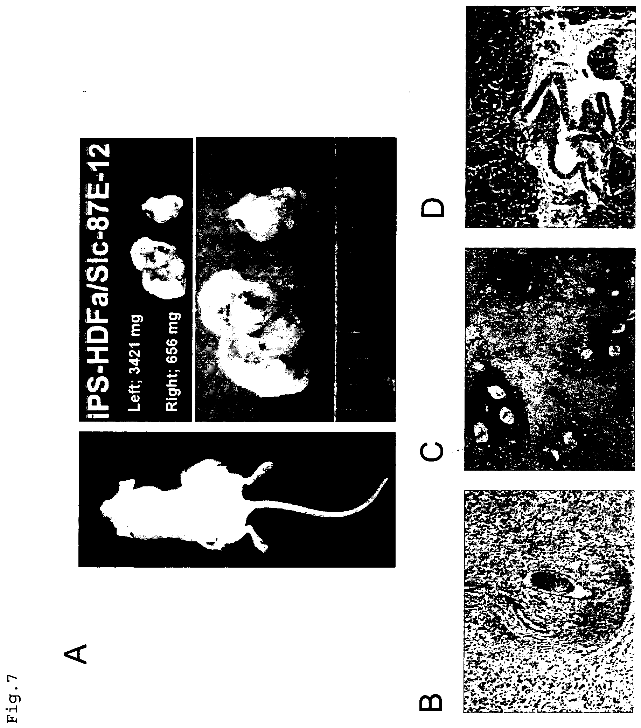

[Fig.7] Fig.7 is a view showing a mouse (A) after five weeks, which was subcutaneously injected with clone iPS-HDFaSlc-87E12, and tumor isolated from the mouse (A), as well as a view showing results of hematoxylin-eosin staining of teratoma-derived tissues isolated from mice subcutaneously injected with clone iPS-HDFaSlc-87E3 (B), clone iPS-HDFaSlc-87E6 (C), and clone iPS-HDFaSlc-87E12 (D), respectively.

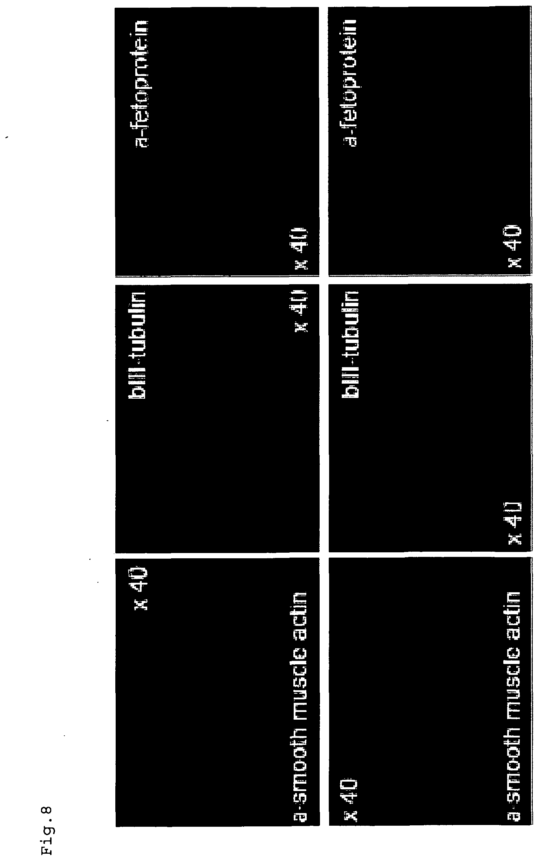

[Fig.8] Fig.8 is a view of immunostaining showing in vitro differentiation of human iPS cells. Human iPS-like cells were cultured on HEMA-coated plates for 7 days, and further cultured on a gelatin-coated dish for 7 days, and then the cells were collected to fix with fixing solution (10% formalin-containing PBS). The cells were reacted with anti-α-smooth muscle actin antibody (a-smooth muscle actin), anti-βIII-tubulin antibody (bIII-tublin), and anti-α-fetoprotein antibody (α-fetoprotein) as a primary antibody, and immunostained with the secondary antibodies.

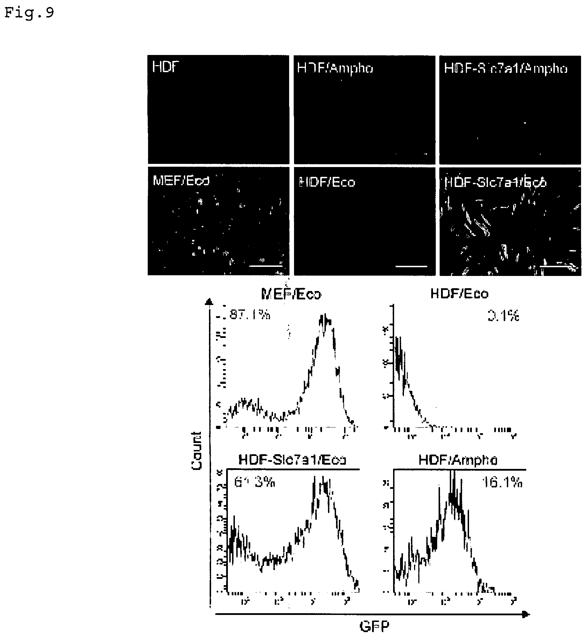

[Fig.9] Fig.9 is a view showing results of improvement in an efficiency of introducing retrovirus genes into human HDF. Ecotropic (Eco) or amphotropic (Ampho) retrovirus containing GFP cDNA was introduced to HDF or HDF (HDF-Slc7a1) expressing mouse Slc7a1 gene. The photographs of the top of the figure shows results observed with fluorescent microscope (bar is 100 µm), and graphs of the bottom of the figure shows results of flow cytometry.

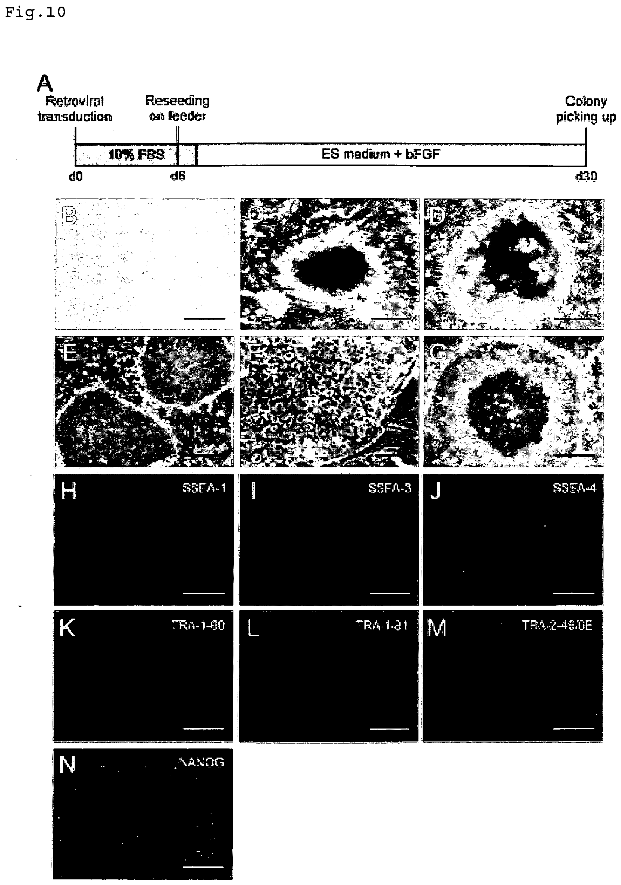

[Fig.10] Fig. 10 is a view showing results of induction of iPS cells from adult HDF. A shows time schedule of iPS cell generation. B shows cell morphology image of HDF, C shows typical image of non-ES cell-like colony, D shows typical image of human ES cell-like colonies, E shows cell morphology of established iPS cell line at passage number 6 (clone 201B7), F shows an image of iPS cells with high magnification, and G shows an image of spontaneously differentiated cells in the center part of human iPS cell colonies, respectively. H to N show results of analysis for immunocytochemistry for SSEA-1 (H), SSEA-3 (I), SSEA-4 (J), TRA-1-60 (K), TRA-1-81 (L), TRA-2-49/6E (M) and Nanog (N), respectively. Nucleuses were stained with Hoechst33342 (blue). Bars of each image show 200 µm (B to E, G), 20 µm(F), and 100 µm (H to N).

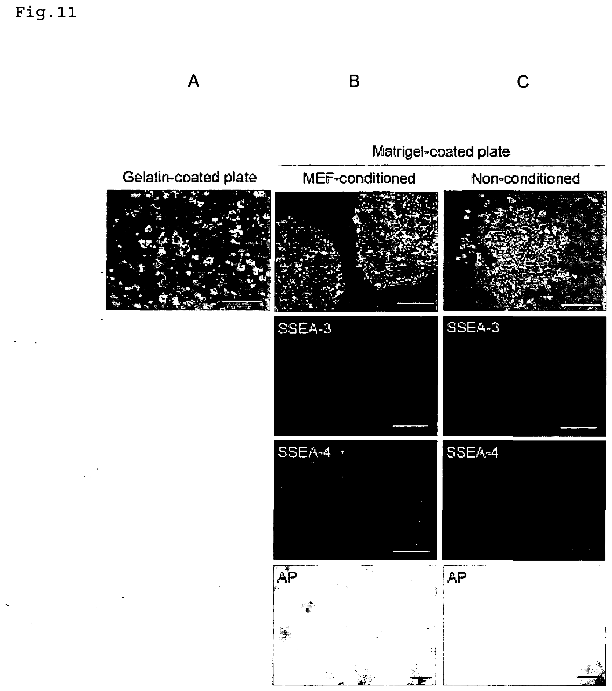

[Fig.11] Fig.11 is a view showing feeder cell dependency of human iPS cell. A shows an image of iPS cells seeded on gelatin-coated plate. B shows an image of iPS cells cultured in MEF-conditioned medium (MEF-CM) on matrigel-coated plate. C shows an image of iPS cells cultured in non-conditioned medium for human ES on matrigel-coated plate.

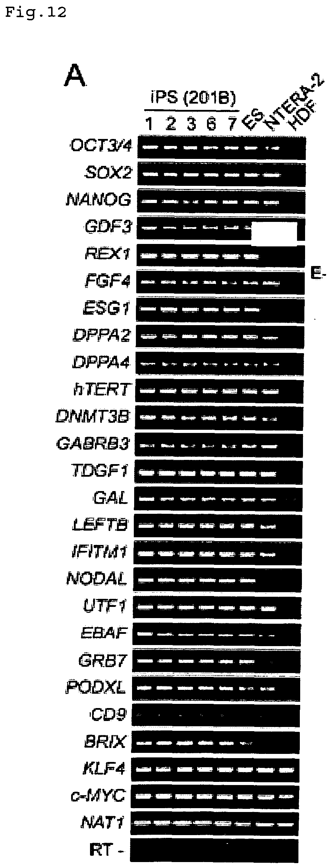

[Fig.12] Fig.12 is a view showing results of RT-PCR analysis of ES cell marker genes for expression of human ES cell marker genes in human iPS cells. Cells, which RNA has been isolated, are iPS cell clone 201B, ES cell, NTERA2 clone D1 human embryonic cancer cell (NTERA2), and adult HDF (HDF) expressing mouse Slc7a1 gene.

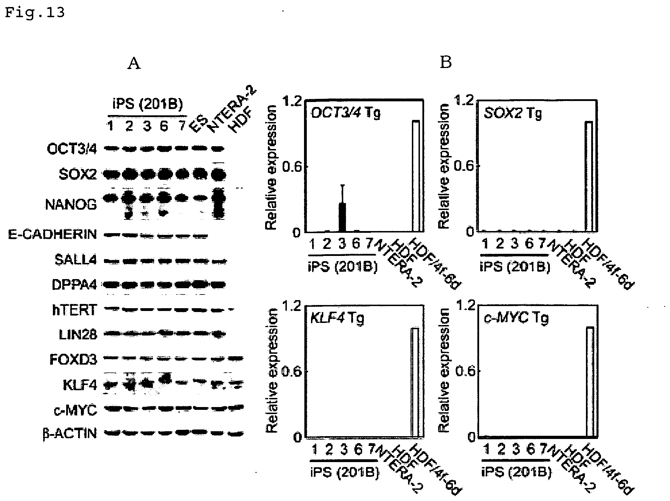

[Fig.13] Fig.13 is a view showing Western blotting analysis of ES cell marker genes (A) and results of quantitative PCR for expression of retrovirus-introduced gene (B), for expression of human ES cell marker genes in human iPS cells. B shows the averages of three independent experiments and a bar shows standard deviation.

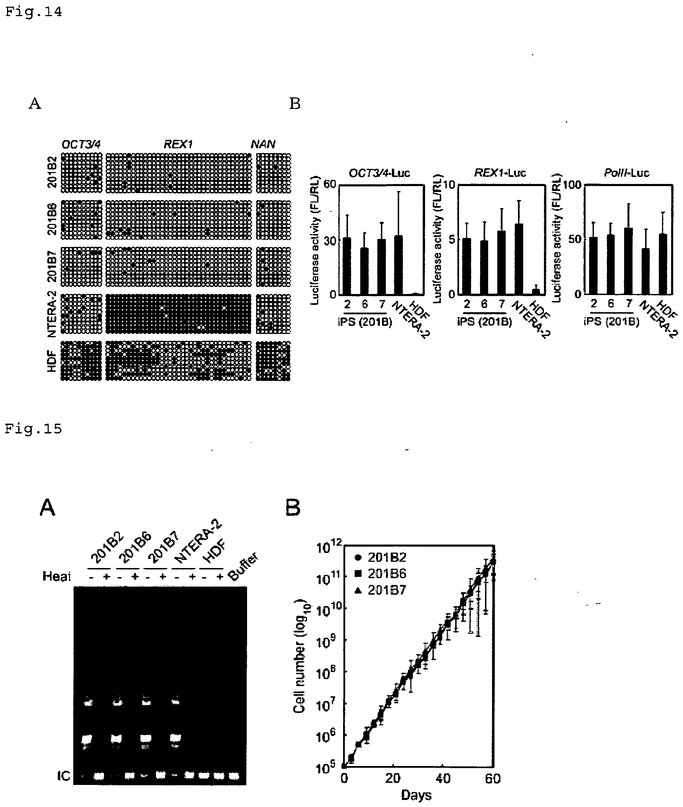

[Fig.14] Fig.14 is a view showing results of bisulfite genomic sequencing of the promoter regions of Oct3/4, REX1 and Nanog (A) and results of luciferase assays (B), for expression of human ES cell marker genes in human iPS cells. In A, open and closed circles indicate unmethylated and methylated CpGs. B shows the average of the result from four assays, and bars indicate standard deviation.
[Fig.15] Fig.15 is a view showing high levels of telomerase activity and exponential proliferation of human iPS cells. A shows results of detection of telomerase activities by the TRAP method. Heat-inactive (+) samples were used as negative controls (IC is internal control). B shows proliferation curve of iPS cells, and averages and standard deviations in quadruplicate.

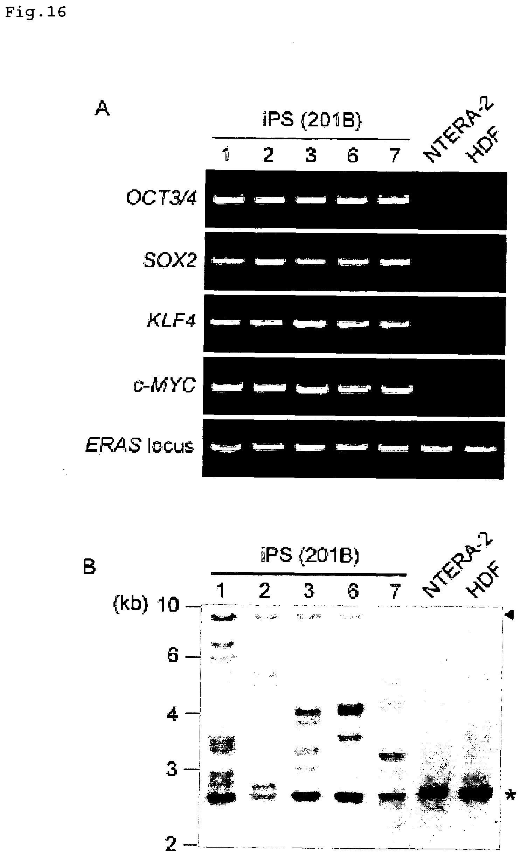

[Fig.16] Fig.16 is a view showing results of gene analysis of human iPS cells. A is results of genomic PCR showing results of integration of all four retroviruses in the chromosomes of all clones. B shows results of Southern blotting analysis by using c-Myc cDNA probe. Asterisks of the figure shows endogeneous c-Myc allele (2.7 kb), and arrows show mouse c-Myc allele (9.8 kb) derived from SNL feeder cells.

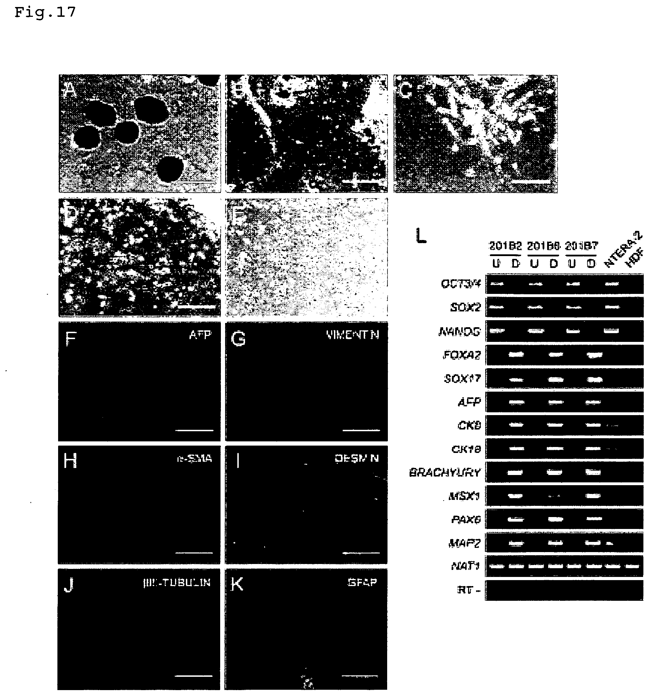

[Fig.17] Fig.17 is a view showing embryoid body-mediated differentiation of human iPS cells. A shows an image of floating culture of iPS cells at day 8. B to E show images of differentiated cells at day 16 (B), neuron-like cells (C), epithelial cells (D), and cobblestone-like cells (E), respectively. F to K show results of immunocytochemistry of α-fetoprotein (F), vimentin (G), α-smooth muscle actin (H), desmin (I), βIII tubulin (J), and GFAP (K), respectively. Bars of the figure show 200µm (A,B) and 100µm (C-K). In F-K, Nucleuses were stained with Hoechst 33342 (blue). L shows results of RT-PCR analyses of various differentiation markers for three germ layers.

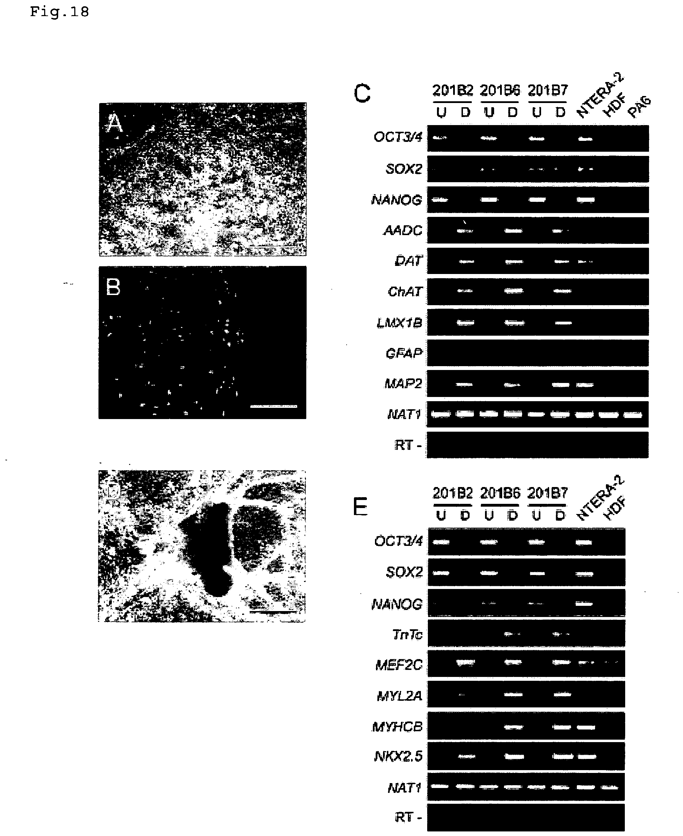

[Fig.18] Fig.18 is a view showing directed differentiation of human iPS cells. A is phase-contrast images of differentiated iPS cells after 18 days cultivation on PA6 cell. B shows results of immunocytochemistry of the cells shown in A with βIII tubulin (red) and tyrosine hydroxylase (green) antibodies. Nucleuses were stained with Hoechst 33342 (blue). C represents results of RT-PCR analyses of dopaminergic neuron markers. D is phase-contrast image of iPS cells differentiated into cardiomyotic cells. E shows results of RT-PCR analyses of cardiomyotic cell markers. Bars in the images of A, B and D are 200µm (A, D) and 100µm (B) .

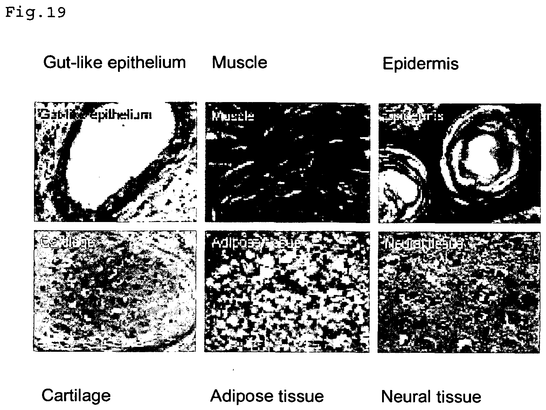

[Fig.19] Fig.19 is a view showing teratoma derived from human human iPS cells. Hematoxylin and eosin staining image of teratoma derived from iPS cells (clone 201B7 is used) is shown.

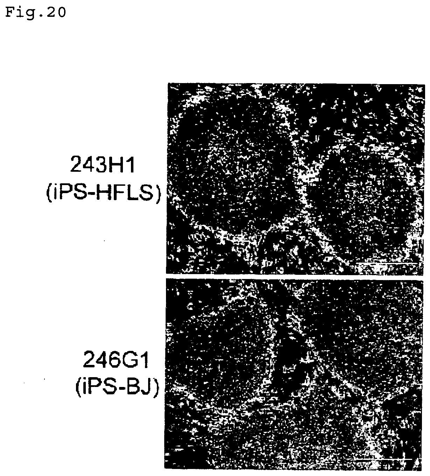

[Fig.20] Fig.20 is phase-contrast images of human iPS cells derived from adult fibroblast-like synoviocytes (HFLS, clone 243H1) and neonate preputium-derived fibroblasts (BJ, clone 246G1). In each image, a bar shows 200 µm.

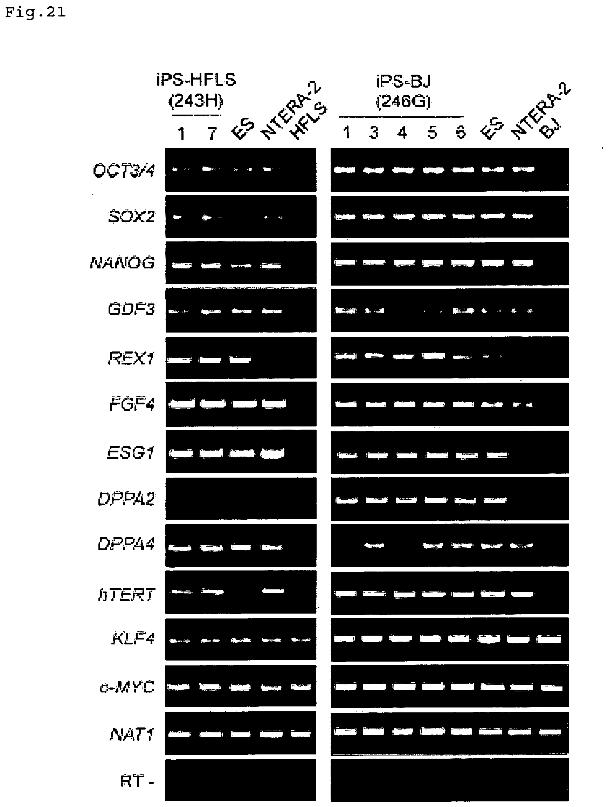

[Fig.21] Fig.21 is a view showing expression of ES cell marker genes in iPS cells derived from HFLS and BJ.

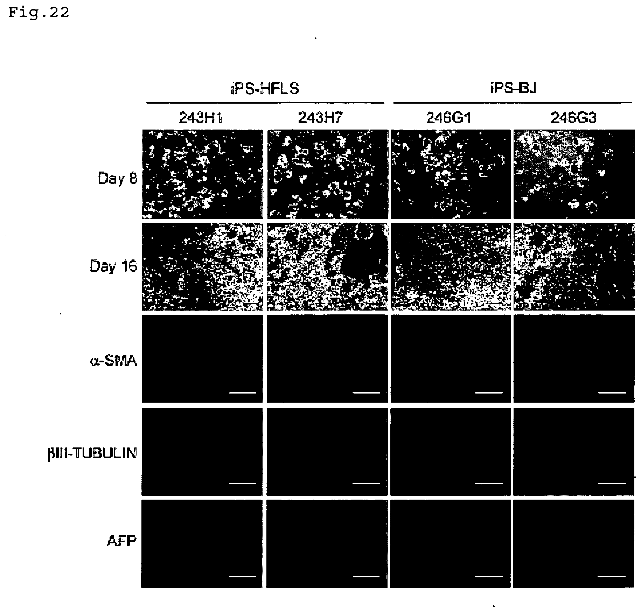

[Fig.22] Fig.22 is a view showing embryoid body-mediated differentiated cells of iPS cells derived from HFLS and BJ. Expressions of α-smooth muscle actin, βIII tubulin, and α-fetoprotein were confirmed by immunostaining.

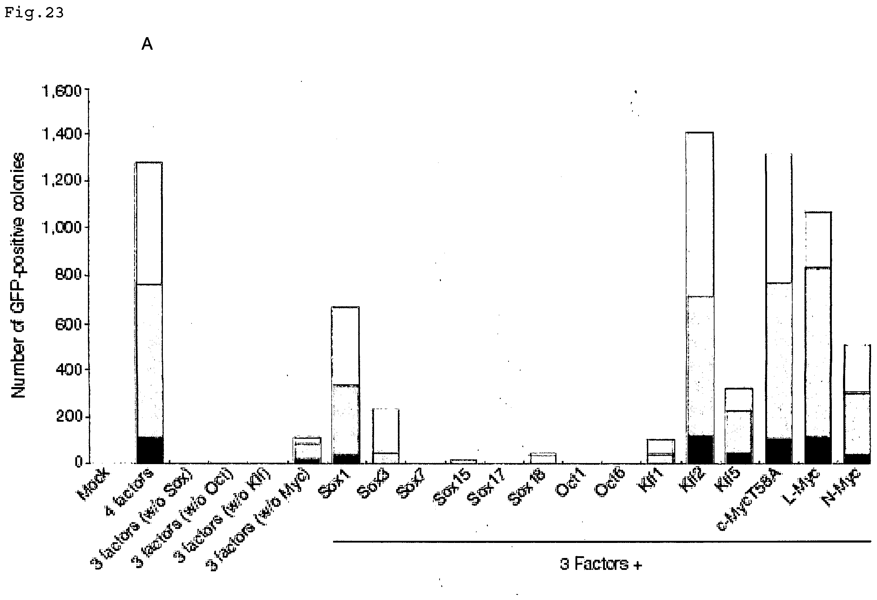

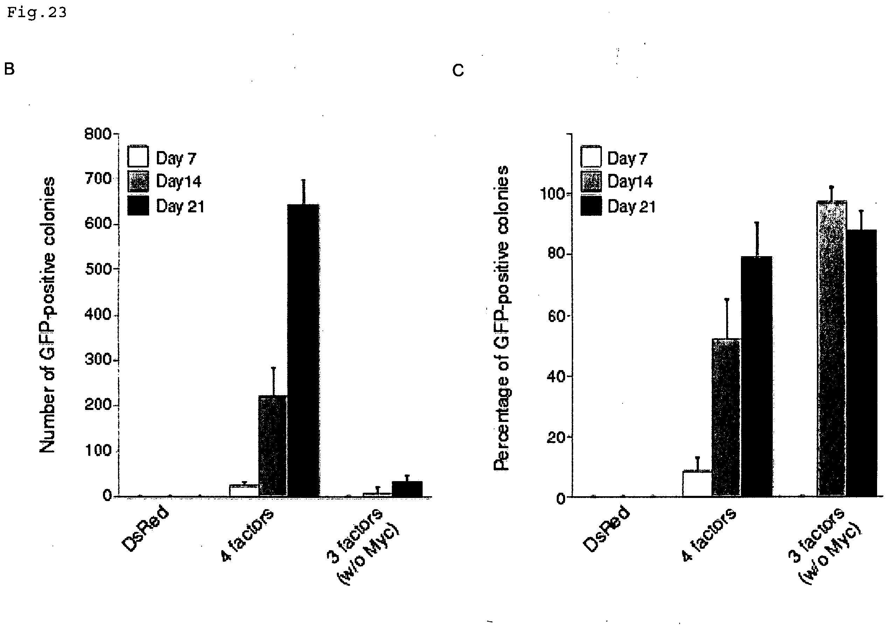

[Fig.23] Fig.23 is a view showing the effect of family gene and ommision of c-Myc on the generation of iPS cells from Nanog-reporter MEF mice. A shows the results of generation of iPS cells with family gene from MEFs by Nanog selection. A graph shows the number of GFP-positive colonies. The results of three independent experiments are shown with different colors (white, gray, and black). "4 factors" shows the combination of Oct3/4, Sox2, Klf4, and c-Myc. B shows the effect of puromycin selection timing on iPS cell generation, and GFP-positive colonies observed 28 days after transduction of the four genes or the three genes devoid of c-Myc. C shows the effect of puromycin selection timing on the percentage of GFP-positive colonies per all colonies.

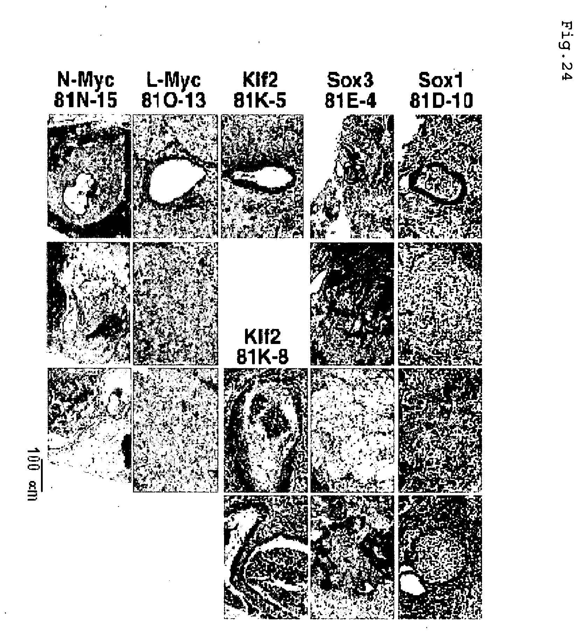

[Fig.24] Fig.24 is a view showing histological images of teratoma derived from iPS cells, which were induced from Fbx15-reporter mice-derived MEF with family proteins of Myc, Klf, and Sox.

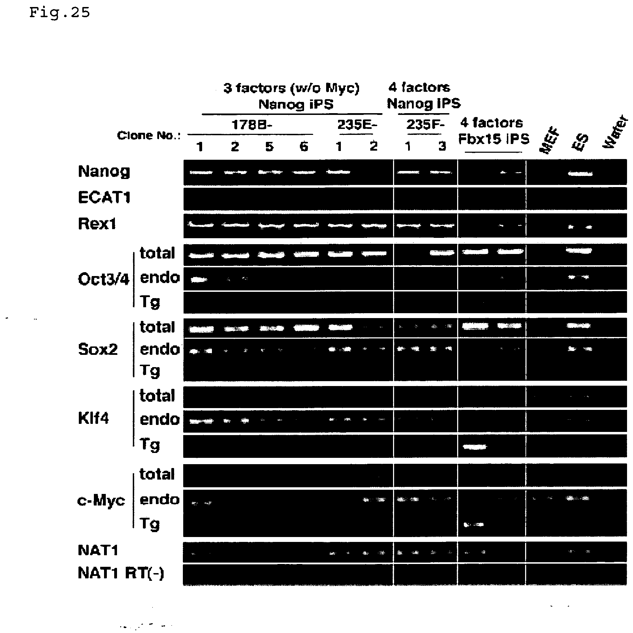

[Fig.25] Fig.25 is a view showing the characterization of iPS cells generated by inducing three genes (Oct3/4, Sox2, Klf4) devoid of c-Myc into Nanog-reporter mice-derived MEF with retroviruses. Results of RT-PCR showing expression levels of ES cell marker genes and the four genes are shown. By using specific primer sets, total transcripts, transcripts from the endogeneous genes (endo), and transcripts from the retroviruses (Tg) were distinguished for the four genes.

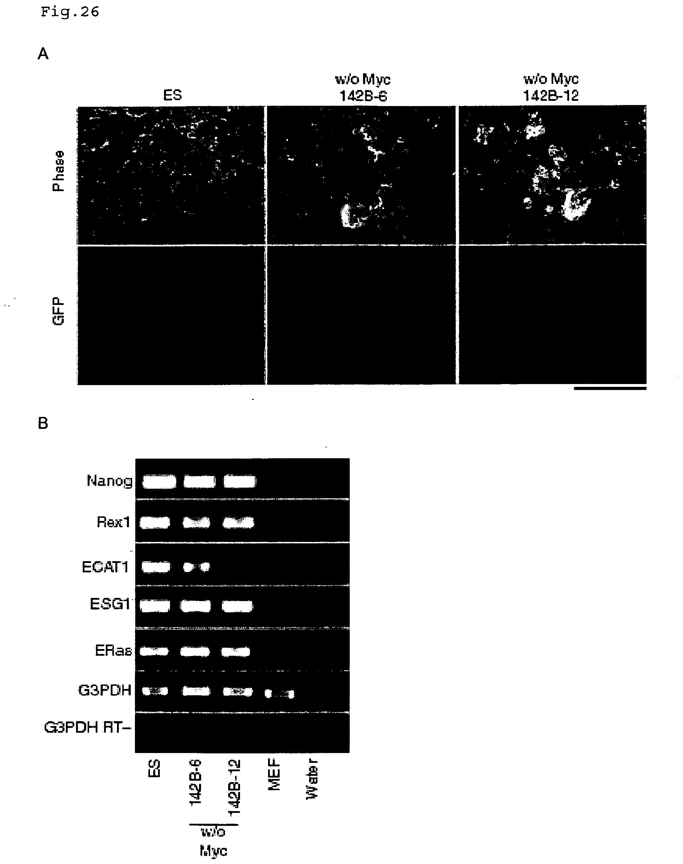

[Fig.26] Fig.26 is a view showing results of induction of iPS cells generated by inducing three genes (Oct3/4, Sox2, Klf4) devoid of c-Myc into Fbx15-reporter mice-derived MEF with retroviruses, as well as results of expression of induced GFP. A shows morphology of iPS cells generated by introduction of the three genes (Oct3/4, Sox2, Klf4) devoid of c-Myc. The bar of the photograph shows 500µm. B shows results of RT-PCR analyses of ES-cell marker genes in ES, MEF, and iPS cells generated by introduction of three genes (Oct3/4, Sox2, Klf4) devoid of c-Myc.

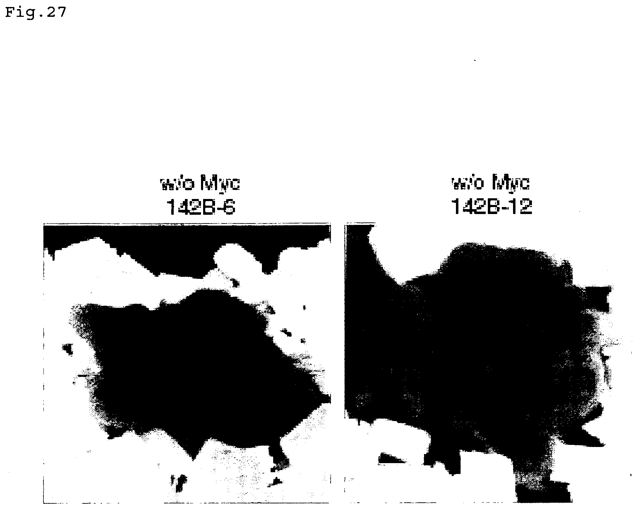

[Fig.27] Fig.27 is a view showing chimeric mice derived from iPS cells (clone 142B-6 and 142B-12) generated by introduction of three genes (Oct3/4, Sox2, Klf4) devoid of c-Myc.

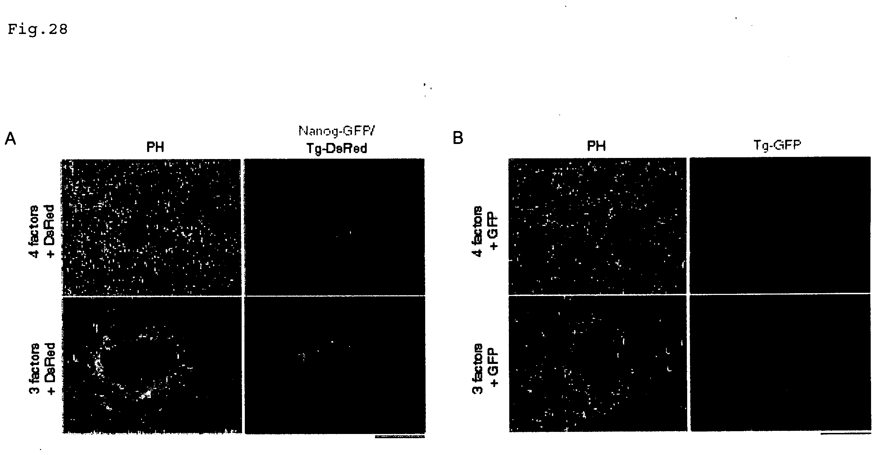

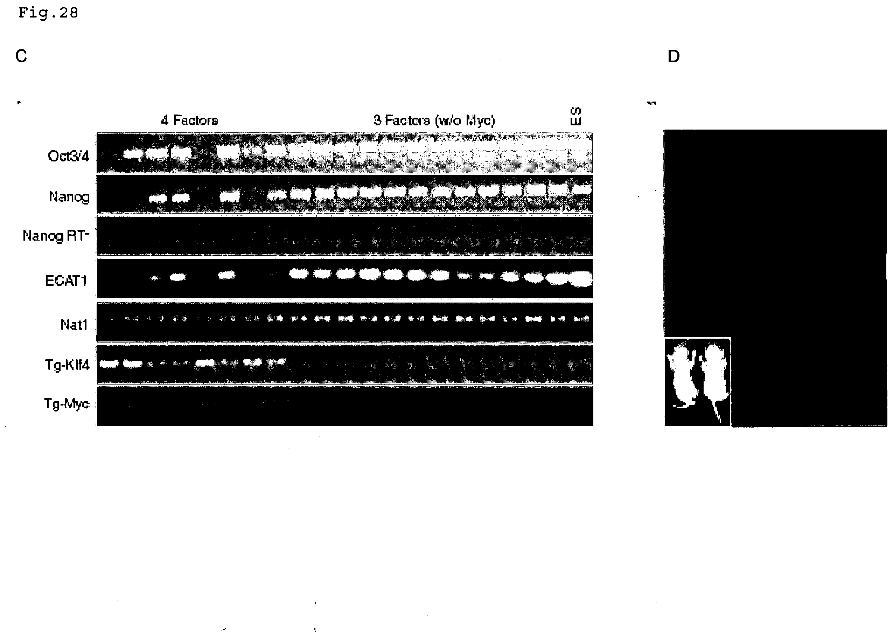

[Fig.28] Fig.28 is a view showing results of the efficient isolation of iPS cells without drug selection. A is images of morphology of iPS cells induced from TTF derived from adult mice containing the Nanog-GFP-IRES-Puro^{r} reporter. Cells were transduced with either the four genes or the three genes devoid of c-Myc, together with DsRed, and then were cultured for 30 days without drug selection. Expression of the Nanog reporter (Nanog-GFP) and the DsRed retrovirus (Tg-DsRed) was examined by fluorescence microscopy. The bar indicates 500 µm. B shows images of morphology of iPS cells induced from TTF derived from adult mice, which contained a DsRed transgene driven by a constitutively active promoter (ACTB, β-actin gene) but lacked the Nanog- or Fbx15- selection cassettes. The cells were transduced with either the four genes or the three genes devoid of c-Myc, together with GFP, and then cultured for 30 days without drug selection. The expression of the GFP retrovirus (Tg-GFP) was examined by a fluorescence microscopy. The bar indicates 500µm. C shows results of RT-PCR analyses of ES marker genes in ES cells and in iPS cells generated from TTFs without drug selection. D is an image of chimeric mice derived from iPS cells, which were generated from adult TTFs without drug selection or the c-Myc retrovirus.

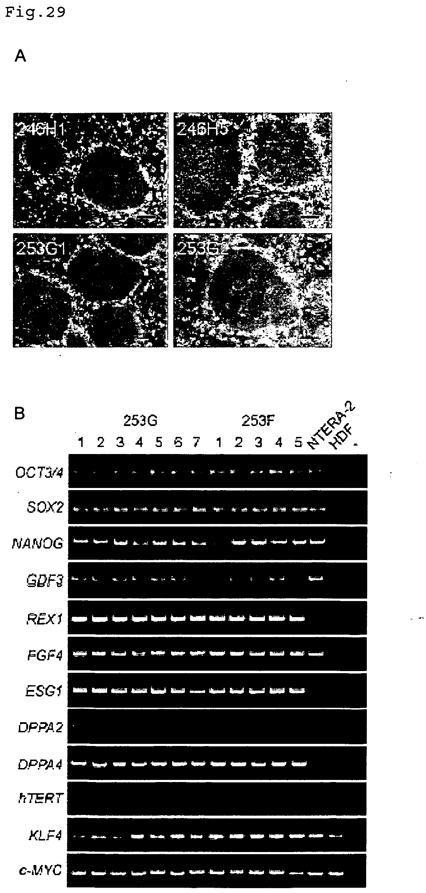

[Fig.29] Fig.29 is a view showing results of induction of iPS cells generated by inducing three genes (Oct3/4, Sox2, Klf4) devoid of c-Myc. A is images showing that established human iPS cells (clone 253G and 246H) exhibit human ES cell-like morphology. B shows results of expression of ES cell marker genes in human iPS cells derived from HDF established using iPS cells (253G) generated by introduction of three genes (Oct3/4, Sox2, Klf4) devoid of c-Myc, or iPS cells (253F) generated by introduction of four genes (Oct3/4, Sox2, Klf4, c-Myc) including c-Myc.

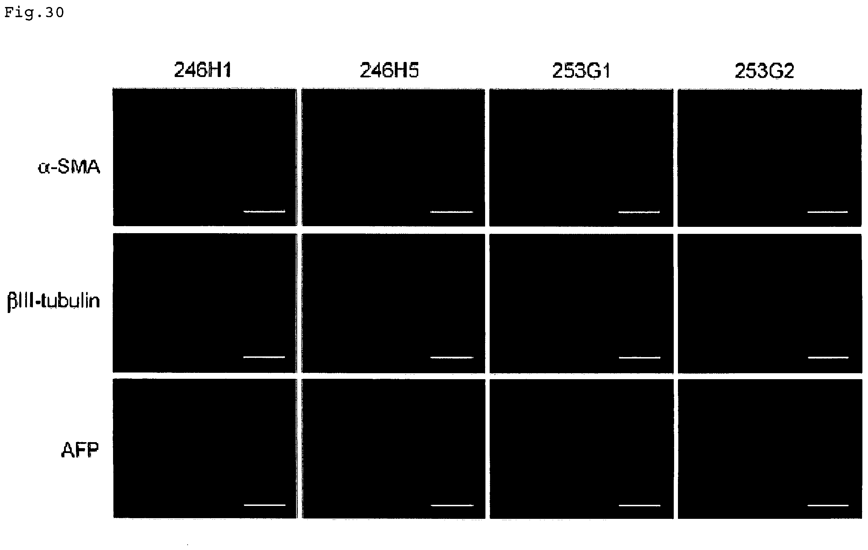

[Fig.30] Fig.30 is a view of induction of embryoid body-mediated differentiation from human iPS cells generated by introduction of three genes (Oct3/4, Sox2, Klf4) devoid of c-Myc into adult fibroblasts (HDL; 253G) and neonate preputium-derived fibroblasts (BJ; 246G). Expression of α-smooth muscle actin, βIII tubulin, and α-fetoprotein was examined by immunostaining.

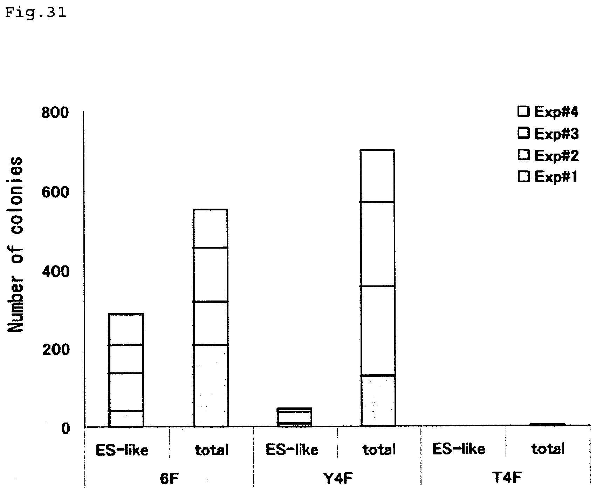

[Fig.31] Fig.31 is a view showing a graph of the number of colonies, which were counted after introduction of six genes (Klf4, c-Myc, Oct3/4, Sox2, Nanog, and Lin28), or two different combinations of four genes (Klf4, c-Myc, Oct3/4, and Sox2 are shown in Y4F; and Oct3/4, Sox2, Nanog, and Lin28 are shown in T4F) into 293FT cells. "ES-like" indicates the number of colonies which are morphologically like to colonies of ES cells, and "total" indicates the total number of ES-like colonies and non-ES-like colonies. Exp#1, Exp#2, Exp#3, and Exp#4 show results independent experiments. The vertical scale indicates the number of colonies.

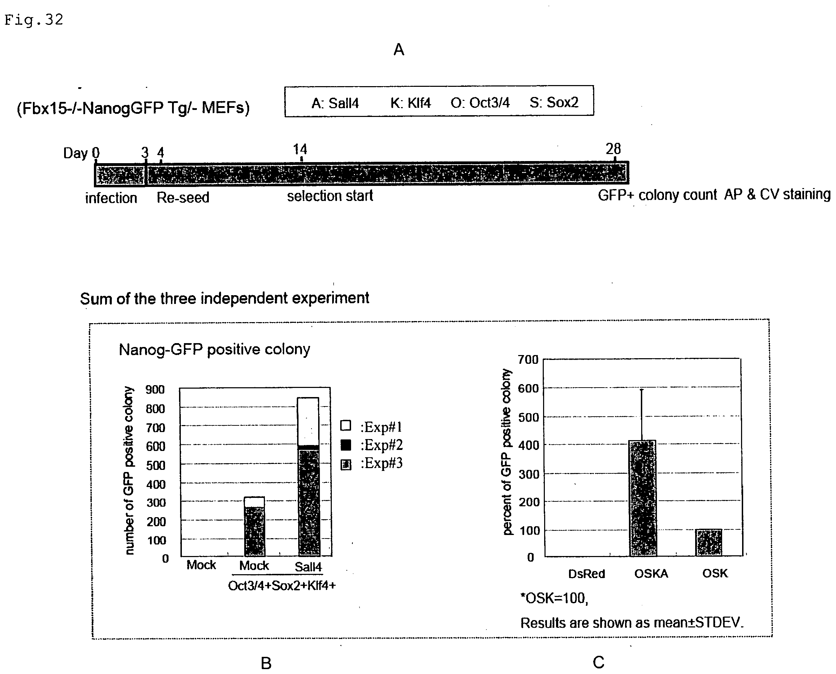

[Fig.32] Fig.32 shows experimental results performed using mouse embryonic fibroblasts (MEF). A indicates the time schedule of the experiment. 1.0×10⁵ MEF cells, which were obtained from Nanog GFP^{tg/-} Fbx15^{-/-} mice, were seeded on gelatin-coated 6-well plates. Next day, four genes (Oct3/4, Klf4, Sox2 and Sall4; OSKA) or three genes (Oct3/4, Klf4, and Sox2; OSK) were introduced into MEFs with retrovirus. On 4 day after infection, the cells were seeded on 6-well plate covered with mitomycin C-treated STO cells at the ratio of 1:2 or 1:6. Drug selection was started on 14 day or 21 day after infection. On 28 day, GFP-positive cells were counted and stained with alkaline phosphatase (AP) and crystal violet (CV). B indicates the total number of GFP-positive colonies in three independent experiments (#1, 2, and 3). C indicates the percentages of GFP-positive colonies in three independent experiments (#1, 2, and 3).

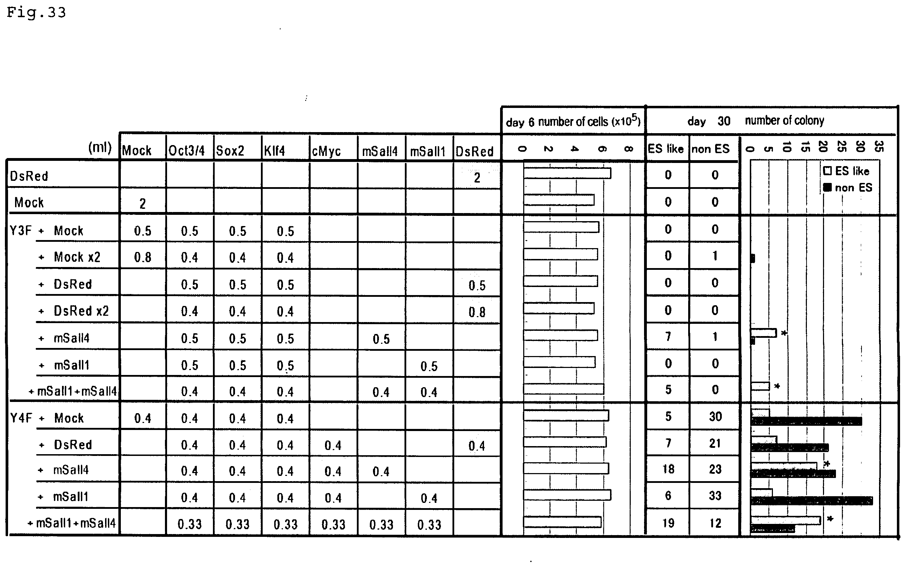

[Fig.33] Fig.33 shows experimental results which were performed using human adult skin-derived fibroblasts (adult HDF). 1.0×10⁵ adult HDF cells expressing mouse retrovirus receptor slc7a were seeded on 6-well plates. Next day, a combination of various genes shown in the figure was introduced into the cells with retrovirus vectors. On 6 days after infection, the number of the cells was adjusted to 5.0×10⁵, and the cells were seeded again on 100 mm plate covered with 1.5×10⁶ mitomycin C-treated STO cells. After 7 days, the medium was exchanged with primate ES cell medium suplemented with 4 ng/ml bFGF. The figure shows the number of colonies on 30 days after infection.

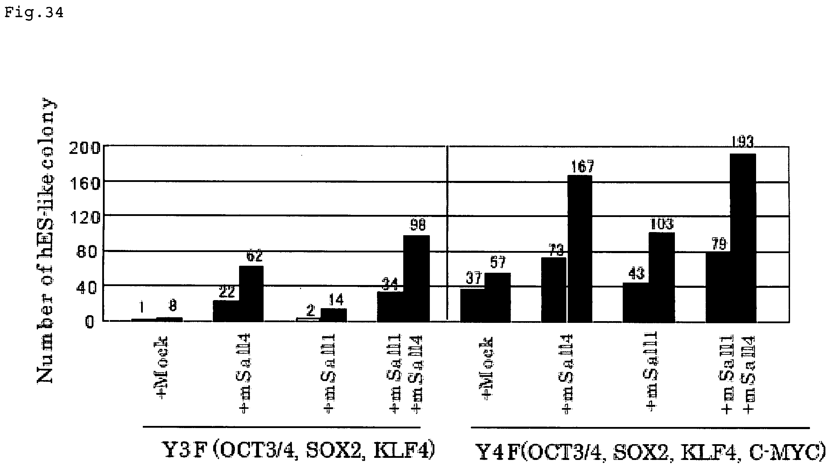

[Fig.34] Fig.34 is a view showing the number of colonies counted on 32 day (left bar) and 40 day (right bar) after infection by introduction of human-derived four genes (Y4F: Oct3/4, Sox2, Klf4, and c-Myc) or three genes (Y3F: Oct3/4, Sox2, and Klf4), and mouse Sall4 (mSall4) or mouse Salll (mSall1) or both of them into human adult skin-derived fibroblasts(HDF) expressing mouse retrovirus receptor Slc7a with retrovirus vectors. "+Mock" indicates a group infected with retroviruses which were replaced with empty vector devoid of Y3F or Y4F, "+mSall4" indicates a group introduced with Sall4 together with Y3F or Y4F, "+mSalll" indicates a group introduced with mSall1 together with Y3F or Y4F, and "+mSall4+mSall1" indicates a group in which both of mSall4 and mSalll were introduced together with Y3F or Y4F. The vertical scale indicates the number of colonies of human iPS cells confirmed on 10 cm petri dishes. The same experiment was repeated in twice, and the total numbers of colonies were shown on the top of graphs per each experiment.

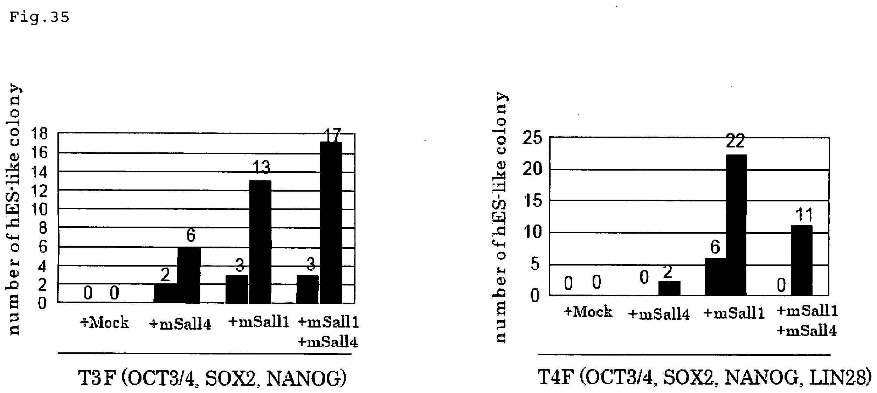

[Fig.35] Fig.35 is a view showing the number of colonies counted on 32 day (left bar) and 40 day (right bar) after infection by introduction of human-derived four genes (T4F: Oct3/4, Sox2, Nanog and Lin28) or three genes (T3F: Oct3/4, Sox2, and Nanog), and mouse Sall4 (mSall4) or mouse Salll (mSall1) or both of them into human adult skin-derived fibroblasts (HDF) expressing mouse retrovirus receptor Slc7a by using retrovirus vectors. "+Mock" indicates a group infected with retroviruses which are empty vectors devoid of T3F or T4F, "+mSall4" indicates a group introduced with mSall4 together with T3F or T4F, "+mSall1" indicates a group introduced with mSall1 together with T3F or T4F, and "+mSall4+mSall1" indicates a group in which both of mSall4 and mSall1 were introduced together with T3F or T4F. The vertical scale indicates the number of colonies of human iPS cells counted on 10 cm petri dishes. The same experiment was repeated in twice, and the total numbers of colonies of each experiment are shown on the top of bar in the graph.

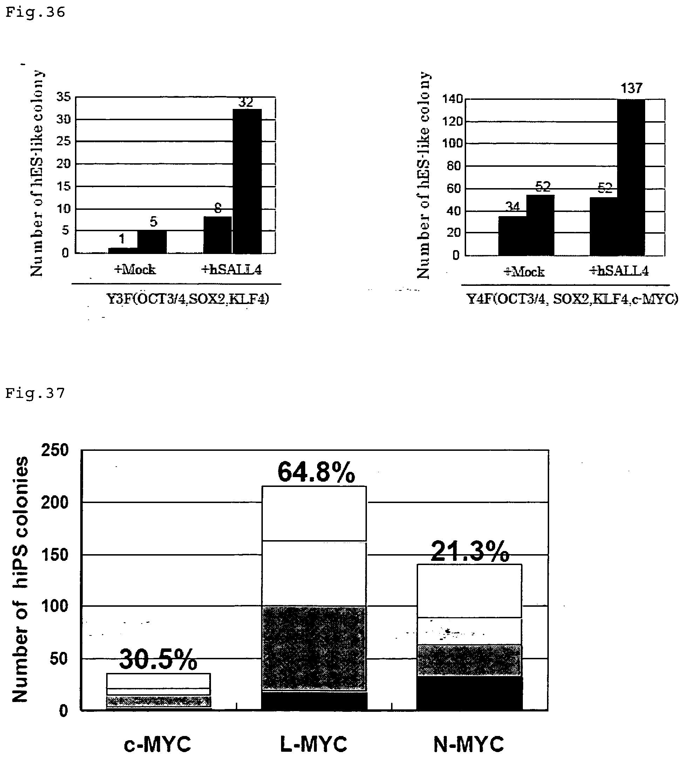

[Fig.36] Fig.36 is a view showing the number of colonies counted on 32 day (left bar) and 40 day (right bar) after infection by introduction of human-derived four genes (Y4F) or three genes (Y3F) and human Sall4 (hSall4) into human adult skin-derived fibroblasts (HDF) expressing mouse retrovirus receptor Slc7a with retrovirus vectors. "+Mock" indicates a group infected with retroviruses which are empty vectors devoid of Y3F or Y4F, and "+hSall4" indicates a group introduced with hSall4 together with Y3F or Y4F. The vertical scale indicates the number of colonies of human iPS cells counted on 10 cm petri dishes. The same experiment was repeated in twice or three times, and the total numbers of colonies in each experiment were shown on the bar of the graph.
[Fig.37] Fig.37 is a graph showing the results of counting the number of colonies of human iPS cells generated by introducing the total four genes of three genes of Oct3/4, Klf4, and Sox2, and c-Myc, L-Myc, or N-Myc gene, into human adult skin-derived fibroblasts (HDFa-Slc7a1) expressing mouse retrovirus receptor Slc7a with lentivirus vectors. The value on each graph indicates the ratio of the number of colonies of iPS cells to total number of the colonies.

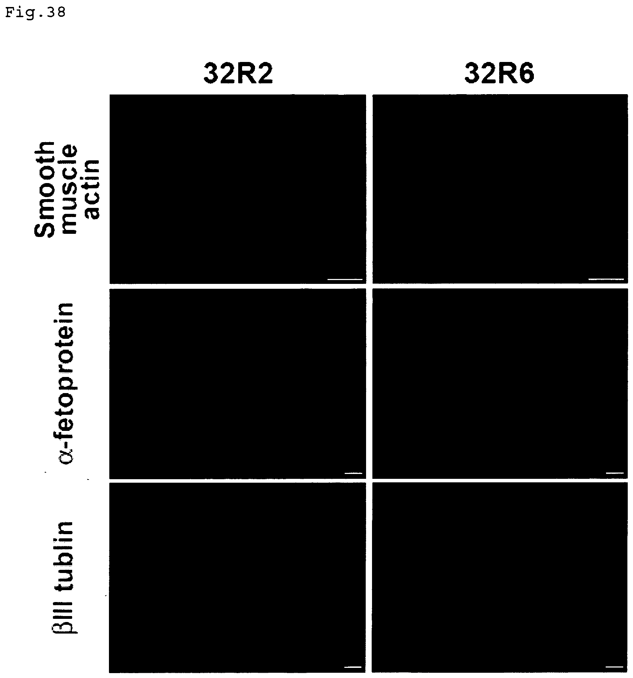

[Fig.38] Fig.38 is a photograph showing results confirmed that human iPS cells (32R2, 32R6) generated by introducing four genes (Oct3/4, Sox2, Klf4, L-Myc) into human adult skin-derived fibroblasts (HDFa-Slc7a1) expressing mouse retrovirus receptor Slc7a with lentivirus vectors have the ability to be differentiated into three germ cell lines, by immunostaining with α-smooth muscle actin, α-fetoprotein, and βIII-tubulin antibody.

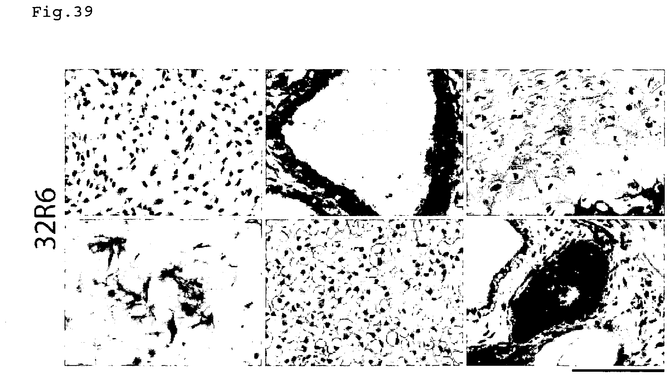

[Fig.39] Fig.39 is histological staining images (hematoxylin-eosin staining) of the teratoma obtained by injecting human iPS cells (32R6) generated by introduction of four genes (Oct3/4, Sox2, Klf4, and L-Myc) into the testis of SCID mouse. Top columns indicate histological images of nerve tissue, intestinal tract-like tissue, and cartilage tissue, from the left side. Bottom columns indicate histological images of hair tissue, adipose tissue, and pigment tissue, from the left side.

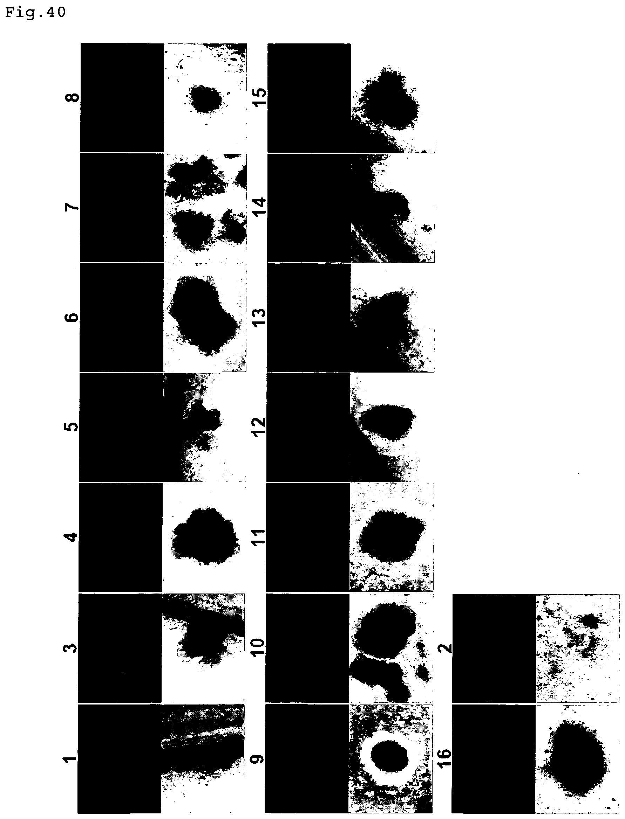

[Fig.40] Fig.40 is photographs showing morphology of GFP-positive colonies (iPS-MEF-Ng-443-3) obtained by introducing three genes (Oct3/4, Klf4, and L-Myc) into Nanog reporter mice-derived MEF (MEF-Ng) with retrovirus vectors. Top columns are the images of GFP-positive colonies, and bottom columns are phase-contrast images.

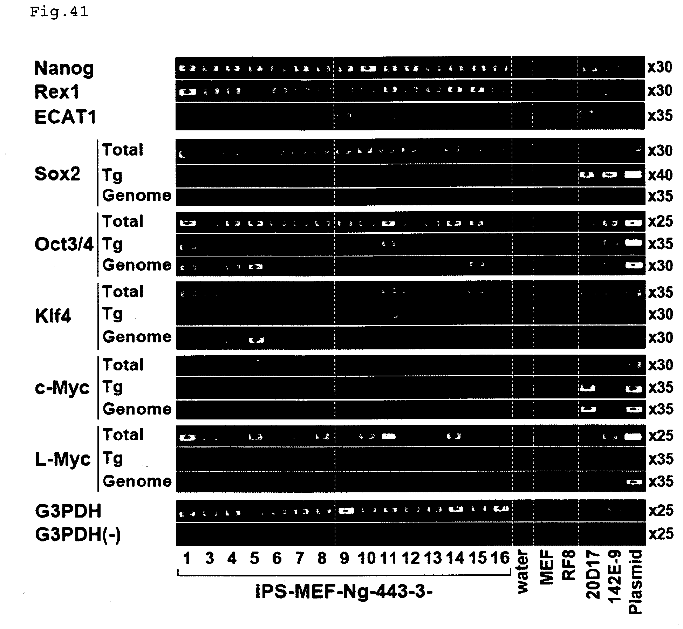

[Fig.41] Fig.41 is a photograph showing results of RT-PCR and Genomic PCR for clones derived from GFP-positive colonies (iPS-MEF-Ng-443-3) shown in Fig.40. In the figure, "Total" shows expression of endogenous and retrovirus-derived genes, "Tg" shows expression of retrovirus-derived genes, and "Genome" shows results of detection for the presence of the retrovirus-derived genes, which were integrated into genome DNA, by using the specific primers for the genes. The number of the right side in the photograph indicates the cycle number of PCR. In the figure, "RF8" indicates ES cells as control, "20D17" indicates iPS cells (Nanog-iPS cell; Nature, 448, pp.313-317, 2007), which were obtained by introducing four genes (Oct3/4, Klf4, L-Myc, and Sox2) into MEF-Ng, and "142E-9" indicates iPS cells (Fbx-iPS), which were obtained by introducing four genes (Oct3/4, Klf4, L-Myc, and Sox2) into MEF-Fbx, and results of the same experiment on these cells are shown. In the figure, "Plasmid" shows results of using plasmids containing each gene (pMXs-Sox2, pMXs-Oct3/4, pMXs-Klf4, pMXs-c-Myc, and pMXs-L-Myc).

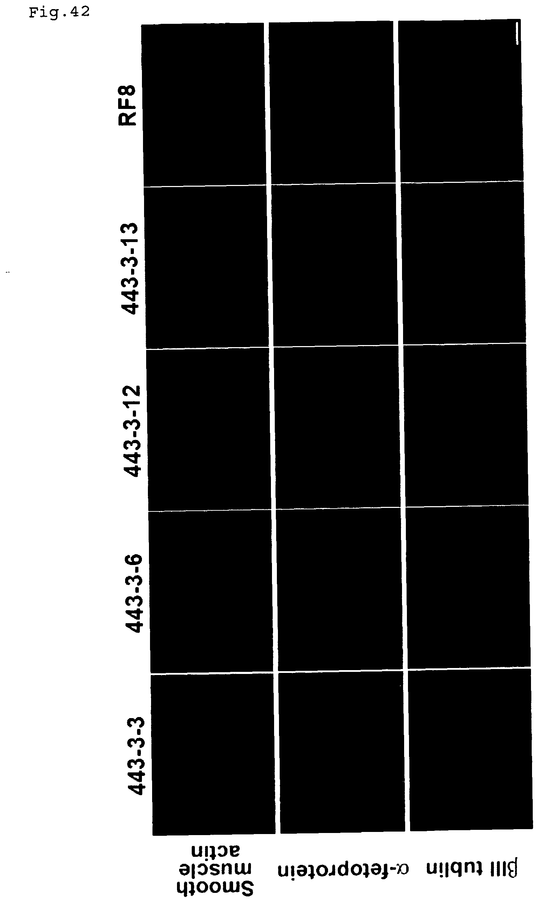

[Fig.42] Fig.42 is a photograph showing results of confirmation that mouse iPS cells (443-3-3, 443-3-6, 443-3-12, and 443-3-13) generated by introduction of three genes (Oct3/4, Klf4, and L-Myc) have the ability to be differentiated into three germ layer cell lines, by staining with anti-α-smooth muscle actin antibody, anti-α-fetoprotein antibody, and anti-βIII-tubulin antibody. RF8 is mouse ES cell as control.

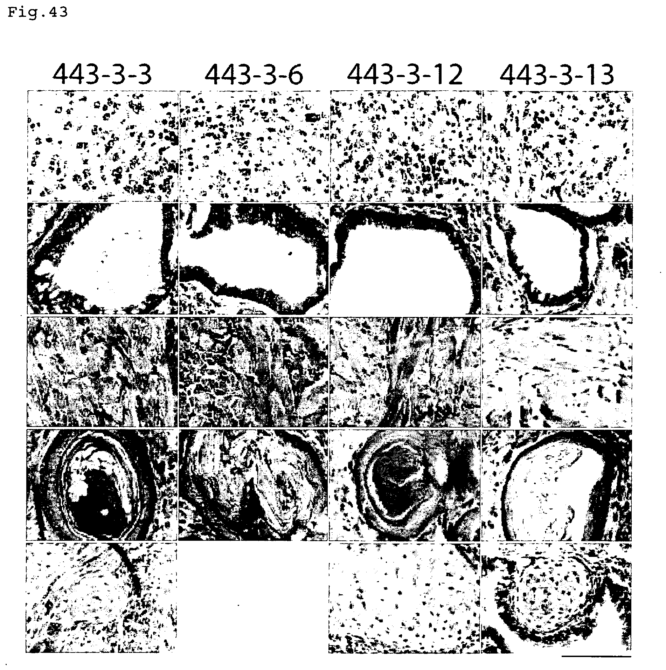

[Fig.43] Fig.43 is histological staining images (hematoxylin-eosin staining) of the teratoma obtained by subcutaneously injecting with mouse iPS cells (443-3-3, 443-3-6, 443-3-12, and 443-3-13) generated by introduction of three genes (Oct3/4, Klf4, and L-Myc). Histological images of the nerve tissue, intestinal tract-like tissue, muscle tissue, epithelium tissue, and cartilage tissue, from the top are shown.

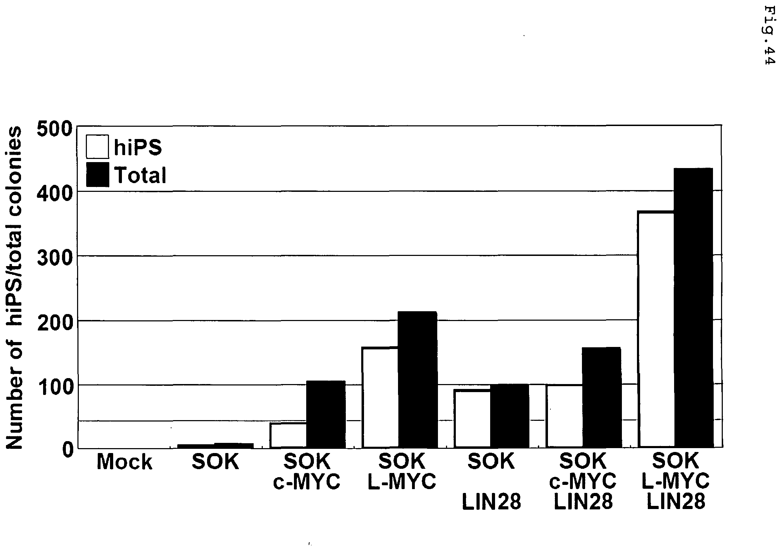

[Fig.44] Fig.44 is a graph showing results of counting the number of colonies of human iPS cells generated by introducing c-Myc, L-Myc, and/or Lin28 genes in addition to three genes of Oct3/4, Klf4, and Sox2 into human adult skin-derived fibroblasts (HDFa-Slc7a1) expressing mouse retrovirus receptor Slc7a with lentivirus vectors. The black bar indicates total number of the colonies, and the white bar indicates the number of iPS cell colonies.

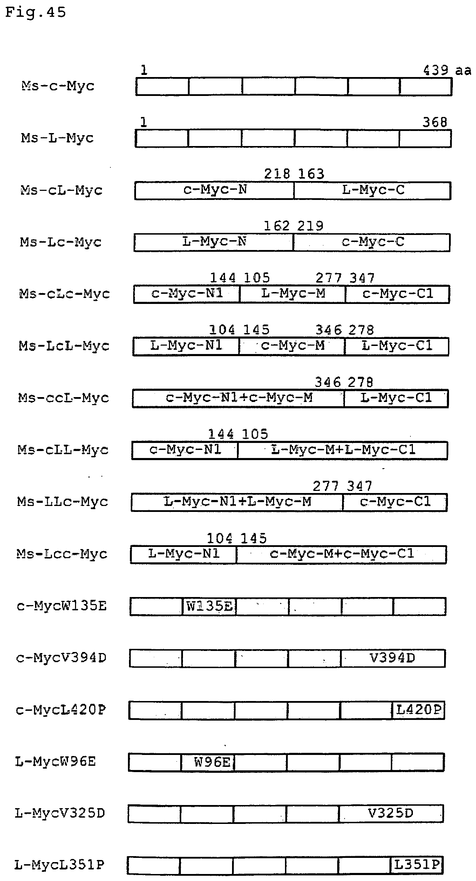

[Fig.45] Fig.45 is a view schematically showing structures of various Myc chimera genes and mutant genes. A nucleotide sequence and an amino acid sequence of Ms-c-Myc are shown in SEQ ID Nos.:153 and 154 of Sequence Listing, a nucleotide sequence and an amino acid sequence of Ms-L-Myc are shown in SEQ ID Nos.:155 and 156 of Sequence Listing, a nucleotide sequence and an amino acid sequence of Ms-cL-Myc are shown in SEQ ID Nos.:157 and 158 of Sequence Listing, a nucleotide sequence and an amino acid sequence of Ms-Lc-Myc are shown in SEQ ID Nos.:159 and 160 of Sequence Listing, a nucleotide sequence and an amino acid sequence of Ms-cLc-Myc are shown in SEQ ID Nos.:161 and 162 of Sequence Listing, a nucleotide sequence and an amino acid sequence of Ms-LcL-Myc are shown in SEQ ID Nos.:163 and 164 of Sequence Listing, a nucleotide sequence and an amino acid sequence of Ms-ccL-Myc are shown in SEQ ID Nos.:165 and 166 of Sequence Listing, a nucleotide sequence and an amino acid sequence of Ms-cLL-Myc are shown in SEQ ID Nos.:167 and 168 of Sequence Listing, a nucleotide sequence and an amino acid sequence of Ms-LLc-Myc are shown in SEQ ID Nos.:169 and 170 of Sequence Listing, a nucleotide sequence and an amino acid sequence of Ms-Lcc-Myc are shown in SEQ ID Nos.:171 and 172 of Sequence Listing, a nucleotide sequence and an amino acid sequence of c-MycW135E are shown in SEQ ID Nos.:173 and 174, a nucleotide sequence and an amino acid sequence of c-MycV394D are shown in SEQ ID Nos.:175 and 176 of Sequence Listing, a nucleotide sequence and an amino acid sequence of c-MycL420P are shown in SEQ ID Nos.:177 and 178 of Sequence Listing, a nucleotide sequence and an amino acid sequence of L-MycW96E are shown in SEQ ID Nos.:179 and 180 of Sequence Listing, a nucleotide sequence and an amino acid sequence of L-MycV325D are shown in SEQ ID Nos.:181 and 182 of Sequence Listing, a nucleotide sequence and an amino acid sequence of LMycL351P are shown in SEQ ID Nos.:183 and 184.

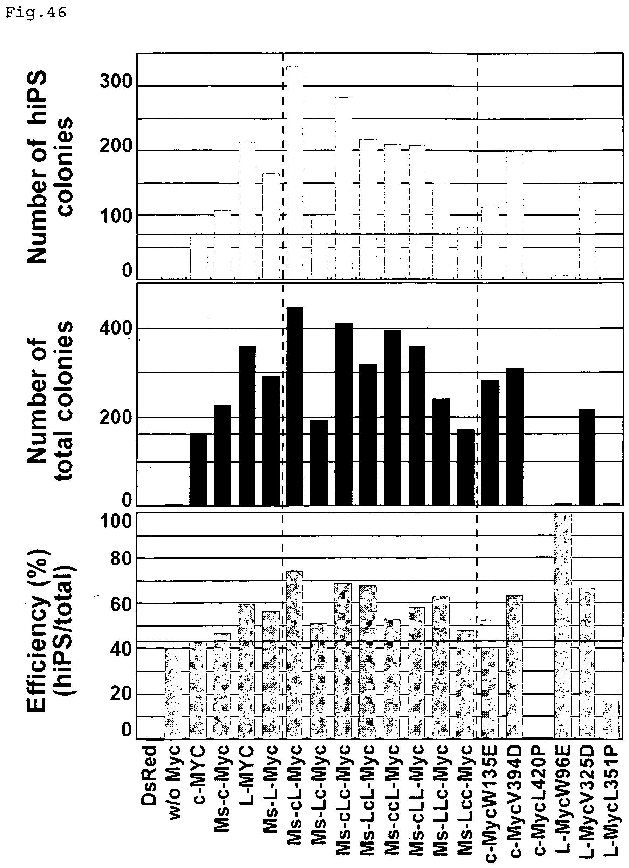

[Fig.46] Fig.46 is a graph showing the results of counting the number of colonies of a human iPS cell established by introducing various Myc chimera genes and mutant genes into human adult skin-derived fibroblasts (HDFa-Slc7a1) expressing mouse retrovirus receptor Slc7a with lentivirus vectors. The number of iPS cell colonies, total colony number, and the ratio (%) of the number of iPS cell colonies to total colony number, from the top, respectively.

### Best Mode for Carrying Out the Invention

The process of the present invention provided from the first viewpoint is a process for generating induced pluripotent stem cells from somatic cells, and comprises the step of introducing the following genes: Oct family gene, Klf family gene, Sox family gene and L-Myc into somatic cells, further comprising the step of introducing Lin28 into somatic cells, and a preferable aspect of the process comprises the step of, after introduction of the following three genes: Oct family gene, Klf family gene, and Sox family gene into somatic cells, culturing the cell over sufficient time for the cells to acquire pluripotency and proliferate.

For the Oct family gene, the Klf family gene, and the Sox family gene, examples of the family genes are shown in International Publication WO2007/69666. As the Oct family gene, Oct3/4 is preferred, as the Klf family gene, Klf4 is preferred and, as the Sox family gene Sox2 is preferred. As these genes, in addition to wild-type genes, mutant genes, in which several (e.g. 1 to 30, preferably 1 to 20, more preferably 1 to 10, further preferably 1 to 5, and particularly preferably 1 or 2) nucleotides are substituted, inserted, and/or deleted, and chimera genes, which are appropriately obtained in combination of the family genes, having the similar function to that of the wild-type genes, or having the improved function, can also be used.

The process of the present invention provided from the second viewpoint is a process for generating induced pluripotent stem cells from somatic cells, and comprises a) the step of introducing Lin28 into somatic cells; and b) the step of introducing a combination of the following two genes: Oct family gene and Sox family gene, and at least one of genes selected from the following three genes: L-Myc, Sall1, and Sall4 into somatic cells, or a combination of the following two genes: Oct family gene and Klf family gene, and at least one of genes selected from the following three genes: L-Myc, Salll, and Sall4 into somatic cells. Without being bound to a specified theory, the gene selected from the group consisting of L-Myc, Salll, and Sall4 is a nuclear reprogramming factor having the activity to enhance an efficiency of nuclear reprogramming and, by using a combination with nuclear reprogramming factors essential for nuclear reprogramming (e.g. a combination of Oct family gene and Sox family gene, or a combination of Oct family gene and Klf family gene), an efficiency of nuclear reprogramming can be remarkably improved.

As more concretely described, the process comprises:
(a) the step of introducing a combination of the following two genes: Oct family gene and Sox family gene, and L-Myc into somatic cells;
(b) the step of introducing a combination of the following two genes: Oct family gene and Sox family gene, and Salll into somatic cells;
(c) the step of introducing a combination of the following two genes: Oct family gene and Sox family gene, and Sall4 into somatic cells;
(d) the step of introducing a combination of the following two genes: Oct family gene and Sox family gene, and L-Myc and Salll into somatic cells;
(e) the step of introducing a combination of the following two genes: Oct family gene and Sox family gene, and L-Myc and Sall4 into somatic cells;
(f) the step of introducing a combination of the following two genes: Oct family gene and Sox family gene, and Salll and Sall4 into somatic cells;
(g) the step of introducing a combination of the following two genes: Oct family gene and Sox family gene, and L-Myc, Salll, and Sall4 into somatic cells.
(h) the step of introducing a combination of the following two genes: Oct family gene and Klf family gene, and L-Myc into somatic cells;
(i) the step of introducing a combination of the following two genes: Oct family gene and Klf family gene, and Salll into somatic cells;
(j) the step of introducing a combination of the following two genes: Oct family gene and Klf family gene, and Sall4 into somatic cells;
(k) the step of introducing a combination of the following two genes: Oct family gene and Klf family gene, and L-Myc and Salll into somatic cells;
(l) the step of introducing a combination of the following two genes: Oct family gene and Klf family gene, and L-Myc and Sall4 into somatic cells;
(m) the step of introducing a combination of the following two genes: Oct family gene and Klf family gene, and Salll and Sall4 into somatic cells;
(o) the step of introducing a combination of the following two genes: Oct family gene and Klf family gene, and L-Myc, Salll and into somatic cells; wherein in any of (a) to (o) the process further comprises the step of introducing Lin28 into somatic cells.

Alternatively, in any aspect of the (a) to (g), the step of introducing Klf family gene into somatic cells may be further comprised. Further, in an aspect of any of the (a) to (o), or in an aspect of adding Klf family gene to any aspect of the (a) to (g), the step of introducing Lin family gene and/or Nanog, preferably into somatic cells may be comprised. Further according to the present disclosure, if desired, the step of introducing c-Myc into somatic cells may be comprised.

As the Oct family gene, Oct3/4 is preferred, as the Sox family gene, Sox2 is preferred, and as the Klf family gene, Klf4 is preffered. In addition, as the Lin family gene, Lin28 and Lin28b are known, and Lin28 is preferred. L-Myc is described in Table 1 of International Publication WO2007/69666, and Sall4 is described in Table 4 and Table 5 of the above International Publication. Salll is the ES cell-specific expressing gene, and is known as one of Zinc finger proteins, and is expected to be involved in development of kidney. The NCBI Accession number of Salll is NM_021390 (mouse) and NM_002968 (human). In addition, the NCBI Accession number of Lin28 is NM_145833 (mouse) and NM_024674 (human).

As these genes, in addition to wild-type genes, mutant genes, in which several (e.g. 1 to 30, preferably 1 to 20, more preferably 1 to 10, further preferably 1 to 5, and particularly preferably 1 or 2) nucleotides are substituted, inserted, and/or deleted, and chimera genes obtained by the arbitrary combination of family gene, having the similar function to that of the wild-type genes, or the improved function, can also be used.

For example, as L-Myc or c-Myc, in addition to wild-type genes, mutant genes in which several (e.g. 1 to 30, preferably 1 to 20, more preferably 1 to 10, further preferably 1 to 5, and particularly preferably 1 or 2) nucleotides are substituted, inserted, and/or deleted, or chimera Myc gene having the similar function to that of wild-type genes, or the improved function, can also be used. For Myc gene, means and results for concretely analyzing the function of various Myc chimera genes and Myc point mutation genes. Therefore, by using this analyzing means, mutant c-Myc genes, mutant L-Myc genes, or chimera Myc genes having the desired improved function can be easily selected and used. Preferred examples of the chimera Myc gene include Ms-cL-Myc (SEQ ID Nos.:157 and 158) and Ms-cLc-Mys (SEQ ID Nos.:161 and 162), more preferably Ms-cL-Myc.

Examples of a preferable aspect of this process include processes comprising:
(a-1) the step of introducing Oct3/4, Sox2, and L-Myc into somatic cells;
(a-2) the step of introducing Oct3/4, Sox2, Klf4, and L-Myc into somatic cells;
(b-1) the step of introducing Oct3/4, Sox2, and Salll into somatic cells;
(b-2) the step of introducing Oct3/4, Sox2, Klf4, and Salll into somatic cells;
(c-1) the step of introducing Oct3/4, Sox2, and Sall4 into somatic cells;
(c-2) the step of introducing Oct3/4, Sox2, Klf4, and Sall4 into somatic cells;
(d-1) the step of introducing Oct3/4, Sox2, L-Myc, and Salll into somatic cells;
(d-2) the step of introducing Oct3/4, Sox2, Klf4, L-Myc, and Salll into somatic cells;
(e-1) the step of introducing Oct3/4, Sox2, L-Myc, and Sall4 into somatic cells;
(e-2) the step of introducing Oct3/4, Sox2, Klf4, L-Myc, and Sall4 into somatic cells;
(f-1) the step of introducing Oct3/4, Sox2, Salll, and Sall4 into somatic cells;
(f-2) the step of introducing Oct3/4, Sox2, Klf4, Salll, and Sall4 into somatic cells;
(g-1) the step of introducing Oct3/4, Sox2, L-Myc, Salll, and Sall4 into somatic cells;
(g-2) the step of introducing Oct3/4, Sox2, Klf4, L-Myc, Salll, and Sall4 into somatic cells;
(h-1) the step of introducing Otc3/4, Klf4, and L-Myc into somatic cells;
(i-1) the step of introducing Otc3/4, Klf4, and Salll into somatic cells;
(j-1) the step of introducing Otc3/4, Klf4, and Sall4 into somatic cells;
(k-1) the step of introducing Otc3/4, Klf4, L-Myc, and Salll into somatic cells;
(l-1) the step of introducing Otc3/4, Klf4, L-Myc, and Sall4 into somatic cells;
(m-1) the step of introducing Otc3/4, Klf4, Salll and Sall4 into somatic cells; or
(o-1) the step of introducing Otc3/4, Klf4, L-Myc, Salll, and Sall4 into somatic cells, in the above process; wherein the above (a-1) to (o-1) further comprise the step of introducing Lin28 into somatic cells.

Further, a process comprising:
(a-3) the step of introducing Oct3/4, Sox2, L-Myc, and Lin28 into somatic cells;
(a-4) the step of introducing Oct3/4, Sox2, Klf4, L-Myc, and Lin28 into somatic cells;
(b-3) the step of introducing Oct3/4, Sox2, Sall1, and Lin28 into somatic cells;
(b-4) the step of introducing Oct3/4, Sox2, Klf4, Sall1, and Lin28 into somatic cells;
(c-3) the step of introducing Oct3/4, Sox2, Sall4, and Lin28 into somatic cells;
(c-4) the step of introducing Oct3/4, Sox2, Klf4, Sall4, and Lin28 into somatic cells;
(d-3) the step of introducing Oct3/4, Sox2, L-Myc, Sall1, and Lin28 into somatic cells;
(d-4) the step of introducing Oct3/4, Sox2, Klf4, L-Myc, Salll, and Lin28 into somatic cells;
(e-3) the step of introducing Oct3/4, Sox2, L-Myc, Sall4, and Lin28 into somatic cells;
(e-4) the step of introducing Oct3/4, Sox2, Klf4, L-Myc, Sall4, and Lin28 into somatic cells;
(f-3) the step of introducing Oct3/4, Sox2, Sall1, Sall4, and Lin28 into somatic cells;
(f-4) the step of introducing Oct3/4, Sox2, Klf4, Sall1, Sall4, and Lin28 into somatic cells;
(g-3) the step of introducing Oct3/4, Sox2, L-Myc, Sall1, Sall4, and Lin28 into somatic cells;
(g-4) the step of introducing Oct3/4, Sox2, Klf4, L-Myc, Salll, Sall4, and Lin28 into somatic cells;
(h-2) the step of introducing Oct3/4, Klf4, L-Myc, and Lin28 into somatic cells;
(i-2) the step of introducing Oct3/4, Klf4, Sall1 and Lin28 into somatic cells;
(j-2) the step of introducing Oct3/4, Klf4, and Lin28 into somatic cells;
(k-2) the step of introducing Oct3/4, Klf4, L-Myc, Sall1, and Lin28 into somatic cells;
(1-2) the step of introducing Oct3/4, Klf4, L-Myc, Sall4, and Lin28 into somatic cells;
(m-2) the step of introducing Oct3/4, Klf4, Sall1 Sall4, and Lin28 into somatic cells; or
(o-2) the step of introducing Oct3/4, Klf4, L-Myc, Sall1, Sall4, and Lin28 into somatic cells,
   in the above process is also preferred.

In each of the above processes, a process comprising the step of introducing Nanog together with Lin28 is also preferred. For example, in an aspect of the (a-4), an aspect of using Nanog together with Lin28m i.e. a process comprising the step of introducing Oct3/4, Sox2, Klf4, L-Myc, Lin28, and Nanog into somatic cells or etc is exemplified.

Alternatively, in each of the above processes, the step of introducing L-Myc together with N-Myc into somatic cells may be included.

Further, in each of the above disclosed processes, the step of introducing c-Myc into somatic cells may be optionally included, but in order to substantially reduce or eliminate development of tumor in the cells or tissues obtained after differentiation induction, it is preferred that the step of introducing c-Myc into somatic cells is not included in the above disclosed process.

For an aspect comprising L-Myc, it is preferred to use human somatic cells as somatic cells to be nuclear-reprogrammed. In addition, if a combination of Oct3/4, Sox2, and Klf4, or a combination of Oct3/4, Sox2, Klf4, and L-Myc (or c-Myc instead of L-Myc) is included, the case where is further combined, or the case where both of Sall1 and Sall4 are further combined is preferred. Further, if a combination of Oct3/4, Sox2, Nanog, and Lin28 is included, the case where Salll is further combined, or the case where both of Sall1 and are further combined is preferred. Also for these cases, it is preferred to use human somatic cells as somatic cells to be nuclear-reprogrammed.

The process of the present disclosure provided from the third viewpoint is a process for generating induced pluripotent stem cells from somatic cells, and includes the step of introducing the following six genes: Oct family gene, Klf family gene, Sox family gene, Myc family gene, Lin28, and Nanog into somatic cells, preferably includes the step of introducing the following six genes: Oct3/4, Klf4, Sox2, c-Myc, Lin28, and Nanog into somatic cells.

In each process provided from the first to third viewpoints, the "induced pluripotent stem cell" is a cell having the characterization like to that of ES cell, more specifically, includes undifferentiated cells having pluripotency and the proliferating ability, and this term must not be limitedly construed in any sense, and needs to be construed most broadly. A method for preparing induced pluripotent stem cells by using nuclear reprogramming factors is specifically described in International Publication WO2007/69666, Cell, 126, pp.663-676, 2006 and Cell, 131, pp.861-872, 2007, and a means for separating induced pluripotent stem cells is also specifically described in the above Gazette.

The "somatic cell" to be reprogrammed by the process of the present disclosure means all cells, but not limited to the cells, devoid of pluripotent cells such as ES cell or the like thereof. For example, in addition to somatic cells at embryonic stage, mature somatic cell may be used. Preferably, somatic cells derived from mammal comprising human are used, more preferably, somatic cells derived from mouse and somatic cells derived from primate are used. Particularly preferably, somatic cells derived from human can be used. Examples of the somatic cells include specifically (1) tissue stem cells (somatic stem cells) such as nerve somatic cell, hematopoietic stem cell, mesenchymal stem cell, dental pulp stem cell and the like, (2) tissue precursor cells, and (3) differentiated cells such as lymphocyte, epithelial cell, muscular cell, fibroblast (skin cell etc.), hair cell, liver cell, stomach cell, and mucosa cell. If the induced pluripotent stem cells are used in treating the disease, it is desired to use somatic cells separated from a patient itself, or somatic cells separated from other person having the same type of HLA antigen and, for example, somatic cells involved in the disease and somatic cells involved in disease therapy can be used.

A method for introducing the above genes into somatic cells is not particularly limited, but for example, it is general to use vectors which can express the above genes. A specific means for using retrovirus vectors as vectors is disclosed in International Publication WO2007/69666, Cell, 126, pp.663-676, 2006 and Cell, 131, pp.861-872, 2007, and the case where lentivirus vectors are used as vectors is disclosed in Science, 318, pp.1917-1920, 2007. In addition, since the case where plasmids which are non-virus vectors is used is described in an article of Okita et al. (Science, Published online: October 9, 2008; 10.1126/Science.1164270), a person skilled in the art can select and adopt an appropriate means among them. If the vector is used, two or more genes selected from the aforementioned genes may be integrated into the vector, and several kinds of vectors integrating one gene may be used. When one or two kinds of the aforementioned genes are already expressed in somatic cells to be reprogrammed, expressed one or two kinds of genes may be removed from a combination of the aforementioned genes to be introduced.

When a virus vector, for example, a retrovirus vector is used as a means for introducing the genes into somatic cells, it is preferred that culture supernatant containing each virus is independently prepared by preparing virus vectors containing each gene to be introduced, thereafter, transfecting separate packaging cells with each virus vector, and culturing the cells, a mixture of those culture supernatants is prepared, and is used in gene introduction by infection with a virus containing the gene. By adopting this means, an efficiency of gene introduction can be remarkably enhanced in some cases, and this means is preferred in some cases particularly where nuclear reprogramming is performed without c-Myc. Indeed, it is also possible to transfect single packaging cell with a plurality of virus vectors, prepare culture supernatant containing a plurality of virus vectors, and use it in gene introduction.

If the gene is introduced into somatic cells, expression vectors may be introduced into somatic cells cultured on feeder cells, or expression vectors may be introduced into somatic cells without feeder cells. In order to enhance an introduction efficiency of expression vectors, the latter method is suitable for some cases. As the feeder cell, feeder cells which are used in culturing embryonic stem cells may be appropriately used, and for example, cells, which cells such as primary culture cells of fibroblasts of mouse embryos at 14-15 days after fertilization or STO cell which is fibroblast-derived cell line are treated with drug such as mitomycin C or radiation, can be used. It is preferred that culturing somatic cells after gene introduction is performed on feeder cells. Alternatively, for about several to 30 days after gene introduction, drug selection with puromycin may be added. Indeed, a procedure which can induce iPS cells without drug selection is also known (Example 9 of International Publication WO2007/69666), and it is also preferred to induce iPS cells without drug selection.

By culturing somatic cells with nuclear reprogramming factors introduced therein under an appropriate condition, nuclear reprogramming autonomously proceeds, and induced pluripotent stem cells can be generated from somatic cells. It is preferred that after the genes are introduced into somatic cells, the cells are cultured over sufficient time for the cells to acquire pluripotency and proliferate. When human induced pluripotent stem cells are generated, it is desirable that culturing density of the cells after introduction of expression vectors is set to be lower than that in the case of animal cell culturing. For example, it is preferred to continue culturing at cell density of 1-100 thousands, preferably about 50 thousands per a dish for cell culture.

Usually, the induced pluripotent stem cells can be obtained, for example, by culturing for 25 days or longer by using suitable medium for animal spices of somatic cells, for example, medium for embryonic stem cell (ES cell) (for example, when the gene is introduced into human somatic cells, medium for primate ES cell, preferably human ES cell). In the process provided from the first viewpoint, an efficiency of nuclear reprogramming is reduced as compared with the case where nuclear reprogramming is performed using a combination containing one kind or two kinds or more of c-Myc, L-Myc, N-Myc, Salll, and Sall4 in some cases and, generally, culturing for a long term is necessary for many cases. For example, in this process, it is necessary to continue culturing preferably for 28 days or longer, more preferably 30 days or longer, further preferably 33 days or longer, and particularly preferably 35 days or longer. Specifically, if the gene is introduced into human somatic cells, culturing for 40 days or longer, and further culturing for 45 days or longer become optimal culturing days in some cases. Indeed, according to the process provided from the first viewpoint, contamination of debris cells which becomes to background is eliminated, and accordingly, induced pluripotent stem cell colonis as pure cell population can be obtained, and it becomes possible to obtain induced pluripotent stem cells having extremely high quality for gene expression and differentiation ability. On the other hand, in the process of the present invention provided from the second viewpoint, as a production efficiency of induced pluripotent stem cells is generally sufficiently high, the desired number of induced pluripotent stem cells can be obtained for shorter culturing days, for example, for 15 to 30 days, preferably around 20 days, in some cases.

Whether the genes-introduced cells gain pluripotency can be easily determined by detecting, for example, various markers specific for undifferentiated cell, such as alkaline phosphatase (ALP), SSEA-3, SSEA-4, ABCG-2, and E-cadherin. Types of the markers and assays are concretely and specifically described in the Publication (e.g. Cell, 126, pp.663-676, 2006 and Cell, 131, pp.861-872, 2007 etc.). If nuclear reprogramming is performed using somatic cells having genes in which marker genes such as GFP are integrated at the downstream of promoters of ES cell-specific expression gene, it is possible to identify the induced pluripotent stem cells by using marker (GFP) positive as an indicator.

In addition, proliferation can be generally determined by colony formation, and since it is known that the shape of colony (usually, a cell population composed of about 500-1,000 induced pluripotent stem cells) exhibits characteristic appearance depending on the animal species, a colony formed by proliferation of induced pluripotent stem cells can be easily identified.

For example, it is known that while mouse induced pluripotent stem cells form raised colonies, human induced pluripotent stem cells form flat colonies, and since the shapes of these colonies are extremely similar to those of mouse ES cells and human ES cells, a person skilled in the art can confirm a degree of proliferation of the induced pluripotent stem cells by detecting the colonies of the generated induced pluripotent stem cells. Medium which can maintain undifferentiated property and pluripotency of ES cells, or medium which can not maintain the properties is variously known in the art and, by using a combination of appropriate mediums, the induced pluripotent stem cells can be effectively separated. The differentiation and proliferation ability of the separated induced pluripotent stem cells can be easily confirmed by a person skilled in the art by utilizing confirmation means which is generally used for ES cells.

In the above process, gene introduction can be performed optionally in the absence or presence of cytokines, preferably in the absence of cytokines. In addition, in the above process, somatic cells after gene introduction can be cultured optionally in the absence or presence of cytokines. Examples of the cytokines typically include, but are not limited to, basic fibroblast growth factor (bFGF), stem cell factor (SCF), leukemia inhibitory factor (LIF) and the like, and gene introduction and/or culturing can be performed in the presence or absence of physiological active substances which are classified as a cytokine in the art. Somatic cells after gene introduction may be cultured on feeder cells modified so that it can express the cytokines.

In the above process, nuclear reprogramming may be performed using gene products encoded by the genes devoid of the genes. If the above process is performed using nuclear reprogramming factors which are gene products, the nuclear reprogramming factors and somatic cells may be contacted in the atmosphere where the somatic cells and the induced pluripotent stem cells can be proliferated. More specifically, for example, a means of adding the gene products to medium can be used. One kind or two kinds of gene products of these genes may be introduced into nucleuses by a procedure such as microinjection into a fused protein or a nucleus, and remaining one kind or two kinds of genes may be introduced by an appropriate gene introducing method, for example, a method using a recombinant vector.

The gene product may be a protein itself produced from the gene, or may be in a form of the fused gene product of the protein and other protein or peptide. For example, a fusion protein with a green fluorescent protein (GFP) or a fused gene product with a peptide such as a histidine tag can also be used. In addition, by preparing and using a fusion protein with TAT peptide derived from HIV virus, uptake of nuclear reprogramming factor from the plasma membrane into a cell can be promoted, and it becomes possible to induce reprogramming only by adding fusion proteins to medium, avoiding complicated operation such as gene introduction. Since such a method of preparing the fused gene product is well known to a person skilled in the art, a person skilled in the art can easily design and prepare an appropriate fused gene product depending on the purpose.

In each of the above processes, introduction of one kind or two kinds or more of the genes into somatic cells can be replaced with contacting of low-molecular compounds with somatic cells in some cases. As such low-molecular compounds, for example, low-molecular compounds having the action of promoting expression of one or more genes selected from the group consisting of Oct family gene, Klf family gene, Sox family gene, Myc family gene, Lin family gene, and Salll, Sall4, and Nanog genes can be used, and such low-molecular compounds can be easily screened by a person skilled in the art with using an expression amount of each gene as an indicator.

In the above processes, in addition to defined each gene, genes encoding factors inducing immortalized cell may be further combined. As disclosed in International Publication WO2007/69666, for example, TERT gene, and one or more genes selected from the group consisting of the following genes: SV40 Large T antigen, HPV16 E6, HPV16 E7, and Bmil can be used alone, or in an appropriate combination. Further, in addition to the above genes, one or more genes selected from the group consisting of Fbx15, ERas, ECAT15-2, Tell, and β-catenin may be combined, and/or one or more genes selected from the group consisting of ECAT1, Esg1, Dnmt3L, ECAT8, Gdf3, Sox15, ECAT15-1, Fthl17, Rexl, UTF1, Stella, Stat3, and Grb2 may also be combined. These combinations are specifically described in International Publication WO2007/69666. Indeed, a gene can be used in the process of the present invention is not limited to genes specifically described as above.

In the process of the present disclosure, in addition to other genes which can function as nuclear reprogramming factors, one kind or two or more kinds of genes encoding factors involved in differentiation, development or proliferation, or other factors having the physiological activity may be included. For example, as a means for confirming nuclear reprogramming factors, a method of screening nuclear reprogramming factors described in International Publication WO2005/80598, and a method of specifying specific nuclear reprogramming factors described in International Publication WO2007/69666 can be utilized. A person skilled in the art can screen nuclear reprogramming factors, and utilize for the process of the present invention by referring to these Publications. Alternatively, a method of appropriate modification or variation of the screening method may be used to confirm nuclear reprogramming factors. A person skilled in the art can easily confirm by the method described in the Publications that a combination of genes used in the above process acts as nuclear reprogramming factors.

In the process of the present disclosure, in order to improve an efficiency of generating induced pluripotent stem cells, introduction and/or addition of various generation efficiency improving agents may be performed. Examples of a substance for improving generation efficiency of iPS cells include, but are not limited to, histone deacetylase (HDAC) inhibitors [e.g. low-molecular inhibitors such as valproic acid (VPA) (Nat. Biotechnol., 26(7), pp.795-797, 2008), trichostatin A, sodium lactate, MC 1293 and M344, and nucleic acidic expression inhibitors such as siRNAd and shRNA for HDAC (e.g. HDAC1 siRNA Smarpool (registered trademark, Millipore), HuSH 29mer shRNA Constructs against HDAC1 (OriGene))etc.], and G9a histone methyltransferase inhibitors [e.g. low-molecular inhibitors such as BIX-01294 (Cell Stem Cell, 2, pp.525-528, 2008) etc., and nucleic acid system expression inhibitors such as siRNA and shRNA for G9a (e.g. G9a siRNA (human) (Santa Cruz Biotechnology)) etc.]. The nucleic acid system expression inhibitor may be in a form of expression vectors containing DNA encoding siRNA or shRNA.

Use of the induced pluripotent stem cells generated by the process of the present invention is not particularly limited, but the cells can be used such as in all tests/studies which are performed using ES cells, and therapy of diseases by using ES cell. For example, by treating the induced pluripotent stem cells obtained by the process of the present invention with retinoic acid, growth factor such as EGF, or glucocorticoid, desired differentiated cell (e.g. nerve cell, cardiomyotic cell, hemocyte cell etc.) can be induced, i.e. appropriate tissues can be formed. A differentiated inducing method is specifically described in International Publication WO2007/69666. By returning these obtained differentiation cell or tissue to a patient, stem cell therapy by autocyto transplantation can be attained. For example, induced pluripotent stem cells with high safety can be effectively produced from somatic cells of a patient itself, and cells obtained by making these cells (e.g. cardiomyotic cell, insulin-production cell, or nerve cell) defferentiated can be safely utilized in stem cell transplantation therapy for a variety of diseases such as heart failure, insulin dependent diabetes, Parkinson's disease and spinal cord damage.

Alternatively, for example, after the induced pluripotent stem cells are generated from human somatic cells by the process of the present invention, these induced pluripotent stem cells are differentiation-induced to prepare somatic cells, tissues, or organs, and the physiological activity or toxicity of a compound, a medicament, a toxin and the like for the somatic cells, the tissues, or the organs can also be assessed. Alternatively, after induced pluripotent stem cells are generated from somatic cells of a patient of a specified disease, these induced pluripotent stem cells are differentiation-induced to prepare somatic cells, tissues, or organs, and drug candidate compounds are acted on the somatic cells, the tissues, or the organs to determine therapeutic and/or preventive effect, and accordingly, drug candidate compounds can be screened. Indeed, use of the induced pluripotent stem cells of the present invention is not limited to the aforementioned specified aspects. Examples

The following Examples further specifically illustrate the present invention and disclosure, but the present invention and disclosure is not limited by the following Examples.

### Example 1: Establishment of iPS cells from adult HDF in mouse ES cell medium

A Slc7a1 (mouse retrovirus receptor) gene was introduced into human adult skin-derived fibroblasts (adult HDF) or human neonate preputium-derived fibroblasts (BJ) with lentivirus (referred to as "HDFa-Slc7al", and "BJ-Slc7a1", respectively), HDFa-Slc7a1 or BJ-Slc7a1 was adjusted to 800,000, and then seeded on feeder cells (mitomycin-treated STO cell), and genes were introduced into retrovirus vectors by the following combinations.
1. Oct3/4, Sox2, Klf4, c-Myc, hTERT, Bmil
2. Oct3/4, Sox2, Klf4, c-Myc, hTERT, HPV16 E6, HPV16 E7
3. Oct3/4, Sox2, Klf4, c-Myc, hTERT, HPV16 E7
4. Oct3/4, Sox2, Klf4, c-Myc, hTERT, SV40 Large T antigen
5. Oct3/4, Sox2, Klf4, c-Myc, hTERT, HPV16 E6
6. Oct3/4, Sox2, Klf4, c-Myc

"-" shown in the figure indicates the combination of "6. Oct3/4, Sox2, Klf4, c-Myc" (Oct3/4, Sox2, Klf4, c-Myc, and TERT are derived from human, and Bmil is derived from mouse).

When culturing was continued without drug selection under the culturing condition of mouse ES cells, in dish into which genes had been introduced by the combination 4, a colony considered to be iPS cells emerged on 8 day after virus infection. Also, in other combinations (1 to 3 and 5), iPS cell-like colonies emerged although not clear as compared to the case of the combination 4. Even when only four genes (6) were introduced, no colony emerged, but cells into which only four genes had been introduced under the aforementioned condition clearly exhibited positive for alkaline phosphatase staining (Fig.1).

Similarly, human neonate preputium-derived fibroblasts (BJ) expressing mouse Slc7a1 gene were adjusted to 80,000, and then seeded on feeder cells (mitomycin C-treated STO cells), and the genes were introduced into retrovirus vectors by the following combinations.
1. 4s (Oct3/4, Sox2, Klf4, c-Myc), hTERT, SV40 Large T antigen
2. 4s (Oct3/4, Sox2, Klf4, c-Myc), hTERT, Bmil
3. hTERT, SV40 Large T antigen
4. 4s (Oct3/4, Sox2, Klf4, c-Myc), hTERT, HPV16 E6
5. 4s (Oct3/4, Sox2, Klf4, c-Myc), hTERT, HPV16 E7
6. 4s (Oct3/4, Sox2, Klf4, c-Myc), hTERT, HPV16 E6, HPV16 E7
7. 4s (Oct3/4, Sox2, Klf4, c-Myc), hTERT
8. 4s (Oct3/4, Sox2, Klf4, c-Myc)
9. DsRed
   (Oct3/4, Sox2, Klf4, c-Myc, and TERT are derived from human, and Bmil is derived from mouse).

When culturing was continued for two weeks under the culturing condition of mouse ES cell, the cells into which only four genes (8) had been introduced exhibited positive for alkaline phosphatase staining (Fig.2).

### Example 2: ECAT expression in iPS cells (1)

Whether human iPS cells established from human adult skin-derived fibroblasts (adult HDF) expresses ECAT (ES cell associated transcript) which is a group of genes specifically expressed in ES cells or not was investigated.

iPS cells (clone iPS-HDFaSlc-87E6) derived from adult HDF were seeded on feeder cells (mitomycin C-treated STO cell) which had been cultured on 6-well plates in advance, at a ratio of 5×10⁴ per each well, and cultured for 4 days. The cell was fixed with PBS containing 10% formalin, a fixing solution was removed, this was washed with PBS and, further, 3% BSA-containing PBS was added, followed by allowing to stand at room temperature for 45 minutes. A primary antibody (anti-human ABCG-2 antibody (mouse IgG), anti-SSEA-3 antibody (rat IgM), and anti-SSEA-4 antibody (mouse IgG)) were diluted 1:100 with 3% BSA-containing PBS, they were reacted at 4°C overnight, the cells were washed with 1% BSA-containing PBS at three times, and a secondary antibody which had been diluted 1:300 with 1% BSA-containing PBS was used to react them under light shielding at room temperature for 1 hour. As the secondary antibody, anti-mouse IgG antibodies (antibody to ABCG-2 and SSEA-4; Chemicon) labeled with Cy-3, and anti-rat IgM antibodies (antibody to SSEA-3; Jackson ImmunoResearch) were used. The cell was washed with PBS and, then, observed and photographed with a microscope (Fig.3). As a result, it was observed that adult HDF-derived iPS cells express ABCG-2, SSEA-3 and SSEA-4.

### Example 3: ECAT expression in iPS cells (2)

iPS cells (clone iPS-HDFaSlc-87E3, 87E4, and 87E12) derived from human adult skin-derived fibroblasts (adult HDF) were seeded on feeder cells (mitomycin C-treated STO cells) which had been seeded on 6-well plates in advance, at a ratio of 5×10⁴ per each well, and cultured for 5 days. As a control, HDF which are cells derived from the iPS cells were seeded on the 6-well plate, and maintained for 2 days. The cells were fixed with PBS containing 10% formalin. After fixing solution was removed, and the cells were washed with PBS, blocking buffer (3% BSA-containing PBS) was added to the cells, and this was allowed to stand at room temperature for 45 minutes. After reacted with a primary antibody (anti-human ABCG-2 antibody (mouse IgG; diluted 1:80 with blocking buffer), anti-E-cadherin (mouse IgG; diluted 1:80 with blocking buffer), anti-SSEA-3 antibody (rat IgM; diluted 1:250 with blocking buffer), and anti-SSEA-4 antibody (mouse IgG; diluted 1:250 with blocking buffer)) at 4°C overnight, the cells were washed with the blocking buffer. After washing, the cells were further reacted with secondary antibodies at room temperature for 1 hour. As the secondary antibodies, an anti-mouse IgG antibody (antibody to ABCG-2, E-cadherin, and SSEA-4) labeled with Cy-3, which had been diluted 1:300 with the blocking buffer, and an anti-rat IgM antibody (antibody to SSEA-3) were used. After the reaction with the secondary antibody, antibody solution was removed, this was washed with PBS, PBS containing 50% glycerol was added to the cell, and this was observed (Fig.4).

iPS cells derived from human adult skin-derived fibroblasts (adult HDF) expressing SSEA-3, SSEA-4, ABCG-2, and E-cadherin which are surface markers of ES cells. To the contrary, HDFa which are cells derived from iPS cells did not express any of SSEA-3, SSEA-4, ABCG-2, and E-cadherin.

### Example 4: ECAT expression in iPS cells (3)

Total RNA was isolated from human iPS cell clones (iPS-HDFaSlc87E-1 to 8, 11 and 12), NTERA2 clone D1 human embryonic cancer cell (passage number 35), and adult HDF (passage number 6) expressing mouse Slc7a1 gene. The first strand cDNA was synthesized with oligo-dT20 primer and Rever Tra Ace-α-kit (Toyobo) according to a protocol of a manufacturer. PCR was performed with primers as follows. For endogeneous OCT3/4, hOct3/4-S1165 and hOct3/4-AS1283 were used; for endogeneous Sox2, hSox2-S1430 and hSox2-AS1555 were used; for Nanog, ECAT4-macaca-968S and ECAT4-macaca-1334AS were used; for REX1, hRex1-RT-U and hRex1-RT-L were used; for FGF4, hFGF4-RT-U and hFGF4-RT-L were used; for GDF3, hGDF3-S243 and hGDF3-AS850 were used; for ECAT15-1, hECAT15-S532 and hECAT15-AS916 were used; for ECAT15-2, hECAT15-2-S85 and hECAT15-2-AS667 were used; for ESG1, hpH34-S40 and hpH34-AS259 were used; for hTERT, hTERT-S3556 and hTERT-AS3713 were used; as well as, for G3PDH, G3PDH-F and G3PDH-R were used (Table 1; SEQ ID Nos.: 1 to 22 of Sequence Listing)

As a result, many Human iPS cell clones (iPS-HDFaS1c87E-1 to 8, 11 and 12) expressed ECAT and, particularly, 87E6 clone expressed various ECATs (Fig.5).

**[Table 1]**

| Primer | Sequence (5' to 3') | Applications |
|---|---|---|
| hOct3/4-S1165 | GAC AGG GGG AGG GGA GGA GCT AGG | Endo Oct3/4 RT-PCR |
| hOct3/4-AS1283 | CTT CCC TCC AAC CAG TTG CCC CAA AC | |
| hSox2-S1430 | GGG AAA TGG GAG GGG TGC AAA AGA GG | Endo Sox2 |
| hSox2-AS1555 | TTG CGT GAG TGT GGA TGG GAT TGG TG | RT-PCR |
| ECAT4(Nanog)-macaca-968S | CAG CCC CGA TTC TTC CAC CAG TCC C | Nanog RT-PCR |
| ECAT4(Nanog)-macaca-1334AS | CGG AAG ATT CCC AGT CGG GTT CAC C | |
| hREXI-RT-U | CAG ATC CTA AAC AGC TCG CAG AAT | REX1 RT-PCR |
| hREXI-RT-L | GCG TAC GCA AAT TAA AGT CCA GA | |
| hFGF4-RT-U | CTA CAA CGC CTA CGA GTC CTA CA | FGF4 RT-PCR |
| hFGF4-RT-L | GTT GCA CCA GAA AAG TCA GAG TTG | |
| hGDF3-S243 | CTT ATG CTA CGT AAA GGA GCT GGG | GDF3 RT-PCR |
| hGDF3-AS850 | GTG CCA ACC CAG GTC CCG GAA GTT | |
| hECAT15-1-S532 | GGA GCC GCC TGC CCT GGA AAA TTC | DPPA4 RT-PCR |
| hECAT15-1-AS916 | TTT TTC CTG ATA TTC TAT TCC CAT | |
| hECAT15-2-S85 | CCG TCC CCG CAA TCT CCT TCC ATC | DPPA2 RT-PCR |
| hECAT15-2-AS667 | ATG ATG CCA ACA TGG CTC CCG GTG | |
| hpH34-S40 | ATA TCC CGC CGT GGG TGA AAG TTC | ESG1 RT-PCR |
| hpH34-AS259 | ACT CAG CCA TGG ACT GGA GCA TCC | |
| hTERT-S3234 | CCT GCT CAA GCT GAC TCG ACA CCG TG | hTERT RT-PCR |
| hTERT-AS3713 | GGA AAA GCT GGC CCT GGG GTG GAG C | |
| G3PDH-F | ACC ACA GTC CAT GCC ATC AC | G3PDH PCR |
| G3PDH-R | TCC ACC ACC CTG TTG CTG TA | |

### Example 5: ECAT expression in iPS cells (4)

Whether human iPS cells derived from human neonate preputium-derived fibroblasts (BJ fibroblast) expresses ECAT or not was confirmed.

Total RNA was isolated from human iPS cells (iPS-BJSlc-97E-1, 2, 4, 5, 6, 7, 8, 10, 11, 12, -97G-3, 5, -97H-3, and 5), NTERA2 clone D1 human embryonic cancer cell (passage number 35), and neonate preputium-derived fibroblast (BJ) (passage number 6) expressing mouse Slc7a1 gene. The first strand cDNA was synthesized with oligo-dT20 primer and Rever Tra Ace-α-kit (Toyobo) according to a protocol of a manufacturer. PCR was performed with primers as follows. For endogeneous OCT3/4, hOct3/4-S1165 and hOct3/4-AS1283 were used; for endogeneous Sox2, hSox2-S1430 and hSox2-AS1555 were used; for Nanog, ECAT4-macaca-968S and ECAT4-macaca-1334AS were used; for REX1, hRex1-RT-U and hRex1-RT-L were used; for FGF4, hFGF4-RT-U and hFGF4-RT-L were used; for GDF3, hGDF3-S243 and hGDF3-AS850 were used; for ECAT15-1, hECAT15-S532 and hECAT15-AS916 were used; for ECAT15-2, hECAT15-2-S85 and hECAT15-2-AS667 were used; for ESG1, hpH34-S40 and hpH34-AS259 were used; for hTERT, hTERT-S3556 and hTERT-AS3713 were used; as well as for G3PDH, G3PDH-F and G3PDH-R were used (Table 2: SEQ ID Nos.: 23 to 44 of Sequence Listing).

As a result, many Human iPS cell clones (iPS-BJSlc-97E-1, 2, 4, 5, 6, 7, 8, 10, 11, 12, -97G-3, 5, -97H-3, and 5) expressed ECAT (Fig.6).

**[Table 2]**

| Primer | Sequence (5' to 3') | Applications |
|---|---|---|
| hOct 3/4-S1165 | GAC AGG GGG AGG GGA GGA GCT AGG | Endo Oct3/4 RT-PCR |
| hOct 3/4-AS1283 | CTT CCC TCC AAC CAG TTG CCC CAA AC | |
| hSox2-S1430 | GGG AAA TGG GAG GGG TGC AAA AGA GG | Endo Sox2 RT-PCR |
| hSox2-AS1555 | TTG CGT GAG TGT GGA TGG GAT TGG TG | |
| ECAT4(Nanog)-macaca-968S | CAG CCC CGA TTC TTC CAC CAG TCC C | Nanog RT-PCR |
| ECAT4(Nanog)-macaca-1334AS | CGG AAG ATT CCC AGT CGG GTT CAC C | |
| hREXI-RT-U | CAG ATC CTA AAC AGC TCG CAG AAT | REX1 RT-PCR |
| hREXI-RT-L | GCG TAC GCA AAT TAA AGT CCA GA | |
| hFGF4-RT-U | CTA CAA CGC CTA CGA GTC CTA CA | FGF4 RT-PCR |
| hFGF4-RT-L | GTT GCA CCA GAA AAG TCA GAG TTG | |
| hGDF3-S243 | CTT ATG CTA CGT AAA GGA GCT GGG | GDF3 RT-PCR |
| hGDF3-AS850 | GTG CCA ACC CAG GTC CCG GAA GTT | |
| hECAT15-1-S532 | GGA GCC GCC TGC CCT GGA AAA TTC | DPPA4 RT-PCR |
| hECAT15-1-AS916 | TTT TTC CTG ATA TTC TAT TCC CAT | |
| hECAT15-2-S85 | CCG TCC CCG CAA TCT CCT TCC ATC | DPPA2 RT-PCR |
| hECAT15-2-AS667 | ATG ATG CCA ACA TGG CTC CCG GTG | |
| hpH34-S40 | ATA TCC CGC CGT GGG TGA AAG TTC | ESG1 RT-PCR |
| hpH34-AS259 | ACT CAG CCA TGG ACT GGA GCA TCC | |
| hTERT-S3234 | CCT GCT CAA GCT GAC TCG ACA CCG TG | hTERT RT-PCR |
| hTERT-AS3713 | GGA AAA GCT GGC CCT GGG GTG GAG C | |
| G3PDH-F | ACC ACA GTC CAT GCC ATC AC | G3PDH PCR |
| G3PDH-R | TCC ACC ACC CTG TTG CTG TA | |

### Example 6: Formation of teratoma by human iPS cells

5.0×10⁶ human iPS cells were subcutaneously injected into back side of SCID mouse (female, 5 week old). On five weeks after injection, big tumor was observed. Tumor was excised, weight was measured, and appearance was photographed. This tumor was fixed with PBS containing 10% formalin. The tumor embedded in paraffin was sliced at 4.5µm sections, and the resulting thin sections were placed on a glass slide, air-dried, and then stained with hematoxylin-eosin. A of Fig.7 indicates mouse subcutaneously injected with iPS-HDFaSlc-87E12 clones, and teratoma thereof. B of Fig.7 indicates histological image derived from teratoma excised from mouse subcutaneously injected with clone iPS-HDFaSlc-87E3, Fig. 7C indicates that with a clone iPS-HDFaSlc-87E6, and Fig. 7D indicates that with a clone iPS-HDFaSlc-87E12.

### Example 7: In vitro differentiation of human iPS cells

By performing floating culture, embryoid bodis (EBs) were formed, and the differentiatiion ability of human iPS cells in vitro was assessed. Human iPS cells (iPS-HDFaSlc-127F2, E3) were floating-cultured for 7 days to form embryoid bodies. Thereafter, the embryoid bodies were transferred to gelatin-coated plates, culturing was further continued for 8 days and, thereafter, immunocytochemical analysis was performed. Primary antibodies used were as follows. Anti-α-smooth muscle actin antibody (Dako), anti-βIII-tubulin antibody (Chemicon), anti-α-fetoprotein antibody (Dako), normal mouse IgG (2 mg/ml, Chemicon), and normal rabbit IgG (2 mg/ml, Chemicon) were diluted 1:100 with 3% BSA-containing PBS, respectively, and these were used as primary antibodies. After the primary antibody was reacted at room temperature for 1 hour, the cells were washed with PBS, and reacted with secondary antibodies (diluted 1:300 with 3% BSA-containing PBS). In addition, nucleuses were stained with DAPI. As a result, α-smooth muscle actin (α-SMA, mesoderm marker), βIII tubulin (ectoderm marker), and α-fetoprotein (endoderm marker) exhibit positive, and accordingly, it was confirmed that human iPS cells are differentiated in vitro via formation of embryoid bodies (Fig.8).

### Example 8: Optimization for gene introduction with retrovirus for generating human iPS cells

For inducing iPS cells from mouse fibroblasts, a retrovirus having high gene introduction efficiency is considered to be effective (Takahashi et al., Cell, 126, pp.663-676, 2006). Consequently, gene introduction method of human adult skin-derived fibroblasts (adult HDF) was optimized. First, a green fluorescent protein (GFP) was introduced into adult HDF with an amphotropic retrovirus prepared in PLAT-A packaging cells. As a control, by using an ecotropic retrovirus prepared in PLAT-E packaging cells (Morita et al., Gene Ther., 7, pp.1063-1066, 2000), GFP was introduced into mouse embryonic fibroblasts (MEF). In MEF, 80% or more of cells expressed GFP (Fig.9). On the other hand, less than 20% of adult HDF was clearly lower in the expression intensity of GFP than that in the case of MEF, and GFP expression rate was less than 20%.

For improving gene introduction efficiency, receptor Slc7a1 (Verrey et al., Pflugers Arch., 447, pp.532-542, 2004) (also known as mCAT1) of mouse retrovirus was introduced into adult HDF with a lentivirus. Next, GFP was introduced into HDFa-Slc7a1 with an ecotropic retrovirus. By this method, gene introduction efficiency of 60% was attained (Fig.9).

### Example 9: Preparation of iPS cells from adult HDF by using primate ES cell medium

An outline of a protocol for inducing human iPS cells is shown in Fig.10A. Human Oct3/4, Sox2, Klf4, and c-Myc were introduced into HDF-Slc7a1 with a retrovirus vector (Fig.10B, 8×10⁵ cells/100 mm dish).

On 6 days after gene introduction, the cells were collected by trypsin treatment, adjusted to 5×10⁴ or 5×10⁵ cells/100 mm dish, and then seeded on mitomycin C-treated SNL feeder cells (McMahon et al., Cell, 62, pp.1073-85, 1990). Next day, medium (DMEM containing 10% FBS) was replaced with primate ES cell medium for primate ES cells (Reprocell Inc.) supplemented with 4 ng/ml of basic fibroblast growth factor (bFGF). After about two weeks, several granuled colonies which are not similar to human ES cells in emerged cell morphology (Fig.10C). On around 25 days, another type of flat colony similar to human ES cell colony was observed (Fig.10D). From 5×10⁴ fibroblasts, about 10 human ES cell-like colonies and about 100 granulated colonies were observed (In six independent experiments, 7/122, 8/84, 8/171, 5/73, 6/122, and 11/213 were observed. Results are summarized in Table 3).

**[Table 3]**

| Experimental number | Parent cell | Number of cells seeded 6 days after gene introduc tion | Number of ES-like colonies | Total colony number | Number of picked up colonies | Number of establ ished clones |
|---|---|---|---|---|---|---|
| 201B | HDF | 50000 | 7 | 129 | 7 | 5 |
| 243H | HFLS | 500000 | 0 | >1000 | | |
| | | 50000 | 17 | 679 | 6 | 2 |
| 246B | HDF | 500000 | 0 | 420 | | |
| | | 500000 | 2 | 508 | | |
| | | 50000 | 8 | 92 | 6 | 6 |
| 246G | BJ | 50000 | 7 | 10 | 6 | 5 |
| | | 500000 | 86 | 98 | | |
| | | 500000 | 106 | 108 | | |
| 249D | HDF | 500000 | 0 | 320 | | |
| | | 500000 | 0 | 467 | | |
| | | 50000 | 8 | 179 | 6 | 4 |
| 253F | HDF | 50000 | 5 | 78 | 3 | 2 |
| | | 50000 | 6 | 128 | 3 | 3 |
| | | 500000 | 10 | 531 | | |
| | | 500000 | 3 | 738 | | |
| 282C | HDF | 50000 | 11 | 224 | 3 | 1 |
| 282H | BJ | 50000 | 13 | 15 | 3 | 2 |
| 282R | HFLS | 5000 | 31 | 98 | 6 | 2 |

day, human ES cell-like colonies were picked up, and mechanically disaggregated into small clamps without enzyme digestion. When started with 5×10⁵ fibroblasts, a dish was almost covered with more than 300 of granuled colonies. Several human ES cell-like colonies were observed between granuled colonies in some cases, but since the granuled colony had a high density, it was difficult to isolate human ES cell-like colonies.

When human iPS cells were proliferated on SNL feeder cells in primate ES cell-like medium containing bFGF, the cells formed flat colonies which were densely aggregated (Fig.10E). Each cell showed the same cell morphology as that of human ES cell characteristic in larger nucleolinus and smaller cytoplasm (Fig.10F). Like the case of human ES cells, spontaneous differentiation was observed in the center of colonies in some cases (Fig.10G). In addition, as observed in human ES cells, these cells exhibited feeder cell dependency, and was not adhered to tissue culturing plate coated with gelatin. On the other hand, these cells maintained the undifferentiated state in the medium for MEF-conditioned primate ES cell (MEF-CM) on matrigel-coated plate, but did not maintain the undifferentiated state in the medium for non-conditioned primate ES cell (Fig.11). Clones of established human iPS cells are summarized in Table 4.

**[Table 4]**

| Clone | Origin | Expression of marker | | Pluipontency | | | |
|---|---|---|---|---|---|---|---|
| | | RT-PCR | IC | EB | PA6 | Cardiomyotic cell | Teratoma |
| 201B1 | HDF | √ | | | | | |
| 201B2 | | √ | √ | √ | √ | √ | |
| 201B3 | | √ | | | | | |
| 201B6 | | | √ | √ | √ | √ | √ |
| 201B7 | | √ | √ | √ | √ | √ | √ |
| 243H1 | HFLS | √ | | √ | | | |
| 243H7 | | √ | | √ | √ | | √ |
| 246B1 | HDF | √ | | | | | |
| 246B2 | | √ | | | | | |
| 246B3 | | √ | | | | | |
| 246B4 | | √ | | | | | |
| 246B5 | | √ | | | | | |
| 246B6 | | √ | | | | | |
| 246G1 | BJ | √ | | √ | √ | | √ |
| 246G3 | | √ | | √ | √ | | |
| 246G4 | | √ | | | | | |
| 246G5 | | √ | | | | | √ |
| 246G6 | | √ | | | | | |
| 253F1 | HDF | √ | | | | | |
| 253F2 | | √ | | | | | |
| 253F3 | | √ | | | | | |
| 253F4 | | √ | | | | | |
| 253F5 | | √ | | | | | |

| | | | | | | | |
|---|---|---|---|---|---|---|---|
| IC: immunocytochemistry, EB: embryoid body | | | | | | | |

### Example 10: Expression of human ES cell markers by human iPS cells

By immunostaining analysis, human iPS cells exhibited expression of human ES cell-specific surface antigens (Adewumi et al., Nat. Biotechnol., 25, pp.803-816, 2007), comprising SSEA-3, SSEA-4, TRA-1-60, TRA-1-81, and TRA-2-49/6E (alkaline phosphatase), as well as Nanog protein (Fig. 10I to N).

It was shown by RT-PCR that human iPS cells express many gene markers (e.g. Oct3/4, Sox2, Nanog, GDF3, REX1, FGF4, ESG1, DPPA2, DPPA4, and (hTERT) etc.) of undifferentiated ES cells at a level equal to or higher than a level in the case of human embryonic cancer cell line NTERA-2 (Fig.12). From results of Western blotting, a protein level of Oct3/4, Sox2, Nanog, Sall4, E-CADHERIN, and hTERT was equal in human iPS cells and human ES cells (Fig.13A). Since expression of introduced gene from a retrovirus incorporated into a chromosome in human iPS cell was effectively silenced, it was suggested that the level is dependent on endogeneous expression of these genes (Fig.13B).

### Example 11: Activity of ES cell-specific gene promoter in human iPS cells

By a bisulfite genomic sequencing method, the state of methylation of cytosine guanine dinucleotide (CpG) in a promoter region of the genes associated with pluipotency of Oct3/4, REX1, and Nanog was assayed and, as a result, it was found out that CpG dinucleotides of that region was highly methlylated in original parent HDF (HDF), while it was highly demethylated in human iPS cells (201B2, 201B6, 201B7) (Fig.14A). From these findings, it was suggested that these promoters are activated in human iPS cells.

Also, in luciferase reporter assay, it was shown that human Oct3/4 and REX1 promoters have the high level transcription activity in human iPS cells, but do not have that in HDF. The promoter activity of gene ubiquitously expressing such as human RNA polymerase II (PolII) exhibited equivalent activity in both of human iPS cells and HDF (Fig.14B).

### Example 12: High telomerase activity and exponential proliferation of human iPS cells

As suggested from high expression level of hTERT, human iPS cells exhibited high telomerase activity (Fig.15A). Human iPS cells were exponentially proliferated for at least 4 months (Fig.15B). A doubling time on calculation of human iPS cells was 46.9±12.4 (clone 201B2), 47.8±6.6 (201B6), and 43.2±11.5 (201B7) hours (Fig.15B). These times were equivalent to the already reported doubling time of human ES cells (Cowan et al., N. Engl. J. Med., 350, pp.1353-56, 2004).

### Example 13: Assay for cross-contamination in HDF-derived human iPS cells

PCR for genome DNA of human iPS cells showed that all clones have incorporation of all four kinds of retroviruses (Fig.16A). By Southern blotting analysis with c-Myc cDNA probe, it was found out that each clone has specific patterns of retrovirus incorporation site (Fig.16B). In addition, patterns of 16 short tandem repeats were completely consistent between human iPS clones and parent HDF. Results of STR analysis of HDF-derived iPS cells are shown in Table 5.

**[Table 5]**

| Locus/ Clone | 201B1 | | 201B2 | | 201B3 | | 201B6 | | 201B7 | | NTERA-2 | | HDF | |
|---|---|---|---|---|---|---|---|---|---|---|---|---|---|---|
| D3S1358 | 15 | 17 | 15 | 17 | 15 | 17 | 15 | 17 | 15 | 17 | 15 | | 15 | 17 |
| TH01 | 5 | | 5 | | 5 | | 5 | | 5 | | 9 | | 5 | |
| D21S11 | 28 | | 28 | | 28 | | 28 | | 28 | | 29 | 30 | 28 | |
| D18S51 | 14 | | 14 | | 14 | | 14 | | 14 | | 13 | | 14 | |
| Penta_E | 7 | 19 | 7 | 19 | 7 | 19 | 7 | 19 | 7 | 19 | 5 | 14 | 7 | 19 |
| D5S818 | 11 | | 11 | | 11 | | 11 | | 11 | | 8 | 11 | 11 | |
| D13S317 | 10 | 14 | 10 | 14 | 10 | 14 | 10 | 14 | 10 | 14 | 14 | | 10 | 14 |
| D7S820 | 9 | 10 | 9 | 10 | 9 | 10 | 9 | 10 | 9 | 10 | 12 | | 9 | 10 |
| D16S539 | 11 | 13 | 11 | 13 | 11 | 13 | 11 | 13 | 11 | 13 | 11 | 16 | 11 | 13 |
| CSF1PO | 10 | | 10 | | 10 | | 10 | | 10 | | 9 | 11 | 10 | |
| Penta_D | 8 | 10 | 8 | 10 | 8 | 10 | 8 | 10 | 8 | 10 | 11 | 12 | 8 | 10 |
| AMEL | X | | X | | X | | X | | X | | X | Y | X | |
| vWA | 15 | 18 | 15 | 18 | 15 | 18 | 15 | 18 | 15 | 18 | 19 | | 15 | 18 |
| D8S1179 | 8 | 10 | 8 | 10 | 8 | 10 | 8 | 10 | 8 | 10 | 13 | 15 | 8 | 10 |
| TPOX | 8 | 9 | 8 | 9 | 8 | 9 | 8 | 9 | 8 | 9 | 8 | 9 | 8 | 9 |
| FGA | 20 | 22 | 20 | 22 | 20 | 22 | 20 | 22 | 20 | 22 | 23 | | 20 | 22 |

These patterns were different from those of established human ES cell lines reported in website of National Institute of Health (http://stemcells.nih.gov/research/scunit/genotyping.htm). In addition, by G horizontal stripe analysis of the chromosome, it was shown that human iPS cells have normal 46XX karyotypes. Therefore, it was concluded that human iPS clones are derived from HDF, and are not due to contamination of ES cells.

### Example 14: Differentiation via embryoid bodies of human iPS cells

In order to determine the differentiation ability of human iPS cells in vitro, floating culture was used to form embryoid bodies (EBs). On 8 days after floating culture, iPS cells formed ball-like structures (Fig.17A). These embryoid body-like structures were transferred to gelatin-coated plates, and culturing was further continued for 8 days. Attached cells showed various cell morphologies such as nerve-like cells, cobblestone-like cells, and epithelial cells (Fig.17B to E). By immunocytechemical analysis, cells positive for βIII tubulin (ecotoderm marker), glia fibril acidic protein (GFAP, ecotoderm), α-smooth muscle actin (α-SMA, mesoderm), desmin (mesoderm), α-fetoprotein (endoderm), and vimentin (mesoderm and endoderm of body wall) were detected (Fig.17F to K). From results of RT-PCR, it was confirmed that FOXA2 (endoderm marker), AFP (endoderm), cytokeratin 8 and 18 (endoderm), SOX 17 (endoderm), BRACHYURY (mesoderm), MSX1 (mesoderm), MAP2 (ecotoderm), and PAX6 (ecotoderm) are expressed in these differentiated cells (Fig.17L). On the other hand, expressions of Oct3/4, Sox2, and Nanog were clearly reduced. From these results, it was shown that iPS cells can be differentiated into three germ layer cells in vitro.

### Example 15: Differentiation of human iPS cells into nerve cells

Whether differentiation from human iPS cells can be induced by the already reported method for human ES cells was determined. Human iPS cells were seeded on PA6 feeder cell layer, and maintained/cultured for 2 weeks (Kawasaki et al., Neuron, 28, pp.31-40, 2000). The cells were greatly broaded, and some nerve structures were observed (Fig.18A). By immunocytochemical analysis, tyrosine hydroxynase and βIII tubulin-positive cells were detected in the culture (Fig.18B). From results of PCR analysis, expression of dopaminergic nerve markers such as AADC, ChAT, DAT, and LMX1B, as well as MAP2 which is other nerve marker were confirmed (Fig.18C). In addition, expression of Oct3/4, Sox2, and Nanog was reduced (Fig.18C). From these results, it was shown that iPS cells can be differentiated into nerve cells containing dopaminergic neuron by coculturing with PA6 cells.

### Example 16: Directed differentiation of human iPS cells into cardiac cells.

According to the reference for differentiation into cardiac cells by using activin A and bone morphogenetic factor (BMP) 4 (Laflamme et al., Nat. Biotechnol., 25, pp.1015-24, 2007), differentiation of human iPS cells into heart was determined. On 12 days after differentiation induction, a cell aggregation starts beating (Fig.18D). From results of RT-PCR, it was shown that these cells express cardiomyotic cell markers such as TnTc, MEF2C, NKX2.5, MYL2A, and MYHCB (Fig.18E). On the other hand, expression of Oct3/4, Sox2, and Nanog was remarkably reduced. From these results, it was shown that human iPS cells can be differentiated into cardiomyotic cells in vitro.

### Example 17: Formation of teratoma from human iPS cells

In order to determine pluripotency in vivo, human iPS cells (clone 201B7) were subcutaneously transplanted into the dorsal flank of an immunodeficient (SCID) mouse. After 9 weeks, tumorigenesis was observed. By histological observation, it was shown that various tissues comprising archenteric canal-like epithelial tissue (ecotoderm), striated muscle (mesoderm), cartilage (muscle), nerve tissue (endoderm), and keratin-containing flat tissue (endoderm) (Fig.19) are contained in tumors.

### Example 18: Preparation of iPS cells from other human somatic cells

In addition to HDF, cell lines (HFLS) established from primary human-derived fibroblast-like synovial cells of adult human synovial tissue and cell lines (BJ cells) established from fibroblasts derived from neonate preputium, were used (Table 3). Preparation of iPS cells from HFLS (5×10⁴ cells/100 mm dish) was examined and, as a result, 600 or more of granuled colonies and 17 human ES cell-like colonies were obtained. When 6 colonies of these 17 colonies were subjected to the determination, only two colonies of them could be proliferated (Fig.20). A dish seeded with 5×10⁵ HFLSs was covered with granuled colonies, and human ES cell-like colonies could not be confirmed. On the other hand, preparation of iPS cells from BJ cells was examined and, as a result, in the case of from 5×10⁴ cells/100 mm dish, 7 to 13 human ES cell-like colonies and slight granuled colonies were recognized, but in the case of from 5×10⁵ /100 mm dish, 100 human ES cell-like colonies were obtained (Table 3). Six human ES cell-like colonies of them were collected, and 5 colonies were confirmed as iPS cells (Fig.20). Human iPS cells derived from HFLS and BJ expressed human ES cell marker genes at a level equal to or higher than a level in the case of human ES cells (Fig.21). These human iPS cells were differentiated into 3 germ layer cells via EBs (Fig.22). By STR analysis, it was confirmed that iPS-HFLS cells and iPS-BJ cells were derived from HFLS cells and BJ cells, respectively. Table 6 shows results of STR analysis of HFLS-derived iPS cells, and Table 7 shows results of STR analysis of BJ-derived iPS cells.

**[Table 6]**

| Locus/ Clone | 243H1 | | 243H7 | | HFLS | |
|---|---|---|---|---|---|---|
| D3S1358 | 16 | 17 | 16 | 17 | 16 | 17 |
| TH01 | 5 | 9 | 5 | 9 | 5 | 9 |
| D21S11 | 28 | 30 | 28 | 30 | 28 | 30 |
| D18S51 | 14 | 17 | 14 | 17 | 14 | 17 |
| Penta_E | 5 | 12 | 5 | 12 | 5 | 12 |
| D5S818 | 10 | 12 | 10 | 12 | 10 | 12 |
| D13S317 | 13 | | 13 | | 13 | |
| D7S820 | 9 | 12 | 9 | 12 | 8 | 12 |
| D16S539 | 11 | 13 | 11 | 13 | 11 | 13 |
| CSF1PO | 10 | 11 | 10 | 11 | 10 | 11 |
| Penta_D | 9 | 11 | 9 | 11 | 9 | 11 |
| AMEL | X | | X | Y | X | Y |
| vWA | 17 | 19 | 17 | 19 | 17 | 19 |
| D8S1179 | 13 | | 13 | | 13 | |
| TPOX | 8 | 11 | 8 | 11 | 8 | 11 |
| FGA | 21 | 22 | 21 | 22 | 21 | 22 |

**[Table 7]**

| Locus/ Clone | 246G1 | | 246G3 | | 246G4 | | 246G5 | | 246G6 | | BJ | |
|---|---|---|---|---|---|---|---|---|---|---|---|---|
| D3S1358 | 13 | 15 | 13 | 15 | 13 | 15 | 13 | 15 | 13 | 15 | 13 | 15 |
| TH01 | 6 | 7 | 6 | 7 | 6 | 7 | 6 | 7 | 6 | 7 | 6 | 7 |
| D21S11 | 28 | | 28 | | 28 | | 28 | | 28 | | 28 | |
| D18S51 | 16 | 18 | 16 | 18 | 16 | 18 | 16 | 18 | 16 | 18 | 16 | 18 |
| Penta_E | 7 | 17 | 7 | 17 | 7 | 17 | 7 | 17 | 7 | 17 | 7 | 17 |
| D5S818 | 11 | | 11 | | 11 | | 11 | | 11 | | 11 | |
| D13S317 | 9 | 10 | 9 | 10 | 9 | 10 | 9 | 10 | 9 | 10 | 9 | 10 |
| D7S820 | 11 | 12 | 11 | 12 | 11 | 12 | 11 | 12 | 11 | 12 | 11 | 12 |
| D16S539 | 9 | 13 | 9 | 13 | 9 | 13 | 9 | 13 | 9 | 13 | 9 | 13 |
| CSF1PO | 9 | 11 | 9 | 11 | 9 | 11 | 9 | 11 | 9 | 11 | 9 | 11 |
| Penta_D | 11 | 12 | 11 | 12 | 11 | 12 | 11 | 12 | 11 | 12 | 11 | 12 |
| AMEL | X | Y | X | Y | X | Y | X | Y | X | Y | X | Y |
| vWA | 16 | 18 | 16 | 18 | 16 | 18 | 16 | 18 | 16 | 18 | 16 | 18 |
| D8S1179 | 9 | 11 | 9 | 11 | 9 | 11 | 9 | 11 | 9 | 11 | 9 | 11 |
| TPOX | 10 | 11 | 10 | 11 | 10 | 11 | 10 | 11 | 10 | 11 | 10 | 11 |
| FGA | 22 | 23 | 22 | 23 | 22 | 23 | 22 | 23 | 22 | 23 | 22 | 23 |

From forgoing results, it was shown that by gene introduction of four genes: Oct3/4, Sox2, Klf4, and c-Myc with a retrovirus, iPS cells can be prepared from adult HDF and other somatic cells. The established human iPS cells were similar to human ES cells in many points comprising cell morphology, proliferation, feeder cell dependency, an expression pattern of surface markers, gene expression, promoter activity, telomerase activity, in vitro differentiation ability, and teratoma forming ability. In addition, four retroviruses were almost completely silenced in human iPS cells. From the above description, it was shown that these cells are completely reprogrammed, and self regeneration does not depend on continuous expression of the transgenes.

Experimental materials and methods in Examples 8 to 18 are as follows.

### 1. Cell culturing

HDFs obtained from the facial skin of 36-year-old Caucasian female, and HFLs obtained from the synovial tissue of 69-year-old Caucasian male were purchased from Cell Applications,Inc. BJ fibroblasts from the neonate preputium, and NTERA-2 clone D1 human embryonic cancer cells were obtained from American Type Culture Collection. Human fibroblast PLAT-E and PLAT-A cells were maintained in Dulbecco's modified Eagle medium (DMEM, Nacalai Tesque) containing 10% bovine fatal serum (FBS), as well as 0.5% penicillin and streptomycin (Invitrogen). 293FT cells were maintained in DMEM containing 10% FBS, 2mM L glutamine (Invitrogen), 1×10⁴ M non-essential amino acid (Invitrogen), and 1mM sodium pyruvate (Sigma), as well as 0.5% penicillin and streptomycin. A PA6 stromal cells (Riken BioResource Center) were maintained in α-MEM containing 10% FBS and 0.5% penicillin and streptomycin. iPS cells were established and maintained in the medium for primate ES cell (ReproCeLL) supplemented with 4 ng/ml recombinant human basic fibroblast growth factor (bFGF, Wako Pure Chemical Industries, Ltd.). In the passage, human iPS cells were washed with PBS once and, thereafter, were incubated in DMEM/F12 containing 1 mg/ml Collagenase IV (Invitrogen) at 37°C. When colonies at the edge of dish start dissociating from the bottom, DMEM/F12/Collagenase was removed and washed with human ES cell medium. The cells were scrapped and collected into 15 ml conical tube. An appropriate volume of the medium was added, and the contents were transferred to SNL feeder cells-harvested on a new dish on. The split ratio was routinely set to 1:3. In order to culture iPS cells under the condition where feeder cells are not contained, plates were coated with 0.3 mg/ml matrigel (BD Biosciences) at 4°C overnight. The plate was warmed to room temperature before use. Unbound matrigel was aspirated off, and washed out with DMEM/F12. iPS cells were seeded on Matrigel-coated plate in MEF-conditioned (MEF-CM) or nonconditioned primate ES cell medium (ReproCELL), both supplemented with 4 ng/ml bFGF. The medium was changed daily. For preparation of MEF-CM, MEFs derived from embryonic day 13.5 embryo pool of ICR mice were plated at 1×10⁶ cells/100 mm dish, and incubated overnight. Next day, the cells were washed once with PBS, and cultured in 10 ml of primate ES cell medium. After culturing for 24 hours, a culture supernatant of MEF culture was collected, filtered through a 0.22 µm pore-size filter, and stored at -20°C until use.

### 2. Plasmid construction

The open reading frame of human Oct3/4 was amplified by RT-PCR, and cloned into pCR2.1-TOPO. An EcoRI fragment in pCR2.1-hOct3/4 was subcloned into the EcoRI site of pMXs retrovirus vector. In order to distinguish each experiment, a 20-bp random sequence named N₂₀ barcode was inserted into the NotI/Sall site in Oct3/4 expression vector. In order to avoid interexperimental contamination, a unique barcode sequence was used in each experiment. The open reading frames of human Sox2, Klf4, and c-Myc were also amplified by RT-PCR, and subcloned into pENTR-D-TOPO (Invitrogen). A full gene subcloned into pENTR-D-TOPO was inserted into pMXs retrovirus vector using Gateway Cloning System (Invitrogen), according to the manufacture's instructions. Mouse Slc7a1 open reading frame was also amplified, subcloned into pENTR-D-TOPO, and inserted into pLenti6/UbC/V5-DEST (Invitrogen) by Gateway System. Regulation regions of human Oct3/4 gene and REX1 gene was amplified by PCR, and subcloned into pCRXL-TOPO (Invitrogen). For PhOCT4-Luc and phREX1-Luc, the KpnI/BglII fragments were removed by KpnI/BglII digestion from pCRXL vector and subcloned into the Kpnl/BglII site in pGV-BM2. For pPolII-Luc, an AatII (blunt end)/NheI fragment of pQBI-polII was inserted into the KpnI (blunt end)/NheI site of pGV-BM2. All of fragments were verified by sequencing. Primer sequences are shown in Table 8 (SEQ ID Nos: 45 to 126 of Sequence Listing).

**[Table 8]**

| Primer | Sequence (5' to 3') | Applications |
|---|---|---|
| hOct3/4-S944 | CCC CAG GGC CCC ATT TTG GTA CC | Oct3/4 Tg PCR |
| hSox2-S691 | GGC ACC CCT GGC ATG GCT CTT GGC TC | Sox2 Tg PCR |
| hKlf4-S1128 | ACG ATC GTG GCC CCG GAA AAG GAC C | Klf4 endo and Tg PCR |
| hMYC-S1011 | CAA CAA CCG AAA ATG CAC CAG CCC CAG | c-Myc Tg PCR |
| pMXs-AS3200 | TTA TCG TCG ACC ACT GTG CTG CTG | Tg PCR |
| pMXs-L3205 | CCC TTT TTC TGG AGA CTA AAT AAA | Tg PCR |
| hOct3/4-S1165 | GAC AGG GGG AGG GGA GGA GCT AGG | Endo Oct3/4 RT-PCR |
| hOct3/4-AS1283 | CTT CCC TCC AAC CAG TTG CCC CAA AC | |
| hSox2-S1430 | GGG AAA TGG GAG GGG TGC AAA AGA GG | Endo Sox2 RT-PCR |
| hSox2-AS1555 | TTG CGT GAG TGT GGA TGG GAT TGG TG | |
| ECAT4-macaca-968S | CAG CCC CGA TTC TTC CAC CAG TCC C | Nanog RT-PCR |
| ECAT4-macaca-1334AS | CGG AAG ATT CCC AGT CGG GTT CAC C | |
| hGDF3-S243 | CTT ATG CTA CGT AAA GGA GCT GGG | GDF3 RT-PCR |
| hGDF3-AS850 | GTG CCA ACC CAG GTC CCG GAA GTT | |
| hREXI-RT-U | CAG ATC CTA AAC AGC TCG CAG AAT | REX1 RT-PCR |
| hREXI-RT-L | GCG TAC GCA AAT TAA AGT CCA GA | |
| hFGF4-RT-U | CTA CAA CGC CTA CGA GTC CTA CA | FGF4 RT-PCR |
| hFGF4-RT-L | GTT GCA CCA GAA AAG TCA GAG TTG | |
| hpH34-S40 | ATA TCC CGC CGT GGG TGA AAG TTC | ESG1 RT-PCR |
| hpH34-AS259 | ACT CAG CCA TGG ACT GGA GCA TCC | |
| hECAT15-1-S532 | GGA GCC GCC TGC CCT GGA AAA TTC | DPPA4 RT-PCR |
| hECAT15-1-AS916 | TTT TTC CTG ATA TTC TAT TCC CAT | |
| hECAT15-2-S85 | CCG TCC CCG CAA TCT CCT TCC ATC | DPPA2 RT-PCR |
| hECAT15-2-AS667 | ATG ATG CCA ACA TGG CTC CCG GTG | |
| hTERT-S3234 | CCT GCT CAA GCT GAC TCG ACA CCG TG | hTERT RT-PCR |
| hTERT-AS3713 | GGA AAA GCT GGC CCT GGG GTG GAG C | |
| hKlf4-AS1826 | TGA TTG TAG TGC TTT CTG GCT GGG CTC C | Endo Klf4 RT-PCR |
| hMYC-S253 | GCG TCC TGG GAA GGG AGA TCC GGA GC | Endo c-Myc RT-PCR |
| hMYC-AS555 | TTG AGG GGC ATC GTC GCG GGA GGC TG | |
| hMSX1-S665 | CGA GAG GAC CCC GTG GAT GCA GAG | MSX1 RT-PCR |
| hMSX1-AS938 | GGC GGC CAT CTT CAG CTT CTC CAG | |
| hBRACHYURY-S1292 | GCC CTC TCC CTC CCC TCC ACG CAC AG | BRACHYURY/T RT-PCR |
| hBRACHYURY-AS1540 | CGG CGC CGT TGC TCA CAG ACC ACA GG | |
| hGFAP-S1040 | GGC CCG CCA CTT GCA GGA GTA CCA GG | GFAP RT-PCR |
| hGFAP-AS1342 | CTT CTG CTC GGG CCC CTC ATG AGA CG | |
| hPAX6-S1206 | ACC CAT TAT CCA GAT GTG TTT GCC CGA G | PAX6 RT-PCR |
| hPAX6-AS1497 | ATG GTG AAG CTG GGC ATA GGC GGC AG | |
| hFOXA2-S208 | TGG GAG CGG TGA AGA TGG AAG GGC AC | FOXA2 RT-PCR |
| hFOXA2-AS398 | TCA TGC CAG CGC CCA CGT ACG ACG AC | |
| hSOX17-S423 | CGC TTT CAT GGT GTG GGC TAA GGA CG | SOX17 RT-PCR |
| hSOX17-AS583 | TAG TTG GGG TGG TCC TGC ATG TGC TG | |
| hAADC-S1378 | CGC CAG GAT CCC CGC TTT GAA ATC TG | AADC RT-PCR |
| hAADC-AS1594 | TCG GCC GCC AGC TCT TTG ATG TGT TC | |
| hChAT-S1360 | GGA GGC GTG GAG CTC AGC GAC ACC | ChAT RT-PCR |
| hChAT-AS1592 | CGG GGA GCT CGC TGA CGG AGT CTG | |
| hMAP2-S5401 | | MAP2 RT-PCR |
| hMAP2-AS5587 | CAC GCT GGA TCT GCC TGG GGA CTG TG | |
| hDAT-S 1935 | ACA GAG GGG AGG TGC GCC AGT TCA CG | SLC6A3/DAT RT-PCR |
| hDAT-AS2207 | ACG GGG TGG ACC TCG CTG CAC AGA TC | |
| hLMX1B-S770 | GGC ACC AGC AGC AGC AGG AGC AGC AG | LMX1B RT-PCR |
| hLMXIB-AS1020 | CCA CGT CTG AGG AGC CGA GGA AGC AG | |
| hMYL2A-S258 | GGG CCC CAT CAA CTT CAC CGT CTT CC | MYL2A RT-PCR |
| hMYL2A-AS468 | TGT AGT CGA TGT TCC CCG CCA GGT CC | |
| hTnTc-S524 | ATG AGC GGG AGA AGG AGC GGC AGA AC | TnTc RT-PCR |
| hTnTc-AS730 | TCA ATG GCC AGC ACC TTC CTC CTC TC | |
| hMEF2C-S1407 | TTT AAC ACC GCC AGC GCT CTT CAC CTT G | MEF2C RT-PCR |
| hMEF2C-AS1618 | TCG TGG CGC GTG TGT TGT GGG TAT CTC G | |
| hMYHCB-S5582 | CTG GAG GCC GAG CAG AAG CGC AAC G | MYHCB RT-PCR |
| hMYHCB-AS5815 | GTC CGC CCG CTC CTC TGC CTC ATC C | |
| dT₂₀ | TTT TTT TTT TTT TTT TTT TT | Reverse transcription |
| hMYC-S857 | GCC ACA GCA AAC CTC CTC ACA GCC CAC | Southern blot probe |
| hMYC-AS1246 | CTC GTC GTT TCC GCA ACA AGT CCT CTT C | |
| hOct3/4-S | CAC CAT GGC GGG ACA CCT GGC TTC AG | Oct3/4 cloning |
| hOct3/4-AS | ACC TCA GTT TGA ATG CAT GGG AGA GC | |
| hSox2-S | | Sox2 cloning |
| hSox2-AS | TCA CAT GTG TGA GAG GGG CAG TGT GC | |
| hKlf4-S | | Klf4 cloning |
| hKlf4-AS | | |
| hMYC-S | | c-Myc cloning |
| hMYC-AS | | |
| Slc7a1-S | CAC CAT GGG CTG CAA AAA CCT GCT CGG | Mouse Slc7al cloning |
| Slc7al-AS | TCA TTT GCA CTG GTC CAA GTT GCT GTC | |
| hREX1-pro5K-S | | Promoter cloning |
| hREXx1-pro5K-AS | | |
| hOct3/4-pro5K-S | | |
| hOct3/4-pro5K-AS | | |
| mehREX1-F1-S | | Bisulfite sequencing |
| mehREX1-F1-AS | CAA ACT ACA ACC ACC CAT CAA C | |
| mehOct3/4 F2-S | | |
| mehOct3/4 F2-AS | | |
| mehNanog-FI-S | TGG TTA GGT TGG TTT TAA ATT TTT G | |
| mehNanog-FI-AS | AAC CCA CCC TTA TAA ATT CTC AAT TA | |

### 3. Preparation and infection of lentivirus

293FT cells (Invitrogen) were plated at 6×10⁶ cells per 100 mm dish and incubated overnight. 293FT cells were transfected with 3 µg of pLenti6/UbC-Slc7a1 along with 9 µg of Virapower packaging mix by using Lipofectamine 2000 (Invitrogen), according to the manufacture's instructions. On next day of transfection, medium changing was performed. Further, after 24 hours, the supernatant of transfectant was collected, and filtered through a 0.45µm pore-size cellulose acetate filter (Whatman). One day before gene introduction, human fibroblasts were seeded at 8×10⁵ cells/100 mm dish. The medium was replaced with virus-containing supernatant supplemented with 4 µg/ml polybrene (Nacalai Tesque), and incubated for 24 hours.

### 4. Retrovirus infection and production of iPS cell

PLAT-E packaging cells were plated at 8×10⁶ cells/100 mm dish, and cultured overnight. Next day, PLAT-E cells were transfected with pMXs vectors with Fugene 6 transfection reagent (Roche). 24 hours after transfection, the medium was replaced with a new medium and, further, after 24 hours, the culture supernatant was collected as virus-containing supernatant. One day before gene introduction, human fibroblasts (HDFa-Slc7a1) expressing mouse Slc7a1 was adjusted to 8×10⁵ cells/100 mm dish, and seeded. The virus-containing supernatants were filtered through a 0.45 µm pore-size filter and supplemented with 4 µm/ml polybrene. Equivalent amounts of supernatants containing each of the four retroviruses were mixed, transferred to the dish of HDFa-Slc7a1, and incubated overnight. After 24 hours, the virus-containing supernatant was removed, and the medium was replaced with a fresh medium. After 6 days, HDFa-Slc7a1 cells were harvested by trypsinization and replated at 5×10⁴ cells/100 mm dish on an SNL feeder layer. Next day, the medium was replaced with primate ES cell medium supplemented with 4 ng/ml bFGF. The medium was changed every other day. 30 days after gene introduction, colonies were picked up, and transferred into 0.2 ml of primate ES cell medium. The colonies were mechanically dissociated to small clamps by mildly pipetting. The cell suspension was transferred on SNL feeder in 24-well plates. This stage was regarded as passage number 1.

### 5. RNA isolation and reverse transcription

Total RNA was purified with Trizol reagent (Invitrogen), and treated with Turbo DNA Free Kit (Ambion) to remove genomic DNA contamination. 1 µg of total RNA was used for reverse transcription reaction with Rever Tra Ace-α (Toyobo) and dT₂₀ primer, according to the manufacture's instructions. PCR was performed with ExTaq (Takara). Quantitative PCR was performed with Platinum SYBR Green qPCR Supermix UDG (Invitrogen), and analyzed with the 7300 Realtime PCR System (Applied Biosystems). Primer sequence are shown in Table 8.

### 6. Alkaline phosphatase staining and immunocytechemical analysis

Alkaline phosphatase staining was performed using the Leukocyte Alkaline Phosphtase Kit (Sigma). For immunocytechemical analysis, cells were fixed with PBS containing 4% paraformaldehyde for 10 minutes at room temperature. After washing with PBS, the cells were treated with PBS containing 5% normal goat or donkey serum (Chemicon), 1% bovine serum albumin (BSA, Nacalai Tesque), and 0.1% Triton X-100 at room temperature for 45 minutes. As a primary antibody, SSEA1 (diluted 1:100, Developmental Studies Hybridoma Bank), SSEA3 (diluted 1:10, gift from Dr.Peter W. Andrews), SSEA4 (diluted 1:100, Developmental Studies Hybridoma Bank), TRA-2-49/6E (diluted 1:20, Developmental Studies Hybridoma Bank), TRA-1-60 (diluted 1:50, gift from Dr.Peter W. Andrews), TRA-1-81 (diluted 1:50, gift from Dr.Peter W. Andrews), Nanog (diluted 1:20, AF1997, R&D Systems), βIII tubulin (diluted 1:100, CB412, Chemicon), glia fibril acidic protein (diluted 1:500, Z0334, Dako), α-smooth muscle actin (diluted, N1584, Dako), desmin (diluted 1:100, Lab Vision), vimentin (diluted 1:100, SC-6260, Santa Cruz), α-fetoprotein (diluted 1:100, MAB1368, R&D Systems), and tyrosine hydroxylase (diluted 1:100, AB152, Chemicon) were used. As a secondary antibody, cyanine3 (Cy3) conjugated goat anti- rat IgM (diluted 1:500, Jackson ImmunoResearch), Alexa 546 conjugated goat anti-mouse IgM (diluted 1:500, Invitrogen), Alexa 488 conjugated goat anti-rabbit IgG (diluted 1:500, Invitrogen), Alexa 488 conjugated donkey anti-goat IgG (diluted 1:500, Invitrogen), Cy3 conjugated goat anti-mouse IgG (diluted 1:500, Chemicon), and Alexa 488 conjugated goat anti-mouse IgM (diluted 1:500, Invitrogen) were used. Nucleuses were stained with 1 µg/ml Hoechst 33342 (Invitrogen).

### 7. In vitro differentiation

Human iPS cells were treated with collagenase IV, transferred to the dish coated with hydroxyethyl methacrylate, and subjected to floating culture using a DMEM/F12 medium containing 20% KSR (Invitrogen), 2 mM L-glutamine, 1×10⁻⁴ M non-essential amino acid, 1×10⁻⁴ M 2-mercaptoethanol (Invitrogen), and 0.5% penicillin and streptomycin, to form embryoid bodies (EBs). The medium was changed every other day. After 8 days as a floating culture, EBs were transferred to gelatin-coated plate, and cultured in the same medium for another 8 days.

For differentiation into dopaminergic neuron, first, PA6 feeder cells were plated on gelatin-coated 6-well plates, and incubated for 4 days to reach confluent. Small clumps of iPS cells were plated on PA6 feeder layer in Glasgow Minimum Essential Medium (Invitrogen) containing 10% KSR (Invitrogen), 1×10⁻⁴ M non-essential amino acid, and 1×10⁻⁴ M 2-mercaptoethanol (Invitrogen), as well as 0.5% penicillin and streptomycin.

For differentiation into a cardiomyotic cell, iPS cells were maintained on Matrigel-coated plate in MEF-CM supplemented with 4 ng/ml bFGF for 6 days. Thereafter, the medium was replaced with RPMI1640 plus B27 supplement (Invitrogen) medium (RPMI/B27), supplemented with 100 ng/ml human recombinant Activin A (R&D Systems) for 24 hours, followed by 10 ng/ml human recombinant bone morphogenetic protein 4 (BMP4, R&D Systems) for 4 days. After stimulation with cytokine, the cells were maintained in RPMI/B27 without any cytokines. The medium was changed every other day.

### 8. Bisulfite sequencing method

Genomic DNA (1 µg) was treated with Cp Genome DNA Modification Kit (Chemicon) according to the method recommended by a manufacturer. Treated DNA was purified with QIA Quick Column (QIAGEN). The promoter regions of the human Oct3/4, Nanog, and Rex1 genes were amplified by PCR. The PCR products were subcloned into pCR2.1-TOPO. Ten clones of each sample were velified by sequencing with the M13 universal primer. Primer sequences used for PCR amplification were shown in Table 8.

### 9. Luciferase assay

Each reporter plasmid (1 µg) containing the firefly luciferase gene was introduced into human iPS cells or HDF with 50 ng of pRL-TK (Promega). 48 hours after gene introduction, the cell were lysed with 1×Passive lysis buffer (Promega), and incubated for 15 minutes at room temperature. Luciferase activities were measured with Dual Luciferase Reporter Assay System (Promega) and Centro LB960 detection system (Barthold) according to the protocol of a manufacturer.

### 10. Formation of teratoma

The cell was harvested by collagenase IV treatment, collected into tubes, and centrifuged, and the pellets were suspended in DMEM/F12. One quarter of the cells from a confluent 100 mm dish were subcutaneously injected to dorsal flank of a SCID mouse (Clea Japan). After 9 weeks, tumors were dissected, weighted, and fixed with PBS containing 4% paraformaldehyde. Paraffin-embedded tissue was sliced, and stained with hematoxylin-eosin.

### 11. Western blotting

The cells at semiconfluent state were lysed with RIPA buffer (50mM Tris-HCl, pH 8.0, 150 mM NaCl, 1% Nonidet P-40 (NP-40), 1% sodium deoxycholate and 0.1% SDS) supplemented with protease inhibitor cocktail (Roche). The cell lysate of MEL-1 human ES cell line was purchased from Abcam. Cell lysates (20 µg) were separated by 8% or 12% SDS polyacrylamide gel electrophoresis, and transferred to a polyvinyldene difluoride membrane (Millipore). The membrane was blocked with TBST (20 mM Tris-HCl, pH 7.6, 136 mM NaCl, and 0.1% Tween-20) containing 1% skim milk and, thereafter, incubated with primary antibody solution at 4°C overnight. After washing with TBST, the membrane was incubated with horseradish peroxidase (HRP)-conjugated secondary antibody for 1 hour at room temperature. Signals were detected with an Immobilon Western chemiluminescent HRP substrate (Millipore) and LAS3000 Imaging System (Fuji Film). As the primary antibody, anti-Oct3/4 (diluted 1:600, SC-5279, Santa Cruz), anti-Sox2 (diluted 1:2000, AB5603, Chemicon), anti-Nanog (diluted 1:200, R&D Systems), anti-Klf4 (diluted 1:200, SC-20691, Santa Cruz), anti-c-Myc (diluted 1:200, SC-764, Santa Cruz), anti-E-cadherin (diluted 1:1000, 610182, BS Bioscience), anti-Dppa4 (diluted 1:500, ab31648 Abcam), anti-FoxD3 (diluted 1:200, AB5687, Chemicon), anti-telomerase (diluted 1:1000, ab23699, Abcam), anti-Sall4 (diluted 1:400, ab29112, Abcam), anti-Lin28 (diluted 1:500, AF3757, R&D Systems), and anti-βactin (diluted 1:5000, A5441, Sigma) were used and, as the secondary antibody, anti-mouse IgG-HRP (diluted 1:3000, #7076, Cell Signaling), anti-rabbit IgG-HRP (diluted 1:2000, #7074, Cell Signaling), and anti-goat IgG-HRP (diluted 1:3000, SC-2056, Santa Cruz) were used.

### 12. Southern blotting

Genomic DNA (5 µg) was digested with BglII, EcoRI, and NcoI overnight. Digested DNA fragments were separated on 0.8% agarose gel, and transferred to a nylon membrane (Amersham). The membrane was reacted with digoxigenin (DIG)-labeled DNA probe in DIG Easy Hyb buffer (Roche) at 42°C overnight with constant agitation. After washing, alkaline phosphatase-conjugated anti-DIG antibody (diluted 1:10000, Roche) was added to the membrane. Signals were raised by CDP-star (Roche) and detected by LAS3000 Imaging System.

### 13. Short tandem repeat analysis and karyotyping

Genomic DNA was used for PCR with Powerplex 16 System (Promega) and analyzed by ABIPRISM 3100 (Genetic analyzer and Gene Mapper v3.5) (Applied Bio Systems). Chromosomal G band analyses were performed at the Nihon Gene Research Laboratories Inc. (Japan).

### 14. Detection of telomerase activity

Telomerase activity was detected with a TRAPEZE telomerase detection kit (Chemicon) according to the manufacture's instructions. The samples were separated by TBE-based 10% acrylamide non-denaturing gel electrophoresis. The gel was stained with SYBR Gold (diluted 1:10000, Invitrogen).

### 15. Chromatin immunoprecipitation assay

Approximately 1×10⁷ cells were crosslinked with 1% formaldehyde for 5 minites at room temperature, and the reaction was stopped by addition of glycine. The cell lysate was sonicated to share chromatin-DNA complex. Immunoprecipitation was performed with Dynabeads Protein G (Invitrogen)-linked anti-trimethyl Lys4 histone H3 (07-473, Upstate), anti-trimethyl Lys27 histone H3 (07-449, Upstate), or normal rabbit IgG antibody. Eluates were used for quantitative PCR as templates.

### 16. DNA microarray

Total RNA from HDF and hiPS cells (clone 201B) was labeled with Cy3. Samples were hybridized with whole human genome microarray 4×44K (G4112F, Agilent). Each sample was hybridized once with the one color protocol. Arrays were scanned with G2565BA microarray scanner system (Agilent), and data was analyzed using GeneSpringGX 7.3.1 Software. Two normalization procedures were applied. First, signal intensities less than 0.01 were set to 0.01. Then, each chip was normailized at 50^{th} percentile of measurements taken from the chip. The microarray data (Tesar et al., Nature, 448, pp.196-199, 2007) of hES H9 cell were retrieved from GEO DataSets (GSM194390, http://www.ncbi.nlm.nih.gov/sites/entrez?db=gds&cmd=search& term=GSE7902). In all three samples, genes with "present" flag value were used for analyses (32,266 genes). The microarray data of HDF and hiPS cells have been deposited in GEO DateSets as accession number GSE9561.

### Example 19: Establishment of iPS cell without c-Myc

iPS clones of mouse iPS cells (Takahashi et al., Cell, 126, pp.663-76, 2006) obtained by introducing Oct3/4, Sox2, Klf4, and c-Myc into mouse fibroblast with retroviruses contain incorporation of a few retroviruses in each gene. Each clone has a total of more than 20 retrovirus incorporation sites, and there is a possibility that a risk of tumorigenesis is increased. In the case of mouse iPS cells, it is known that tumorigenesis occurs in ∼20% of an iPS cell-derived chimera mouse and its progeny (Okita et al., Nature, 448, pp.313-17, 2007), and there is a possibility that re-activation of c-Myc retrovirus increases tumorigenetic occurrence rate in chimera mouse generated by using iPS cells and its progeny mouse. Consequently, a method for establishing iPS cells without c-Myc was studied.

In addition, whether iPS cells can be established by using family genes of four genes of Oct3/4, Sox2, Klf4, and c-Myc was determined. For this purpose, mouse embryonic fibroblasts (MEF) containing a green fluorescent protein (GFP)-IRES-Puro^{r} transgene driven by the Nanog gene regulatory elements were used (Okita et al., Nature, 448, pp.313-17, 2007). Nanog is specifically expressed in mouse ES cells and preimplantation embryos (Chambers et al., Cell, 113, pp.643-655, 2003; Mitsui et al., Cell, 113, pp.631-642, 2003) and can serve as a selection marker during iPS cell induction. When reprogramming is induced, GFP is expressed, and this becomes an index of being an iPS cell. It is shown that Nanog-selected iPS cells are indistinguishable from ES cells, and give rise to germline-competent chimera mice (Wernig et al., Nature, 448, pp.318-324, 2007; Okita et al., Nature, 448, pp.313-17, 2007; Maherali et al., Cell Stem Cell, 1, pp.55-70, 2007).

Oct3/4 belongs to the Oct family of transcription factors, which contain the POU domain (Ryan et al., Genes Dev 11: 1207-25, 1997). A homologue which is closest to Oct3/4 is Oct1 and Oct6. Oct3/4, Oct1 or Oct6 together with remaining 3 genes were introduced into Nanog reporter MEF with a retrovirus. If Oct3/4 is used, many GFP positive colonies were observed (Fig.23a).

Sox2 belongs to the Sox (SRY-associated HMG box) transcription factors, which characterized by the presence of a high-mobility group (HMG) domain (Schepers et al., Dev. Cell, 3, pp.167-170, 2002). Sox1, Sox3, Sox7, Sox15, Sox17, and Sox18 were tested and, when Sox1 was used, GFP positive colonies were obtained. In addition, if Sox3, Sox15, and Sox18 are used, a few GFP-positive colonies were obtained (Fig.23a).

Klf4 belongs to the Kruppel-like factor (Klfs), zinc finger protein that contain an amino acid sequences similar to those of the drosophila embryic pattern regulator Kruppel (Dang et al., Int. J. Biochem. Cell Biol., 32, pp.1103-1121, 2000). Klfl, Klf2, and Klf5 were tested and, if Klf2 is used, GFP-expressing colonies were obtained (Fig.23a). Also if Klfl and Klf5 ware used, iPS cells could be induced.

c-Myc has two related genes (N-Myc and L-Myc) (Adhikary et al., Nat. Rev. Mol. Cell Biol., 6, pp.635-645, 2005). By using N-Myc or L-Myc, GFP positive colonies emerged (Fig.23a). Therefore, it was shown that iPS cells can be induced by family genes of four genes.

Whether iPS cells can be induced from MEF in which βgeo is knocked into the Fbx15 gene locus (Tokuzawa et al., Mol. Cell Biol., 23, pp.2699-2708, 2003) was studied for family genes, and results similar to results obtained by selection based on Nanog were obtained. That is, Sox2 could be replaced by Sox1 or Sox3, Klf4 could be replaced by Klf2, and c-Myc could be replaced by N-Myc and L-Myc. The cell generated by the family genes could be proliferated and showed morphology indistinguishable from that of ES cells, and caused teratomas in nude mice (Fig.24). Therefore, it was shown that these family genes can induce iPS cells from both of Nanog reporter MEFs and a Fbx15 reporter MEFs.

A few ES cell-like GFP-positive colonies were obtained from Nanog reporter MEF without using c-Myc (Fig.23a). Although Okita et al. previously reported that GFP-positive colonies is not obtained without c-Myc (Okita et al., Nature, 448, pp.313-17, 2007), one difference between the method reported in this publication and the aforementioned method is the timing of the drug selection. In the method described in the publication, puromycin selection was initiated 7 days after gene introduction, but drug selection was initiated on 14^{th} day in this method. Either of four genes of Oct3/4, Sox2, Klf4, and c-Myc or three genes devoid of c-Myc was introduced into Nanog reporter MEFs, and puromycin selection was initiated 7 days, 14 days or 21 days after gene introduction (Fig.23b). When four genes were used, GFP-positive colonies were observed in all conditions and, when puromycin selection was delayed, the number of colonies was remarkably increased. In the case of use of without c-Myc, when selection was initiated 7 days after gene introduction, GFP-positive colonies were not observed, but when selection was initiated 14 days or 21 days after gene introduction, GFP-positive colonies emerged. The number of colonies was smaller in the case of using three genes than in the case of using four genes, in each condition. Nanog-selected iPS cells generated by using three genes (Oct3/4, Sox2, Klf4) without the Myc retrovirus expressed ES-cell marker genes at similar levels to those in ES cells (Fig.25) and, gave rise to adult chimera mice when transplanted into blastocysts (Table 9).

**[Table 9]**

| iPS clones | Genotype derived from * | Selection | Injected blastocys | Born mouse | Chimera mouse |
|---|---|---|---|---|---|
| 142B-6 | MEF-FB/GFP | G418 | 39 | 7 | 3 |
| 142B-12 | | | 46 | 12 | 5 |
| 178B-1 | MEF-Ng | Puro | 156 | 50 | 5 |
| 178B-2 | | | 142 | 43 | 17 |
| 178B-5 | | | 60 | 20 | 5 |
| 178B-6 | | | 28 | 10 | 4 |
| 256H- 4 | TTF-ACTB-DsRed | No | 72 | 6 | 5 |
| 256H-13 | | | 96 | 8 | 5 |
| 256H-18 | | | 90 | 17 | 11 |

| | | | | | |
|---|---|---|---|---|---|
| All of iPS clones were induced with three genes without using MYC from MEF or TTF. *FB: Fbx15- βgeo Reporter ; Ng: Nanog-GFP-IRES-Puro^{r} Reporter ; GFP: CAG-EGFP | | | | | |

Another difference is that, when iPS cells are induced using three genes without using c-Myc, fewer GFP negative colonies and lower background cells were observed (Fig.23c). Therefore, the method for inducing iPS cells without using c-Myc has an advantage that induction is slow, and efficient is reduced as compared with induction of iPS cells together with c-Myc, but specificity of iPS cell induction is improved.

A few iPS cells could be produced from MEF in which βgeo was knocked into Fbx15 locus (Tokuzawa et al., Mol. Cell Biol., 23, pp.2699-2708, 2003) without using c-Myc (Fig.26A). Takahashi et al. previously reported that iPS cells could not be obtained without c-Myc (Takahashi et al., Cell, 126, pp.663-676, 2006) and, in the method reported in this publication and the aforementioned method, G418 selection is initiated at the same timing, that is, 3 days after gene introduction. However, the colonies are selected at 14 to 21 days after gene introduction in the method reported in the publication, whereas colony selection is performed at about 30 day after in this experiment. In addition, in this experiment, retroviruses containing four genes (or three genes) were separately prepared using separate independent PLAT-E cells (Morita et al., Gene Ther., 7, pp.1063-1066, 2000). By this operation, a transfection efficiency of retrovirus was improved, and remarkably increase in the number of iPS cell colonies was observed as compared with the already reported method of preparing all of four genes in single PLAT-E cell.

An expression level of ES cell marker genes is lower in Fbx15 selected iPS cells by introduced four genes as compared with ES cells (Takahashi et al., Cell, 126, pp.663-676, 2006). This iPS cell could not produce to adult chimera mouse when microinjected into blastocysts, but iPS cells prepared without using c-Myc expressed ES cell marker genes at the level comparable with a level of ES cells also when Fbx15 selection was done (Fig.26B). In addition, adult chimera mice were obtained at a high iPS cell contribution rate from iPS cells prepared without using c-Myc (Fig.27, Table 9). Increase in an occurrence rate of tumorigenesis was not observed in these chimera mice. That is, in chimera mice derived from iPS cells established with four genes, 6 of 37 chimera mice died of tumor within 100 days after birth, but in chimera mice derived from iPS cells established with three genes without c-Myc, since all 26 chimeras survived within an observation period (shorter than 4 months), it was made clear that the risk of tumorigenesis is reduced without using c-Myc.

Whether iPS cells can be effectively isolated without using c-Myc and without drug selection was investigated. Four genes (Oct3/4, Sox2, Klf4, and c-Myc) or three genes without using c-Myc were introduced into adult tail tip fibroblasts (TTF) containing the Nanog reporter, and culturing was continued without puromycin selection. In order to visualize the cells with genes introduced therein, DsRed retroviruses together with four genes or three genes were introduced. 30 days after retroviral introduction, the dishes with cells introduced with the four genes were covered with many GFP negative colonies and background cells (Fig.28A, Table 10: Nanog-GFP reporter TTF + no drug selection). A numerical value of the parenthesis in Table indicates the number of GFP-positive colonies or clones. A ratio of retroviruses (Oct3/4, Sox2, Klf4, (c-Myc), and DsRed) was 1:1:1: (1):4 in No.256, and 1:1:1: (1):4 in Nos.272 and 309. In No.220, DsRed was not introduced.

**[Table 10]**

| Experimental number | Gene | Number of seeded cells | Total of colonies | Number of picked-up colonies | Number of established colonies |
|---|---|---|---|---|---|
| 220 | 4 | 5x10⁴ | many (107) | 26 (24) | 25(22) |
| 256 | 4 | 5x10⁴ | many (4) | | |
| | 3 | 3.5x10⁵ | 7(4) | 7(4) | 6(5) |
| 272 | 4 | | many (132)' | 6 (6) | 5(4) |
| | 3 | 3.1x10⁵ | 21 (8) | 4(4) | 2(2) |
| 309 | 4 | | many (424) | | |

Using fluorescent microscope, GFP-positive colonies were observed among colonies (4, 132, 424 colonies in three independent experiments). The GFP-positive colonies were negative for DsRed, which was similar to the retroviral silencing observed in iPS cells selected with Nanog (Okita et al., Nature, 448, pp.313-17, 2007). When three genes without using c-Myc were used, colonies were observed as colonies having few background cells among colonies (7, 21, and 43 in three independent experiments). Approximately half of these colonies expressed GFP in a patchy manner. DsRed was detected in only a small portion of some colonies, and indicating that it was largely silenced. Overlapping was not observed between GFP and DsRed. Most of these colonies were expandable and consist of iPS cells, which became posive for GFP and negative for DsRed at passage number of 2. Therefore, it was shown that specifically generation of iPS cells could be enhanced in the case of without drug selection and without using c-Myc. Nanog-GFP is activated and the retroviruses are silenced in the iPS cells.

Preparation of iPS cells was tried from adult TTF that did not have selection markers but had the DsRed transgene driven by a constitutively active promotor (Vintersten et al., Genesis, 40, pp.241-246, 2004). The four genes (Oct3/4, Sox2, Klf4, and c-Myc) or three genes without using c-Myc were introduced into the cells. Further, a GFP retrovirus was introduced to monitor silencing. After 30 days without drug selection, about 1,000 colonies emerged from 0.5×10⁵ cells introduced with the four genes. Most of them were GFP-positive, indicating that retroviral silencing did not take place in these cells. On the other hand, from 3.5×10⁵ cells with three genes without using c-Myc introduced therein, 16 colonies emerged (Fig.28B). Most of these colonies did not express GFP, and the remaining colonies expressed GFP slightly. All of these colonies could be proliferated and, even at a passage number of 2, they showed morphology of an iPS cell (ES cell-like morphology). In addition, it was shown that these cells are all GFP-negative, and retroviral silencing is generated. By RT-PCR, it was shown that in these cells, ES cell marker genes are expressed at a level comparable with that of an ES cells (Fig.28C). Further, in iPS cells prepared by using the three genes, the retroviral silencing of Klf4 and the absence of the c-Myc transgene were confirmed by RT-PCR and, when these iPS cells were transplanted into blastocysts, chimera mouse was generated (Fig.28D and Table 9). From the foregoing results, it was shown that iPS cells having the excellent properties can be obtained without using c-Myc, and iPS cells can be effectively prepared from adult TTF without using drug selection.

Next, retroviruses for Oct3/4, Sox2, and Klf4 were introduced into human neonate preputium-derived fibroblasts (BJ) (clone 246H) or human adult skin-derived fibroblast HDF (253G). After 30 days, a few human iPS cell colonies emerged. These cells showed human ES cell-like colony morphology, and could be proliferated (Fig.29A). When three genes without using c-Myc were introduced (253G), or when c-Myc in addition to the three genes were introduced at the same time (253F), it was confirmed that HDF-derived human iPS cells express ES cell marker genes (Fig.29B). In addition, differentiation of human iPS cells induced without using retrovirus of c-Myc via embryoid bodies was confirmed (Fig.30).

Experimental materials and methods in Example 19 are as follows.

### 1. Plasmid construction

The coding regions of family genes were amplified by RT-PCR with primers listed in Table 11 (SEQ ID Nos.:127 to 152 of Sequence Listing), subcloned into pDONR201 or pENTR-D-TOPO (Invitrogen), and recombined to pMXs-gw by the LR reaction (Invitrogen).

**[Table 11]**

| Genes | Sequences |
|---|---|
| Sox1 | CAC CAT GTA CAG CAT GAT GAT GGA GAC CGA CCT |
| | CTA GAT ATG CGT CAG GGG CAC CGT GC |
| Sox3 | CAC CAT GTA CAG CCT GCT GGA GAC TGA ACT CAA G |
| | TCA GAT GTG GGT CAG CGG CAC CGT TCC ATT |
| Sox7 | CAC CTC GGC CAT GGC CTC GCT GCT GGG |
| | CTC CAT TCC TCC AGC TCT ATG ACA CAC |
| Sox15 | CAC CAT GGC GCT GAC CAG CTC CTC ACA A |
| | TTA AAG GTG GGT TAC TGG CAT GGG |
| Sox17 | CAC CAG AGC CAT GAG CAG CCC GGA TG |
| | CGT CAA ATG TCG GGG TAG TTG CAA TA |
| Sox18 | CAC CAT GCA GAG ATC GCC GCC CGG CTA CG |
| | CTA GCC TGA GAT GCA AGC ACT GTA ATA GAC |
| Oct1 | CAC CAT GAA TAA TCC ATC AGA AAC CAA T |
| | GCT CTG CAC TCA GCT CAC TGT GCC |
| Oct6 | CAC CAT GGC CAC CAC CGC GCA GTA TCT G |
| | GGA ACC CAG TCC GCA GGG TCA CTG |
| Klf1 | CAC CAT GAG GCA GAA GAG AGA GAG GAG GC |
| | TCA GAG GTG ACG CTT CAT GTG CAG AGC TAA |
| Klf2 | CAC CAT GGC GCT CAG CGA GCC TAT CTT GCC |
| | CTA CAT ATG TCG CTT CAT GTG CAA GGC CAG |
| Klf5 | CAC CAT GCC CAC GCG GGT GCT GAC CAT G |
| | TCG CTC AGT TCT GGT GGC GCT TCA |
| L-MycWT | CAC CAT GGA CTT CGA CTC GTA TCA GCA CTA TTT C |
| | TTA GTA GCC ACT GAG GTA CGC GAT TCT CTT |
| N-MycWT | CAC CAT GCC CAG CTG CAC CGC GTC CAC CAT |
| | TTA GCA AGT CCG AGC GTG TTC GAT CT |

### 2. Retrovirus transduction

Retrovirus vectors based on pMXs were transfected into PLAT-E cells (Morita et al., Gene Ther., 7, pp.1063-1066, 2000) using a Fugene6 reagent (Roche) according to instructions of a manufacturer. After 24 hours, the medium was replaced, further, after 24 hours, the virus-containing supernatant was taken, and gene introduction by retrovirus infection was performed. In a "mixed" protocol, the mixture of plasmids for the four genes was transfected into a single dish of PLAT-E cells. In a "separated" method, each plasmid was transfected into separate dishes of PLAT-E cells. The virus-containing supernatant was mixed before gene introduction. In the separate method, significantly high gene introduction efficiency was observed.

### 3. Induction of iPS cells with drug selection.

Induction of iPS cells was performed by modification of the already reported method (Takahashi et al., Cell., 126, pp.663-676, 2006; Okita et al., Nature, 448, pp.313-17, 2007). MEFs, which contained either the Nanog-GFP-IRES-Puro^{r} reporter or the Fbx15-βgeo reporter, or both, were seeded at a ratio of 1.3×10⁵ cells/well (6 well plate) and 8.5×10⁵ cells/well (100mm dish) on SNL feeder cell-harvested 6-well plates and 100 mm dish, respectively, with (McMahon et al., Cell., 62, pp.1073-1085, 1990). The cells with genes introduced therein were cultivated with ES cell medium containing LIF (Meiner et al., Proc. Natl. Acad. Sci. U.S.A., 93, pp.14041-14046, 1996). Selection with G418 (300µg/ml) or puromycin (1.5µg/ml) was initiated as in the already reported method. 25 days to 30 days after gene introduction, the colony number was counted. Some colonies were selected for expansion.

### 4. Induction of iPS cell without using drug selection

TTFs were isolated from adult Nanog reporter mice or adult DsRed-transgenic mice (Vintersten et al., Genesis, 40, pp.241-246, 2004). Retrovirus-containing supernatant was prepared in the separated method. For introducing the four genes, retrovirus-containing supernatants of Klf4, c-Myc, Oct3/4, Sox2, and DsRed were mixed at a ratio of 1:1:1:1:4. When the three genes were introduced, retrovirus containing supernatants of Klf4, Oct3/4, Sox2, Mock (empty vector), and DsRed were mixed at a ratio of 1:1:1:1:4. With the DsRed transgenic mouse, the GFP retrovirus was used instead of DsRed. For transfection, TTFs were seeded at a ratio of 8.0×10⁵ cells per 100 mm dish, which did not have feeder cells. TTFs were incubated in the virus/polybrene-containing supernatant for 24 hours. 4 days after gene introduction, TTFs with three genes introduced therein was reseeded at a ratio of 3.5×10⁵ cells per 100 mm dish with SNL feeder cells, and cultured with ES cell medium. TTFs with four genes introduced therein were reseeded at a ratio of 0.5×10⁵ cells per 100 mm dish with SNL feeder cells. 30 days to 40 days after gene introduction, the colony number was counted. Some colonies were selected for expansion.

### 5. Assessment of iPS cells

RT-PCR and teratoma formation were performed according to the already reported method. For chimera experiments, 15 to 20 iPS cells were injected into BDF1-derived blastocysts, which were then transplanted into the uterus of pseudo-pregnant mice.

### Example 20: Generation of human iPS cells from epithelial cell using six genes.

Using a combination of the following genes: Klf4, c-Myc, Oct3/4, Sox2, Nanog, and Lin28 (NCBI accession number NM_145833 (mouse) or NM_024674 (human)), or a combination devoid of one or two or more of genes from the combination, iPS cells were induced.

6×10⁶ 293 FT cells were seeded on 10 cm petri dishes, and cultured overnight, and these petri dish were transfected with 3 µg of a pLenti6/UbC-Slc7al lentivirus vector using 9 µg of Virapower packaging mix together with lipofectoamine 2000 (Invitrogen). After 24 hours, the medium was replaced with a new medium. Further, after 20 hours, the culture supernatant was taken, and filtered through a 0.45µm pore-size cellulose acetate filter (Whatman). 5×10⁵ epithelial cells were prepared on the previous day. To the petri dishes of the epithelial cell from which the culture supernatant had been removed, was added the filtered culture supernatant with 4 µg/ml polybrene (Nacalai Tesque) added thereto, and the cells were cultured for 24 hours.

1.0×10⁶ PLAT-E cells were seeded on 6 cm petri dishes, and cultured. On the next day, the cells were transfected with 9.0 µg of a retrovirus vector based on pMX containing Klf4, c-Myc, Oct3/4, Sox2, Nanog, and Lin28 with 27 µl of a Fugene6 transfection reagent (Roche). After 24 hours, the medium was replaced with a new medium. Further, on the next day, the supernatant of the PLAT-E cell was collected, and filtered through a 0.45µm pore-size cellulose acetate filter (Whatman). 7 days after lentivirus infection, epithelial cells were reseeded at 3.0×10⁵ /6 cm dish, and the aforementioned culture supernatant containing the retrovirus and polybrene was added thereto.

Results are shown in Table 12. In Table, "6F" indicates 6 genes (Klf4, c-Myc, Oct3/4, Sox2, Nanog, and Lin28), "L" indicates Lin28, "N" indicates Nanog, "M" indicates c-Myc, "O" indicates Oct3/4, "S" indicates Sox2, and "K" indicates Klf4, respectively. "-" before a letter means the case where a gene indicated by a letter subsequent to "-" is removed from the six genes and, for example, "-L" means remaining five genes obtained by removing Lin-28 from six genes, and "-KS" means four genes devoid of Klf4 and Sox2 from six genes. A numerical value in Table indicates the colony number, "non-ES like" indicates a colony number having non-ES like morphology, and "ES like" indicates a colony number having ES-like cell morphology.

**[Table 12]**

| | | | | | | |
|---|---|---|---|---|---|---|
| | Day 23 | | Day 29 | | Dav 23 | |
| | non ES like | ES like | non ES like | ES like | non ES like | ES like |
| 6F | 59 | 39 | 167 | 42 | 16 | 27 |
| -L | 49 | 5 | 53 | 14 | | |
| -N | 220 | 11 | 216 | | | |
| -M | 2 | 0 | 15 | 47 0 | | |
| -O | 0 | 0 | 0 | 0 | | |
| -S | 491 | 0 | 489 | 0 | | |
| -K | 61 | 0 | 51 | 0 | | |
| -KS | 1206 | 0 | 1305 | 0 | | |
| -KO | 0 | 0 | 0 | 0 | | |
| -KM | 0 | 0 | 0 | 0 | 0 | 0 |
| -KN | 51 | 0 | 57 | 0 | | |
| -KL | 28 | 0 | 41 | 0 | | |
| -so | 0 | 0 | 0 | 0 | | |
| -SM | 0 | 0 | 0 | 0 | | |
| -SN | 188 | 0 | 171 | 0 | | |
| -SL | 112 | 0 | 136 | 0 | | |
| -OM | 0 | 0 | 0 | 0 | | |
| -ON | 0 | 0 | 0 | 0 | | |
| -OL | 0 | 0 | 0 | 0 | | |
| -MN | 3 | 0 | 8 | 0 | | |
| -ML | 0 | 0 | 0 | 0 | | |
| -NL | 98 | 1 | 119 | 9 | 17 | 6 |
| GFP | 0 | 0 | 0 | 0 | | |
| KO | 0 | 0 | 0 | 0 | | |
| KS | 0 | 0 | 0 | 0 | | |

Table 12 shows results of two times experiments. Results of the first experiment shows the colony number of the cells induced by introducing a combination of each gene on 23^{rd} day or 29^{th} day after gene introduction, and results of the second experiment (Day 23 of right column) indicate the colony number in the case of "6F", "-KM", and "-NL". Since the colony number of cells without introduction of Lin-28 like "-L" was smaller than the colony number when Lin-28 was introduced, it was shown that Lin28 is responsible for an important role to improve an generation efficiency of iPS cells.

Further, iPS cell induction was performed using six genes (Klf4, c-Myc, Oct3/4, Sox2, Nanog, and Lin28), and two different combinations of four genes (Klf4, c-Myc, Oct3/4, and Sox2 represented by Y4F in Fig.31, and Oct3/4, Sox2, Nanog, and Lin28 represented by T4F in Fig.31). T4F is the same combination as that disclosed in Yu et al., Science, 318, pp.1917-1920, 2007. In Fig.31, "ES-like" indicates the number of colonies morphologically similar to that of colonies of ES cells, and "total" indicates a total of the number of ES-like colonies and the number of non-ES-like colonies. Exp#1, Exp#2, Exp#3, and Exp#4 indicate results of separate experiments. In these experiments, iPS cell colonies having morphology similar to those of ES-like cell colonies were obtained by using six genes and a combination of four genes of Y4F. However, in a combination of T4F, formation of colonies having morphology similar to that of ES-like cell colonies was not recognized.

### Example 21: Effective production of iPS cell using Sall4

An experiment was performed using mouse embryonic fibroblasts (MEF) and human adult dermal fibroblasts (adult HDF) and, as a result, it was found out that induction of iPS cells by using three genes (Klf4, Oct3/4, and Sox2) increases a generation efficiency of iPS cells when Sall4 is added to this combination, that is, when Klf4, Oct3/4, Sox2, and Sall4 are used (Figs.32 and 33). When Sall4 was added to the four genes (Klf4, Oct3/4, Sox2, and c-Myc), more iPS cell colonies were observed. From these experiments, it was shown that an efficiency of inducing iPS cells can be improved by adding Sall4 to the nuclear reprogramming factors.

### Example 22: Effect of promoting iPS cell induction of mouse Sall4 and/or mouse Sall1

According to the already reported method (Cell, 131, pp.861-872, 2007), and using a retrovirus, human-derived four genes (Y4F: Oct3/4, Sox2, Klf4, and c-Myc) or three genes (Y3F: Oct3/4, Sox2, and Klf4), and mouse Sall4 (mSall4) gene or mouse Sall1 (mSall1) gene were introduced into adult dermal fibroblasts (adult HDF), which was made to express mouse ecotropic receptor Slc7a gene with a lentivirus.

On 6^{th} day after introduction, HDF was once collected and, thereafter, HDF adjusted at 5×10⁵ was seeded on 1.5×10⁶ STO cells treated with mitomycin C. After the next day, cells were cultured in a medium for culturing primate ES cells (ReproCELL) containing 4 ng/ml of recombinant human bFGF (WAKO). On 32^{nd} day and 40^{th} day after infection, results of counting of the ES cell-like colony number are shown in Fig.34. On 32^{nd} day after infection, the colony number when Y3F+Mock (empty vector) were introduced was 1, and the colony number when Y4F+Mock were introduced was 37, while when Y3F or Y4F and Sall4 were introduced at the same time, 22 colonies were recognized in a Y3F+mSall4-introduced group and 73 colonies were recognized in a Y4F+mSall4-introduced group. When mSall1 was introduced into Y3F or Y4F, 2 colonies were recognized in a Y3F+mSall1-introduced group, and 43 colonies were recognized in a Y4F+mSall1-introduced group, and a difference in the colony number was not significant as compared with the case where only Y3F or Y4F was introduced. Further, when mSall4 and mSall1 were introduced into Y3F or Y4F at the same time, the colony number was 34 in a Y3F+mSall4+mSall1-introduced group, and 79 in a Y4F+mSall4+mSall1-introduced group.

On 40^{th} day after infection, the colony number when Y3F+Mock were introduced was 8, and the colony number when Y4F+ Mock were introduced was 57, while when Y3F or Y4F and mSall4 were introduced at the same time, 62 colonies were recognized in a Y3F+mSall4-introduced group, and 167 colonies were recognized in a Y4F+mSall4-introduced group. When mSall1 was introduced into Y3F or Y4F, 14 colonies were recognized in a Y3F+mSall1-introdued group, and 103 colonies were recognized in a Y4F+mSall1-introduced group. Further, when mSall4 and mSall1 were introduced into Y3F or Y4F at the same time, the colony number was 98 in a Y3F+mSall4+mSall1-introduced group, and 193 in a Y4F+mSall4+mSall1-introduced group. From the foregoing results, it was shown that by introducing mSall4 into Y3F, or introducing mSall4 and mSall1 at the same time, human iPS cells can be introduced at a 20- to 100-fold efficiency. In addition, it was shown that by introducing mSall4 into Y4F, or introducing mSall4 and mSall1 at the same time, human iPS cells can be introduced at a 2- to 4-fold efficiency.

### Example 23: Effect of promoting iPS cell induction of mouse Sall4 and/or mouse Sall1 (2)

According to the already reported method (Cell, 131, pp.861-872, 2007), mouse Sall4 (mSall4) or mouse Sall1 (mSall1) or both of them together with human-derived four genes (T4F: Oct3/4, Sox2, Nanog, and Lin28) or three genes (T3F: Oct3/4, Sox2, and Nanog) were introduced into human adult dermal fibroblasts (HDFa-Slc7a1) which were made to express mouse ecotropic receptor Slc7a with a lentivirus.

On 6^{th} day after introduction, HDF-Slc7a1 was collected once and, thereafter, HDF-Slc7a1 adjusted at 5×10⁵ was seeded on 1.5×10⁶ STO cells treated with mitomycin C. Further, after cultured for 7 days, the cells were cultured in a medium for culturing primate ES cells (ReproCeLL) containing 4 ng/ml recombinant human bFGF (WAKO). On 32^{nd} day and 40^{th} day after infection, results of counting of the ES cell-like colony number are shown in Fig.35. On 32^{nd} day after infection, the colony number when T3F+Mock and T4F+Mock were introduced was 0, while when T3F or T4F and mSall4 were introduced at the same time, two colonies were detected in a T3F+mSall4-introduced group. In a T4F+mSall4-intorduced group, human ES cell-like colony could not be confirmed.

When T3F or T4F and mSall1 were introduced at the same time, 3 colonies were recognized in a T3F+mSall1-introduced group, and 6 colonies were recognized in a T4F+mSall1-introduced group. Further, when mSall4 and mSall1 together with T3F or T4F were introduced at the same time, 3 colonies were recognized in a T3F+mSall4+mSall1-introduced group. In a T4F+mSall4+mSall1-introduced group, human ES-like colonies could not be confirmed. On 40^{th} day after infection, the colony number when T3F+Mock and T4F+Mock were introduced was 0, while when T3F or T4F and mSall4 were introduced at the same time, 6 colonies were recognized in a T3F+mSall4-introduced group, and 2 colonies were recognized in a T4F+mSall4-introduced group.

When T3F or T4F and mSall1 were introduced at the same time, 13 colonies were recognized in a T3F+mSall1-inroduced group, and 22 colonies were recognized in a T4F+mSall1-introduced group. Further, when mSall4 and mSall1 were introduced into T3F or T4F at the same time, 17 colonies were recognized in a T3F+mSall4+mSall1-introduced group, and 11 colonies were recognized in a T4F+mSall4+mSall1-introduced group. In generation of iPS cells by introduction of T3F and T4F, the promoting effect in iPS cell colony formation was recognized by adding mSall4, and mSall1 more effectively induced formation of human iPS cell colony. Further, it was seen that colony formation of human iPS cell can be more promoted by introducing both of mSall1 and mSall4 together with T3F or T4F.

### Example 24: Effect of promoting of iPS cell induction of human Sall4

According to the method described in Cell, 131, pp.861-872, 2007, human adult dermal fibroblasts which was made to express mouse ecotropic receptor Slc7a1 with a lentivirus(HDF-Slc7a1) was prepared and, on the next day, human-derived four genes (Y4F: Oct3/4, Sox2, Klf4, and c-Myc) or three genes (Y3F: Oct3/4, Sox2, and Klf4) and human Sall4 (hSall4) were introduced with a retrovirus. On 6^{th} day after introduction, HDF was collected once and, thereafter, HDF adjusted at 5×10⁵ was seeded on 1.5×10 STO cells treated with mitomycin C. After the next day, the cells were cultured in a medium for culturing primate ES cells (ReproCELL) containing 4 ng/ml recombinant human bFGF (WAKO). On 32^{nd} day and 40^{th} day after infection, results of counting of the ES cell-like colony number are shown in Fig.36.

On 32^{nd} day after infection, the colony number when Y3F+Mock were introduced was 1, and the colony number when Y4F+Mock were introduced was 34, while when Y3F or Y4F and hSall4 were introduced at the same time, 8 colonies were observed in a Y3F+hSall4-introduced group, and 52 colonies were recognized in a Y4F+hSall4-introduced group. On 40^{th} day after infection, the colony number when Y3F+Mock were introduced was 5, and the colony number when Y4F+Mock were introduced was 52, while when Y3F and Y4F and hSall4 were introduced at the same time, 32 colonies were recognized in a Y3F+Sall4-introduced group, and 137 colonies were recognized in a Y4F+hSall4-introduced group. As a result, it was shown that human iPS cell colony can be obtained at a 6- to 8- fold efficiency by introducing hSall4 into Y3F at the same time, and human iPS cell colony can be obtained at a 1.5- to 2.5-fold efficiency by introducing Y4F and hSall4 at the same time.

### Example 25: Effect of L-Myc

### (A) Determination of nuclear reprogramming by introduction of the four genes (Oct3/4, Klf4, Sox2, and L-Myc)

### (1) Effect on human iPS cell induction

Adult skin-derived fibroblasts which were made to express mouse ecotropic virus receptor Slc7a1 gene (aHDF-Slc7a1) were prepared according to the already reported method (Cell, 131, pp.861-872, 2007). aHDF-Slc7a1 was seeded at a ratio of 3×10⁵/60 mm dish and, on the next day, human-derived four genes (total of 4 genes of L-Mycl (hereinafter, simply referred to as "L-Myc"), c-Myc, or N-Myc gene in addition to three genes of Oct3/4, Klf4, and Sox2) were introduced with a retrovirus according to the method described in the aforementioned publication. 6 days after virus infection, cells were collected, and reseeded on MSTO cells (5×10⁵/100 mm dish). From the next day, cells were cultured in a primate ES cell medium (ReproCELL) supplemented with 4 ng/ml of recombinant human bFGF (WAKO) .

The number of human iPS cell colonies which emerged on 37^{th} day after retrovirus infection was counted. A summary of four times experimental results are shown in Table 13 and Fig.37 (Fig.37 is a graph of Table 13, and a numerical value on the graph indicates a ratio of the iPS cell colonies relative to the total number of colonies). By using L-Myc, an efficiency of inducing iPS cell colony became 6-fold higher as compared with c-Myc, and a ratio of the number of iPS cell colonies relative to the number of total colony was also about 2-fold increased in the case of using L-Myc as compared with that of c-Myc. Also when N-Myc was used, increase in the number of iPS cell colonies was recognized as compared with the case of c-Myc. In induction of mouse embryonic fibroblast (MEF)-derived iPS cells, it was reported that there is no difference between the case of using c-Myc and the case of using L-Myc (Nature Biotech., 26, pp.101-106, 2008), but it was made clear that when a human cell is used, an efficiency of inducing iPS cells is improved more remarkably in use of L-Myc than in use of c-Myc.

**[Table 13]**

| **< Number of hiPS colonies >** | | | | |
|---|---|---|---|---|
| | | ES-like | total | % |
| h32 ■ | c-MYC | 3 | 38 | 7.89 |
| | L-MYC | 18 | 35 | 51.4 |
| | N-MYC | 32 | 431 | 7.42 |
| h44 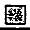 | c-MYC | 11 | 14 | 78.6 |
| | L-MYC | 82 | 100 | 82.0 |
| | N-MYC | 31 | 63 | 49.2 |
| h51 □ | c-MYC | 7 | 69 | 10.1 |
| | L-MYC | 62 | 124 | 50.0 |
| | N-MYC | 25 | 225 | 11.1 |
| h58 □ | c-MYC | 14 | 55 | 25.5 |
| | L-MYC | 53 | 70 | 75.7 |
| | N-MYC | 52 | 298 | 17.4 |

### (2) In vitro differentiation induction

Human iPS cells (32R2, 32R6) established by introduction of the four genes of Oct3/4, Klf4, Sox2, and L-Myc were seeded on a low-binding dish, and embryoid bodies (EB) were formed (100 mm dish) according to the already reported method (Cell, 131, pp.861-872, 2007). After culturing for 2 weeks, staining was performed using each antibody for α-fetoprotein (R&D Systems) which is a differentiation marker of an endoderm cell, α-smooth muscle actin (Wako) which is a differentiation marker of a mesoderm cell, and βIII-tubulin (Chemicon) which is a differentiation marker of an ectoderm cell. Results are shown in Fig.38. By the staining, expression of each marker was confirmed, and it was confirmed that the resulting human iPS cell had the ability of being differentiated into three derm layer cells.

### (3) Teratoma forming ability

Human iPS cells (32R6) established by introduction of the four genes of Oct3/4, Klf4, Sox2, and L-Myc were cultured in a medium for culturing primate ES cells (ReproCeLL) containing recombinant human bFGF (4 ng/ml) and Rho kinase inhibitor Y-27632 (10µM). After 1 hour, the cells were collected by treatment with collagenase IV, and the cells were recovered by centrifugation to suspend them in DMEM/F12 containing Y-27632 (10µM). A 1/4 amount of confluent cells (100 mm dish) were injected into the testis of SCID mouse. After 2 to 3 months, tumor was chopped, and fixed with PBS (-) containing 4% formaldehyde. A paraffin-embedded tissue was sliced, and stained with hematoxylin-eosin. This result is shown in Fig.39. Since tumor is histologically constructed of a plurality of kinds of cells, and nerve tissue, intestinal tract-like tissue, cartilage tissue, hair tissue, fat cell, and pigment tissue were recognized, pluripotency of iPS cells was certified.

### (4) Influence of chimera mouse on tumorigenesis

MEFs were isolated (MEF-Ng) from mouse having Nanog reporter, which was prepared by incorporating EGFP and puromycin resistant gene into Nanog locus (Nature, 448, pp.313-317, 2007). Similarly, MEF was isolated (MEF-Fbx) from a mouse having Fbx15 reporter, which was prepared by incorporating β-geo gene into Fbx15 locus (Cell, 126, pp.663-676, 2006). Four genes (Oct3/4, Klf4, Sox2, and L-Myc) were introduced into these MEFs with a retrovirus and iPS cells were established by selecting with puromycin regarding MEF-Ng, and iPS cells were established by selecting with G418 regarding MEF-Fbx (referred to as Nanog-iPS and Fbx-iPS, respectively). By transplanting these iPS cells into blastocysts of C57BL/6 mouse, chimera mice were generated. Tumorigenesis in chimera mice and F1 mice were studied and, as a result, at this time, mice which were survived for the following days, respectively, and occurrence of tumor was not recognized in any of living mice.

Chimera mouse Fbx-iPS: 19/19 animals living for 349 days Chimera mouse Nanog-iPS: 15/16 animals living for 363 days F1 Nanog-iPS: 1/1 animal living for 255 days F1 Nanog-iPS: 3/3 animals living for 181 days F1 Nanog-iPS: 1/1 animal living for 132 days Chimera mouse Nanog-iPS: 30/30 animals living for 63 days Chimera mouse Nanog-iPS: 12/14 animals living for 62 days

When iPS cells reprogrammed by the four genes of Oct3/4, Klf4, Sox2, and L-Myc was used, 6 of 37 chimera mice died of tumor within 100 days after birth (Nature Biotech., 26, pp.101-106, 2008), however, remarkable improvement was confirmedin survived days by using L-Myc instead of c-Myc, and it was made clear that tumor occurrence is dramatically decreased.

### (B) Determination of nuclear reprogramming by three genes (Oct3/4, Klf4, L-Myc) introduction

### (1) Presence or absence of iPS cell colony formation

Establishment of iPS by introduction of the three genes (Oct3/4, Klf4, and L-Myc) was examined according to the already reported method (Nature Biotech., 26, pp.101-106, 2008). MEF-Ng described in the (A) (1) to (4) was seeded on 6-well culturing plate coated with gelatin at a ratio of 1.3×10⁵/well and, after 24 hours, mouse-derived three genes (Oct3/4, Klf4, and L-Myc) were introduced by using a retrovirus. 4 days after virus infection, cells were collected, and reseeded on MSTO cells (1.5×10⁵/6-well plate). From the next day, cells were cultured using a medium for culturing ES cell with LIF added thereto (DMEM (Nacalai-Tesque) with 15% bovine fetal serum, 2mM L-glutamine (Invitrogen), 100 µM non-essential amino acid (Invitrogen), 100 µM 2-mercaptoethanol (Invitrogen), 50U/mL penicillin (Invitroge), and 50 mg/mL streptomycin (Invitrogen) added thereto). On 46^{th} day after virus infection, GFP-positive colonies were picked up (iPS-MEF-Ng-443-3 clone). Photographs of picked up 16 cells are shown in Fig.40. Any cells were iPS cell colonies exhibiting GFP-positive.

Regarding these GPF-positive colony-derived clones, RT-PCR and genomic-PCR were performed in order to analys gene expression. Results are shown in Fig.41. Any clone expressed Nanog, Rex1, and ECAT1, which are marker genes of ES cell. In addition, in all iPS cell colonies, introduced three genes (Oct3/4, Klf4, and L-Myc) were incorporated into the genomes. On the other hand, expression of mRNA was not detected in some clones, and this is considered that they undergo silencing.

### (2) In vitro differentiation induction

The four clones (iPS-MEF-Ng-443-3-3, iPS-MEF-Ng-443-3-6, iPS-MEF-Ng-443-3-12, and iPS-MEF-Ng-443-3-13) established in the above (1) were seeded on a low-binding dish (2×10⁶/ 6-well plate) to form embryoid bodies (EB). After culturing in a medium for culturing an ES cell for 2 weeks, staining was performed using each antibody against α-fetoprotein (R&D Systems) which is a differentiation marker of the endoderm cell, α-smooth muscle induction (Dako) which is a differentiation marker of the mesoderm cell, and βIII-tubulin (Chemicon) which is a differentiation marker of the ectoderm cell. Results are shown in Fig.42. By staining, expression of these markers was confirmed, and it was confirmed that the resulting mouse iPS cells have the ability of being differentiated into three germ layer cells.

### (3) Teratoma forming ability

The four clones established in the above (1) (iPS-MEF-Ng-443-3-3, iPS-MEF-Ng-443-3-6, iPS-MEF-Ng-443-3-12, and iPS-MEF-Ng-443-3-13) were subcutaneously injected into nude mice, respectively. After four weeks, formation of the teratoma was confirmed in all examples. Since tumor is histologically constructed of a plurality of the cells, and the nerve tissue, intestinal tract-like tissue, muscle tissue, epidermal tissue, and cartilage tissue were recognized (Fig.43), pluripotency of iPS cells was certified.

### Example 26: Effect of L-Myc and Lin28 in induction of human iPS cells

Adult dermal fibroblasts, which were made to express mouse ecotropic virus receptor Slc7a1 gene (aHDF-Slc7a1), were prepared according to the method described in the already reported method (Cell, 131, pp.861-872, 2007). aHDF-Slc7a1 were seeded at a ratio of 3×10⁵/60 mm dish and, on the next day, human-derived the following three genes, four genes, or five genes were introduced with a retrovirus according to the method described in the above publication.
- Sox2, Oct3/4, and Klf4
- Sox2, Oct3/4, Klf4, and c-Myc
- Sox2, Oct3/4, Klf4, and L-Myc
- Sox2, Oct3/4, Klf4, and Lin28
- Sox2, Oct3/4, Klf4, c-Myc, and Lin28
- Sox2, Oct3/4, Klf4, L-Myc, and Lin28

6 days after virus infection, the cells were collected, and reseeded on MSTO cells (5×10⁵/100 mm dish). From the next day, the cells were cultured in a primate ES cell medium (ReproCELL) supplemented with 4 ng/ml of recombinant human bFGF (Wako) to a medium for culturing. After retrovirus infection, the number of the total colonies and the number of hiPS colonies which emerged on 37^{th} day were counted. Results are shown in Table 14 and Fig.44 (Fig.44 is a graph of Table 14).

**[Table 14]**

| | | | | | | | hIPS colonies | Total colonies | hIPS (%) |
|---|---|---|---|---|---|---|---|---|---|
| 1 | Mock | DsRed | Mock | Mock | Mock | A | 0 | 0 | 0 |
| 2 | SOX2 | OCT4 | KLF4 | Mock | Mock | D | 3 | 5 | 60.0 |
| 3 | SOX2 | OCT4 | KLF4 | c-MYC | Mock | B | 38 | 103 | 36.9 |
| 4 | SOX2 | OCT4 | KLF4 | L-MYC | Mock | C | 156 | 211 | 73.9 |
| 5 | SOX2 | OCT4 | KLF4 | Mock | LIN28 | C | 90 | 97 | 92.8 |
| 6 | SOX2 | OCT4 | KLF4 | c-MYC | LIN28 | E | 98 | 154 | 63.6 |
| 7 | SOX2 | OCT4 | KLF4 | L-MYC | LIN28 | F | 366 | 432 | 84.7 |

By adding Lin28 to the four genes of Sox2, Oct3/4, Klf4, and L-Myc, the number of iPS cell colonies was remarkably increased. This number of iPS cell colonies was remarkably more than the case where Lin28 was added to the four genes of Sox2, Oct3/4, Klf4, and c-Myc. In addition, Lin28 exhibited the synergistic effect on the action of c-Myc, and exhibited further strong synergistic effect on the action of L-Myc (Table 14). Also, regarding a ratio of the number of iPS cell colony relative to that of the total colony, extremely high ratio (84.7%) was imparted when five genes of Sox2, Oct3/4, Klf4, L-Myc, and Lin28 were used. From the foregoing results, it was made clear that, in order to improve an efficiency of inducing human iPS cells, a combination of the five genes of Sox2, Oct3/4, Klf4, L-Myc, and Lin28 is extremely effective.

### Example 27: Effect of Myc chimera gene and mutant gene in human iPS cell induction

Various chimera genes (Ms-cL-Myc, Ms-Lc-Myc, Ms-cLc-Myc, Ms-LcL-Myc, Ms-ccL-Myc, Ms-cLL-Myc, Ms-LLc-Myc, Ms-Lcc-Myc), and Myc point mutant genes (c-MycW135E, c-MycV394D, c-MycL420P, L-MycW96E, L-MycV325D, L-MycL351P) shown in Fig.45 were constructed as follows: First, mouse c-Myc(Ms-c-Myc) and L-Myc (Ms-L-Myc) were inserted into pENTR-D-TOPO (Invitrogen), respectively, to prepare pENTR-D-TOPO-Ms-c-Myc and pENTR-D-TOPO-Ms-L-Myc. Next, based on sequence information of mouse c-Myc (NCBI Acc.No. NM_010849) and L-Myc (NCBI Acc.No. NM_008506), primers for introducing point mutation shown in Fig. 45 were prepared, and these were used to perform PCR by using pENTR-D-TOPO-Ms-c-Myc and pENTR-D-TOPO-Ms-L-Myc as a template. After each mutation was confirmed by sequencing, this was subcloned into pMXs of retrovirus vectors by the LR reaction. Regarding chimera Myc shown in Fig.45, each fragment was amplified by PCR, PCR was performed using a mixture as a template depending on each combination, and this was subcloned into pMXs of retrovirus vectors as described above.

The obtained each retrovirus vector above, and each retrovirus vector of Sox2, Oct3/4, and Klf4 used in Example 25 were introduced into adult skin-derived fibroblasts (aHDF-Slc7a1) in the same as in Example 25 and, after retrovirus infection, the number of total colonies and hiPS colonies which emerged on 31^{st} day was counted. Results are shown in Fig.46 and Table 15 (Fig.46 is a graph of Table 15)

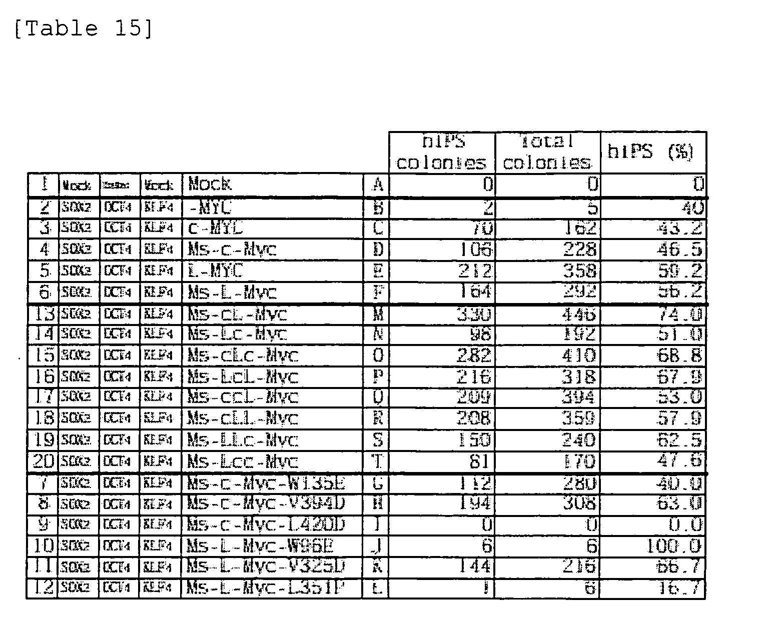

Both of c-Myc and L-Myc have identity near 100% at an amino acid level in a human and a mouse. Since as shown in Fig.46 and Table 15, human iPS cell colonies were generated with human genes (c-Myc and L-Myc) also when mouse genes (Ms-c-Myc and Ms-L-Myc) were used, it was confirmed that mouse c-Myc and L-Myc have substantially the same functions as those of human c-Myc and L-Myc.

As a result of comparing results in various Myc chimera genes and point mutant genes with those of the wild-type, when Ms-cL-Myc and Ms-cLc-Myc were used, the iPS cell colony number is increased as compared with the wild-type L-Myc (L-Myc) and, particularly, when Ms-cL-Myc was used, the best results were obtained. In addition, from results of point mutant genes, it was also made clear that one amino acid (c-Myc420P and L-MycL351P) at a C-terminus is very important in both of c-Myc and L-Myc. From the forgoing results, it was suggested that an important functional region of Myc in establishment of human iPS cells is a part which is 1/3 on the N-terminus of c-Myc, the central part of L-Myc, and the C-terminal part of c/L-Myc.

### Industrial Applicability

According to the present invention, a process for generating the safer induced pluripotent stem cells, and more effective process for generating induced pluripotent stem cells are provided, as defined in the claims.

## Claims

1. A process for generating induced pluripotent stem cells from somatic cells, comprising the step of introducing the following genes: Oct family gene, Klf family gene, Sox family gene and L-Myc into somatic cells, further comprising the step of introducing Lin28 into somatic cells.

2. The process according to claim 1, comprising the step of, after introduction of the said genes into somatic cells, culturing the cells over sufficient time for the cells to acquire pluripotency and proliferate.

3. The process according to claim 1, comprising the step of introducing the following genes: Oct3/4, Klf4, Sox2, L-Myc and Lin28 into somatic cells.

4. The process according to any one of claims 1 to 3, comprising the step of transfecting virus vectors containing Oct family gene, virus vectors containing Klf family gene, and virus vectors containing Sox family gene into separate packaging cells, respectively, and culturing the cells to obtain the three culture supernatants, and the step of preparing a mixture of the three culture supernatants obtained by the above step, and reprogramming somatic cells by using the mixture.

5. The process according to any one of claims 1 to 4, wherein the induced pluripotent stem cells are generated without drug selection.

6. A process for generating induced pluripotent stem cells from somatic cells, comprising
I) a) the step of introducing Lin28 into somatic cells; and b) the step of introducing a combination of the following two genes: Oct family gene and Sox family gene, and an L-Myc gene, into somatic cells, or a combination of the following two genes: Oct family gene and Klf family gene, and an L-Myc gene into somatic cells; or
II) a) the step of introducing Lin28 into somatic cells; and b) the step of introducing a combination of the following three genes: Oct family gene, Sox family gene and Klf gene, and at least one of genes selected from the following two genes: Sall1, and Sall4 into somatic cells.

7. The process according to claim 6, comprising the step of introducing a combination of the following two genes: Oct3/4 and Sox2 or a combination of the following two genes: Oct3/4 and Klf4, and at least one of genes selected from the following three genes: L-Myc, Sall1, and Sall4 into somatic cells, further comprising the step of introducing Lin28 into somatic cells.

8. The process according to claim 6, comprising the step of introducing Oct3/4, Sox2, Klf4, L-Myc, and Lin28 into somatic cells.

9. The process for generating induced pluripotent stem cells from somatic cells according to claim 1, comprising the step of introducing Nanog into somatic cells.

10. The process according to any one of claims 1 to 9, wherein the somatic cells are somatic cells of mammal including human.

11. The process according to any one of claims 1 to 10, wherein the somatic cells are human somatic cells.

12. An induced pluripotent stem cell obtainable by the process as defined in any one of claims 1 to 11.

13. A process for producing somatic cells, comprising a step of producing iPS cells according to a process of any one of claims 1 to 11, and the step of differentiation-inducing the induced pluripotent stem cells.

14. A process for producing a tissue, an organ, or a body fluid, which is a population of somatic cells obtainable by the process as defined in claim 13.

## Patentansprüche

1. Verfahren zum Herstellen von induzierten pluripotenten Stammzellen aus somatischen Zellen, umfassend den Schritt des Einbringens der folgenden Gene: Oct-Familie-Gen, Klf-Familie-Gen, Sox-Familie-Gen und L-Myc in somatische Zellen, weiterhin umfassend den Schritt des Einbringens von Lin28 in somatische Zellen.

2. Verfahren gemäß Anspruch 1, umfassend den Schritt, nach Einbringen der Gene in somatische Zellen, des Kultivierens der Zellen über einen ausreichenden Zeitraum, dass die Zellen Pluripotenz erlangen und proliferieren.

3. Verfahren gemäß Anspruch 1, umfassend den Schritt des Einbringens der folgenden Gene: Oct3/4, Klf4, Sox2, L-Myc und Lin28 in somatische Zellen.

4. Verfahren gemäß einem der Ansprüche 1 bis 3, umfassend den Schritt des Transfizierens von Oct-Familie-Genenthaltenden viralen Vektoren, Klf-Familie-Genenthaltenden viralen Vektoren und Sox-Familie-Genenthaltenden viralen Vektoren jeweils in getrennte Verpackungszellen und Kultivieren der Zellen, um die drei Kulturüberstände zu erhalten, und den Schritt des Herstellens einer Mischung der drei durch den obigen Schritt erhaltenen Kulturüberstände und Reprogrammieren von somatischen Zellen unter Verwendung der Mischung.

5. Verfahren gemäß einem der Ansprüche 1 bis 4, wobei die induzierten pluripotenten Stammzellen ohne Wirkstoffselektion hergestellt werden.

6. Verfahren zum Herstellen von induzierten pluripotenten Stammzellen aus somatischen Zellen, umfassend:
I)
a) den Schritt des Einbringens von Lin28 in somatische Zellen; und
b) den Schritt des Einbringens einer Kombination aus den folgenden zwei Genen: Oct-Familie-Gen und Sox-Familie-Gen, und eines L-Myc-Gens in somatische Zellen, oder einer Kombination aus den folgenden zwei Genen: Oct-Familie-Gen und Klf-Familie-Gen, und eines L-Myc-Gens in somatische Zellen; oder
II)
a) den Schritt des Einbringens von Lin28 in somatische Zellen; und
b) den Schritt des Einbringens einer Kombination aus den folgenden drei Genen: Oct-Familie-Gen, Sox-Familie-Gen und Klf-Gen, und wenigstens eines Gens, ausgewählt aus den folgenden zwei Genen: Sall1 und Sall4, in somatische Zellen.

7. Verfahren gemäß Anspruch 6, umfassend den Schritt des Einbringens einer Kombination aus den folgenden zwei Genen: Oct3/4 und Sox2, oder einer Kombination aus den folgenden zwei Genen: Oct3/4 und Klf4, und wenigstens eines Gens, ausgewählt aus der folgenden drei Genen: L-Myc, Sall1 und Sall4, in somatische Zellen, weiterhin umfassend den Schritt des Einbringens von Lin28 in somatische Zellen.

8. Verfahren gemäß Anspruch 6, umfassend den Schritt des Einbringens von Oct3/4, Sox2, Klf4, L-Myc und Lin28 in somatische Zellen.

9. Verfahren zum Herstellen von induzierten pluripotenten Stammzellen aus somatischen Zellen gemäß Anspruch 1, umfassend den Schritt des Einbringens von Nanog in somatische Zellen.

10. Verfahren gemäß einem der Ansprüche 1 bis 9, wobei die somatischen Zellen somatische Zellen von einem Säugetier, einschließlich Mensch, sind.

11. Verfahren gemäß einem der Ansprüche 1 bis 10, wobei die somatischen Zellen menschliche somatische Zellen sind.

12. Induzierte pluripotente Stammzelle, erhältlich durch das in einem der Ansprüche 1 bis 11 definierte Verfahren.

13. Verfahren zum Produzieren von somatischen Zellen, umfassend einen Schritt des Produzierens von iPS-Zellen gemäß einem Verfahren gemäß einem der Ansprüche 1 bis 11 und den Schritt des Induzierens von Differenzierung der induzierten pluripotenten Stammzellen.

14. Verfahren zum Produzieren eines Gewebes, eines Organs oder einer Körperflüssigkeit, welche(s) eine Population von somatischen Zellen, erhältlich durch das in Anspruch 13 definierte Verfahren, ist.

## Revendications

1. Procédé de génération de cellules souches pluripotentes induites à partir de cellules somatiques, comprenant l'étape d'introduction des gènes suivants : gène de la famille Oct, gène de la famille Klf, gène de la famille Sox et L-Myc dans des cellules somatiques, comprenant en outre l'étape d'introduction de Lin28 dans des cellules somatiques.

2. Procédé selon la revendication 1, comprenant l'étape, après l'introduction desdits gènes dans des cellules somatiques, de cultiver les cellules suffisamment longtemps pour que les cellules deviennent pluripotentes et prolifèrent.

3. Procédé selon la revendication 1, comprenant l'étape d'introduction des gènes suivants : Oct3/4, Klf4, Sox2, L-Myc et Lin28 dans des cellules somatiques.

4. Procédé selon l'une quelconque des revendications 1 à 3, comprenant l'étape de transfection de vecteurs viraux contenant le gène de la famille Oct, de vecteurs viraux contenant le gène de la famille Klf et de vecteurs viraux contenant le gène de la famille Sox dans des cellules d'empaquetage séparées, respectivement, et de cultiver les cellules pour obtenir les trois surnageants de culture, et l'étape de préparation d'un mélange des trois surnageants de culture obtenus par l'étape ci-dessus, et de reprogrammation de cellules somatiques en utilisant le mélange.

5. Procédé selon l'une quelconque des revendications 1 à 4, dans lequel les cellules souches pluripotentes induites sont générées sans sélection de médicament.

6. Procédé de génération de cellules souches pluripotentes induites à partir de cellules somatiques, comprenant
I)
a) l'étape d'introduction de Lin28 dans des cellules somatiques ; et
b) l'étape d'introduction d'une combinaison des deux gènes suivants : gène de la famille Oct et gène de la famille Sox, et un gène L-Myc, dans des cellules somatiques, ou une combinaison des deux gènes suivants : gène de la famille Oct et gène de la famille Klf, et un gène L-Myc dans des cellules somatiques ; ou
II)
a) l'étape d'introduction de Lin28 dans des cellules somatiques ; et
b) l'étape d'introduction d'une combinaison des trois gènes suivants : gène de la famille Oct, gène de la famille Sox et gène Klf, et au moins un des gènes sélectionnés parmi les deux gènes suivants : Sall1 et Sall4 dans des cellules somatiques.

7. Procédé selon la revendication 6, comprenant l'étape d'introduction d'une combinaison des deux gènes suivants : Oct3/4 et Sox2 ou une combinaison des deux gènes suivants : Oct3/4 et Klf4, et au moins un des gènes sélectionnés parmi les trois gènes suivants : L-Myc, Sall1 et Sall4 dans des cellules somatiques, comprenant en outre l'étape d'introduction de Lin28 dans des cellules somatiques.

8. Procédé selon la revendication 6, comprenant l'étape d'introduction d'Oct3/4, Sox2, Klf4, L-Myc et Lin28 dans des cellules somatiques.

9. Procédé de génération de cellules souches pluripotentes induites à partir de cellules somatiques selon la revendication 1, comprenant l'étape d'introduction de Nanog dans des cellules somatiques.

10. Procédé selon l'une quelconque des revendications 1 à 9, dans lequel les cellules somatiques sont des cellules somatiques de mammifère, y compris humaines.

11. Procédé selon l'une quelconque des revendications 1 à 10, dans lequel les cellules somatiques sont des cellules somatiques humaines.

12. Cellule souche pluripotente induite pouvant être obtenue par le procédé tel que défini dans l'une quelconque des revendications 1 à 11.

13. Procédé de production de cellules somatiques, comprenant une étape de production de cellules iPS selon un procédé selon l'une quelconque des revendications 1 à 11, et l'étape de différenciation induisant les cellules souches pluripotentes induites.

14. Procédé de production d'un tissu, d'un organe ou d'un fluide corporel, qui est une population de cellules somatiques pouvant être obtenue par le procédé tel que défini dans la revendication 13.
